# EUROPEAN PATENT APPLICATION

(11) **EP 1 223 218 A1**
(43) Date of publication of application: **17.07.2002**
(21) Application number: 01309339.8
(22) Date of filing: 02.11.2001
(51) Int. Cl.: C12N 15/12, C07K 14/705, C12N 5/00, C12N 15/62, G01N 33/50, G01N 33/53, C12Q 1/68, A61K 39/395

(54) **CD2000 and CD2001 molecules and uses thereof**

(30) Priority: 03.11.2000 US 706167
(71) Applicant: Millennium Pharmaceuticals, Inc., Cambridge, Massachusetts 02139 (US)
(72) Inventor: Fraser, Christopher C., Lexington, MA 02421 (US)
(74) Representative: Jump, Timothy John Simon

(57) **Abstract**

The invention provides isolated nucleic acid molecules, designated CD2000, which encode polypeptide molecules containing Ig and Ig-like domains and SLAM associated protein (SAP) motifs. The invention also provides isolated nucleic acid molecules, designated CD2001, which encode polypeptide molecules containing an Ig and Ig-like domains. The invention also provides antisense nucleic acid molecules, expression vectors containing the nucleic acid molecules of the invention, host cells into which the expression vectors have been introduced, and non-human transgenic animals in which a nucleic acid molecule of the invention has been introduced or disrupted. The invention still further provides isolated polypeptides, fusion polypeptides, antigenic peptides and antibodies. Diagnostic, screening and therapeutic methods utilizing compositions of the invention are also provided.

SEQUENCE LISTING

<110>  Millennium Pharmaceuticals

<120>  CD2000 AND CD2001 MOLECULES, AND USES THEREOF

<130>  7853-244-227

<160>  100

<170>  PatentIn version 3.1

<210>  1
<211>  2713
<212>  DNA
<213>  Homo sapiens

<400>  1
   gtcgacccac gcgtccgatt gactgcctca aggtctcaag caccagtctt caccgcggaa      60
   agcatgttgt ggctgttcca atcgctcctg tttgtcttct gctttggccc agggaatgta     120
   gtttcacaaa gcagcttaac cccattgatg gtgaacggga ttctggggga gtcagtaact     180
   cttcccctgg agtttcctgc aggagagaag gtcaacttca tcacttggct tttcaatgaa     240
   acatctcttg ccttcatagt accccatgaa accaaaagtc cagaaatcca cgtgactaat     300
   ccgaaacagg gaaagcgact gaacttcacc cagtcctact ccctgcaact cagcaacctg     360
   aagatggaag acacaggctc ttacagagcc cagatatcca caaagacctc tgcaaagctg     420
   tccagttaca ctctgaggat attaagacaa ctgaggaaca tacaagttac caatcacagt     480
   cagctatttc agaatatgac ctgtgagctc catctgactt gctctgtgga ggatgcagat     540
   gacaatgtct cattcagatg ggaggccttg ggaaacacac tttcaagtca gccaaacctc     600
   actgtctcct gggaccccag gatttccagt gaacaggact acacctgcat agcagagaat     660
   gctgtcagta atttatcctt ctctgtctct gcccagaagc tttgcgaaga tgttaaaatt     720
   caatatacag ataccaaaat gattctgttt atggtttctg ggatatgcat agtcttcggt     780
   ttcatcatac tgctgttact tgttttgagg aaaagaagag attccctatc tttgtctact     840
   cagcgaacac agggccccga gtccgcaagg aacctagagt atgtttcagt gtctccaacg     900
   aacaacactg tgtatgcttc agtcactcat tcaaacaggg aaacagaaat ctggacacct     960
   agagaaaatg atactatcac aatttactcc acaattaatc attccaaaga gagtaaaccc    1020
   actttttcca gggcaactgc ccttgacaat gtcgtgtaag ttgctgaaag gcctcagagg    1080
   aattcgggaa tgacacgtct tctgatccca tgagacagaa caaagaacag gaagcttggt    1140
   tcctgttgtt cctggcaaca gaatttgaat atctaggata ggatgatcac ctccagtcct    1200
   tcggacttaa acctgcctac ctgagtcaaa cacctaagga taacatcatt tccagcatgt    1260
   ggttcaaata atattttcca atccacttca ggccaaaaca tgctaaagat aacacaccag    1320
   cacattgact ctctctttga taactaagca aatggaatta tggttgacag agagtttatg    1380
   atccagaaga caaccacttc tctcctttta gaaagcagca ggattgactt attgagaaat    1440
   aatgcagtgt gttggttaca tgtgtagtct ctggagttgg atgggcccat cctgatacaa    1500
   gttgagcatc ccttgtctga aatgcttggg attagaaatg tttcagattt caattttttt    1560
   tcagattttg gaatatttgc attatattta gcggttgagt atccaaatcc aaaaatccaa    1620
   aattcaaaat gctccaataa gcatttccct tgagtttcat tgatgtcgat gcagtgctca    1680
   aaatctcaga ttttggagca ttttggatat tggatttttg gatttgggat gctcaacttg    1740
   tacaatgttt attagacaca tctcctggga catactgcct aaccttttgg agccttagtc    1800
   tcccagactg aaaaaggaag aggatggtat tacatcagct ccattgtttg agccaagaat    1860
   ctaagtcatc cctgactcca gtgtctttgt caccaggccc tttggactct acctcagaaa    1920
   tatttcttgg accttccact tctcctccaa ctccttgacc accatcctgt atccaaccat    1980
   caccacctct aacctgaatc ctaccttaag atcagaacag ttgtcctcac ttttgttctt    2040
   gtccctctta aaaccctgca ggtttcccaa tgcactcaga aagaaatcca gtttccatgg    2100
   ccctggatgg tctggcccac ctccagcctc agctagcatt accctctgga cactctctat    2160
   gtagcctccc tgatcttctt tcagctcctc tattaaagga aaagttcttt atgttaatta    2220
   tttacatctt cctgcaggcc cttcctctgc ctgctggggt cctcctattc tttaggttta    2280
   attttaaata tgtcacctcc taagagaaac cttcccagac cactctttct aaaatgaatc    2340
   ttctaggctg ggcatggtgg ctcacacctg taatcccagt actttgggag gccaaggggg    2400
   gagatcactt gaggtcagga gttcaagacc agcctggcca acttggtgaa accccgtctt    2460
   tactaaaaat acaaaaaaat tagccaggcg tggtggtgca cccctaaaat cccagctact    2520
   tgagagactg aggcaggaga atcgcttgaa cccaggaggt ggaggttcca gtgagccaaa    2580
   atcatgccaa tgtattccag tctgggtgac agagtgagac tctgtctcaa aaaataaata     2640
   aataaaataa aatgaaaaaa aaaaaaaaaa aaaaaaaaaa aaaaaaaaaa aaaaaaaaaa    2700
   aaagggcggc cgc                                                       2713

<210>  2
<211>  993
<212>  DNA
<213>  Homo sapiens

<400> 2
atgttgtggc tgttccaatc gctcctgttt gtcttctgct ttggcccagg gaatgtagtt 60
tcacaaagca gcttaacccc attgatggtg aacgggattc tgggggagtc agtaactctt 120
cccctggagt ttcctgcagg agagaaggtc aacttcatca cttggctttt caatgaaaca 180
tctcttgcct tcatagtacc ccatgaaacc aaaagtccag aaatccacgt gactaatccg 240
aaacagggaa agcgactgaa cttcacccag tcctactccc tgcaactcag caacctgaag 300
atggaagaca caggctctta cagagcccag atatccacaa agacctctgc aaagctgtcc 360
agttacactc tgaggatatt aagacaactg aggaacatac aagttaccaa tcacagtcag 420
ctatttcaga atatgacctg tgagctccat ctgacttgct ctgtggagga tgcagatgac 480
aatgtctcat tcagatggga ggccttggga aacacacttt caagtcagcc aaacctcact 540
gtctcctggg accccaggat ttccagtgaa caggactaca cctgcatagc agagaatgct 600
gtcagtaatt tatccttctc tgtctctgcc cagaagcttt gcgaagatgt taaaattcaa 660
tatacagata ccaaaatgat tctgtttatg gtttctggga tatgcatagt cttcggtttc 720
atcatactgc tgttacttgt tttgaggaaa agaagagatt ccctatcttt gtctactcag 780
cgaacacagg gccccgagtc cgcaaggaac ctagagtatg tttcagtgtc tccaacgaac 840
aacactgtgt atgcttcagt cactcattca aacagggaaa cagaaatctg gacacctaga 900
gaaaatgata ctatcacaat ttactccaca attaatcatt ccaaagagag taaacccact 960
ttttccaggg caactgccct tgacaatgtc gtg 993

<210> 3
<211> 331
<212> PRT
<213> Homo sapiens

<400> 3
Met Leu Trp Leu Phe Gln Ser Leu Leu Phe Val Phe Cys Phe Gly Pro
1               5                   10                  15

Gly Asn Val Val Ser Gln Ser Ser Leu Thr Pro Leu Met Val Asn Gly
            20                  25                  30

Ile Leu Gly Glu Ser Val Thr Leu Pro Leu Glu Phe Pro Ala Gly Glu
        35                  40                  45

Lys Val Asn Phe Ile Thr Trp Leu Phe Asn Glu Thr Ser Leu Ala Phe
    50                  55                  60

Ile Val Pro His Glu Thr Lys Ser Pro Glu Ile His Val Thr Asn Pro
65                  70                  75                  80

Lys Gln Gly Lys Arg Leu Asn Phe Thr Gln Ser Tyr Ser Leu Gln Leu
                85                  90                  95

Ser Asn Leu Lys Met Glu Asp Thr Gly Ser Tyr Arg Ala Gln Ile Ser
            100                 105                 110

Thr Lys Thr Ser Ala Lys Leu Ser Ser Tyr Thr Leu Arg Ile Leu Arg
        115                 120                 125

Gln Leu Arg Asn Ile Gln Val Thr Asn His Ser Gln Leu Phe Gln Asn
    130                 135                 140

Met Thr Cys Glu Leu His Leu Thr Cys Ser Val Glu Asp Ala Asp Asp
145                 150                 155                 160

Asn Val Ser Phe Arg Trp Glu Ala Leu Gly Asn Thr Leu Ser Ser Gln
                165                 170                 175

Pro Asn Leu Thr Val Ser Trp Asp Pro Arg Ile Ser Ser Glu Gln Asp
            180                 185                 190

Tyr Thr Cys Ile Ala Glu Asn Ala Val Ser Asn Leu Ser Phe Ser Val
        195                 200                 205

Ser Ala Gln Lys Leu Cys Glu Asp Val Lys Ile Gln Tyr Thr Asp Thr
    210                 215                 220

Lys Met Ile Leu Phe Met Val Ser Gly Ile Cys Ile Val Phe Gly Phe

225                 230                 235                 240

Ile Ile Leu Leu Leu Leu Val Leu Arg Lys Arg Arg Asp Ser Leu Ser
                245                 250                 255

Leu Ser Thr Gln Arg Thr Gln Gly Pro Glu Ser Ala Arg Asn Leu Glu
            260                 265                 270

Tyr Val Ser Val Ser Pro Thr Asn Asn Thr Val Tyr Ala Ser Val Thr
        275                 280                 285

His Ser Asn Arg Glu Thr Glu Ile Trp Thr Pro Arg Glu Asn Asp Thr
    290                 295                 300

Ile Thr Ile Tyr Ser Thr Ile Asn His Ser Lys Glu Ser Lys Pro Thr
305                 310                 315                 320

Phe Ser Arg Ala Thr Ala Leu Asp Asn Val Val
                325                 330

<210>  4
<211>  22
<212>  PRT
<213>  Homo sapiens

<400>  4
Met Leu Trp Leu Phe Gln Ser Leu Leu Phe Val Phe Cys Phe Gly Pro
1               5                   10                  15

Gly Asn Val Val Ser Gln
            20

<210>  5
<211>  309
<212>  PRT
<213>  Homo sapiens

<400>  5
Ser Ser Leu Thr Pro Leu Met Val Asn Gly Ile Leu Gly Glu Ser Val
1               5                   10                  15

Thr Leu Pro Leu Glu Phe Pro Ala Gly Glu Lys Val Asn Phe Ile Thr
            20                  25                  30

Trp Leu Phe Asn Glu Thr Ser Leu Ala Phe Ile Val Pro His Glu Thr
        35                  40                  45

Lys Ser Pro Glu Ile His Val Thr Asn Pro Lys Gln Gly Lys Arg Leu
    50                  55                  60

Asn Phe Thr Gln Ser Tyr Ser Leu Gln Leu Ser Asn Leu Lys Met Glu
65                  70                  75                  80

Asp Thr Gly Ser Tyr Arg Ala Gln Ile Ser Thr Lys Thr Ser Ala Lys
                85                  90                  95

Leu Ser Ser Tyr Thr Leu Arg Ile Leu Arg Gln Leu Arg Asn Ile Gln
            100                 105                 110

Val Thr Asn His Ser Gln Leu Phe Gln Asn Met Thr Cys Glu Leu His
        115                 120                 125

Leu Thr Cys Ser Val Glu Asp Ala Asp Asp Asn Val Ser Phe Arg Trp
    130                 135                 140

Glu Ala Leu Gly Asn Thr Leu Ser Ser Gln Pro Asn Leu Thr Val Ser
145                 150                 155                 160

Trp Asp Pro Arg Ile Ser Ser Glu Gln Asp Tyr Thr Cys Ile Ala Glu

165                 170                 175

Asn Ala Val Ser Asn Leu Ser Phe Ser Val Ser Ala Gln Lys Leu Cys
            180                 185                 190

Glu Asp Val Lys Ile Gln Tyr Thr Asp Thr Lys Met Ile Leu Phe Met
        195                 200                 205

Val Ser Gly Ile Cys Ile Val Phe Gly Phe Ile Ile Leu Leu Leu Leu
    210                 215                 220

Val Leu Arg Lys Arg Arg Asp Ser Leu Ser Leu Ser Thr Gln Arg Thr
225                 230                 235                 240

Gln Gly Pro Glu Ser Ala Arg Asn Leu Glu Tyr Val Ser Val Ser Pro
            245                 250                 255

Thr Asn Asn Thr Val Tyr Ala Ser Val Thr His Ser Asn Arg Glu Thr
            260                 265                 270

Glu Ile Trp Thr Pro Arg Glu Asn Asp Thr Ile Thr Ile Tyr Ser Thr
        275                 280                 285

Ile Asn His Ser Lys Glu Ser Lys Pro Thr Phe Ser Arg Ala Thr Ala
    290                 295                 300

Leu Asp Asn Val Val
305

<210>  6
<211>  27
<212>  PRT
<213>  Homo sapiens

<400>  6
Ser Ser Leu Thr Pro Leu Met Val Asn Gly Ile Leu Gly Glu Ser Val
1               5                   10                  15

Thr Leu Pro Leu Glu Phe Pro Ala Gly Glu Lys
            20                  25

<210>  7
<211>  17
<212>  PRT
<213>  Homo sapiens

<400>  7
Val Asn Phe Ile Thr Trp Leu Phe Asn Glu Thr Ser Leu Ala Phe Ile
1               5                   10                  15

Val

<210>  8
<211>  159
<212>  PRT
<213>  Homo sapiens

<400>  8
Pro His Glu Thr Lys Ser Pro Glu Ile His Val Thr Asn Pro Lys Gln
1               5                   10                  15

Gly Lys Arg Leu Asn Phe Thr Gln Ser Tyr Ser Leu Gln Leu Ser Asn
            20                  25                  30

Leu Lys Met Glu Asp Thr Gly Ser Tyr Arg Ala Gln Ile Ser Thr Lys
        35                  40                  45

Thr Ser Ala Lys Leu Ser Ser Tyr Thr Leu Arg Ile Leu Arg Gln Leu
    50                  55                  60

Arg Asn Ile Gln Val Thr Asn His Ser Gln Leu Phe Gln Asn Met Thr
65                  70                  75                  80

Cys Glu Leu His Leu Thr Cys Ser Val Glu Asp Ala Asp Asp Asn Val
                85                  90                  95

Ser Phe Arg Trp Glu Ala Leu Gly Asn Thr Leu Ser Ser Gln Pro Asn
            100                 105                 110

Leu Thr Val Ser Trp Asp Pro Arg Ile Ser Ser Glu Gln Asp Tyr Thr
        115                 120                 125

Cys Ile Ala Glu Asn Ala Val Ser Asn Leu Ser Phe Ser Val Ser Ala
    130                 135                 140

Gln Lys Leu Cys Glu Asp Val Lys Ile Gln Tyr Thr Asp Thr Lys
145                 150                 155

<210>  9
<211>  23
<212>  PRT
<213>  Homo sapiens

<400>  9
Met Ile Leu Phe Met Val Ser Gly Ile Cys Ile Val Phe Gly Phe Ile
1               5                   10                  15

Ile Leu Leu Leu Leu Val Leu
            20

<210>  10
<211>  83
<212>  PRT
<213>  Homo sapiens

<400>  10
Arg Lys Arg Arg Asp Ser Leu Ser Leu Ser Thr Gln Arg Thr Gln Gly
1               5                   10                  15

Pro Glu Ser Ala Arg Asn Leu Glu Tyr Val Ser Val Ser Pro Thr Asn
            20                  25                  30

Asn Thr Val Tyr Ala Ser Val Thr His Ser Asn Arg Glu Thr Glu Ile
        35                  40                  45

Trp Thr Pro Arg Glu Asn Asp Thr Ile Thr Ile Tyr Ser Thr Ile Asn
    50                  55                  60

His Ser Lys Glu Ser Lys Pro Thr Phe Ser Arg Ala Thr Ala Leu Asp
65                  70                  75                  80

Asn Val Val

<210>  11
<211>  331
<212>  PRT
<213>  Mus sp.

<400>  11
Met Ala Val Ser Arg Ala Pro Ala Pro Asp Ser Ala Cys Gln Arg Met
1               5                   10                  15

Val Trp Leu Phe Pro Leu Val Phe Cys Leu Gly Ser Gly Ser Glu Val
            20                  25                  30

Ser Gln Ser Ser Ser Asp Pro Gln Leu Met Asn Gly Val Leu Gly Glu
        35                  40                  45

Ser Ala Val Leu Pro Leu Lys Leu Pro Ala Gly Lys Ile Ala Asn Ile
    50                  55                  60

Ile Ile Trp Asn Tyr Glu Trp Glu Ala Ser Gln Val Thr Ala Leu Val
65                  70                  75                  80

Ile Asn Leu Ser Asn Pro Glu Ser Pro Gln Ile Met Asn Thr Asp Val
                85                  90                  95

Lys Lys Arg Leu Asn Ile Thr Gln Ser Tyr Ser Leu Gln Ile Ser Asn
            100                 105                 110

Leu Thr Met Ala Asp Thr Gly Ser Tyr Thr Ala Gln Ile Thr Thr Lys
        115                 120                 125

Asp Ser Glu Val Ile Thr Phe Lys Tyr Ile Leu Arg Val Phe Glu Arg
    130                 135                 140

Leu Gly Asn Leu Glu Thr Thr Asn Tyr Thr Leu Leu Leu Glu Asn Gly
145                 150                 155                 160

Thr Cys Gln Ile His Leu Ala Cys Val Leu Lys Asn Gln Ser Gln Thr
                165                 170                 175

Val Ser Val Glu Trp Gln Ala Thr Gly Asn Ile Ser Leu Gly Gly Pro
            180                 185                 190

Asn Val Thr Ile Phe Trp Asp Pro Arg Asn Ser Gly Asp Gln Thr Tyr
        195                 200                 205

Val Cys Arg Ala Lys Asn Ala Val Ser Asn Leu Ser Val Ser Val Ser
    210                 215                 220

Thr Gln Ser Leu Cys Lys Gly Val Leu Thr Asn Pro Pro Trp Asn Ala
225                 230                 235                 240

Val Trp Phe Met Thr Thr Ile Ser Ile Ile Ser Ala Val Ile Leu Ile
                245                 250                 255

Phe Val Cys Trp Ser Ile His Val Trp Lys Arg Arg Gly Ser Leu Pro
            260                 265                 270

Leu Thr Ser Gln His Pro Glu Ser Ser Gln Ser Thr Asp Gly Pro Gly
        275                 280                 285

Ser Pro Gly Asn Thr Val Tyr Ala Gln Val Thr Arg Pro Met Gln Glu
    290                 295                 300

Met Lys Ile Pro Lys Pro Ile Lys Asn Asp Ser Met Thr Ile Tyr Ser
305                 310                 315                 320

Ile Val Asn His Ser Arg Glu Ala Glu Tyr Ser
                325                 330

<210>  12
<211>  7
<212>  PRT
<213>  Homo sapiens

<400>  12
Met Leu Trp Leu Phe Gln Ser
1               5

<210>  13

<211>  24
<212>  PRT
<213>  Homo sapiens

<400>  13
Ser Leu Leu Phe Val Phe Cys Phe Gly Pro Gly Asn Val Val Ser Gln
1               5                   10                  15

Ser Ser Leu Thr Pro Leu Met Val
            20

<210>  14
<211>  19
<212>  PRT
<213>  Homo sapiens

<400>  14
Asn Gly Ile Leu Gly Glu Ser Val Thr Leu Pro Leu Glu Phe Pro Ala
1               5                   10                  15

Gly Glu Lys


<210>  15
<211>  195
<212>  PRT
<213>  Homo sapiens

<400>  15
Asn Gly Ile Leu Gly Glu Ser Val Thr Leu Pro Leu Glu Phe Pro Ala
1               5                   10                  15

Gly Glu Lys Val Asn Phe Ile Thr Trp Leu Phe Asn Glu Thr Ser Leu
            20                  25                  30

Ala Phe Ile Val Pro His Glu Thr Lys Ser Pro Glu Ile His Val Thr
        35                  40                  45

Asn Pro Lys Gln Gly Lys Arg Leu Asn Phe Thr Gln Ser Tyr Ser Leu
    50                  55                  60

Gln Leu Ser Asn Leu Lys Met Glu Asp Thr Gly Ser Tyr Arg Ala Gln
65                  70                  75                  80

Ile Ser Thr Lys Thr Ser Ala Lys Leu Ser Ser Tyr Thr Leu Arg Ile
                85                  90                  95

Leu Arg Gln Leu Arg Asn Ile Gln Val Thr Asn His Ser Gln Leu Phe
            100                 105                 110

Gln Asn Met Thr Cys Glu Leu His Leu Thr Cys Ser Val Glu Asp Ala
        115                 120                 125

Asp Asp Asn Val Ser Phe Arg Trp Glu Ala Leu Gly Asn Thr Leu Ser
    130                 135                 140

Ser Gln Pro Asn Leu Thr Val Ser Trp Asp Pro Arg Ile Ser Ser Glu
145                 150                 155                 160

Gln Asp Tyr Thr Cys Ile Ala Glu Asn Ala Val Ser Asn Leu Ser Phe
                165                 170                 175

Ser Val Ser Ala Gln Lys Leu Cys Glu Asp Val Lys Ile Gln Tyr Thr
            180                 185                 190

Asp Thr Lys
        195

<210>  16

<211>  203
<212>  PRT
<213>  Homo sapiens

<400>  16
Ser Ser Leu Thr Pro Leu Met Val Asn Gly Ile Leu Gly Glu Ser Val
1               5                   10                  15

Thr Leu Pro Leu Glu Phe Pro Ala Gly Glu Lys Val Asn Phe Ile Thr
            20                  25                  30

Trp Leu Phe Asn Glu Thr Ser Leu Ala Phe Ile Val Pro His Glu Thr
        35                  40                  45

Lys Ser Pro Glu Ile His Val Thr Asn Pro Lys Gln Gly Lys Arg Leu
    50                  55                  60

Asn Phe Thr Gln Ser Tyr Ser Leu Gln Leu Ser Asn Leu Lys Met Glu
65                  70                  75                  80

Asp Thr Gly Ser Tyr Arg Ala Gln Ile Ser Thr Lys Thr Ser Ala Lys
                85                  90                  95

Leu Ser Ser Tyr Thr Leu Arg Ile Leu Arg Gln Leu Arg Asn Ile Gln
            100                 105                 110

Val Thr Asn His Ser Gln Leu Phe Gln Asn Met Thr Cys Glu Leu His
        115                 120                 125

Leu Thr Cys Ser Val Glu Asp Ala Asp Asp Asn Val Ser Phe Arg Trp
    130                 135                 140

Glu Ala Leu Gly Asn Thr Leu Ser Ser Gln Pro Asn Leu Thr Val Ser
145                 150                 155                 160

Trp Asp Pro Arg Ile Ser Ser Glu Gln Asp Tyr Thr Cys Ile Ala Glu
                165                 170                 175

Asn Ala Val Ser Asn Leu Ser Phe Ser Val Ser Ala Gln Lys Leu Cys
            180                 185                 190

Glu Asp Val Lys Ile Gln Tyr Thr Asp Thr Lys
        195                 200

<210>  17
<211>  83
<212>  PRT
<213>  Homo sapiens

<400>  17
Arg Lys Arg Arg Asp Ser Leu Ser Leu Ser Thr Gln Arg Thr Gln Gly
1               5                   10                  15

Pro Glu Ser Ala Arg Asn Leu Glu Tyr Val Ser Val Ser Pro Thr Asn
            20                  25                  30

Asn Thr Val Tyr Ala Ser Val Thr His Ser Asn Arg Glu Thr Glu Ile
        35                  40                  45

Trp Thr Pro Arg Glu Asn Asp Thr Ile Thr Ile Tyr Ser Thr Ile Asn
    50                  55                  60

His Ser Lys Glu Ser Lys Pro Thr Phe Ser Arg Ala Thr Ala Leu Asp
65                  70                  75                  80

Asn Val Val

<210>  18
<211>  52
<212>  PRT
<213>  Homo sapiens

<400>  18
Thr Cys Glu Leu His Leu Thr Cys Ser Val Glu Asp Ala Asp Asp Asn
1               5                   10                  15

Val Ser Phe Arg Trp Glu Ala Leu Gly Asn Thr Leu Ser Ser Gln Pro
            20                  25                  30

Asn Leu Thr Val Ser Trp Asp Pro Arg Ile Ser Ser Glu Gln Asp Tyr
        35                  40                  45

Thr Cys Ile Ala
    50

<210>  19
<211>  77
<212>  PRT
<213>  Homo sapiens

<400>  19
Gly Glu Ser Val Thr Leu Pro Leu Glu Phe Pro Ala Gly Glu Lys Val
1               5                   10                  15

Asn Phe Ile Thr Trp Leu Phe Asn Glu Thr Ser Leu Ala Phe Ile Val
            20                  25                  30

Pro His Glu Thr Lys Ser Pro Glu Ile His Val Thr Asn Pro Lys Gln
        35                  40                  45

Gly Lys Arg Leu Asn Phe Thr Gln Ser Tyr Ser Leu Gln Leu Ser Asn
    50                  55                  60

Leu Lys Met Glu Asp Thr Gly Ser Tyr Arg Ala Gln Ile
65                  70                  75

<210>  20
<211>  75
<212>  PRT
<213>  Homo sapiens

<400>  20
Leu Ser Thr Gln Arg Thr Gln Gly Pro Glu Ser Ala Arg Asn Leu Glu
1               5                   10                  15

Tyr Val Ser Val Ser Pro Thr Asn Asn Thr Val Tyr Ala Ser Val Thr
            20                  25                  30

His Ser Asn Arg Glu Thr Glu Ile Trp Thr Pro Arg Glu Asn Asp Thr
        35                  40                  45

Ile Thr Ile Tyr Ser Thr Ile Asn His Ser Lys Glu Ser Lys Pro Thr
    50                  55                  60

Phe Ser Arg Ala Thr Ala Leu Asp Asn Val Val
65                  70                  75

<210>  21
<211>  6
<212>  PRT
<213>  Homo sapiens

<400>  21
Thr Val Tyr Ala Ser Val
1               5

<210>  22
<211>  6
<212>  PRT
<213>  Homo sapiens

<400>  22
Thr Ile Tyr Ser Thr Ile
1               5

<210>  23
<211>  22
<212>  DNA
<213>  Abies alba

<400>  23
agagactgag gcaggagaat cg                                              22

<210>  24
<211>  23
<212>  DNA
<213>  Artificial Sequence

<220>
<223> Description of Artificial Sequence: Primer

<400>  24
tggaatacat tggcatgatt ttg                                             23

<210>  25
<211>  26
<212>  DNA
<213>  Artificial Sequence

<220>
<223> Description of Artificial Sequence: Primer

<400>  25
ttgaacccag gaggtggagg ttccag                                          26

<210>  26
<211>  21
<212>  DNA
<213>  Artificial Sequence

<220>
<223> Description of Artificial Sequence: Primer

<400>  26
   cacccccact gaaaaagatg a                                                21

<210>  27
<211>  26
<212>  DNA
<213>  Artificial Sequence

<220>
<223> Description of Artificial Sequence: Primer

<400>  27
cttaactatc ttgggctgtg acaaag                                          26

<210>  28
<211>  23
<212>  DNA
<213>  Artificial Sequence

<220>
<223> Description of Artificial Sequence: Primer

<400>  28
atgcctgccg tgtgaaccac gtg                                              23

<210>  29
<211>  1040
<212>  DNA
<213>  Homo sapiens

<400>  29
aattccggtg cttttccaca gaaggttaga ccctgaaaga gatggctcag caccacctat      60
ggatcttgct cctttgcctg caaacctggc cggaagcagc tggaaaagac tcagaaatct     120
tcacagtgaa tgggattctg ggagagtcag tcactttccc tgtaaatatc caagaaccac     180
ggcaagttaa aatcattgct tggacttcta aaacatctgt tgcttatgta acaccaggag     240
actcagaaac agcacccgta gttactgtga cccacagaaa ttattatgaa cggatacatg     300
ccttaggtcc gaactacaat ctggtcatta gcgatctgag gatggaagac gcaggagact     360
acaaagcaga cataaataca caggctgatc cctacaccac caccaagcgc tacaacctgc     420
aaatctatcg tcggcttggg aaaccaaaaa ttacacagag tttaatggca tctgtgaaca     480
gcacctgtaa tgtcacactg acatgctctg tagagaaaga agaaaagaat gtgacataca     540
attggagtcc cctgggagaa gagggtaatg tccttcaaat cttccagact cctgaggacc     600
aagagctgac ttacacgtgt acagcccaga accctgtcag caacaattct gactccatct     660
ctgcccggca gctctgtgca gacatcgcaa tgggcttccg tactcaccac accgggttgc     720
tgagcgtgct ggctatgttc tttctgcttg ttctcattct gtcttcagtg tttttgttcc     780
gtttgttcaa gagaagacaa gatgctgcct caaagaaaac catatacaca tatatcatgg     840
cttcaaggaa cacccagcca gcagagtcca gaatctatga tgaaatcctg cagtccaagg     900
tgcttccctc caaggaagag ccagtgaaca cagtttattc cgaagtgcag tttgctgata     960
agatggggaa agccagcaca caggacagta aacctcctgg gacttcaagc tatgaaattg    1020
tgatctaggc tgctgggctg                                                1040

<210>  30
<211>  328
<212>  PRT
<213>  Homo sapiens

<400>  30
Met Ala Gln His His Leu Trp Ile Leu Leu Leu Cys Leu Gln Thr Trp
1               5                   10                  15

Pro Glu Ala Ala Gly Lys Asp Ser Glu Ile Phe Thr Val Asn Gly Ile
            20                  25                  30

Leu Gly Glu Ser Val Thr Phe Pro Val Asn Ile Gln Glu Pro Arg Gln
        35                  40                  45

Val Lys Ile Ile Ala Trp Thr Ser Lys Thr Ser Val Ala Tyr Val Thr
    50                  55                  60

Pro Gly Asp Ser Glu Thr Ala Pro Val Val Thr Val Thr His Arg Asn
65                  70                  75                  80

Tyr Tyr Glu Arg Ile His Ala Leu Gly Pro Asn Tyr Asn Leu Val Ile
                85                  90                  95

Ser Asp Leu Arg Met Glu Asp Ala Gly Asp Tyr Lys Ala Asp Ile Asn
            100                 105                 110

Thr Gln Ala Asp Pro Tyr Thr Thr Thr Lys Arg Tyr Asn Leu Gln Ile
        115                 120                 125

Tyr Arg Arg Leu Gly Lys Pro Lys Ile Thr Gln Ser Leu Met Ala Ser
    130                 135                 140

Val Asn Ser Thr Cys Asn Val Thr Leu Thr Cys Ser Val Glu Lys Glu
145                 150                 155                 160

Glu Lys Asn Val Thr Tyr Asn Trp Ser Pro Leu Gly Glu Glu Gly Asn
                165                 170                 175

Val Leu Gln Ile Phe Gln Thr Pro Glu Asp Gln Glu Leu Thr Tyr Thr

180                 185                 190

Cys Thr Ala Gln Asn Pro Val Ser Asn Asn Ser Asp Ser Ile Ser Ala
        195                 200                 205

Arg Gln Leu Cys Ala Asp Ile Ala Met Gly Phe Arg Thr His His Thr
    210                 215                 220

Gly Leu Leu Ser Val Leu Ala Met Phe Phe Leu Leu Val Leu Ile Leu
225                 230                 235                 240

Ser Ser Val Phe Leu Phe Arg Leu Phe Lys Arg Arg Gln Asp Ala Ala
                245                 250                 255

Ser Lys Lys Thr Ile Tyr Thr Tyr Ile Met Ala Ser Arg Asn Thr Gln
            260                 265                 270

Pro Ala Glu Ser Arg Ile Tyr Asp Glu Ile Leu Gln Ser Lys Val Leu
        275                 280                 285

Pro Ser Lys Glu Glu Pro Val Asn Thr Val Tyr Ser Glu Val Gln Phe
    290                 295                 300

Ala Asp Lys Met Gly Lys Ala Ser Thr Gln Asp Ser Lys Pro Pro Gly
305                 310                 315                 320

Thr Ser Ser Tyr Glu Ile Val Ile
                325

<210>  31
<211>  2713
<212>  DNA
<213>  Homo sapiens

<400>  31
gtcgacccac gcgtccgatt gactgcctca aggtctcaag caccagtctt caccgcggaa      60
agcatgttgt ggctgttcca atcgctcctg tttgtcttct gctttggccc agggaatgta     120
gtttcacaaa gcagcttaac cccattgatg gtgaacggga ttctggggga gtcagtaact     180
cttcccctgg agtttcctgc aggagagaag gtcaacttca tcacttggct tttcaatgaa     240
acatctcttg tcttcatagt accccatgaa accaaaagtc cagaaatcca cgtgactaat     300
ccgaaacagg gaaagcgact gaacttcacc cagtcctact ccctgcaact cagcaacctg     360
aagatggaag acacaggctc ttacagagcc cagatatcca caaagacctc tgcaaagctg     420
tccagttaca ctctgaggat attaagacaa ctgaggaaca tacaagttac caatcacagt     480
cagctatttc agaatatgac ctgtgagctc catctgactt gctctgtgga ggatgcagat     540
gacaatgtct cattcagatg ggaggccttg ggaaacacac tttcaagtca gccaaacctc     600
actgtctcct gggaccccag gatttccagt gaacaggact acacctgcat agcagagaat     660
gctgtcagta atttatcctt ctctgtctct gcccagaagc tttgcgaaga tgttaaaatt     720
caatatacag ataccaaaat gattctgttt atggtttctg ggatatgcat agtcttcggt     780
ttcatcatac tgctgttact tgttttgagg aaaagaagag attccctatc tttgtctact     840
cagcgaacac agggccccga gtccgcaagg aacctagagt atgtttcagt gtctccaacg     900
aacaacactg tgtatgcttc agtcactcat tcaaacaggg aaacagaaat ctggacacct     960
agagaaaatg atactatcac aatttactcc acaattaatc attccaaaga gagtaaaccc    1020
actttttcca gggcaactgc ccttgacaat gtcgtgtaag ttgctgaaag gcctcagagg    1080
aattcgggaa tgacacgtct tctgatccca tgagacagaa caaagaacag gaagcttggt    1140
tcctgttgtt cctggcaaca gaatttgaat atctaggata ggatgatcac ctccagtcct    1200
tcggacttaa acctgcctac ctgagtcaaa cacctaagga taacatcatt tccagcatgt    1260
ggttcaaata atattttcca atccacttca ggccaaaaca tgctaaagat aacacaccag    1320
cacattgact ctctctttga taactaagca aatggaatta tggttgacag agagtttatg    1380
atccagaaga caaccacttc tctcctttta gaaagcagca ggattgactt attgagaaat    1440
aatgcagtgt gttggttaca tgtgtagtct ctggagttgg atgggcccat cctgatacaa    1500
gttgagcatc ccttgtctga aatgcttggg attagaaatg tttcagattt caattttttt    1560
tcagattttg gaatatttgc attatattta gcggttgagt atccaaatcc aaaaatccaa    1620
aattcaaaat gctccaataa gcatttccct tgagtttcat tgatgtcgat gcagtgctca    1680
aaatctcaga ttttggagca ttttggatat tggatttttg gatttgggat gctcaacttg    1740
tacaatgttt attagacaca tctcctggga catactgcct aacctttgg agccttagtc    1800
tcccagactg aaaaaggaag aggatggtat tacatcagct ccattgtttg agccaagaat    1860
ctaagtcatc cctgactcca gtgtctttgt caccaggccc tttggactct acctcagaaa    1920
tatttcttgg accttccact tctcctccaa ctccttgacc accatcctgt atccaaccat    1980
caccacctct aacctgaatc ctaccttaag atcagaacag ttgtcctcac ttttgttctt    2040

gtccctctta aaaccctgca ggtttcccaa tgcactcaga aagaaatcca gtttccatgg 2100
ccctggatgg tctggcccac ctccagcctc agctagcatt acccttctga cactctctat 2160
gtagcctccc tgatcttctt tcagctcctc tattaaagga aaagttcttt atgttaatta 2220
tttacatctt cctgcaggcc cttcctctgc ctgctggggt cctcctattc tttaggttta 2280
attttaaata tgtcacctcc taagagaaac cttcccagac cactctttct aaaatgaatc 2340
ttctaggctg ggcatggtgg ctcacacctg taatcccagt actttgggag gccaaggggg 2400
gagatcactt gaggtcagga gttcaagacc agcctggcca acttggtgaa accccgtctt 2460
tactaaaaat acaaaaaaat tagccaggcg tggtggtgca cccctaaaat cccagctact 2520
tgagagactg aggcaggaga atcgcttgaa cccaggaggt ggaggttcca gtgagccaaa 2580
atcatgccaa tgtattccag tctgggtgac agagtgagac tctgtctcaa aaaataaata 2640
aataaaataa aatgaaaaaa aaaaaaaaaa aaaaaaaaaa aaaaaaaaaa aaaaaaaaaa 2700
aaagggcggc cgc 2713

<210> 32
<211> 2713
<212> DNA
<213> Homo sapiens

<400> 32
   gtcgacccac gcgtccgatt gactgcctca aggtctcaag caccagtctt caccgcggaa 60
   agcatgttgt ggctgttcca atcgctcctg tttgtcttct gctttggccc agggaatgta 120
   gtttcacaaa gcagcttaac cccattgatg gtgaacggga ttctggggga gtcagtaact 180
   cttcccctgg agtttcctgc aggagagaag gtcaacttca tcacttggct tttcaatgaa 240
   acatctcttg ccttcatagt accccatgaa accaaaagtc cagaaatcca cgtgactaat 300
   ccgaaacagg gaaagcgact gaacttcacc cagtcctact ccctgcaact cagcaacctg 360
   aagatggaag acacaggctc ttacagagcc cagatatcca caaagacctc tgcaaagctg 420
   tccagttaca ctctgaggct attaagacaa ctgaggaaca tacaagttac caatcacagt 480
   cagctatttc agaatatgac ctgtgagctc catctgactt gctctgtgga ggatgcagat 540
   gacaatgtct cattcagatg ggaggccttg ggaaacacac tttcaagtca gccaaacctc 600
   actgtctcct gggaccccag gatttccagt gaacaggact acacctgcat agcagagaat 660
   gctgtcagta atttatcctt ctctgtctct gcccagaagc tttgcgaaga tgttaaaatt 720
   caatatacag ataccaaaat gattctgttt atggtttctg ggatatgcat agtcttcggt 780
   ttcatcatac tgctgttact tgttttgagg aaaagaagag attccctatc tttgtctact 840
   cagcgaacac agggccccga gtccgcaagg aacctagagt atgtttcagt gtctccaacg 900
   aacaacactg tgtatgcttc agtcactcat tcaaacaggg aaacagaaat ctggacacct 960
   agagaaaatg atactatcac aatttactcc acaattaatc attccaaaga gagtaaaccc 1020
   actttttcca gggcaactgc ccttgacaat gtcgtgtaag ttgctgaaag gcctcagagg 1080
   aattcgggaa tgacacgtct tctgatccca tgagacagaa caaagaacag gaagcttggt 1140
   tcctgttgtt cctggcaaca gaatttgaat atctaggata ggatgatcac ctccagtcct 1200
   tcggacttaa acctgcctac ctgagtcaaa cacctaagga taacatcatt tccagcatgt 1260
   ggttcaaata atattttcca atccacttca ggccaaaaca tgctaaagat aacacaccag 1320
   cacattgact ctctctttga taactaagca aatggaatta tggttgacag agagtttatg 1380
   atccagaaga caaccacttc tctcctttta gaaagcagca ggattgactt attgagaaat 1440
   aatgcagtgt gttggttaca tgtgtagtct ctggagttgg atgggcccat cctgatacaa 1500
   gttgagcatc ccttgtctga aatgcttggg attagaaatg tttcagattt caattttttt 1560
   tcagattttg gaatatttgc attatattta gcggttgagt atccaaatcc aaaaatccaa 1620
   aattcaaaat gctccaataa gcatttccct tgagtttcat tgatgtcgat gcagtgctca 1680
   aaatctcaga ttttggagca ttttggatat tggatttttg gatttgggat gctcaacttg 1740
   tacaatgttt attagacaca tctcctggga catactgcct aaccttttgg agccttagtc 1800
   tcccagactg aaaaaggaag aggatggtat tacatcagct ccattgtttg agccaagaat 1860
   ctaagtcatc cctgactcca gtgtctttgt caccaggccc tttggactct acctcagaaa 1920
   tatttcttgg accttccact tctcctccaa ctccttgacc accatcctgt atccaaccat 1980
   caccacctct aacctgaatc ctaccttaag atcagaacag ttgtcctcac ttttgttctt 2040
   gtccctctta aaaccctgca ggtttcccaa tgcactcaga aagaaatcca gtttccatgg 2100
   ccctggatgg tctggcccac ctccagcctc agctagcatt acccttctga cactctctat 2160
   gtagcctccc tgatcttctt tcagctcctc tattaaagga aaagttcttt atgttaatta 2220
   tttacatctt cctgcaggcc cttcctctgc ctgctggggt cctcctattc tttaggttta 2280
   attttaaata tgtcacctcc taagagaaac cttcccagac cactctttct aaaatgaatc 2340
   ttctaggctg ggcatggtgg ctcacacctg taatcccagt actttgggag gccaaggggg 2400
   gagatcactt gaggtcagga gttcaagacc agcctggcca acttggtgaa accccgtctt 2460
   tactaaaaat acaaaaaaat tagccaggcg tggtggtgca cccctaaaat cccagctact 2520
   tgagagactg aggcaggaga atcgcttgaa cccaggaggt ggaggttcca gtgagccaaa 2580
   atcatgccaa tgtattccag tctgggtgac agagtgagac tctgtctcaa aaaataaata 2640
   aataaaataa aatgaaaaaa aaaaaaaaaa aaaaaaaaaa aaaaaaaaaa aaaaaaaaaa 2700
   aaagggcggc cgc 2713

<210> 33
<211> 2713
<212> DNA

<213>  Homo sapiens

<400>  33
gtcgacccac gcgtccgatt gactgcctca aggtctcaag caccagtctt caccgcggaa      60
agcatgttgt ggctgttcca atcgctcctg tttgtcttct gctttggccc agggaatgta     120
gtttcacaaa gcagcttaac cccattgatg gtgaacggga ttctggggga gtcagtaact     180
cttcccctgg agtttcctgc aggagagaag gtcaacttca tcacttggct tttcaatgaa     240
acatctcttg ccttcatagt accccatgaa accaaaagtc cagaaatcca cgtgactaat     300
ccgaaacagg gaaagcgact gaacttcacc cagtcctact ccctgcaact cagcaacctg     360
aagatggaag acacaggctc ttacagagcc cagatatcca caaagacctc tgcaaagctg     420
tccagttaca ctctgaggat attaagacaa ctgaggaaca tacaagttac caatcacagt     480
cagctatttc agaatatgac ctgtgagctc catctgactt gctctgtgga ggatgcagat     540
gacaatgtct cattcagatg ggaggccttg ggaaacacac tttcaagtca gccaaacctc     600
actgtctcct gggaccccag gatttccagt gaacaggact acacctgcat agcagagaat     660
gttgtcagta atttatcctt ctctgtctct gcccagaagc tttgcgaaga tgttaaaatt     720
caatatacag ataccaaaat gattctgttt atggtttctg ggatatgcat agtcttcggt     780
ttcatcatac tgctgttact tgttttgagg aaaagaagag attccctatc tttgtctact     840
cagcgaacac agggccccga gtccgcaagg aacctagagt atgtttcagt gtctccaacg     900
aacaacactg tgtatgcttc agtcactcat tcaaacaggg aaacagaaat ctggacacct     960
agagaaaatg atactatcac aatttactcc acaattaatc attccaaaga gagtaaaccc    1020
actttttcca gggcaactgc ccttgacaat gtcgtgtaag ttgctgaaag gcctcagagg    1080
aattcgggaa tgacacgtct tctgatccca tgagacagaa caaagaacag gaagcttggt    1140
tcctgttgtt cctggcaaca gaatttgaat atctaggata ggatgatcac ctccagtcct    1200
tcggacttaa acctgcctac ctgagtcaaa cacctaagga taacatcatt tccagcatgt    1260
ggttcaaata atattttcca atccacttca ggccaaaaca tgctaaagat aacacaccag    1320
cacattgact ctctctttga taactaagca aatggaatta tggttgacag agagtttatg    1380
atccagaaga caaccacttc tctcctttta gaaagcagca ggattgactt attgagaaat    1440
aatgcagtgt gttggttaca tgtgtagtct ctggagttgg atgggcccat cctgatacaa    1500
gttgagcatc ccttgtctga aatgcttggg attagaaatg tttcagattt caattttttt    1560
tcagattttg gaatatttgc attatattta gcggttgagt atccaaatcc aaaaatccaa    1620
aattcaaaat gctccaataa gcatttccct tgagtttcat tgatgtcgat gcagtgctca    1680
aaatctcaga ttttggagca ttttggatat tggatttttg gatttgggat gctcaacttg    1740
tacaatgttt attagacaca tctcctggga catactgcct aaccttttgg agccttagtc    1800
tcccagactg aaaaaggaag aggatggtat tacatcagct ccattgtttg agccaagaat    1860
ctaagtcatc cctgactcca gtgtctttgt caccaggccc tttggactct acctcagaaa    1920
tatttcttgg accttccact tctcctccaa ctccttgacc accatcctgt atccaaccat    1980
caccacctct aacctgaatc ctaccttaag atcagaacag ttgtcctcac ttttgttctt    2040
gtccctctta aaaccctgca ggtttcccaa tgcactcaga aagaaatcca gtttccatgg    2100
ccctggatgg tctggcccac ctccagcctc agctagcatt acccttctga cactctctat    2160
gtagcctccc tgatcttctt tcagctcctc tattaaagga aaagttcttt atgttaatta    2220
tttacatctt cctgcaggcc cttcctctgc ctgctggggt cctcctattc tttaggttta    2280
attttaaata tgtcacctcc taagagaaac cttcccagac cactctttct aaaatgaatc    2340
ttctaggctg ggcatggtgg ctcacacctg taatcccagt actttgggag gccaaggggg    2400
gagatcactt gaggtcagga gttcaagacc agcctggcca acttggtgaa accccgtctt    2460
tactaaaaat acaaaaaaat tagccaggcg tggtggtgca cccctaaaat cccagctact    2520
tgagagactg aggcaggaga atcgcttgaa cccaggaggt ggaggttcca gtgagccaaa    2580
atcatgccaa tgtattccag tctgggtgac agagtgagac tctgtctcaa aaaataaata    2640
aataaaataa aatgaaaaaa aaaaaaaaaa aaaaaaaaaa aaaaaaaaaa aaaaaaaaaa    2700
aaagggcggc cgc                                                       2713

<210>  34
<211>  2713
<212>  DNA
<213>  Homo sapiens

<400>  34
   gtcgacccac gcgtccgatt gactgcctca aggtctcaag caccagtctt caccgcggaa      60
   agcatgttgt ggctgttcca atcgctcctg tttgtcttct gctttggccc agggaatgta     120
   gtttcacaaa gcagcttaac cccattgatg gtgaacggga ttctggggga gtcagtaact     180
   cttcccctgg agtttcctgc aggagagaag gtcaacttca tcacttggct tttcaatgaa     240
   acatctcttg ccttcatagt accccatgaa accaaaagtc cagaaatcca cgtgactaat     300
   ccgaaacagg gaaagcgact gaacttcacc cagtcctact ccctgcaact cagcaacctg     360
   aagatggaag acacaggctc ttacagagcc cagatatcca caaagacctc tgcaaagctg     420
   tccagttaca ctctgaggat attaagacaa ctgaggaaca tacaagttac caatcacagt     480
   cagctatttc agaatatgac ctgtgagctc catctgactt gctctgtgga ggatgcagat     540
   gacaatgtct cattcagatg ggaggccttg ggaaacacac tttcaagtca gccaaacctc     600
   actgtctcct gggaccccag gatttccagt gaacaggact acacctgcat agcagagaat     660
   gctgtcagta atttatcctt ctctgcctct gcccagaagc tttgcgaaga tgttaaaatt     720
   caatatacag ataccaaaat gattctgttt atggtttctg ggatatgcat agtcttcggt     780
   ttcatcatac tgctgttact tgttttgagg aaaagaagag attccctatc tttgtctact 840
   cagcgaacac agggccccga gtccgcaagg aacctagagt atgtttcagt gtctccaacg 900
   aacaacactg tgtatgcttc agtcactcat tcaaacaggg aaacagaaat ctggacacct 960
   agagaaaatg atactatcac aatttactcc acaattaatc attccaaaga gagtaaaccc 1020
   actttttcca gggcaactgc ccttgacaat gtcgtgtaag ttgctgaaag gcctcagagg 1080
   aattcgggaa tgacacgtct tctgatccca tgagacagaa caaagaacag gaagcttggt 1140
   tcctgttgtt cctggcaaca gaatttgaat atctaggata ggatgatcac ctccagtcct 1200
   tcggacttaa acctgcctac ctgagtcaaa cacctaagga taacatcatt tccagcatgt 1260
   ggttcaaata atattttcca atccacttca ggccaaaaca tgctaaagat aacacaccag 1320
   cacattgact ctctctttga taactaagca aatggaatta tggttgacag agagtttatg 1380
   atccagaaga caaccacttc tctcctttta gaaagcagca ggattgactt attgagaaat 1440
   aatgcagtgt gttggttaca tgtgtagtct ctggagttgg atgggcccat cctgatacaa 1500
   gttgagcatc ccttgtctga aatgcttggg attagaaatg tttcagattt caattttttt 1560
   tcagattttg gaatatttgc attatattta gcggttgagt atccaaatcc aaaaatccaa 1620
   aattcaaaat gctccaataa gcatttccct tgagtttcat tgatgtcgat gcagtgctca 1680
   aaatctcaga ttttggagca ttttggatat tggatttttg gatttgggat gctcaacttg 1740
   tacaatgttt attagacaca tctcctggga catactgcct aaccttttgg agccttagtc 1800
   tcccagactg aaaaaggaag aggatggtat tacatcagct ccattgtttg agccaagaat 1860
   ctaagtcatc cctgactcca gtgtctttgt caccaggccc tttggactct acctcagaaa 1920
   tatttcttgg accttccact tctcctccaa ctccttgacc accatcctgt atccaaccat 1980
   caccacctct aacctgaatc ctaccttaag atcagaacag ttgtcctcac ttttgttctt 2040
   gtccctctta aaaccctgca ggtttcccaa tgcactcaga aagaaatcca gtttccatgg 2100
   ccctggatgg tctggcccac ctccagcctc agctagcatt acccttctga cactctctat 2160
   gtagcctccc tgatcttctt tcagctcctc tattaaagga aaagttcttt atgttaatta 2220
   tttacatctt cctgcaggcc cttcctctgc ctgctggggt cctcctattc tttaggttta 2280
   attttaaata tgtcacctcc taagagaaac cttcccagac cactctttct aaaatgaatc 2340
   ttctaggctg ggcatggtgg ctcacacctg taatcccagt actttgggag gccaaggggg 2400
   gagatcactt gaggtcagga gttcaagacc agcctggcca acttggtgaa accccgtctt 2460
   tactaaaaat acaaaaaaat tagccaggcg tggtggtgca cccctaaaat cccagctact 2520
   tgagagactg aggcaggaga atcgcttgaa cccaggaggt ggaggttcca gtgagccaaa 2580
   atcatgccaa tgtattccag tctgggtgac agagtgagac tctgtctcaa aaaataaata 2640
   aataaaataa aatgaaaaaa aaaaaaaaaa aaaaaaaaaa aaaaaaaaaa aaaaaaaaaa 2700
   aaagggcggc cgc 2713

<210> 35
<211> 993
<212> DNA
<213> Homo sapiens

<400> 35
atgttgtggc tgttccaatc gctcctgttt gtcttctgct ttggcccagg gaatgtagtt 60
tcacaaagca gcttaacccc attgatggtg aacgggattc tgggggagtc agtaactctt 120
cccctggagt ttcctgcagg agagaaggtc aacttcatca cttggctttt caatgaaaca 180
tctcttgtct tcatagtacc ccatgaaacc aaaagtccag aaatccacgt gactaatccg 240
aaacagggaa agcgactgaa cttcacccag tcctactccc tgcaactcag caacctgaag 300
atggaagaca caggctctta cagagcccag atatccacaa agacctctgc aaagctgtcc 360
agttacactc tgaggatatt aagacaactg aggaacatac aagttaccaa tcacagtcag 420
ctatttcaga atatgacctg tgagctccat ctgacttgct ctgtggagga tgcagatgac 480
aatgtctcat tcagatggga ggccttggga aacacacttt caagtcagcc aaacctcact 540
gtctcctggg accccaggat ttccagtgaa caggactaca cctgcatagc agagaatgct 600
gtcagtaatt tatccttctc tgtctctgcc cagaagcttt gcgaagatgt taaaattcaa 660
tatacagata ccaaaatgat tctgtttatg gtttctggga tatgcatagt cttcggtttc 720
atcatactgc tgttacttgt tttgaggaaa agaagagatt ccctatcttt gtctactcag 780
cgaacacagg gccccgagtc cgcaaggaac ctagagtatg tttcagtgtc tccaacgaac 840
aacactgtgt atgcttcagt cactcattca aacagggaaa cagaaatctg gacacctaga 900
gaaaatgata ctatcacaat ttactccaca attaatcatt ccaaagagag taaacccact 960
tttccagggg caactgccct tgacaatgtc gtg 993

<210> 36
<211> 993
<212> DNA
<213> Homo sapiens

<400> 36
atgttgtggc tgttccaatc gctcctgttt gtcttctgct ttggcccagg gaatgtagtt 60
tcacaaagca gcttaacccc attgatggtg aacgggattc tgggggagtc agtaactctt 120
cccctggagt ttcctgcagg agagaaggtc aacttcatca cttggctttt caatgaaaca 180
tctcttgcct tcatagtacc ccatgaaacc aaaagtccag aaatccacgt gactaatccg 240
aaacagggaa agcgactgaa cttcacccag tcctactccc tgcaactcag caacctgaag 300

atggaagaca caggctctta cagagcccag atatccacaa agacctctgc aaagctgtcc 360
agttacactc tgaggctatt aagacaactg aggaacatac aagttaccaa tcacagtcag 420
ctatttcaga atatgacctg tgagctccat ctgacttgct ctgtggagga tgcagatgac 480
aatgtctcat tcagatggga ggccttggga aacacacttt caagtcagcc aaacctcact 540
gtctcctggg accccaggat ttccagtgaa caggactaca cctgcatagc agagaatgct 600
gtcagtaatt tatccttctc tgtctctgcc cagaagcttt gcgaagatgt taaaattcaa 660
tatacagata ccaaaatgat tctgtttatg gtttctggga tatgcatagt cttcggtttc 720
atcatactgc tgttacttgt tttgaggaaa agaagagatt ccctatcttt gtctactcag 780
cgaacacagg gccccgagtc cgcaaggaac ctagagtatg tttcagtgtc tccaacgaac 840
aacactgtgt atgcttcagt cactcattca aacagggaaa cagaaatctg gacacctaga 900
gaaaatgata ctatcacaat ttactccaca attaatcatt ccaaagagag taaacccact 960
ttttccaggg caactgccct tgacaatgtc gtg 993

<210> 37
<211> 993
<212> DNA
<213> Homo sapiens

<400> 37
atgttgtggc tgttccaatc gctcctgttt gtcttctgct ttggcccagg gaatgtagtt 60
tcacaaagca gcttaacccc attgatggtg aacgggattc tgggggagtc agtaactctt 120
cccctggagt ttcctgcagg agagaaggtc aacttcatca cttggctttt caatgaaaca 180
tctcttgcct tcatagtacc ccatgaaacc aaaagtccag aaatccacgt gactaatccg 240
aaacagggaa agcgactgaa cttcacccag tcctactccc tgcaactcag caacctgaag 300
atggaagaca caggctctta cagagcccag atatccacaa agacctctgc aaagctgtcc 360
agttacactc tgaggatatt aagacaactg aggaacatac aagttaccaa tcacagtcag 420
ctatttcaga atatgacctg tgagctccat ctgacttgct ctgtggagga tgcagatgac 480
aatgtctcat tcagatggga ggccttggga aacacacttt caagtcagcc aaacctcact 540
gtctcctggg accccaggat ttccagtgaa caggactaca cctgcatagc agagaatgtt 600
gtcagtaatt tatccttctc tgtctctgcc cagaagcttt gcgaagatgt taaaattcaa 660
tatacagata ccaaaatgat tctgtttatg gtttctggga tatgcatagt cttcggtttc 720
atcatactgc tgttacttgt tttgaggaaa agaagagatt ccctatcttt gtctactcag 780
cgaacacagg gccccgagtc cgcaaggaac ctagagtatg tttcagtgtc tccaacgaac 840
aacactgtgt atgcttcagt cactcattca aacagggaaa cagaaatctg gacacctaga 900
gaaaatgata ctatcacaat ttactccaca attaatcatt ccaaagagag taaacccact 960
ttttccaggg caactgccct tgacaatgtc gtg 993

<210> 38
<211> 993
<212> DNA
<213> Homo sapiens

<400> 38
atgttgtggc tgttccaatc gctcctgttt gtcttctgct ttggcccagg gaatgtagtt 60
tcacaaagca gcttaacccc attgatggtg aacgggattc tgggggagtc agtaactctt 120
cccctggagt ttcctgcagg agagaaggtc aacttcatca cttggctttt caatgaaaca 180
tctcttgcct tcatagtacc ccatgaaacc aaaagtccag aaatccacgt gactaatccg 240
aaacagggaa agcgactgaa cttcacccag tcctactccc tgcaactcag caacctgaag 300
atggaagaca caggctctta cagagcccag atatccacaa agacctctgc aaagctgtcc 360
agttacactc tgaggatatt aagacaactg aggaacatac aagttaccaa tcacagtcag 420
ctatttcaga atatgacctg tgagctccat ctgacttgct ctgtggagga tgcagatgac 480
aatgtctcat tcagatggga ggccttggga aacacacttt caagtcagcc aaacctcact 540
gtctcctggg accccaggat ttccagtgaa caggactaca cctgcatagc agagaatgct 600
gtcagtaatt tatccttctc tgcctctgcc cagaagcttt gcgaagatgt taaaattcaa 660
tatacagata ccaaaatgat tctgtttatg gtttctggga tatgcatagt cttcggtttc 720
atcatactgc tgttacttgt tttgaggaaa agaagagatt ccctatcttt gtctactcag 780
cgaacacagg gccccgagtc cgcaaggaac ctagagtatg tttcagtgtc tccaacgaac 840
aacactgtgt atgcttcagt cactcattca aacagggaaa cagaaatctg gacacctaga 900
gaaaatgata ctatcacaat ttactccaca attaatcatt ccaaagagag taaacccact 960
ttttccaggg caactgccct tgacaatgtc gtg 993

<210> 39
<211> 331
<212> PRT
<213> Homo sapiens

<400> 39
Met Leu Trp Leu Phe Gln Ser Leu Leu Phe Val Phe Cys Phe Gly Pro
1               5                   10                  15

Gly Asn Val Val Ser Gln Ser Ser Leu Thr Pro Leu Met Val Asn Gly
            20                  25                  30

Ile Leu Gly Glu Ser Val Thr Leu Pro Leu Glu Phe Pro Ala Gly Glu
        35                  40                  45
Lys Val Asn Phe Ile Thr Trp Leu Phe Asn Glu Thr Ser Leu Val Phe
    50                  55                  60
Ile Val Pro His Glu Thr Lys Ser Pro Glu Ile His Val Thr Asn Pro
65                  70                  75                  80
Lys Gln Gly Lys Arg Leu Asn Phe Thr Gln Ser Tyr Ser Leu Gln Leu
                85                  90                  95
Ser Asn Leu Lys Met Glu Asp Thr Gly Ser Tyr Arg Ala Gln Ile Ser
            100                 105                 110
Thr Lys Thr Ser Ala Lys Leu Ser Ser Tyr Thr Leu Arg Ile Leu Arg
        115                 120                 125
Gln Leu Arg Asn Ile Gln Val Thr Asn His Ser Gln Leu Phe Gln Asn
    130                 135                 140
Met Thr Cys Glu Leu His Leu Thr Cys Ser Val Glu Asp Ala Asp Asp
145                 150                 155                 160
Asn Val Ser Phe Arg Trp Glu Ala Leu Gly Asn Thr Leu Ser Ser Gln
                165                 170                 175
Pro Asn Leu Thr Val Ser Trp Asp Pro Arg Ile Ser Ser Glu Gln Asp
            180                 185                 190
Tyr Thr Cys Ile Ala Glu Asn Ala Val Ser Asn Leu Ser Phe Ser Val
        195                 200                 205
Ser Ala Gln Lys Leu Cys Glu Asp Val Lys Ile Gln Tyr Thr Asp Thr
    210                 215                 220
Lys Met Ile Leu Phe Met Val Ser Gly Ile Cys Ile Val Phe Gly Phe
225                 230                 235                 240
Ile Ile Leu Leu Leu Leu Val Leu Arg Lys Arg Arg Asp Ser Leu Ser
                245                 250                 255
Leu Ser Thr Gln Arg Thr Gln Gly Pro Glu Ser Ala Arg Asn Leu Glu
            260                 265                 270
Tyr Val Ser Val Ser Pro Thr Asn Asn Thr Val Tyr Ala Ser Val Thr
        275                 280                 285
His Ser Asn Arg Glu Thr Glu Ile Trp Thr Pro Arg Glu Asn Asp Thr
    290                 295                 300
Ile Thr Ile Tyr Ser Thr Ile Asn His Ser Lys Glu Ser Lys Pro Thr
305                 310                 315                 320
Phe Ser Arg Ala Thr Ala Leu Asp Asn Val Val
                325                 330

<210>  40
<211>  331
<212>  PRT
<213>  Homo sapiens

<400>  40
Met Leu Trp Leu Phe Gln Ser Leu Leu Phe Val Phe Cys Phe Gly Pro
 1               5                  10                  15
Gly Asn Val Val Ser Gln Ser Ser Leu Thr Pro Leu Met Val Asn Gly
            20                  25                  30
Ile Leu Gly Glu Ser Val Thr Leu Pro Leu Glu Phe Pro Ala Gly Glu
        35                  40                  45
Lys Val Asn Phe Ile Thr Trp Leu Phe Asn Glu Thr Ser Leu Ala Phe
    50                  55                  60
Ile Val Pro His Glu Thr Lys Ser Pro Glu Ile His Val Thr Asn Pro
65                  70                  75                  80
Lys Gln Gly Lys Arg Leu Asn Phe Thr Gln Ser Tyr Ser Leu Gln Leu
                85                  90                  95
Ser Asn Leu Lys Met Glu Asp Thr Gly Ser Tyr Arg Ala Gln Ile Ser
            100                 105                 110
Thr Lys Thr Ser Ala Lys Leu Ser Ser Tyr Thr Leu Arg Leu Leu Arg
        115                 120                 125
Gln Leu Arg Asn Ile Gln Val Thr Asn His Ser Gln Leu Phe Gln Asn
    130                 135                 140
Met Thr Cys Glu Leu His Leu Thr Cys Ser Val Glu Asp Ala Asp Asp
145                 150                 155                 160

Asn Val Ser Phe Arg Trp Glu Ala Leu Gly Asn Thr Leu Ser Ser Gln
                165                 170                 175
Pro Asn Leu Thr Val Ser Trp Asp Pro Arg Ile Ser Ser Glu Gln Asp
            180                 185                 190
Tyr Thr Cys Ile Ala Glu Asn Ala Val Ser Asn Leu Ser Phe Ser Val
        195                 200                 205
Ser Ala Gln Lys Leu Cys Glu Asp Val Lys Ile Gln Tyr Thr Asp Thr
    210                 215                 220
Lys Met Ile Leu Phe Met Val Ser Gly Ile Cys Ile Val Phe Gly Phe
225                 230                 235                 240
Ile Ile Leu Leu Leu Leu Val Leu Arg Lys Arg Arg Asp Ser Leu Ser
                245                 250                 255
Leu Ser Thr Gln Arg Thr Gln Gly Pro Glu Ser Ala Arg Asn Leu Glu
            260                 265                 270
Tyr Val Ser Val Ser Pro Thr Asn Asn Thr Val Tyr Ala Ser Val Thr
        275                 280                 285
His Ser Asn Arg Glu Thr Glu Ile Trp Thr Pro Arg Glu Asn Asp Thr
    290                 295                 300
Ile Thr Ile Tyr Ser Thr Ile Asn His Ser Lys Glu Ser Lys Pro Thr
305                 310                 315                 320
Phe Ser Arg Ala Thr Ala Leu Asp Asn Val Val
                325                 330

<210>  41
<211>  331
<212>  PRT
<213>  Homo sapiens

<400>  41
Met Leu Trp Leu Phe Gln Ser Leu Leu Phe Val Phe Cys Phe Gly Pro
 1               5                  10                  15
Gly Asn Val Val Ser Gln Ser Ser Leu Thr Pro Leu Met Val Asn Gly
            20                  25                  30
Ile Leu Gly Glu Ser Val Thr Leu Pro Leu Glu Phe Pro Ala Gly Glu
        35                  40                  45
Lys Val Asn Phe Ile Thr Trp Leu Phe Asn Glu Thr Ser Leu Ala Phe
    50                  55                  60
Ile Val Pro His Glu Thr Lys Ser Pro Glu Ile His Val Thr Asn Pro
65                  70                  75                  80
Lys Gln Gly Lys Arg Leu Asn Phe Thr Gln Ser Tyr Ser Leu Gln Leu
                85                  90                  95
Ser Asn Leu Lys Met Glu Asp Thr Gly Ser Tyr Arg Ala Gln Ile Ser
            100                 105                 110
Thr Lys Thr Ser Ala Lys Leu Ser Ser Tyr Thr Leu Arg Ile Leu Arg
        115                 120                 125
Gln Leu Arg Asn Ile Gln Val Thr Asn His Ser Gln Leu Phe Gln Asn
    130                 135                 140
Met Thr Cys Glu Leu His Leu Thr Cys Ser Val Glu Asp Ala Asp Asp
145                 150                 155                 160

Asn Val Ser Phe Arg Trp Glu Ala Leu Gly Asn Thr Leu Ser Ser Gln
                165                 170                 175
Pro Asn Leu Thr Val Ser Trp Asp Pro Arg Ile Ser Ser Glu Gln Asp
            180                 185                 190
Tyr Thr Cys Ile Ala Glu Asn Val Val Ser Asn Leu Ser Phe Ser Val
        195                 200                 205
Ser Ala Gln Lys Leu Cys Glu Asp Val Lys Ile Gln Tyr Thr Asp Thr
    210                 215                 220
Lys Met Ile Leu Phe Met Val Ser Gly Ile Cys Ile Val Phe Gly Phe
225                 230                 235                 240
Ile Ile Leu Leu Leu Leu Val Leu Arg Lys Arg Arg Asp Ser Leu Ser
                245                 250                 255
Leu Ser Thr Gln Arg Thr Gln Gly Pro Glu Ser Ala Arg Asn Leu Glu
            260                 265                 270
Tyr Val Ser Val Ser Pro Thr Asn Asn Thr Val Tyr Ala Ser Val Thr
        275                 280                 285
His Ser Asn Arg Glu Thr Glu Ile Trp Thr Pro Arg Glu Asn Asp Thr
    290                 295                 300
Ile Thr Ile Tyr Ser Thr Ile Asn His Ser Lys Glu Ser Lys Pro Thr

305                 310                 315                 320
Phe Ser Arg Ala Thr Ala Leu Asp Asn Val Val
                325                 330

<210>  42
<211>  331
<212>  PRT
<213>  Homo sapiens

<400>  42
Met Leu Trp Leu Phe Gln Ser Leu Leu Phe Val Phe Cys Phe Gly Pro
1               5                   10                  15
Gly Asn Val Val Ser Gln Ser Ser Leu Thr Pro Leu Met Val Asn Gly
            20                  25                  30
Ile Leu Gly Glu Ser Val Thr Leu Pro Leu Glu Phe Pro Ala Gly Glu
        35                  40                  45
Lys Val Asn Phe Ile Thr Trp Leu Phe Asn Glu Thr Ser Leu Ala Phe
    50                  55                  60
Ile Val Pro His Glu Thr Lys Ser Pro Glu Ile His Val Thr Asn Pro
65                  70                  75                  80
Lys Gln Gly Lys Arg Leu Asn Phe Thr Gln Ser Tyr Ser Leu Gln Leu
                85                  90                  95
Ser Asn Leu Lys Met Glu Asp Thr Gly Ser Tyr Arg Ala Gln Ile Ser
            100                 105                 110
Thr Lys Thr Ser Ala Lys Leu Ser Ser Tyr Thr Leu Arg Ile Leu Arg
        115                 120                 125
Gln Leu Arg Asn Ile Gln Val Thr Asn His Ser Gln Leu Phe Gln Asn
    130                 135                 140
Met Thr Cys Glu Leu His Leu Thr Cys Ser Val Glu Asp Ala Asp Asp
145                 150                 155                 160
Asn Val Ser Phe Arg Trp Glu Ala Leu Gly Asn Thr Leu Ser Ser Gln
                165                 170                 175
Pro Asn Leu Thr Val Ser Trp Asp Pro Arg Ile Ser Ser Glu Gln Asp
            180                 185                 190
Tyr Thr Cys Ile Ala Glu Asn Ala Val Ser Asn Leu Ser Phe Ser Ala
        195                 200                 205
Ser Ala Gln Lys Leu Cys Glu Asp Val Lys Ile Gln Tyr Thr Asp Thr
    210                 215                 220

Lys Met Ile Leu Phe Met Val Ser Gly Ile Cys Ile Val Phe Gly Phe
225                 230                 235                 240
Ile Ile Leu Leu Leu Leu Val Leu Arg Lys Arg Arg Asp Ser Leu Ser
                245                 250                 255
Leu Ser Thr Gln Arg Thr Gln Gly Pro Glu Ser Ala Arg Asn Leu Glu
            260                 265                 270
Tyr Val Ser Val Ser Pro Thr Asn Asn Thr Val Tyr Ala Ser Val Thr
        275                 280                 285
His Ser Asn Arg Glu Thr Glu Ile Trp Thr Pro Arg Glu Asn Asp Thr
    290                 295                 300
Ile Thr Ile Tyr Ser Thr Ile Asn His Ser Lys Glu Ser Lys Pro Thr
305                 310                 315                 320
Phe Ser Arg Ala Thr Ala Leu Asp Asn Val Val
                325                 330

<210>  43
<211>  66
<212>  DNA
<213>  Homo sapiens

<400>  43
atgttgtggc tgttccaatc gctcctgttt gtcttctgct ttggcccagg gaatgtagtt      60
tcacaa                                                                  66

<210>  44
<211>  927
<212>  DNA
<213>  Homo sapiens

<400>  44

agcagcttaa ccccattgat ggtgaacggg attctggggg agtcagtaac tcttcccctg 60
gagtttcctg caggagagaa ggtcaacttc atcacttggc ttttcaatga aacatctctt 120
gccttcatag taccccatga aaccaaaagt ccagaaatcc acgtgactaa tccgaaacag 180
ggaaagcgac tgaacttcac ccagtcctac tccctgcaac tcagcaacct gaagatggaa 240
gacacaggct cttacagagc ccagatatcc acaaagacct ctgcaaagct gtccagttac 300
actctgagga tattaagaca actgaggaac atacaagtta ccaatcacag tcagctattt 360
cagaatatga cctgtgagct ccatctgact tgctctgtgg aggatgcaga tgacaatgtc 420
tcattcagat gggaggcctt gggaaacaca ctttcaagtc agccaaacct cactgtctcc 480
tgggacccca ggatttccag tgaacaggac tacacctgca tagcagagaa tgctgtcagt 540
aatttatcct tctctgtctc tgcccagaag ctttgcgaag atgttaaaat tcaatataca 600
gataccaaaa tgattctgtt tatggtttct gggatatgca tagtcttcgg tttcatcata 660
ctgctgttac ttgttttgag gaaaagaaga gattccctat ctttgtctac tcagcgaaca 720
cagggccccg agtccgcaag gaacctagag tatgtttcag tgtctccaac gaacaacact 780
gtgtatgctt cagtcactca ttcaaacagg gaaacagaaa tctggacacc tagagaaaat 840
gatactatca caatttactc cacaattaat cattccaaag agagtaaacc cactttttcc 900
agggcaactg cccttgacaa tgtcgtg 927

<210> 45
<211> 81
<212> DNA
<213> Homo sapiens

<400> 45
agcagcttaa ccccattgat ggtgaacggg attctggggg agtcagtaac tcttcccctg 60
gagtttcctg caggagagaa g 81

<210> 46
<211> 51
<212> DNA
<213> Homo sapiens

<400> 46
gtcaacttca tcacttggct tttcaatgaa acatctcttg ccttcatagt a 51

<210> 47
<211> 480
<212> DNA
<213> Homo sapiens

<400> 47
ccccatgaaa ccaaaagtcc agaaatccac gtgactaatc cgaaacaggg aaagcgactg 60
aacttcaccc agtcctactc cctgcaactc agcaacctga agatggaaga cacaggctct 120
tacagagccc agatatccac aaagacctct gcaaagctgt ccagttacac tctgaggata 180
ttaagacaac tgaggaacat acaagttacc aatcacagtc agctatttca gaatatgacc 240
tgtgagctcc atctgacttg ctctgtggag gatgcagatg acaatgtctc attcagatgg 300
gaggccttgg gaaacacact ttcaagtcag ccaaacctca ctgtctcctg ggaccccagg 360
atttccagtg aacaggacta cacctgcata gcagagaatg ctgtcagtaa tttatccttc 420
tctgtctctg cccagaagct ttgcgaagat gttaaaattc aatatacaga taccaaaatg 480

<210> 48
<211> 69
<212> DNA
<213> Homo sapiens

<400> 48
atgattctgt ttatggtttc tgggatatgc atagtcttcg gtttcatcat actgctgtta 60
cttgttttg 69

<210> 49
<211> 249
<212> DNA
<213> Homo sapiens

<400> 49
aggaaaagaa gagattccct atctttgtct actcagcgaa cacagggccc cgagtccgca 60
aggaacctag agtatgtttc agtgtctcca acgaacaaca ctgtgtatgc ttcagtcact 120
cattcaaaca gggaaacaga aatctggaca cctagagaaa atgatactat cacaatttac 180
tccacaatta atcattccaa agagagtaaa cccacttttt ccagggcaac tgcccttgac 240
aatgtcgtg 249

<210>  50
<211>  609
<212>  DNA
<213>  Homo sapiens

<400>  50
agcagcttaa ccccattgat ggtgaacggg attctggggg agtcagtaac tcttcccctg      60
gagtttcctg caggagagaa ggtcaacttc atcacttggc ttttcaatga aacatctctt     120
gccttcatag taccccatga aaccaaaagt ccagaaatcc acgtgactaa tccgaaacag     180
ggaaagcgac tgaacttcac ccagtcctac tccctgcaac tcagcaacct gaagatggaa     240
gacacaggct cttacagagc ccagatatcc acaaagacct ctgcaaagct gtccagttac     300
actctgagga tattaagaca actgaggaac atacaagtta ccaatcacag tcagctattt     360
cagaatatga cctgtgagct ccatctgact tgctctgtgg aggatgcaga tgacaatgtc     420
tcattcagat gggaggcctt gggaaacaca ctttcaagtc agccaaacct cactgtctcc     480
tgggacccca ggatttccag tgaacaggac tacacctgca tagcagagaa tgctgtcagt     540
aatttatcct tctctgtctc tgcccagaag ctttgcgaag atgttaaaat tcaatataca     600
gataccaaa                                                             609

<210>  51
<211>  21
<212>  DNA
<213>  Homo sapiens

<400>  51
atgttgtggc tgttccaatc g                                                21

<210>  52
<211>  69
<212>  DNA
<213>  Homo sapiens

<400>  52
ctcctgtttg tcttctgctt tggcccaggg aatgtagttt cacaaagcag cttaacccca      60
ttgatggtg                                                              69

<210>  53
<211>  57
<212>  DNA
<213>  Homo sapiens

<400>  53
aacgggattc tgggggagtc agtaactctt cccctggagt ttcctgcagg agagaag         57

<210>  54
<211>  585
<212>  DNA
<213>  Homo sapiens

<400>  54
aacgggattc tgggggagtc agtaactctt cccctggagt ttcctgcagg agagaaggtc      60
aacttcatca cttggctttt caatgaaaca tctcttgcct tcatagtacc ccatgaaacc     120
aaaagtccag aaatccacgt gactaatccg aaacagggaa agcgactgaa cttcacccag     180
tcctactccc tgcaactcag caacctgaag atggaagaca caggctctta cagagcccag     240
atatccacaa agacctctgc aaagctgtcc agttacactc tgaggatatt aagacaactg     300
aggaacatac aagttaccaa tcacagtcag ctatttcaga atatgacctg tgagctccat     360
ctgacttgct ctgtggagga tgcagatgac aatgtctcat tcagatggga ggccttggga     420
aacacacttt caagtcagcc aaacctcact gtctcctggg accccaggat ttccagtgaa     480
caggactaca cctgcatagc agagaatgct gtcagtaatt tatccttctc tgtctctgcc     540
cagaagcttt gcgaagatgt taaaattcaa tatacagata ccaaa                     585

<210>  55
<211>  609
<212>  DNA
<213>  Homo sapiens

<400>  55

agcagcttaa ccccattgat ggtgaacggg attctggggg agtcagtaac tcttcccctg 60
gagtttcctg caggagagaa ggtcaacttc atcacttggc ttttcaatga aacatctctt 120
gccttcatag taccccatga aaccaaaagt ccagaaatcc acgtgactaa tccgaaacag 180
ggaaagcgac tgaacttcac ccagtcctac tccctgcaac tcagcaacct gaagatggaa 240
gacacaggct cttacagagc ccagatatcc acaaagacct ctgcaaagct gtccagttac 300
actctgagga tattaagaca actgaggaac atacaagtta ccaatcacag tcagctattt 360
cagaatatga cctgtgagct ccatctgact tgctctgtgg aggatgcaga tgacaatgtc 420
tcattcagat gggaggcctt gggaaacaca ctttcaagtc agccaaacct cactgtctcc 480
tgggacccca ggatttccag tgaacaggac tacacctgca tagcagagaa tgctgtcagt 540
aatttatcct tctctgtctc tgcccagaag ctttgcgaag atgttaaaat tcaatataca 600
gataccaaa 609

<210> 56
<211> 249
<212> DNA
<213> Homo sapiens

<400> 56
aggaaaagaa gagattccct atctttgtct actcagcgaa cacagggccc cgagtccgca 60
aggaacctag agtatgtttc agtgtctcca acgaacaaca ctgtgtatgc ttcagtcact 120
cattcaaaca gggaaacaga aatctggaca cctagagaaa atgatactat cacaatttac 180
tccacaatta atcattccaa agagagtaaa cccacttttt ccagggcaac tgcccttgac 240
aatgtcgtg 249

<210> 57
<211> 156
<212> DNA
<213> Homo sapiens

<400> 57
acctgtgagc tccatctgac ttgctctgtg gaggatgcag atgacaatgt ctcattcaga 60
tgggaggcct tgggaaacac actttcaagt cagccaaacc tcactgtctc ctgggacccc 120
aggatttcca gtgaacagga ctacacctgc atagca 156

<210> 58
<211> 231
<212> DNA
<213> Homo sapiens

<400> 58
ggggagtcag taactcttcc cctggagttt cctgcaggag agaaggtcaa cttcatcact 60
tggcttttca atgaaacatc tcttgccttc atagtacccc atgaaaccaa aagtccagaa 120
atccacgtga ctaatccgaa acagggaaag cgactgaact tcacccagtc ctactccctg 180
caactcagca acctgaagat ggaagacaca ggctcttaca gagcccagat a 231

<210> 59
<211> 365
<212> PRT
<213> Homo sapiens

<400> 59
Met Leu Gly Gln Val Val Thr Leu Ile Leu Leu Leu Leu Leu Lys Val
1               5                   10                  15

Tyr Gln Gly Lys Gly Cys Gln Gly Ser Ala Asp His Val Val Ser Ile
            20                  25                  30

Ser Gly Val Pro Leu Gln Leu Gln Pro Asn Ser Ile Gln Thr Lys Val
        35                  40                  45

Asp Ser Ile Ala Trp Lys Lys Leu Leu Pro Ser Gln Asn Gly Phe His
    50                  55                  60

His Ile Leu Lys Trp Glu Asn Gly Ser Leu Pro Ser Asn Thr Ser Asn
65                  70                  75                  80

Asp Arg Phe Ser Phe Ile Val Lys Asn Leu Ser Leu Leu Ile Lys Ala
                85                  90                  95

Ala Gln Gln Gln Asp Ser Gly Leu Tyr Cys Leu Glu Val Thr Ser Ile
            100                 105                 110

Ser Gly Lys Val Gln Thr Ala Thr Phe Gln Val Phe Val Phe Asp Lys
        115                 120                 125

Val Glu Lys Pro Arg Leu Gln Gly Gln Gly Lys Ile Leu Asp Arg Gly
    130                 135                 140

Arg Cys Gln Val Ala Leu Ser Cys Leu Val Ser Arg Asp Gly Asn Val
145                 150                 155                 160

Ser Tyr Ala Trp Tyr Arg Gly Ser Lys Leu Ile Gln Thr Ala Gly Asn
                165                 170                 175

Leu Thr Tyr Leu Asp Glu Glu Val Asp Ile Asn Gly Thr His Thr Tyr
            180                 185                 190

Thr Cys Asn Val Ser Asn Pro Val Ser Trp Glu Ser His Thr Leu Asn
        195                 200                 205

Leu Thr Gln Asp Cys Gln Asn Ala His Gln Glu Phe Arg Phe Trp Pro
    210                 215                 220

Phe Leu Val Ile Ile Val Ile Leu Ser Ala Leu Phe Leu Gly Thr Leu
225                 230                 235                 240

Ala Cys Phe Cys Val Trp Arg Arg Lys Arg Lys Glu Lys Gln Ser Glu
                245                 250                 255

Thr Ser Pro Lys Glu Phe Leu Thr Ile Tyr Glu Asp Val Lys Asp Leu
            260                 265                 270

Lys Thr Arg Arg Asn His Glu Gln Glu Gln Thr Phe Pro Gly Gly Gly
        275                 280                 285

Ser Thr Ile Tyr Ser Met Ile Gln Ser Gln Ser Ser Ala Pro Thr Ser
    290                 295                 300

Gln Glu Pro Ala Tyr Thr Leu Tyr Ser Leu Ile Gln Pro Ser Arg Lys
305                 310                 315                 320

Ser Gly Ser Arg Lys Arg Asn His Ser Pro Ser Phe Asn Ser Thr Ile
                325                 330                 335

Tyr Glu Val Ile Gly Lys Ser Gln Pro Lys Ala Gln Asn Pro Ala Arg
            340                 345                 350

Leu Ser Arg Lys Glu Leu Glu Asn Phe Asp Val Tyr Ser
        355                 360                 365

<210>  60
<211>  335
<212>  PRT
<213>  Homo sapiens

<400>  60
Met Ala Gly Ser Pro Thr Cys Leu Thr Leu Ile Tyr Ile Leu Trp Gln
1               5                   10                  15

Leu Thr Gly Ser Ala Ala Ser Gly Pro Val Lys Glu Leu Val Gly Ser
            20                  25                  30

Val Gly Gly Ala Val Thr Phe Pro Leu Lys Ser Lys Val Lys Gln Val
        35                  40                  45

Asp Ser Ile Val Trp Thr Phe Asn Thr Thr Pro Leu Val Thr Ile Gln
    50                  55                  60

Pro Glu Gly Gly Thr Ile Ile Val Thr Gln Asn Arg Asn Arg Glu Arg
65                  70                  75                  80

Val Asp Phe Pro Asp Gly Gly Tyr Ser Leu Lys Leu Ser Lys Leu Lys
                85                  90                  95

Lys Asn Asp Ser Gly Ile Tyr Tyr Val Gly Ile Tyr Ser Ser Ser Leu
            100                 105                 110

Gln Gln Pro Ser Thr Gln Glu Tyr Val Leu His Val Tyr Glu His Leu
        115                 120                 125

Ser Lys Pro Lys Val Thr Leu Gly Leu Gln Ser Asn Lys Asn Gly Thr
    130                 135                 140

Cys Val Thr Asn Leu Thr Cys Cys Met Glu His Gly Glu Glu Asp Val
145                 150                 155                 160

Ile Tyr Thr Trp Lys Ala Leu Gly Gln Ala Ala Asn Glu Ser His Asn
                165                 170                 175

Gly Ser Ile Leu Pro Ile Ser Trp Arg Trp Gly Glu Ser Asp Met Thr
            180                 185                 190

Phe Ile Cys Val Ala Arg Asn Pro Val Ser Arg Asn Phe Ser Ser Pro
        195                 200                 205

Ile Leu Ala Arg Lys Leu Cys Glu Gly Ala Ala Asp Asp Pro Asp Ser
    210                 215                 220

Ser Met Val Leu Leu Cys Leu Leu Leu Val Pro Leu Leu Leu Ser Leu
225                 230                 235                 240

Phe Val Leu Gly Leu Phe Leu Trp Phe Leu Lys Arg Glu Arg Gln Glu
                245                 250                 255

Glu Tyr Ile Glu Glu Lys Lys Arg Val Asp Ile Cys Arg Glu Thr Pro
            260                 265                 270

Asn Ile Cys Pro His Ser Gly Glu Asn Thr Glu Tyr Asp Thr Ile Pro
        275                 280                 285

His Thr Asn Arg Thr Ile Leu Lys Glu Asp Pro Ala Asn Thr Val Tyr
    290                 295                 300

Ser Thr Val Glu Ile Pro Lys Lys Met Glu Asn Pro His Ser Leu Leu
305                 310                 315                 320

Thr Met Pro Asp Thr Pro Arg Leu Phe Ala Tyr Glu Asn Val Ile
                325                 330                 335

<210>  61
<211>  250
<212>  PRT
<213>  Homo sapiens

<400>  61
Met Val Ala Gly Ser Asp Ala Gly Arg Ala Leu Gly Val Leu Ser Val
1               5                   10                  15

Val Cys Leu Leu His Cys Phe Gly Phe Ile Ser Cys Phe Ser Gln Gln
            20                  25                  30

Ile Tyr Gly Val Val Tyr Gly Asn Val Thr Phe His Val Pro Ser Asn
        35                  40                  45

Val Pro Leu Lys Glu Val Leu Trp Lys Lys Gln Lys Asp Lys Val Ala

50                  55                  60

Glu Leu Glu Asn Ser Glu Phe Arg Ala Phe Ser Ser Phe Lys Asn Arg
65                  70                  75                  80

Val Tyr Leu Asp Thr Val Ser Gly Ser Leu Thr Ile Tyr Asn Leu Thr
                85                  90                  95

Ser Ser Asp Glu Asp Glu Tyr Glu Met Glu Ser Pro Asn Ile Thr Asp
            100                 105                 110

Thr Met Lys Phe Phe Leu Tyr Val Leu Glu Ser Leu Pro Ser Pro Thr
        115                 120                 125

Leu Thr Cys Ala Leu Thr Asn Gly Ser Ile Glu Val Gln Cys Met Ile
    130                 135                 140

Pro Glu His Tyr Asn Ser His Arg Gly Leu Ile Met Tyr Ser Trp Asp
145                 150                 155                 160

Cys Pro Met Glu Gln Cys Lys Arg Asn Ser Thr Ser Ile Tyr Phe Lys
                165                 170                 175

Met Glu Asn Asp Leu Pro Gln Lys Ile Gln Cys Thr Leu Ser Asn Pro
            180                 185                 190

Leu Phe Asn Thr Thr Ser Ser Ile Ile Leu Thr Thr Cys Ile Pro Ser
        195                 200                 205

Ser Gly His Ser Arg His Arg Tyr Ala Leu Ile Pro Ile Pro Leu Ala
    210                 215                 220

Val Ile Thr Thr Cys Ile Val Leu Tyr Met Asn Gly Ile Leu Lys Cys
225                 230                 235                 240

Asp Arg Lys Pro Asp Arg Thr Asn Ser Asn
                245                 250

<210>  62
<211>  243
<212>  PRT
<213>  Homo sapiens

<400>  62
Met Cys Ser Arg Gly Trp Asp Ser Cys Leu Ala Leu Glu Leu Leu Leu
1               5                   10                  15

Leu Pro Leu Ser Leu Leu Val Thr Ser Ile Gln Gly His Leu Val His
            20                  25                  30

Met Thr Val Val Ser Gly Ser Asn Val Thr Leu Asn Ile Ser Glu Ser
        35                  40                  45

Leu Pro Glu Asn Tyr Lys Gln Leu Thr Trp Phe Tyr Thr Phe Asp Gln
    50                  55                  60

Lys Ile Val Glu Trp Asp Ser Arg Lys Ser Lys Tyr Phe Glu Ser Lys
65                  70                  75                  80

Phe Lys Gly Arg Val Arg Leu Asp Pro Gln Ser Gly Ala Leu Tyr Ile
                85                  90                  95

Ser Lys Val Gln Lys Glu Asp Asn Ser Thr Tyr Ile Met Arg Val Leu
            100                 105                 110

Lys Lys Thr Gly Asn Glu Gln Glu Trp Lys Ile Lys Leu Gln Val Leu
        115                 120                 125

Asp Pro Val Pro Lys Pro Val Ile Lys Ile Glu Lys Ile Glu Asp Met
    130                 135                 140

Asp Asp Asn Cys Tyr Leu Lys Leu Ser Cys Val Ile Pro Gly Glu Ser
145                 150                 155                 160

Val Asn Tyr Thr Trp Tyr Gly Asp Lys Arg Pro Phe Pro Lys Glu Leu
                165                 170                 175

Gln Asn Ser Val Leu Glu Thr Thr Leu Met Pro His Asn Tyr Ser Arg
            180                 185                 190

Cys Tyr Thr Cys Gln Val Ser Asn Ser Val Ser Ser Lys Asn Gly Thr
        195                 200                 205

Val Cys Leu Ser Pro Pro Cys Thr Leu Ala Arg Ser Phe Gly Val Glu
    210                 215                 220

Trp Ile Ala Ser Trp Leu Val Val Thr Val Pro Thr Ile Leu Gly Leu
225                 230                 235                 240

Leu Leu Thr

<210>  63
<211>  285
<212>  PRT
<213>  Homo sapiens

<400>  63
Met Val Met Arg Pro Leu Trp Ser Leu Leu Leu Trp Glu Ala Leu Leu
1               5                   10                  15

Pro Ile Thr Val Thr Gly Ala Gln Val Leu Ser Lys Val Gly Gly Ser
            20                  25                  30

Val Leu Leu Val Ala Ala Arg Pro Pro Gly Phe Gln Val Arg Glu Ala
        35                  40                  45

Ile Trp Arg Ser Leu Trp Pro Ser Glu Glu Leu Leu Ala Thr Phe Phe
    50                  55                  60

Arg Gly Ser Leu Glu Thr Leu Tyr His Ser Arg Phe Leu Gly Arg Ala
65                  70                  75                  80

Gln Leu His Ser Asn Leu Ser Leu Glu Leu Gly Pro Leu Glu Ser Gly
                85                  90                  95

Asp Ser Gly Asn Phe Ser Val Leu Met Val Asp Thr Arg Gly Gln Pro
            100                 105                 110

Trp Thr Gln Thr Leu Gln Leu Lys Val Tyr Asp Ala Val Pro Arg Pro
        115                 120                 125

Val Val Gln Val Phe Ile Ala Val Glu Arg Asp Ala Gln Pro Ser Lys
    130                 135                 140

Thr Cys Gln Val Phe Leu Ser Cys Trp Ala Pro Asn Ile Ser Glu Ile
145                 150                 155                 160

Thr Tyr Ser Trp Arg Arg Glu Thr Thr Met Asp Phe Gly Met Glu Pro
                165                 170                 175

His Ser Leu Phe Thr Asp Gly Gln Val Leu Ser Ile Ser Leu Gly Pro
            180                 185                 190

Gly Asp Arg Asp Val Ala Tyr Ser Cys Ile Val Ser Asn Pro Val Ser

195                 200                 205

Trp Asp Leu Ala Thr Val Thr Pro Trp Asp Ser Cys His His Glu Ala
    210                 215                 220

Ala Pro Gly Lys Ala Ser Tyr Lys Asp Val Leu Leu Val Val Val Pro
225                 230                 235                 240

Val Ser Leu Leu Leu Met Leu Val Thr Leu Phe Ser Ala Trp His Trp
                245                 250                 255

Cys Pro Cys Ser Gly Lys Lys Lys Lys Asp Val His Ala Asp Arg Val
            260                 265                 270

Gly Pro Glu Thr Glu Asn Pro Leu Val Gln Asp Leu Pro
        275                 280                 285

<210>  64
<211>  335
<212>  PRT
<213>  Homo sapiens

<400>  64
Met Asp Pro Lys Gly Leu Leu Ser Leu Thr Phe Val Leu Phe Leu Ser
1               5                   10                  15

Leu Ala Phe Gly Ala Ser Tyr Gly Thr Gly Gly Arg Met Met Asn Cys
            20                  25                  30

Pro Lys Ile Leu Arg Gln Leu Gly Ser Lys Val Leu Leu Pro Leu Thr
        35                  40                  45

Tyr Glu Arg Ile Asn Lys Ser Met Asn Lys Ser Ile His Ile Val Val
    50                  55                  60

Thr Met Ala Lys Ser Leu Glu Asn Ser Val Glu Asn Lys Ile Val Ser
65                  70                  75                  80

Leu Asp Pro Ser Glu Ala Gly Pro Pro Arg Tyr Leu Gly Asp Arg Tyr
                85                  90                  95

Lys Phe Tyr Leu Glu Asn Leu Thr Leu Gly Ile Arg Glu Ser Arg Lys
            100                 105                 110

Glu Asp Glu Gly Trp Tyr Leu Met Thr Leu Glu Lys Asn Val Ser Val
        115                 120                 125

Gln Arg Phe Cys Leu Gln Leu Arg Leu Tyr Glu Gln Val Ser Thr Pro
    130                 135                 140

Glu Ile Lys Val Leu Asn Lys Thr Gln Glu Asn Gly Thr Cys Thr Leu
145                 150                 155                 160

Ile Leu Gly Cys Thr Val Glu Lys Gly Asp His Val Ala Tyr Ser Trp
                165                 170                 175

Ser Glu Lys Ala Gly Thr His Pro Leu Asn Pro Ala Asn Ser Ser His
            180                 185                 190

Leu Leu Ser Leu Thr Leu Gly Pro Gln His Ala Asp Asn Ile Tyr Ile
        195                 200                 205

Cys Thr Val Ser Asn Pro Ile Ser Asn Asn Ser Gln Thr Phe Ser Pro
    210                 215                 220

Trp Pro Gly Cys Arg Thr Asp Pro Ser Glu Thr Lys Pro Trp Ala Val
225                 230                 235                 240

Tyr Ala Gly Leu Leu Gly Gly Val Ile Met Ile Leu Ile Met Val Val
                245                 250                 255

Ile Leu Gln Leu Arg Arg Arg Gly Lys Thr Asn His Tyr Gln Thr Thr
            260                 265                 270

Val Glu Lys Lys Ser Leu Thr Ile Tyr Ala Gln Val Gln Lys Pro Gly
        275                 280                 285

Pro Leu Gln Lys Lys Leu Asp Ser Phe Pro Ala Gln Asp Pro Cys Thr
    290                 295                 300

Thr Ile Tyr Val Ala Ala Thr Glu Pro Val Pro Glu Ser Val Gln Glu
305                 310                 315                 320

Thr Asn Ser Ile Thr Val Tyr Ala Ser Val Thr Leu Pro Glu Ser
                325                 330                 335

<210>  65
<211>  360
<212>  PRT
<213>  Homo sapiens

<400>  65
Met Ser Phe Pro Cys Lys Phe Val Ala Ser Phe Leu Leu Ile Phe Asn
1               5                   10                  15

Val Ser Ser Lys Gly Ala Val Ser Lys Glu Ile Thr Asn Ala Leu Glu
            20                  25                  30

Thr Trp Gly Ala Leu Gly Gln Asp Ile Asn Leu Asp Ile Pro Ser Phe
        35                  40                  45

Gln Met Ser Asp Asp Ile Asp Asp Ile Lys Trp Glu Lys Thr Ser Asp
    50                  55                  60

Lys Lys Lys Ile Ala Gln Phe Arg Lys Glu Lys Glu Thr Phe Lys Glu
65                  70                  75                  80

Lys Asp Thr Tyr Lys Leu Phe Lys Asn Gly Thr Leu Lys Ile Lys His
                85                  90                  95

Leu Lys Thr Asp Asp Gln Asp Ile Tyr Lys Val Ser Ile Tyr Asp Thr
            100                 105                 110

Lys Gly Lys Asn Val Leu Glu Lys Ile Phe Asp Leu Lys Ile Gln Glu
        115                 120                 125

Arg Val Ser Lys Pro Lys Ile Ser Trp Thr Cys Ile Asn Thr Thr Leu
    130                 135                 140

Thr Cys Glu Val Met Asn Gly Thr Asp Pro Glu Leu Asn Leu Tyr Gln
145                 150                 155                 160

Asp Gly Lys His Leu Lys Leu Ser Gln Arg Val Ile Thr His Lys Trp
                165                 170                 175

Thr Thr Ser Leu Ser Ala Lys Phe Lys Cys Thr Ala Gly Asn Lys Val
            180                 185                 190

Ser Lys Glu Ser Ser Val Glu Pro Val Ser Cys Pro Glu Lys Gly Leu
        195                 200                 205

Asp Ile Tyr Leu Ile Ile Gly Ile Cys Gly Gly Gly Ser Leu Leu Met
    210                 215                 220

Val Phe Val Ala Leu Leu Val Phe Tyr Ile Thr Lys Arg Lys Lys Gln
225                 230                 235                 240

Arg Ser Arg Arg Asn Asp Glu Glu Leu Glu Thr Arg Ala His Arg Val
                245                 250                 255

Ala Thr Glu Glu Arg Gly Arg Lys Pro His Gln Ile Pro Ala Ser Thr
            260                 265                 270

Pro Gln Asn Pro Ala Thr Ser Gln His Pro Pro Pro Pro Pro Gly His
        275                 280                 285

Arg Ser Gln Ala Pro Ser His Arg Pro Pro Pro Pro Gly His Arg Val
    290                 295                 300

Gln His Gln Pro Gln Lys Arg Pro Pro Ala Pro Ser Gly Thr Gln Val
305                 310                 315                 320

His Gln Gln Lys Gly Pro Pro Leu Pro Arg Pro Arg Val Gln Pro Lys
                325                 330                 335

Pro Pro Met Gly Gln Gln Lys Thr His Cys Pro Leu Pro Leu Ile Lys
            340                 345                 350

Lys Asp Arg Asn Cys Leu Phe Gln
        355                 360

<210>  66
<211>  18
<212>  DNA
<213>  Homo sapiens

<400>  66
actgtgtatg cttcagtc                                                    18

<210>  67
<211>  18
<212>  DNA
<213>  Homo sapiens

<400>  67
acaatttact ccacaatt                                                    18

<210>  68
<211>  27
<212>  DNA
<213>  Artificial Sequence

<220>
<223> Description of Artificial Sequence: Primer

<400>  68
cccgaattcg gcaggaaaag aagagat                                          27

<210>  69
<211>  27
<212>  DNA
<213>  Artificial Sequence

<220>
<223> Description of Artificial Sequence: Primer

<400>  69
cccggatcct tacacgacat tgtcaag                                          27

<210>  70
<211>  27
<212>  DNA
<213>  Artificial Sequence

<220>

<223> Description of Artificial Sequence: Primer

<400> 70
cccggatcct tagatagtat cattttc 27

<210> 71
<211> 27
<212> DNA
<213> Artificial Sequence

<220>
<223> Description of Artificial Sequence: Primer

<400> 71
cccgaattcg gcactcattc aaacagg 27

<210> 72
<211> 30
<212> DNA
<213> Artificial Sequence

<220>
<223> Description of Artificial Sequence: Primer

<400> 72
atcgaattca tggacgcagt ggctgtgtat 30

<210> 73
<211> 30
<212> DNA
<213> Artificial Sequence

<220>
<223> Description of Artificial Sequence: Primer

<400> 73
atcggatcct catggggctt tcaggcagac 30

<210> 74
<211> 1139
<212> DNA
<213> Homo sapiens

<400> 74
actaggagcc tcctaatgca gtcttctgca cagtcctggg gactgactga ctgaatcaca 60
cctctggggc tgggggctgc tgacatgtgt gcctttcctt ggctgcttct tctcctgctg 120
ctccaggagg gcagccaaag gagactctgg agatggtgtg gatccgagga agtggttgcg 180
gtccttcagg agtccatcag cctccccctg gaaataccac cagatgaaga ggttgagaac 240
atcatctggt cctctcacaa aagtcttgcc actgtggtgc cagggaaaga gggacatcca 300
gctaccatca tggtgaccaa tccacactac cagggccaag tgagcttcct ggaccccagc 360
tattccctgc atatcagcaa tctgagctgg gaggattcag ggctttacca agctcaagtc 420
aacctgagaa catcccagat ctctaccatg cagcagtaca atctatgtgt ctaccgatgg 480
ctgtcagagc cccagatcac tgtgaacttt gagagttctg gggaaggtgc ctgcagtatg 540
tccctggtgt gctctgtgga gaaggcaggc atggatatga cctacagctg gctctcccgg 600
ggggatagca cttatacatt ccatgaaggc cctgtcctca gcacatcctg gaggccgggg 660
gacagtgccc tctcctacac ctgcagagcc aacaacccca tcagcaacgt cagttcttgc 720
cccatccctg atgggccctt ctatgcagat cctaactatg cttctgagaa gccttcaaca 780
gccttctgcc tcctggccaa gggattgctc atcttcttgc tcttggtaat tctggccatg 840
ggactctggg tcatccgagt ccagaaaaga cacaaaatgc caaggatgaa gaaactcatg 900
agaaacagaa tgaaattgag gaaggaggca aagcctggct ccagccctgc ctgactgctc 960
cttgggaacc ccagtcctga gcttggtttc ttcccagcac ccagagaatc cttcctcagc 1020
tctcttcttt ccaggggaag gaggtgctca ggggtgggta tccagagagc catacttctg 1080
agggaagact ggctggcaat aaagtcaaat taagtgacca ccaaaaaaaa aaaaaaaaa 1139

<210> 75
<211> 870
<212> DNA
<213> Homo sapiens

<400>  75
atgtgtgcct ttccttggct gcttcttctc ctgctgctcc aggagggcag ccaaaggaga      60
ctctggagat ggtgtggatc cgaggaagtg gttgcggtcc ttcaggagtc catcagcctc     120
cccctggaaa taccaccaga tgaagaggtt gagaacatca tctggtcctc tcacaaaagt     180
cttgccactg tggtgccagg gaaagaggga catccagcta ccatcatggt gaccaatcca     240
cactaccagg gccaagtgag cttcctggac cccagctatt ccctgcatat cagcaatctg     300
agctgggagg attcagggct ttaccaagct caagtcaacc tgagaacatc ccagatctct     360
accatgcagc agtacaatct atgtgtctac cgatggctgt cagagcccca gatcactgtg     420
aactttgaga gttctgggga aggtgcctgc agtatgtccc tggtgtgctc tgtggagaag     480
gcaggcatgg atatgaccta cagctggctc tcccgggggg atagcactta tacattccat     540
gaaggccctg tcctcagcac atcctggagg ccgggggaca gtgccctctc ctacacctgc     600

agagccaaca accccatcag caacgtcagt tcttgcccca tccctgatgg gcccttctat     660
gcagatccta actatgcttc tgagaagcct tcaacagcct tctgcctcct ggccaaggga     720
ttgctcatct tcttgctctt ggtaattctg gccatgggac tctgggtcat ccgagtccag     780
aaaagacaca aaatgccaag gatgaagaaa ctcatgagaa acagaatgaa attgaggaag     840
gaggcaaagc ctggctccag ccctgcctga                                      870

<210>  76
<211>  289
<212>  PRT
<213>  Homo sapiens

<400>  76
Met Cys Ala Phe Pro Trp Leu Leu Leu Leu Leu Leu Leu Gln Glu Gly
1               5                   10                  15

Ser Gln Arg Arg Leu Trp Arg Trp Cys Gly Ser Glu Glu Val Val Ala
            20                  25                  30

Val Leu Gln Glu Ser Ile Ser Leu Pro Leu Glu Ile Pro Pro Asp Glu
        35                  40                  45

Glu Val Glu Asn Ile Ile Trp Ser Ser His Lys Ser Leu Ala Thr Val
    50                  55                  60

Val Pro Gly Lys Glu Gly His Pro Ala Thr Ile Met Val Thr Asn Pro
65                  70                  75                  80

His Tyr Gln Gly Gln Val Ser Phe Leu Asp Pro Ser Tyr Ser Leu His
                85                  90                  95

Ile Ser Asn Leu Ser Trp Glu Asp Ser Gly Leu Tyr Gln Ala Gln Val
            100                 105                 110

Asn Leu Arg Thr Ser Gln Ile Ser Thr Met Gln Gln Tyr Asn Leu Cys
        115                 120                 125

Val Tyr Arg Trp Leu Ser Glu Pro Gln Ile Thr Val Asn Phe Glu Ser
    130                 135                 140

Ser Gly Glu Gly Ala Cys Ser Met Ser Leu Val Cys Ser Val Glu Lys
145                 150                 155                 160

Ala Gly Met Asp Met Thr Tyr Ser Trp Leu Ser Arg Gly Asp Ser Thr
                165                 170                 175

Tyr Thr Phe His Glu Gly Pro Val Leu Ser Thr Ser Trp Arg Pro Gly
            180                 185                 190

Asp Ser Ala Leu Ser Tyr Thr Cys Arg Ala Asn Asn Pro Ile Ser Asn
        195                 200                 205

Val Ser Ser Cys Pro Ile Pro Asp Gly Pro Phe Tyr Ala Asp Pro Asn
    210                 215                 220

Tyr Ala Ser Glu Lys Pro Ser Thr Ala Phe Cys Leu Leu Ala Lys Gly
225                 230                 235                 240

Leu Leu Ile Phe Leu Leu Leu Val Ile Leu Ala Met Gly Leu Trp Val
                245                 250                 255

Ile Arg Val Gln Lys Arg His Lys Met Pro Arg Met Lys Lys Leu Met
            260                 265                 270

Arg Asn Arg Met Lys Leu Arg Lys Glu Ala Lys Pro Gly Ser Ser Pro
        275                 280                 285

Ala

<210>  77
<211>  270
<212>  PRT
<213>  Homo sapiens

<400>  77
Arg Leu Trp Arg Trp Cys Gly Ser Glu Glu Val Val Ala Val Leu Gln
1               5                   10                  15

Glu Ser Ile Ser Leu Pro Leu Glu Ile Pro Pro Asp Glu Glu Val Glu
            20                  25                  30

Asn Ile Ile Trp Ser Ser His Lys Ser Leu Ala Thr Val Val Pro Gly
        35                  40                  45

Lys Glu Gly His Pro Ala Thr Ile Met Val Thr Asn Pro His Tyr Gln
    50                  55                  60

Gly Gln Val Ser Phe Leu Asp Pro Ser Tyr Ser Leu His Ile Ser Asn
65                  70                  75                  80

Leu Ser Trp Glu Asp Ser Gly Leu Tyr Gln Ala Gln Val Asn Leu Arg
                85                  90                  95

Thr Ser Gln Ile Ser Thr Met Gln Gln Tyr Asn Leu Cys Val Tyr Arg
            100                 105                 110

Trp Leu Ser Glu Pro Gln Ile Thr Val Asn Phe Glu Ser Ser Gly Glu
        115                 120                 125

Gly Ala Cys Ser Met Ser Leu Val Cys Ser Val Glu Lys Ala Gly Met
    130                 135                 140

Asp Met Thr Tyr Ser Trp Leu Ser Arg Gly Asp Ser Thr Tyr Thr Phe
145                 150                 155                 160

His Glu Gly Pro Val Leu Ser Thr Ser Trp Arg Pro Gly Asp Ser Ala
                165                 170                 175

Leu Ser Tyr Thr Cys Arg Ala Asn Asn Pro Ile Ser Asn Val Ser Ser
            180                 185                 190

Cys Pro Ile Pro Asp Gly Pro Phe Tyr Ala Asp Pro Asn Tyr Ala Ser
        195                 200                 205

Glu Lys Pro Ser Thr Ala Phe Cys Leu Leu Ala Lys Gly Leu Leu Ile
    210                 215                 220

Phe Leu Leu Leu Val Ile Leu Ala Met Gly Leu Trp Val Ile Arg Val
225                 230                 235                 240

Gln Lys Arg His Lys Met Pro Arg Met Lys Lys Leu Met Arg Asn Arg
                245                 250                 255

Met Lys Leu Arg Lys Glu Ala Lys Pro Gly Ser Ser Pro Ala

260                 265                 270

<210>  78
<211>  19
<212>  PRT
<213>  Homo sapiens

<400>  78
Met Cys Ala Phe Pro Trp Leu Leu Leu Leu Leu Leu Leu Gln Glu Gly
1               5                   10                  15

Ser Gln Arg


<210>  79
<211>  217
<212>  PRT
<213>  Homo sapiens

<400>  79
Arg Leu Trp Arg Trp Cys Gly Ser Glu Glu Val Val Ala Val Leu Gln
1               5                   10                  15

Glu Ser Ile Ser Leu Pro Leu Glu Ile Pro Pro Asp Glu Glu Val Glu
            20                  25                  30

Asn Ile Ile Trp Ser Ser His Lys Ser Leu Ala Thr Val Val Pro Gly
        35                  40                  45

Lys Glu Gly His Pro Ala Thr Ile Met Val Thr Asn Pro His Tyr Gln
    50                  55                  60

Gly Gln Val Ser Phe Leu Asp Pro Ser Tyr Ser Leu His Ile Ser Asn
65                  70                  75                  80

Leu Ser Trp Glu Asp Ser Gly Leu Tyr Gln Ala Gln Val Asn Leu Arg
                85                  90                  95

Thr Ser Gln Ile Ser Thr Met Gln Gln Tyr Asn Leu Cys Val Tyr Arg
            100                 105                 110

Trp Leu Ser Glu Pro Gln Ile Thr Val Asn Phe Glu Ser Ser Gly Glu
        115                 120                 125

Gly Ala Cys Ser Met Ser Leu Val Cys Ser Val Glu Lys Ala Gly Met
    130                 135                 140

Asp Met Thr Tyr Ser Trp Leu Ser Arg Gly Asp Ser Thr Tyr Thr Phe
145                 150                 155                 160

His Glu Gly Pro Val Leu Ser Thr Ser Trp Arg Pro Gly Asp Ser Ala
                165                 170                 175

Leu Ser Tyr Thr Cys Arg Ala Asn Asn Pro Ile Ser Asn Val Ser Ser
            180                 185                 190

Cys Pro Ile Pro Asp Gly Pro Phe Tyr Ala Asp Pro Asn Tyr Ala Ser
        195                 200                 205

Glu Lys Pro Ser Thr Ala Phe Cys Leu
    210                 215

<210>  80
<211>  27
<212>  PRT
<213>  Homo sapiens

<400>  80

Leu Ala Lys Gly Leu Leu Ile Phe Leu Leu Leu Val Ile Leu Ala Met
1               5                   10                  15

Gly Leu Trp Val Ile Arg Val Gln Lys Arg His
            20                  25

<210>  81
<211>  26
<212>  PRT
<213>  Homo sapiens

<400>  81
Lys Met Pro Arg Met Lys Lys Leu Met Arg Asn Arg Met Lys Leu Arg
1               5                   10                  15

Lys Glu Ala Lys Pro Gly Ser Ser Pro Ala
            20                  25

<210>  82
<211>  236
<212>  PRT
<213>  Homo sapiens

<400>  82
Met Cys Ala Phe Pro Trp Leu Leu Leu Leu Leu Leu Leu Gln Glu Gly
1               5                   10                  15

Ser Gln Arg Arg Leu Trp Arg Trp Cys Gly Ser Glu Glu Val Val Ala
            20                  25                  30

Val Leu Gln Glu Ser Ile Ser Leu Pro Leu Glu Ile Pro Pro Asp Glu
        35                  40                  45

Glu Val Glu Asn Ile Ile Trp Ser Ser His Lys Ser Leu Ala Thr Val
    50                  55                  60

Val Pro Gly Lys Glu Gly His Pro Ala Thr Ile Met Val Thr Asn Pro
65                  70                  75                  80

His Tyr Gln Gly Gln Val Ser Phe Leu Asp Pro Ser Tyr Ser Leu His
                85                  90                  95

Ile Ser Asn Leu Ser Trp Glu Asp Ser Gly Leu Tyr Gln Ala Gln Val
            100                 105                 110

Asn Leu Arg Thr Ser Gln Ile Ser Thr Met Gln Gln Tyr Asn Leu Cys
        115                 120                 125

Val Tyr Arg Trp Leu Ser Glu Pro Gln Ile Thr Val Asn Phe Glu Ser
    130                 135                 140

Ser Gly Glu Gly Ala Cys Ser Met Ser Leu Val Cys Ser Val Glu Lys
145                 150                 155                 160

Ala Gly Met Asp Met Thr Tyr Ser Trp Leu Ser Arg Gly Asp Ser Thr
                165                 170                 175

Tyr Thr Phe His Glu Gly Pro Val Leu Ser Thr Ser Trp Arg Pro Gly
            180                 185                 190

Asp Ser Ala Leu Ser Tyr Thr Cys Arg Ala Asn Asn Pro Ile Ser Asn
        195                 200                 205

Val Ser Ser Cys Pro Ile Pro Asp Gly Pro Phe Tyr Ala Asp Pro Asn
    210                 215                 220

Tyr Ala Ser Glu Lys Pro Ser Thr Ala Phe Cys Leu
225                 230                 235

<210> 83
<211> 59
<212> DNA
<213> Homo sapiens

<400> 83
atgtgtgcct ttccttggct gcttcttctc ctgctgctcc aggagggcag ccaaaggag 59

<210> 84
<211> 652
<212> DNA
<213> Homo sapiens

<400> 84
actctggaga tggtgtggat ccgaggaagt ggttgcggtc cttcaggagt ccatcagcct 60
ccccctggaa ataccaccag atgaagaggt tgagaacatc atctggtcct ctcacaaaag 120
tcttgccact gtggtgccag ggaaagaggg acatccagct accatcatgg tgaccaatcc 180
acactaccag ggccaagtga gcttcctgga ccccagctat tccctgcata tcagcaatct 240
gagctgggag gattcagggc tttaccaagc tcaagtcaac ctgagaacat cccagatctc 300
taccatgcag cagtacaatc tatgtgtcta ccgatggctg tcagagcccc agatcactgt 360
gaactttgag agttctgggg aaggtgcctg cagtatgtcc ctggtgtgct ctgtggagaa 420
ggcaggcatg gatatgacct acagctggct ctcccggggg gatagcactt atacattcca 480
tgaaggccct gtcctcagca catcctggag gccgggggac agtgccctct cctacacctg 540
cagagccaac aaccccatca gcaacgtcag ttcttgcccc atccctgatg ggcccttcta 600
tgcagatcct aactatgctt ctgagaagcc ttcaacagcc ttctgcctcc tg 652

<210> 85
<211> 81
<212> DNA
<213> Homo sapiens

<400> 85
gccaagggat tgctcatctt cttgctcttg gtaattctgg ccatgggact ctgggtcatc 60
cgagtccaga aaagacacaa a 81

<210> 86
<211> 78
<212> DNA
<213> Homo sapiens

<400> 86
atgccaagga tgaagaaact catgagaaac agaatgaaat tgaggaagga ggcaaagcct 60
ggctccagcc ctgcctga 78

<210> 87
<211> 711
<212> DNA
<213> Homo sapiens

<400> 87
atgtgtgcct ttccttggct gcttcttctc ctgctgctcc aggagggcag ccaaaggaga 60
ctctggagat ggtgtggatc cgaggaagtg gttgcggtcc ttcaggagtc catcagcctc 120
cccctggaaa taccaccaga tgaagaggtt gagaacatca tctggtcctc tcacaaaagt 180
cttgccactg tggtgccagg gaaagaggga catccagcta ccatcatggt gaccaatcca 240
cactaccagg gccaagtgag cttcctggac cccagctatt ccctgcatat cagcaatctg 300
agctgggagg attcagggct ttaccaagct caagtcaacc tgagaacatc ccagatctct 360
accatgcagc agtacaatct atgtgtctac cgatggctgt cagagcccca gatcactgtg 420
aactttgaga gttctgggga aggtgcctgc agtatgtccc tggtgtgctc tgtggagaag 480
gcaggcatgg atatgaccta cagctggctc tcccgggggg atagcactta tacattccat 540
gaaggccctg tcctcagcac atcctggagg ccgggggaca gtgccctctc ctacacctgc 600
agagccaaca accccatcag caacgtcagt tcttgcccca tccctgatgg gcccttctat 660
gcagatccta actatgcttc tgagaagcct tcaacagcct tctgcctcct g 711

<210> 88
<211> 289
<212> PRT
<213> Homo sapiens

<400>  88
Met Cys Ala Phe Pro Trp Leu Leu Leu Leu Leu Leu Leu Gln Glu Gly
1               5                   10                  15

Ser Gln Arg Arg Leu Trp Arg Trp Cys Gly Ser Glu Glu Leu Val Ala
            20                  25                  30

Val Leu Gln Glu Ser Ile Ser Leu Pro Leu Glu Ile Pro Pro Asp Glu
        35                  40                  45

Glu Val Glu Asn Ile Ile Trp Ser Ser His Lys Ser Leu Ala Thr Val
    50                  55                  60

Val Pro Gly Lys Glu Gly His Pro Ala Thr Ile Met Val Thr Asn Pro
65                  70                  75                  80

His Tyr Gln Gly Gln Val Ser Phe Leu Asp Pro Ser Tyr Ser Leu His
                85                  90                  95

Ile Ser Asn Leu Ser Trp Glu Asp Ser Gly Leu Tyr Gln Ala Gln Val
            100                 105                 110

Asn Leu Arg Thr Ser Gln Ile Ser Thr Met Gln Gln Tyr Asn Leu Cys
        115                 120                 125

Val Tyr Arg Trp Leu Ser Glu Pro Gln Ile Thr Val Asn Phe Glu Ser
    130                 135                 140

Ser Gly Glu Gly Ala Cys Ser Met Ser Leu Val Cys Ser Val Glu Lys
145                 150                 155                 160

Ala Gly Met Asp Met Thr Tyr Ser Trp Leu Ser Arg Gly Asp Ser Thr
                165                 170                 175

Tyr Thr Phe His Glu Gly Pro Val Leu Ser Thr Ser Trp Arg Pro Gly
            180                 185                 190

Asp Ser Ala Leu Ser Tyr Thr Cys Arg Ala Asn Asn Pro Ile Ser Asn
        195                 200                 205

Val Ser Ser Cys Pro Ile Pro Asp Gly Pro Phe Tyr Ala Asp Pro Asn
    210                 215                 220

Tyr Ala Ser Glu Lys Pro Ser Thr Ala Phe Cys Leu Leu Ala Lys Gly
225                 230                 235                 240

Leu Leu Ile Phe Leu Leu Leu Val Ile Leu Ala Met Gly Leu Trp Val
                245                 250                 255

Ile Arg Val Gln Lys Arg His Lys Met Pro Arg Met Lys Lys Leu Met
            260                 265                 270

Arg Asn Arg Met Lys Leu Arg Lys Glu Ala Lys Pro Gly Ser Ser Pro
        275                 280                 285

Ala


<210>  89
<211>  289
<212>  PRT
<213>  Homo sapiens

<400>  89
Met Cys Ala Phe Pro Trp Leu Leu Leu Leu Leu Leu Leu Gln Glu Gly
1               5                   10                  15

Ser Gln Arg Arg Leu Trp Arg Trp Cys Gly Ser Glu Glu Val Val Ala
            20                  25                  30

Val Leu Gln Glu Ser Ile Ser Leu Pro Leu Glu Ile Pro Pro Asp Glu
        35                  40                  45

Glu Val Glu Asn Ile Ile Trp Ser Ser His Lys Ser Leu Ala Thr Val
    50                  55                  60

Val Pro Gly Lys Glu Gly His Pro Ala Thr Ile Met Val Thr Asn Pro
65                  70                  75                  80

His Tyr Gln Gly Gln Val Ser Phe Leu Asp Pro Ser Tyr Ser Leu His
                85                  90                  95

Ile Ser Asn Leu Ser Trp Glu Asp Ser Gly Leu Tyr Gln Ala Gln Val
            100                 105                 110

Asn Leu Arg Thr Ser Gln Ile Ser Thr Met Gln Gln Tyr Asn Leu Cys
        115                 120                 125

Val Tyr Arg Trp Leu Ser Glu Pro Gln Ile Thr Val Asn Phe Glu Ser
    130                 135                 140

Ser Gly Glu Gly Ala Cys Ser Met Ser Leu Val Cys Ser Val Glu Lys
145                 150                 155                 160

Ala Gly Met Asp Met Thr Tyr Ser Trp Leu Ser Arg Gly Asp Ser Thr
                165                 170                 175

Tyr Thr Phe His Glu Gly Pro Val Leu Ser Thr Ser Trp Arg Pro Gly
            180                 185                 190

Asp Ser Ala Leu Ser Tyr Thr Cys Arg Ala Asn Asn Pro Ile Ser Asn
        195                 200                 205

Val Ser Ser Cys Pro Ile Pro Asp Gly Pro Phe Tyr Ala Asp Pro Asn
    210                 215                 220

Tyr Ala Ser Glu Lys Pro Ser Thr Ala Phe Cys Leu Leu Ala Lys Gly
225                 230                 235                 240

Leu Leu Ile Phe Leu Leu Leu Val Ile Leu Ala Met Gly Leu Trp Val
                245                 250                 255

Ile Arg Val Gln Lys Arg His Lys Met Pro Arg Met Arg Lys Leu Met
            260                 265                 270

Arg Asn Arg Met Lys Leu Arg Lys Glu Ala Lys Pro Gly Ser Ser Pro
        275                 280                 285

Ala

<210>  90
<211>  289
<212>  PRT
<213>  Homo sapiens

<400>  90
Met Cys Ala Phe Pro Trp Leu Leu Leu Leu Leu Leu Leu Gln Glu Gly
1               5                   10                  15

Ser Gln Arg Arg Leu Trp Arg Trp Cys Gly Ser Glu Glu Val Val Ala
            20                  25                  30

Val Leu Gln Glu Ser Ile Ser Leu Pro Leu Glu Ile Pro Pro Asp Glu
        35                  40                  45

Glu Val Glu Asn Ile Ile Trp Ser Ser His Lys Ser Leu Ala Thr Val
    50                  55                  60

Val Pro Gly Lys Glu Gly His Pro Ala Thr Ile Met Val Thr Asn Pro
65                  70                  75                  80

His Tyr Gln Gly Gln Val Ser Phe Leu Asp Pro Ser Tyr Ser Leu His
                85                  90                  95

Ile Ser Asn Leu Ser Trp Glu Asp Ser Gly Leu Tyr Gln Ala Gln Val
            100                 105                 110

Asn Leu Arg Thr Ser Gln Ile Ser Thr Met Gln Gln Tyr Asn Leu Cys
        115                 120                 125

Val Tyr Arg Trp Leu Ser Glu Pro Gln Ile Thr Val Asn Phe Glu Ser
    130                 135                 140

Ser Gly Glu Gly Ala Cys Ser Met Ser Leu Val Cys Ser Val Glu Lys
145                 150                 155                 160

Ala Gly Met Asp Met Thr Tyr Ser Trp Leu Ser Arg Gly Asp Ser Thr
                165                 170                 175

Tyr Thr Phe His Glu Gly Pro Val Leu Ser Thr Ser Trp Arg Pro Gly
            180                 185                 190

Asp Ser Ala Leu Ser Tyr Thr Cys Arg Ala Asn Asn Pro Ile Thr Asn
        195                 200                 205

Val Ser Ser Cys Pro Ile Pro Asp Gly Pro Phe Tyr Ala Asp Pro Asn
    210                 215                 220

Tyr Ala Ser Glu Lys Pro Ser Thr Ala Phe Cys Leu Leu Ala Lys Gly
225                 230                 235                 240

Leu Leu Ile Phe Leu Leu Leu Val Ile Leu Ala Met Gly Leu Trp Val
                245                 250                 255

Ile Arg Val Gln Lys Arg His Lys Met Pro Arg Met Lys Lys Leu Met
            260                 265                 270

Arg Asn Arg Met Lys Leu Arg Lys Glu Ala Lys Pro Gly Ser Ser Pro
        275                 280                 285

Ala


<210>  91
<211>  289
<212>  PRT
<213>  Homo sapiens

<400>  91
Met Cys Ala Phe Pro Trp Leu Leu Leu Leu Leu Leu Leu Gln Glu Gly
1               5                   10                  15

Ser Gln Arg Arg Leu Trp Arg Trp Cys Gly Ser Glu Glu Val Val Ala
            20                  25                  30

Val Leu Gln Glu Ser Ile Ser Leu Pro Leu Glu Ile Pro Pro Asp Glu
        35                  40                  45

Glu Val Glu Asn Ile Ile Trp Ser Ser His Lys Ser Leu Ala Thr Val
    50                  55                  60

Val Pro Gly Lys Glu Gly His Pro Ala Thr Ile Met Val Thr Asn Pro
65                  70                  75                  80

His Tyr Gln Gly Gln Val Ser Phe Leu Asp Pro Ser Tyr Ser Leu His
                85                  90                  95

Ile Ser Asn Leu Ser Trp Glu Asp Ser Gly Leu Tyr Gln Ala Gln Val
            100                 105                 110

Asn Leu Arg Thr Ser Gln Ile Ser Thr Met Gln Gln Tyr Asn Leu Cys
        115                 120                 125

Val Tyr Arg Trp Leu Ser Glu Pro Gln Ile Thr Val Asn Phe Glu Ser
    130                 135                 140

Ser Gly Glu Gly Ala Cys Ser Met Ser Leu Val Cys Ser Val Glu Lys
145                 150                 155                 160

Ala Gly Met Asp Met Thr Tyr Ser Trp Leu Ser Arg Gly Asp Ser Thr
                165                 170                 175

Tyr Thr Phe His Glu Gly Pro Val Leu Ser Thr Ser Trp Arg Pro Gly
            180                 185                 190

Asp Ser Ala Leu Ser Tyr Thr Cys Arg Ala Asn Asn Pro Ile Ser Asn
        195                 200                 205

Val Ser Ser Cys Pro Ile Pro Asp Gly Pro Phe Tyr Gly Asp Pro Asn
    210                 215                 220

Tyr Ala Ser Glu Lys Pro Ser Thr Ala Phe Cys Leu Leu Ala Lys Gly
225                 230                 235                 240

Leu Leu Ile Phe Leu Leu Leu Val Ile Leu Ala Met Gly Leu Trp Val
                245                 250                 255

Ile Arg Val Gln Lys Arg His Lys Met Pro Arg Met Lys Lys Leu Met
            260                 265                 270

Arg Asn Arg Met Lys Leu Arg Lys Glu Ala Lys Pro Gly Ser Ser Pro
        275                 280                 285

Ala

<210>  92
<211>  870
<212>  DNA
<213>  Homo sapiens

<400>  92
atgtgtgcct ttccttggct gcttcttctc ctgctgctcc aggagggcag ccaaaggaga      60
ctctggagat ggtgtggatc cgaggaactg gttgcggtcc ttcaggagtc catcagcctc     120
cccctggaaa taccaccaga tgaagaggtt gagaacatca tctggtcctc tcacaaaagt     180
cttgccactg tggtgccagg gaaagaggga catccagcta ccatcatggt gaccaatcca     240
cactaccagg gccaagtgag cttcctggac cccagctatt ccctgcatat cagcaatctg     300
agctgggagg attcagggct ttaccaagct caagtcaacc tgagaacatc ccagatctct     360
accatgcagc agtacaatct atgtgtctac cgatggctgt cagagcccca gatcactgtg     420
aactttgaga gttctgggga aggtgcctgc agtatgtccc tggtgtgctc tgtggagaag     480
gcaggcatgg atatgaccta cagctggctc tcccgggggg atagcactta tacattccat     540
gaaggccctg tcctcagcac atcctggagg ccgggggaca gtgccctctc ctacacctgc     600
agagccaaca accccatcag caacgtcagt tcttgcccca tccctgatgg gcccttctat     660
gcagatccta actatgcttc tgagaagcct tcaacagcct tctgcctcct ggccaaggga     720
ttgctcatct tcttgctctt ggtaattctg gccatgggac tctgggtcat ccgagtccag     780
aaaagacaca aaatgccaag gatgaagaaa ctcatgagaa acagaatgaa attgaggaag     840
gaggcaaagc ctggctccag ccctgcctga                                      870

<210>  93
<211>  870
<212>  DNA
<213>  Homo sapiens

<400> 93
atgtgtgcct ttccttggct gcttcttctc ctgctgctcc aggagggcag ccaaaggaga 60
ctctggagat ggtgtggatc cgaggaagtg gttgcggtcc ttcaggagtc catcagcctc 120
cccctggaaa taccaccaga tgaagaggtt gagaacatca tctggtcctc tcacaaaagt 180
cttgccactg tggtgccagg gaaagaggga catccagcta ccatcatggt gaccaatcca 240
cactaccagg gccaagtgag cttcctggac cccagctatt ccctgcatat cagcaatctg 300
agctgggagg attcagggct ttaccaagct caagtcaacc tgagaacatc ccagatctct 360
accatgcagc agtacaatct atgtgtctac cgatggctgt cagagcccca gatcactgtg 420
aactttgaga gttctgggga aggtgcctgc agtatgtccc tggtgtgctc tgtggagaag 480
gcaggcatgg atatgaccta cagctggctc tcccgggggg atagcactta tacattccat 540
gaaggccctg tcctcagcac atcctggagg ccgggggaca gtgccctctc ctacacctgc 600
agagccaaca accccatcag caacgtcagt tcttgcccca tccctgatgg gcccttctat 660
gcagatccta actatgcttc tgagaagcct tcaacagcct tctgcctcct ggccaaggga 720
ttgctcatct tcttgctctt ggtaattctg gccatgggac tctgggtcat ccgagtccag 780
aaaagacaca aaatgccaag gatgaggaaa ctcatgagaa acagaatgaa attgaggaag 840
gaggcaaagc ctggctccag ccctgcctga 870

<210> 94
<211> 870
<212> DNA
<213> Homo sapiens

<400> 94
atgtgtgcct ttccttggct gcttcttctc ctgctgctcc aggagggcag ccaaaggaga 60
ctctggagat ggtgtggatc cgaggaagtg gttgcggtcc ttcaggagtc catcagcctc 120
cccctggaaa taccaccaga tgaagaggtt gagaacatca tctggtcctc tcacaaaagt 180
cttgccactg tggtgccagg gaaagaggga catccagcta ccatcatggt gaccaatcca 240
cactaccagg gccaagtgag cttcctggac cccagctatt ccctgcatat cagcaatctg 300
agctgggagg attcagggct ttaccaagct caagtcaacc tgagaacatc ccagatctct 360
accatgcagc agtacaatct atgtgtctac cgatggctgt cagagcccca gatcactgtg 420
aactttgaga gttctgggga aggtgcctgc agtatgtccc tggtgtgctc tgtggagaag 480
gcaggcatgg atatgaccta cagctggctc tcccgggggg atagcactta tacattccat 540
gaaggccctg tcctcagcac atcctggagg ccgggggaca gtgccctctc ctacacctgc 600
agagccaaca accccatcac caacgtcagt tcttgcccca tccctgatgg gcccttctat 660
gcagatccta actatgcttc tgagaagcct tcaacagcct tctgcctcct ggccaaggga 720
ttgctcatct tcttgctctt ggtaattctg gccatgggac tctgggtcat ccgagtccag 780
aaaagacaca aaatgccaag gatgaagaaa ctcatgagaa acagaatgaa attgaggaag 840
gaggcaaagc ctggctccag ccctgcctga 870

<210> 95
<211> 870
<212> DNA
<213> Homo sapiens

<400> 95
atgtgtgcct ttccttggct gcttcttctc ctgctgctcc aggagggcag ccaaaggaga 60
ctctggagat ggtgtggatc cgaggaagtg gttgcggtcc ttcaggagtc catcagcctc 120
cccctggaaa taccaccaga tgaagaggtt gagaacatca tctggtcctc tcacaaaagt 180
cttgccactg tggtgccagg gaaagaggga catccagcta ccatcatggt gaccaatcca 240
cactaccagg gccaagtgag cttcctggac cccagctatt ccctgcatat cagcaatctg 300
agctgggagg attcagggct ttaccaagct caagtcaacc tgagaacatc ccagatctct 360
accatgcagc agtacaatct atgtgtctac cgatggctgt cagagcccca gatcactgtg 420
aactttgaga gttctgggga aggtgcctgc agtatgtccc tggtgtgctc tgtggagaag 480
gcaggcatgg atatgaccta cagctggctc tcccgggggg atagcactta tacattccat 540
gaaggccctg tcctcagcac atcctggagg ccgggggaca gtgccctctc ctacacctgc 600
agagccaaca accccatcag caacgtcagt tcttgcccca tccctgatgg gcccttctat 660
ggagatccta actatgcttc tgagaagcct tcaacagcct tctgcctcct ggccaaggga 720
ttgctcatct tcttgctctt ggtaattctg gccatgggac tctgggtcat ccgagtccag 780
aaaagacaca aaatgccaag gatgaagaaa ctcatgagaa acagaatgaa attgaggaag 840
gaggcaaagc ctggctccag ccctgcctga 870

<210> 96
<211> 98
<212> PRT
<213> Homo sapiens

<400> 96
Gln Glu Ser Ile Ser Leu Pro Leu Glu Ile Pro Pro Asp Glu Glu Val
1               5                   10                  15

Glu Asn Ile Ile Trp Ser Ser His Lys Ser Leu Ala Thr Val Val Pro
            20                  25                  30

Gly Lys Glu Gly His Pro Ala Thr Ile Met Val Thr Asn Pro His Tyr
        35                  40                  45

Gln Gly Gln Val Ser Phe Leu Asp Pro Ser Tyr Ser Leu His Ile Ser
    50                  55                  60

Asn Leu Ser Trp Glu Asp Ser Gly Leu Tyr Gln Ala Gln Val Asn Leu
65                  70                  75                  80

Arg Thr Ser Gln Ile Ser Thr Met Gln Gln Tyr Asn Leu Cys Val Tyr
                85                  90                  95

Arg Trp


<210>  97
<211>  70
<212>  PRT
<213>  Homo sapiens

<400>  97
Glu Pro Gln Ile Thr Val Asn Phe Glu Ser Ser Gly Glu Gly Ala Cys
1               5                   10                  15

Ser Met Ser Leu Val Cys Ser Val Glu Lys Ala Gly Met Asp Met Thr
            20                  25                  30

Tyr Ser Trp Leu Ser Arg Gly Asp Ser Thr Tyr Thr Phe His Glu Gly
        35                  40                  45

Pro Val Leu Ser Thr Ser Trp Arg Pro Gly Asp Ser Ala Leu Ser Tyr
    50                  55                  60

Thr Cys Arg Ala Asn Asn
65                  70

<210>  98
<211>  28
<212>  DNA
<213>  Artificial Sequence

<220>
<223> Description of Artificial Sequence: Primer

<400>  98
atgtgtgcct ttccttggct gcttcttc                                        28

<210>  99
<211>  28
<212>  DNA
<213>  Artificial Sequence

<220>
<223> Description of Artificial Sequence: Primer

<400>  99
ccgtttcgga ccgaggtcgg gacggact                                        28

<210>  100
<211>  285
<212>  PRT
<213>  Homo sapiens

<400>  100
Met Gly Ala Leu Leu Trp Ser Leu Leu Leu Leu Leu Gln Glu Ala Lys

1               5                   10                  15

Gly Phe Ser Gly Asp Asp Glu Asp Pro Glu Glu Val Val Gly Val Leu
            20                  25                  30

Gln Glu Ser Ile Asn Leu Ser Leu Glu Ile Pro Ser Asn Glu Glu Ile
        35                  40                  45

Lys His Ile Asp Trp Leu Phe Gln Asn Asn Ile Ala Ile Val Lys Pro
    50                  55                  60

Gly Lys Lys Gly Gln Pro Ala Val Ile Met Ala Val Asp Pro Arg Tyr
65                  70                  75                  80

Arg Gly Arg Val Ser Ile Ser Glu Ser Ser Tyr Ser Leu His Ile Ser
                85                  90                  95

Asn Leu Thr Trp Glu Asp Ser Gly Leu Tyr Asn Ala Gln Val Asn Leu
            100                 105                 110

Lys Thr Ser Glu Ser His Ile Thr Lys Ser Tyr His Leu Arg Val Tyr
        115                 120                 125

Arg Arg Leu Ser Lys Pro His Ile Thr Val Asn Ser Asn Ile Ser Glu
    130                 135                 140

Glu Gly Val Cys Asn Ile Ser Leu Thr Cys Ser Ile Glu Arg Ala Gly
145                 150                 155                 160

Met Asp Val Thr Tyr Ile Trp Leu Ser Ser Gln Asp Ser Thr Asn Thr
                165                 170                 175

Ser His Glu Gly Ser Val Leu Ser Thr Ser Trp Arg Pro Gly Asp Lys
            180                 185                 190

Ala Pro Ser Tyr Thr Cys Arg Val Ser Asn Pro Ile Ser Asn Ile Ser
        195                 200                 205

Ser Arg Arg Ile Ser Val Gly Ser Phe Cys Ala Asp Pro Gly Tyr Leu
    210                 215                 220

Glu Lys Pro Ser Met Leu Cys Leu Leu Val Lys Ser Leu Phe Leu Leu
225                 230                 235                 240

Leu Leu Leu Ala Ile Leu Thr Val Gly Leu Cys Leu Phe Arg Ala Gln
                245                 250                 255

Lys Ser Tyr Glu Thr Pro Arg Val Arg Lys Leu Lys Arg Asn Arg Ile
            260                 265                 270

Lys Leu Arg Lys Lys Gly Lys Ser Gly Pro Thr Pro Val
        275                 280                 285

## Description

This application is a continuation-in-part of U.S. application Serial No. 09/706,167, filed November 3, 2000, which is incorporated herein by reference in its entirety.

### Background of the Invention

Many secreted proteins, for example, cytokines and cytokine receptors, play a vital role in the regulation of cell growth, cell differentiation, and a variety of specific cellular responses. A number of medically useful proteins, including erythropoietin, granulocyte-macrophage colony stimulating factor, human growth hormone, and various interleukins, are secreted proteins. Thus, an important goal in the design and development of new therapies is the identification and characterization of secreted and transmembrane proteins and the genes which encode them.

Many secreted proteins are receptors which bind a ligand and transduce an intracellular signal, leading to a variety of cellular responses. Other secreted proteins are extracellular proteins that act as ligands by binding a receptor which leads to transduction of intracellular signals and ultimately results in downstream cellular responses. The identification and characterization of receptors and/or ligands permits identification of other molecules and of the signal transduction pathways associated with the receptors and/or ligands, permitting one to identify or design modulators of activity, *e.g.*, receptor agonists or antagonists, modulators of signal transduction, and modulators of downstream cellular responses.

Virtually all cell types respond to extracellular and intercellular cues and induce one or more signal transduction pathways. Among such cell types are cells involved in the development, differentiation, activation, function, and maintenance of hematopoietic cells. These cell types include ones (*e.g*., B cells and T cells, and subpopulations thereof, such as T helper (TH) cells, including TH1 and TH2 cells) involved in a variety of immune and autoimmune responses, such as inflammatory responses. These responses can contribute to such diverse disorders as pulmonary disorders such as asthma and emphysema, arthritis, inflammatory bowel diseases such as Crohn's disease and ulcerative colitis, multiple sclerosis, graft-versus-host disease and tissue rejection. As such, identification of secreted proteins involved in some aspect of these cell types is particularly desirable.

### Summary of the Invention

The present invention is based, at least in part, on the discovery of cDNA molecules which encode the CD2000 proteins, which are transmembrane proteins. The present invention relates to CD2000 proteins and uses therefor. The present invention also relates to CD2001 proteins and uses therefor. CD2000 proteins and CD2001 proteins represent CD2 family members.

The CD2000 and CD2001 proteins, and fragments, derivatives, and variants, including allelic variants, thereof are collectively referred to herein as "polypeptides of the invention" or "proteins of the invention."

The term "CD2000 polypeptide of the invention" or CD2000 protein of the invention" as used herein refers to CD2000 proteins, and fragments, derivative and variants, including allelic variants, thereof.

The term "CD2001 polypeptide of the invention" or "CD2001 protein of the invention" as used herein refers to CD2001 proteins, and fragments, derivative and variants, including allelic variants, thereof.

The term "nucleic acids of the invention," as used herein, refers to: 1) nucleic acid molecules encoding the polypeptides of the invention; 2) nucleic acid molecules present as part of CD2000 transcripts or cDNA molecules encoding the polypeptides of the invention *(e.g.,* upstream and/or downstream untranslated sequences); 3) nucleic acid molecules present as part of CD2001 transcripts or cDNA molecules encoding the polypeptides of the invention *(e.g.,* upstream and/or downstream untranslated sequences); 4) nucleic acid molecules that hybridize to 1), 2) and/or 3) under the particular conditions discussed herein; and 5) nucleic acid molecules complementary to 1), 2), 3) and/or 4).

The term "CD2000 nucleic acids of the invention" as used herein refers to: 1) nucleic acid molecules encoding the polypeptides of the invention; 2) nucleic acid molecules present as part of CD2000 transcripts or cDNA molecules encoding the polypeptides of the invention (*e.g*., upstream and/or downstream untranslated sequences); 3) nucleic acid molecules that hybridize to 1) and/or 2) under the particular conditions discussed herein; and 4) nucleic acid molecules complementary to 1), 2) and/or 3).

The term "CD2001 nucleic acids of the invention" as used herein refers to: 1) nucleic acid molecules encoding the polypeptides of the invention; 2) nucleic acid molecules present as part of CD2001 transcripts or cDNA molecules encoding the polypeptides of the invention (*e.g*., upstream and/or downstream untranslated sequences); 3) nucleic acid molecules that hybridize to 1) and/or 2) under the particular conditions discussed herein; and 4) nucleic acid molecules complementary to 1), 2) and/or 3).

The polypeptides of the invention are useful for a variety of purposes. The polypeptides of the invention are involved in immune disorders such as lymphoproliferative disorders (*e.g.,* leukemia and X-linked lymphoproliferative disease), inflammatory bowel disease (*e.g.,* Crohn's disease and ulcerative colitis), autoimmune disorders (*e.g.,* multiple sclerosis), inflammatory disorders (*e.g*., rheumatoid arthritis and asthma), and chronic obstructive pulmonary disorders, and viral, bacterial, fungal or parasitic infections. As such, polypeptides of the invention are useful, for example, as modulators of immune disorders such as lymphoproliferative disorders (*e.g.,* leukemia and X-linked lymphoproliferative disease), inflammatory bowel disease (*e.g.,* Crohn's disease and ulcerative colitis), autoimmune disorders (*e.g*., multiple sclerosis), inflammatory disorders (*e.g.,* rheumatoid arthritis and asthma), and chronic obstructive pulmonary disorders, and viral, bacterial, fungal or parasitic infections. Further, the polypeptides of the invention are useful for identification of additional modulators of immune disorders such as lymphoproliferative disorders (*e.g.,* leukemia and X-linked lymphoproliferative disease), inflammatory bowel disease (*e.g.,* Crohn's disease and ulcerative colitis), autoimmune disorders *(e.g.,* multiple sclerosis), inflammatory disorders *(e.g.,* rheumatoid arthritis and asthma), and chronic obstructive pulmonary disorders, and viral, bacterial, fungal or parasitic infections.

The polypeptides of the invention are expressed by immune cells *(e.g.,* T cells (*e.g*., CD4⁺ and CD8⁺ T cells), B cells, eosinophils, dendritic cells and granulocytes). As such, the polypeptides of the invention can be used as markers for identification, isolation, depletion, or tracking of immune cells, in particular T cells, B cells, eosinophils, dendritic cells and granulocytes, in a sample. The polypeptides of the invention can also be used to modulate the differentiation, replication and/or effector functions of immune cells such as, for example, T cells, B cells, eosinophils, dendritic cells and granulocytes. Higher levels of CD2000 are expressed by activated CD4⁺ T cells than resting CD4⁺, resting CD19⁺ B cells than activated CD19⁺ B cells, activated eosinophils than resting eosinophils, resting granulocytes than activated granulocytes, and TH1 cells than TH0 or TH2 cells. As such, the CD2000 polypeptides of the invention can be used to detect activated CD4⁺ T cells, resting CD19⁺ B cells, activated eosinophils, resting granulocytes, and TH1 cells. Higher levels of CD2000 are detected in synovium from rheumatoid arthritis patients than normal synovium, and higher levels of CD2000 are detected on colons from patients with Crohn's disease and colitis than on normal colons. As such, the CD2000 polypeptides of the invention can be used as a marker for rheumatoid arthritis, Crohn's disease, and colitis.

The polypeptides of the invention can also be used as antigens to make antibodies that can, in turn, be used for identification, isolation, depletion, or tracking of immune cells, in particular T cells, B cells, eosinophils, dendritic cells and granulocytes, or CD2000 or CD2001 in a sample, or to track immune disorders such as lymphoproliferative disorders (*e.g*., leukemia and X-linked lymphoproliferative disease), inflammatory bowel disease (*e.g*., Crohn's disease and ulcerative colitis), autoimmune disorders (*e.g*., multiple sclerosis), inflammatory disorders (*e.g*., rheumatoid arthritis and asthma), and chronic obstructive pulmonary disorders, and viral, bacterial, fungal or parasitic infections. The polypeptides of the invention are useful to generate antibodies that, in turn, can be used to detect and/or diagnose immune disorders such as lymphoproliferative disorders *(e.g.,* leukemia and X-linked lymphoproliferative disease), inflammatory bowel disease (*e.g.*, Crohn's disease and ulcerative colitis), autoimmune disorders *(e.g.,* multiple sclerosis), inflammatory disorders (*e.g*., rheumatoid arthritis and asthma), and chronic obstructive pulmonary disorders, and viral, bacterial, fungal or parasitic infections.

The nucleic acids of the invention are also useful for variety of purposes. First, among the nucleic acids of the invention are ones that encode a polypeptide of the invention. As such, these nucleic acids of the invention can be used to encode a polypeptide of the invention that is a modulator of immune disorders such as lymphoproliferative disorders ((*e.g*., leukemia and X-linked lymphoproliferative disease), inflammatory bowel disease (*e.g.,* Crohn's disease and ulcerative colitis), autoimmune disorders *(e.g.,* multiple sclerosis), inflammatory disorders (*e.g.,* rheumatoid arthritis and asthma), and chronic obstructive pulmonary disorders, and viral, bacterial, fungal or parasitic infections. The nucleic acids of the invention encoding polypeptides of the invention can also be used as part of methods for identifying additional modulators of immune disorders such as lymphoproliferative disorders *(e.g.,* leukemia and X-linked lymphoproliferative disease), inflammatory bowel disease *(e.g.,* Crohn's disease and ulcerative colitis), autoimmune disorders *(e.g.,* multiple sclerosis), inflammatory disorders (*e.g*., rheumatoid arthritis and asthma), and chronic obstructive pulmonary disorders, and viral, bacterial, fungal or parasitic infections.

The nucleic acids of the invention also include nucleic acid molecules present as part of CD2000 or CD2001 transcripts or cDNA molecules, nucleic acid molecules that hybridize to such sequences under particular hybridization conditions discussed herein, and nucleic acid molecules complementary to such sequences. As such, nucleic acid molecules of the invention can be used for the identification and tracking of immune cells, in particular T cells, B cells, eosinophils, dendritic cells and granulocytes, based on expression of CD2000 or CD2001. Further, nucleic acids of the invention encoding polypeptides of the invention can be used to modulate the differentiation, replication and/or effector functions of immune cells such as T cells, B cells, eosinophils, dendritic cells and granulocytes. Still further, the nucleic acids of the invention can be used to detect, diagnosis, or track the onset, development or progression of immune disorders such as lymphoproliferative disorders *(e.g.,* leukemia and X-linked lymphoproliferative disease), inflammatory bowel disease *(e.g.,* Crohn's disease and ulcerative colitis), autoimmune disorders (*e.g*., multiple sclerosis), inflammatory disorders *(e.g.,* rheumatoid arthritis and asthma), and chronic obstructive pulmonary disorders, and viral, bacterial, fungal or parasitic infections.

The invention features nucleic acid molecules comprising a contiguous nucleotide sequence identical to the nucleotide sequence of SEQ ID NO:1, the nucleotide sequence of the cDNA insert of a EpCD2000 clone deposited with the American Type Culture Collection ("ATCC®") as patent deposit Number PTA-2267, or a complement thereof.

The invention features nucleic acid molecules comprising a contiguous nucleotide sequence identical to the nucleotide sequence of SEQ ID NO:1, 2, 31, 32, 33, 34, 35, 36, 37, 38, 43, 44, 45, 46, 47, 48, 49, 50, 51, 52, 53, 54, 55, 56, 57, 58, 66 or 67, or a complement thereof.

The invention features nucleic acid molecules that are at least 25%, preferably at least 30%, at least 35%, at least 40%, at 45%, at least 50%, at least 55%, at least 60%, at least 65%, at least 70%, at least 75%, at least 80%, at least 85%, at least 90%, at least 95%, or at least 98% identical to the nucleotide sequence of SEQ ID NO:1, 31, 32, 33, or 34, the nucleotide sequence of the cDNA insert of a EpCD2000 clone deposited with the American Type Culture Collection ("ATCC®") as patent deposit Number PTA-2267, or a complement thereof. The invention also features nucleic acid molecules that are at least 35%, preferably at least 40%, at 45%, at least 50%, at least 55%, at least 60%, at least 65%, at least 70%, at least 75%, at least 80%, at least 85%, at least 90%, at least 95%, or at least 98% identical to the nucleotide sequence of SEQ ID NO:2, 35, 36, 37, 38, 43, 44, 45, 46, 47, 48, 49, 50, 51, 52, 53, 54, 55, 56, 57, 58, 66 or 67.

The invention features nucleic acid molecules which are at least 25%, preferably at least 30%, at least 35%, at least 40%, at 45%, at least 50%, at least 55%, at least 60%, at least 65%, at least 70%, at least 75%, at least 80%, at least 85%, at least 90%, at least 95%, or at least 98 % identical to the nucleotide sequence of SEQ ID NO:1, 2, 31, 32, 33, 34, 35, 36, 37, 38, 43, 44, 45, 46, 47, 48, 49, 50, 51, 52, 53, 54, 55, 56, 57, 58, 66 or 67, or the nucleotide sequence of the cDNA insert of a EpCD2000 clone deposited with the American Type Culture Collection ("ATCC®") as patent deposit Number PTA-2267, wherein said nucleic acid molecules encode polypeptides that exhibit at least one structural and/or functional feature of a CD2000 polypeptide of the invention.

The invention features nucleic acid molecules which comprise at least 15, preferably at least 25, at least 35, at least 40, at least 50, at least 75, at least 100, at least 125, at least 150, at least 175, at least 200, at least 225, at least 250, at least 275, at least 300, at least 325, at least 350, at least 375, at least 400, at least 425, at least 450, at least 475, at least 500, at least 525, at least 550, at least 575, at least 600, at least 625, at least 650, at least 675, at least 700, at least 725, at least 750, at least 775, at least 800, at least 825, at least 850, at least 875, at least 900, at least 925, at least 950, at least 975, at least 1000, at least 1050, at least 1100, at least 1150, at least 1200, at least 1250, at least 1300, at least 1350, at least 1400, at least 1450, at least 1500, at least 1550, at least 1600, at least 1650, at least 1700, at least 1750, at least 1800, at least 1850, at least 1900, at least 1950, at least 2000, at least 2050, at least 2100, at least 2150, at least 2200, at least 2250, at least 2300, at least 2350, at least 2400, at least 2450, at least 2500, at least 2550, at least 2600, at least 2650, or at least 2700 contiguous nucleotides of the nucleotide sequence of SEQ ID NO:1, the nucleotide sequence of a EpCD2000 cDNA of ATCC® patent deposit Number PTA-2267, or a complement thereof. The invention also features nucleic acid molecules which comprise at least 15, preferably at least 25, at least 35, at least 40, at least 50, at least 75, at least 100, at least 125, at least 150, at least 175, at least 200, at least 225, at least 250, at least 275, at least 300, at least 325, at least 350, at least 375, at least 400, at least 425, at least 450, at least 475, at least 500, at least 525, at least 550, at least 575, at least 600, at least 625, at least 650, at least 675, at least 700, at least 725, at least 750, at least 775, at least 800, at least 825, at least 850, at least 875, at least 900, at least 925, at least 950, at least 975, at least 1000, or at least 1050 contiguous nucleotides of the nucleotide sequence of SEQ ID NO:2, the nucleotide sequence of a EpCD2000 cDNA of ATCC® patent deposit Number PTA-2267, or a complement thereof.

The invention features isolated nucleic acid molecules comprising a nucleotide sequence that is at least about 15, preferably at least 25, at least 35, at least 40, at least 50, at least 75, at least 100, at least 125, at least 150, at least 200, at least 250, at least 300, at least 350, at least 400 or more contiguous nucleotides identical to the nucleic acid sequence of SEQ ID NO:1, 2, 31, 32, 33, 34, 35, 36, 37, 38, 43, 44, 45, 46, 47, 48, 49, 50, 51, 52, 53, 54, 55, 56, 57, 58, 66 or 67, or the nucleotide sequence of the cDNA insert of EpCD2000 of ATCC® patent deposit Number PTA-2267, wherein said nucleic acid molecules encode polypeptides that exhibit at least one structural and/or functional feature of a CD2000 polypeptide of the invention.

The invention also features nucleic acid molecules comprising a contiguous nucleotide sequence that encodes a polypeptide of the amino acid sequence of SEQ ID NO: 3, 4, 5, 6, 7, 8, 9, 10, 12, 13, 14, 15, 16, 17, 18, 19, 21, 22, 39, 40, 41, 42, or the amino acid sequence encoded by the cDNA insert of a EpCD2000 of ATCC® patent deposit Number PTA-2267.

The invention also features nucleic acid molecules comprising a contiguous nucleotide sequence that encodes a polypeptide of an amino acid sequence that is at least 30%, preferably at least 35%, at least 40%, at least 45%, at least 50%, at least 55%, at least 60%, at least 65%, at least 70%, at least 75%, at least 80%, at least 85%, at least 90%, at least 95% or at least 98% identical to the amino acid sequence of SEQ ID NO:3, 5, 39, 40, 41 or 42, or the amino acid sequence encoded by a EpCD2000 cDNA of ATCC® patent deposit Number PTA-2267.

The invention also features nucleic acid molecules comprising a contiguous nucleotide sequence that encodes a polypeptide of an amino acid sequence that is at least 30%, preferably at least 35%, at least 40%, at least 45%, at least 50%, at least 55%, at least 60%, at least 65%, at least 70%, at least 75%, at least 80%, at least 85%, at least 90%, at least 95% or at least 98% identical to the amino acid sequence of SEQ ID NO:4, 6, 7, 8, 9, 10, 14, 15, 16, 17, 18, 19, 21, or 22.

The invention also features nucleic acid molecules comprising a nucleotide sequence encoding a polypeptide of an amino acid sequence that is at least 25%, preferably at least 30%, at least 35%, at least 38%, at least 40%, at least 45%, at least 50%, at least 55%, at least 60%, at least 65%, at least 70%, at least 75%, at least 80%, at least 85%, at least 90%, at least 95% or at least 98% identical to the amino acid sequence of SEQ ID NO:3, 4, 5, 6, 7, 8, 9, 10, 12,13, 14, 15, 16, 17, 18, 19, 21, 22, 39, 40, 41 or 42, or the amino acid sequence encoded by a EpCD2000 cDNA of ATCC® patent deposit Number PTA-2267, wherein the polypeptide encoded by the nucleotide sequence also exhibits at least one structural and/or functional feature of a CD2000 polypeptide of the invention.

The invention includes nucleic acid molecules comprising a nucleotide sequence that encodes a polypeptide comprising at least 15, preferably at least 25, at least 50, at least 75, at least 100, at least 125, at least 150, at least 175, at least 200, at least 225, at least 250, at least 275, at least 300, at least 325, at least 330 or more contiguous amino acids of SEQ ID NO:3, or the amino acid sequence encoded by a EpCD2000 cDNA of ATCC® patent deposit Number PTA-2267. The invention also includes nucleic acid molecules comprising a nucleotide sequence that encodes a polypeptide comprising at least 15, preferably at least 25, at least 50, at least 75, at least 100, at least 125, at least 150, at least 175, at least 200, at least 225, at least 250, at least 275, at least 300, at least 325, at least 330 or more contiguous amino acids of SEQ ID NO:3, or the amino acid sequence encoded by a EpCD2000 cDNA of ATCC® patent deposit Number PTA-2267, wherein the polypeptide encoded by the nucleotide sequence also exhibits at least one structural and/or functional feature of a CD2000 polypeptide of the invention.

The invention also includes nucleic acid molecules comprising a nucleotide sequence that encodes a polypeptide comprising at least 15, preferably at least 25, at least 50, at least 75, at least 100, at least 125, or at least 150 contiguous amino acids of amino acid residues 173 to 331 of SEQ ID NO:3. The invention also includes nucleic acid molecules comprising a nucleotide sequence that encodes a polypeptide comprising at least 15, preferably at least 25, at least 50, at least 75, at least 100, at least 125, or at least 150 contiguous amino acids of SEQ ID NO:3, wherein the polypeptide encoded by the nucleotide sequence also exhibits at least one structural and/or functional feature of a CD2000 polypeptide of the invention.

The invention also includes nucleic acid molecules which encode a naturally occurring variant, *e.g.*, a naturally occurring allelic variant, of a polypeptide comprising the amino acid sequence of SEQ ID NO:3, 4, 5, 6, 7, 8, 9,10, 12, 13, 14, 15, 16, 17, 18, 19, 21, 22, 39, 40, 41, or 42, or encoding the amino acid sequence encoded by a EpCD2000 cDNA of ATCC® patent deposit Number PTA-2267, wherein the nucleic acid molecule hybridizes to a nucleic acid molecule consisting of a nucleic acid sequence encoding SEQ ID NO:NO:3, 4, 5, 6, 7, 8, 9, 10, 12, 13, 14, 15, 16, 17, 18, 19, 21, 22, 39, 40, 41, or 42, or encoding the amino acid sequence encoded by a EpCD2000 cDNA of ATCC® patent deposit Number PTA-2267, or a complement thereof, under stringent conditions.

The invention also features nucleic acid molecules that hybridize under stringent conditions to a nucleic acid molecule having the nucleotide sequence of SEQ ID NO: 1 or 2, a EpCD2000 cDNA of ATCC® patent deposit Number PTA-2267, or a complement thereof. In certain embodiments, the nucleic acid molecules are at least 15, preferably at least 25, at least 30, at least 35, at least 40, at least 45, at least 50, at least 55, at least 60, at least 65, at least 75, at least 80, at least 85, at least 90, at least 95, at least 100, at least 125, at least 150, at least 175, at least 200, at least 225, at least 250, at least 275, at least 300, at least 325, at least 350, at least 375, at least 400, at least 425, at least 450, at least 475, at least 500, at least 525, at least 550, at least 575, at least 600, at least 625, at least 650, at least 700, at least 725, at least 750, at least 775, at least 800, at least 825, at least 850, at least 875, at least 900, at least 925, at least 950, at least 975, at least 1000, at least 1050, at least 1200, at least 1250, at least 1300, at least 1350, at least 1400, at least 1450, at least 1500, at least 1550, at least 1600, at least 1650, at least 1700, at least 1750, at least 1800, at least 1850 or more contiguous nucleotides in length and hybridize under stringent conditions to a nucleic acid molecule comprising the nucleotide sequence of SEQ ID NO:1 or 2, EpCD2000 cDNA of ATCC® patent deposit Number PTA-2267, or a complement thereof. In other embodiments, the invention provides nucleic acid molecules that hybridize under stringent conditions over their full length to a nucleic acid molecule comprising the nucleotide sequence of SEQ ID NO:1 or 2, EpCD2000 cDNA of ATCC® patent deposit Number PTA-2267, or a complement thereof. In yet other embodiments, the invention provides nucleic acid molecules that hybridize under stringent conditions over their full length to a nucleic acid molecule consisting of the nucleotide sequence of SEQ ID NO:1 or 2, EpCD2000 cDNA of ATCC® patent deposit Number PTA-2267, or a complement thereof.

The invention also features nucleic acid molecules that hybridize under stringent conditions to a nucleic acid molecule having the nucleotide sequence of SEQ ID NO:1, 2, 31, 32, 33, 34, 35, 36, 37, 38, 43, 44, 45, 46, 47, 48, 49, 50, 51, 52, 53, 54, 55, 56, 57, 58, 66 or 67, or EpCD2000 cDNA of ATCC® patent deposit Number PTA-2267, or complement thereof, wherein such nucleic acid molecules encode polypeptides that exhibit at least one structural and/or functional feature of a CD2000 polypeptide of the invention.

The invention also features nucleic acid molecules at least 15, preferably at least 25, at least 30, at least 35, at least 40, at least 45, at least 50, at least 55, at least 60, at least 65, at least 75, at least 80, at least 85, at least 90, at least 95, at least 100, at least 125, at least 150, at least 175, at least 200, at least 225, at least 250, at least 275, at least 300, at least 325, at least 350, at least 375, at least 400, at least 425, at least 450, at least 475, at least 500, at least 525, at least 550, at least 575, at least 600, at least 625, at least 650, at least 700, at least 725, at least 750, at least 775, at least 800, at least 825, at least 850, at least 875, at least 900, at least 925, at least 950, at least 975, at least 1000, at least 1050, at least 1200, at least 1250, at least 1300, at least 1350, at least 1400, at least 1450, at least 1500, at least 1550, at least 1600, at least 1650, at least 1700, at least 1750, at least 1800, at least 1850 or more contiguous nucleotides in length which hybridize under stringent conditions to a nucleic acid molecule comprising the nucleotide sequence of SEQ ID NO.1, 2, 31, 32, 33, 34, 35, 36, 37, 38, 43, 44, 45, 46, 47, 48, 49, 50, 51, 52, 53, 54, 55, 56, 57, 58, 66 or 67, or EpCD2000 cDNA of ATCC® patent deposit Number PTA-2267, or a complement thereof, wherein said nucleic acid molecules encode polypeptides or proteins that exhibit at least one structural and/or functional feature of a CD2000 polypeptide of the invention.

The invention also features nucleic acid molecules that hybridize under stringent conditions over their full length to a nucleic acid molecule comprising the nucleotide sequence SEQ ID NO.1, 2, 31, 32, 33, 34, 35, 36, 37, 38, 43, 44, 45, 46, 47, 48, 49, 50, 51, 52, 53, 54, 55, 56, 57, 58, 66 or 67, EpCD2000 cDNA of ATCC® patent deposit Number PTA-2267, or a complement thereof, wherein said nucleic acid molecules encode polypeptides or proteins that exhibit at least one structural and/or functional feature of a CD2000 polypeptide of the invention. The invention further features nucleic acid molecules that hybridize under stringent conditions over their full length to a nucleic acid molecule consisting of the nucleotide sequence of SEQ ID NO.1, 2, 31, 32, 33, 34, 35, 36, 37, 38, 43, 44, 45, 46, 47, 48, 49, 50, 51, 52, 53, 54, 55, 56, 57, 58, 66 or 67, EpCD2000 cDNA of ATCC® patent deposit Number PTA-2267, or a complement thereof, wherein said nucleic acid molecules encode polypeptides or proteins that exhibit at least one structural and/or functional feature of a CD2000 polypeptide of the invention.

The invention features nucleic acid molecules comprising a contiguous nucleotide sequence identical to the nucleotide sequence of SEQ ID NO:74 or a complement thereof. The invention features nucleic acid molecules comprising a contiguous nucleotide sequence identical to the nucleotide sequence of SEQ ID NO:74, 75, 84, 85, 86, 87, 92, 93, 94, or 95, or a complement thereof.

The invention features nucleic acid molecules that are at least 80%, preferably at least 85%, at least 90%, at least 95%, or at least 98% identical to the nucleotide sequence of SEQ ID NO:74, 75, 92, 93, 94, or 95, or a complement thereof. The invention also features nucleic acid molecules that are at least 95%, preferably at least 98% identical to the nucleotide sequence of SEQ ID NO:84 or 87.

The invention features nucleic acid molecules which are at least 80%, preferably at least 85%, at least 90%, at least 95%, or at least 98 % identical to the nucleotide sequence of SEQ ID NO:74, 75, 92, 93, 94, or 95, or a complement thereof, wherein said nucleic acid molecules encode polypeptides that exhibit at least one structural and/or functional feature of a CD2001 polypeptide of the invention. The invention also features nucleic acid molecules that are at least 95%, preferably at least 98% identical to the nucleotide sequence of SEQ ID NO:84 or 87, wherein said nucleic acid molecules encode polypeptides that exhibit at least one structural and/or functional feature of a CD2001 polypeptide of the invention.

The invention features nucleic acid molecules which comprise at least 25, preferably at least 50, at least 75, at least 100, at least 125, at least 150, at least 175, at least 200, at least 225, at least 250, at least 275, at least 300, at least 325, at least 350, at least 400, at least 425, at least 450, at least 475 or more contiguous nucleotides of the nucleotide sequence of SEQ ID NO:74 or 75, or a complement thereof. The invention also features isolated nucleic acid molecules comprising a nucleotide sequence that is at least 25, preferably at least 50, at least 75, at least 100, at least 125, at least 150, at least 175, at least 200, at least 225, at least 250, at least 275, at least 300, at least 325, at least 350, at least 400, at least 425, at least 450, at least 475 or more contiguous nucleotides of the nucleotide sequence of SEQ ID NO:74 or 75, or a complement thereof, wherein said nucleic acid molecules encode polypeptides that exhibit at least one structural and/or functional feature of a CD2001 polypeptide of the invention.

The invention also features nucleic acid molecules comprising a contiguous nucleotide sequence that encodes a polypeptide of the amino acid sequence of SEQ ID NO:76, 77, 79, 80, 81, 82, 88, 89, 90, or 91, or a complement thereof.

The invention also features nucleic acid molecules comprising a contiguous nucleotide sequence that encodes a polypeptide of an amino acid sequence that is at least 70%, at least 75%, at least 80%, at least 85%, at least 90%, at least 95% or at least 98% identical to the amino acid sequence of SEQ ID NO:76, 77, 79, 80, 81, 82, 88, 89, 90, or 91. The invention also features nucleic acid molecules comprising a contiguous nucleotide sequence that encodes a polypeptide of an amino acid sequence that is at least 70%, at least 75%, at least 80%, at least 85%, at least 90%, at least 95% or at least 98% identical to the amino acid sequence of SEQ ID NO:76, 77, 79, 80, 81, 82, 88, 89, 90, or 91, wherein the polypeptide encoded by the nucleotide sequence also exhibits at least one structural and/or functional feature of a CD2001 polypeptide of the invention.

The invention includes nucleic acid molecules comprising a nucleotide sequence that encodes a polypeptide comprising at least 15, preferably at least 25, at least 50, at least 75, at least 100, at least 125, at least 150, at least 175, at least 180, at least 190 or more contiguous amino acid residues of SEQ ID NO:76. The invention includes nucleic acid molecules comprising a nucleotide sequence that encodes a polypeptide comprising at least 15, preferably at least 25, at least 50, at least 75, at least 100, at least 125, at least 150, at least 175, at least 180, at least 190 or more contiguous amino acid residues of SEQ ID NO:76, wherein the polypeptide encoded by the nucleotide sequence also exhibits at least one structural and/or functional feature of a CD2001 polypeptide of the invention.

The invention also includes nucleic acid molecules which encode a naturally occurring variant, *e.g.*, a naturally occurring allelic variant, of a polypeptide comprising the amino acid sequence of SEQ ID NO:76, 77, 79, 80, 81, 82, 88, 89, 90, or 91, wherein the nucleic acid molecule hybridizes to a nucleic acid molecule consisting of a nucleic acid sequence encoding SEQ ID NO:76, 77, 79, 80, 81, 82, 88, 89, 90, or 91, or a complement thereof, under stringent conditions.

The invention also features nucleic acid molecules that hybridize under stringent conditions to a nucleic acid molecule having the nucleotide sequence of SEQ ID NO:74 or 75, or a complement thereof. In certain embodiments, the nucleic acid molecules hybridize under stringent conditions over their full length to a nucleic acid molecule comprising the nucleotide sequence of SEQ ID NO:74 or 75, or a complement thereof. In other embodiments, the nucleic acid molecules hybridize under stringent conditions over their full length to a nucleic acid molecule consisting of the nucleotide sequence of SEQ ID NO:74 or 75, or a complement thereof.

The invention also features nucleic acid molecules that hybridize under stringent conditions to a nucleic acid molecule having the nucleotide sequence of SEQ ID NO:74 or 75, or a complement thereof, wherein such nucleic acid molecules encode polypeptides that exhibit at least one structural and/or functional feature of a CD2001 polypeptide of the invention.

In one embodiment, the invention provides an isolated nucleic acid molecule which is antisense to the coding strand of a nucleic acid of the invention. CD2000 or CD2001 antisense molecules can be used, for example, as modulators of immune disorders such as lymphoproliferative disorders (*e.g*., leukemia and X-linked lymphoproliferative disease), inflammatory bowel disease *(e.g.,* Crohn's disease and ulcerative colitis), autoimmune disorders *(e.g.,* multiple sclerosis), inflammatory disorders (*e.g*., rheumatoid arthritis and asthma), and chronic obstructive pulmonary disorders, and viral, bacterial, fungal or parasitic infections.

Preferred nucleic acid molecules of the invention are ones that encode a protein or polypeptide of the invention that possesses at least one biological activity possessed by the corresponding naturally-occurring human polypeptide.

Another aspect of the invention provides vectors, *e.g.,* recombinant expression vectors, comprising a nucleic acid molecule of the invention, and methods for producing such vectors. In another embodiment, the invention provides host cells containing such a vector or engineered to contain and/or express a nucleic acid molecule of the invention, and methods for producing such host cells. The invention also provides methods for producing a polypeptide of the invention by culturing, in a suitable medium, a host cell of the invention such that a polypeptide of the invention is produced.

The invention also features isolated CD2000 polypeptides of the invention. In one embodiment, a CD2000 protein includes at least one or more of the following domains: a signal sequence, an immunoglobulin domain, an immunoglobulin-like domain, or an amino acid sequence sufficiently identical to an identified domain of a CD2000 polypeptide of the invention.

As used herein, the term "sufficiently identical" refers to a first amino acid which contains a sufficient or minimum number of identical or equivalent (*e.g*., with a similar side chain) amino acid residues to a second amino acid, such that the first and second amino acid have a common structural domain and/or common functional activity. For example, amino acid sequences which contain or encode a common structural domain having about 60% identity, preferably 65% identity, more preferably 75%, 85%, 95%, 98% or more identity are defined herein as sufficiently identical. Likewise, as used herein, the term "sufficiently identical" refers to a first nucleotide sequence which contains a sufficient or minimum number of identical or equivalent (*e.g*., degenerate nucleotides for amino acid coding purposes) nucleotides to a nucleotide sequence such that the first and second nucleotide sequences encode polypeptides with a common structural domain and/or common functional activity.

The invention features isolated polypeptides comprising the amino acid sequence of SEQ ID NO:3, 4, 5, 6, 7, 8, 9, 10, 12, 13, 14, 15, 16, 17, 18, 19, 21, 22, 39, 40, 41 or 42, or the amino acid sequence encoded by a EpCD2000 cDNA of ATCC® patent deposit Number PTA-2267.

The invention also includes isolated polypeptides comprising an amino acid sequence that is at least about 35%, preferably at least 40%, at least 45%, at least 50%, at least 55%, at least 60%, at least 65%, at least 70%, at least 75%, at least 80%, at least 85%, at least 90%, at least 95% or at least 98% identical to the amino acid sequence of SEQ ID NO:3, 5, 39, 40, 41, 42, or the amino acid sequence encoded by a EpCD2000 cDNA of ATCC® patent deposit Number PTA-2267. The invention also includes isolated polypeptides comprising an amino acid sequence that is at least 30%, preferably at least 35%, at least 40%, at least 45%, at least 50%, at least 55%, at least 60%, at least 65%, at least 70%, at least 75%, at least 80%, at least 85%, at least 90%, at least 95% or at least 98% identical to the amino acid sequence of SEQ ID NO:4, 5, 6, 7, 8, 9, 10, 13, 14, 15, 16, 17, 18, 19, 21, or 22.

The invention also features isolated polypeptides comprising an amino acid sequence that is at least about 25%, preferably at least 30%, at least 35%, at least 40%, at least 45%, at least 50%, at least 55%, at least 60%, at least 65%, at least 70%, at least 75%, at least 80%, at least 85%, at least 90%, at least 95% or at least 98% identical to the amino acid sequence of SEQ ID NO:3, 4, 5, 6, 7, 8, 9, 10, 12, 13, 14, 15, 16, 17, 18, 19, 21, 22, 39, 40, 41, or 42, or the amino acid sequence encoded by a EpCD2000 cDNA of ATCC® patent deposit Number PTA-2267, wherein the polypeptides also exhibit at least one structural and/or functional feature of a polypeptide of the invention.

The invention also includes isolated polypeptides which are encoded by a nucleic acid molecule having a nucleotide sequence that is at least about 25%, preferably at least 30%, at least 35%, at least 40%, at least 45%, at least 50%, at least 55%, at least 60%, at least 65%, at least 70%, at least 75%, at least 80%, at least 85%, at least 90%, at least 95% or at least 98% identical to the nucleic acid sequence encoding SEQ ID NO:3. The invention also includes isolated polypeptides which are encoded by a nucleic acid molecule having a nucleotide sequence which hybridizes under stringent hybridization conditions to a nucleic acid molecule having the nucleotide sequence of SEQ ID NO:1 or 2, a complement thereof, or the non-coding strand of a EpCD2000 cDNA of ATCC® patent deposit Number PTA-2267.

The invention also features isolated polypeptides which are encoded by a nucleic acid molecule having a nucleotide sequence that is at least about 25%, preferably at least 30%, at least 35%, at least 40%, at least 45%, at least 50%, at least 55%, at least 60%, at least 65%, at least 70%, at least 75%, at least 80%, at least 85%, at least 90%, at least 95% or 98% identical to a nucleic acid sequence encoding SEQ ID NO:3, 4, 5 ,6, 7, 8, 9, 10, 12, 13, 14, 15, 16, 17, 18, 19, 21, 22, 39, 40, 41 or 42, wherein the polypeptides also exhibit at least one structural and/or functional feature of a CD2000 polypeptide of the invention. The invention also features isolated polypeptides which are encoded by a nucleic acid molecule having a nucleotide sequence which hybridizes under stringent hybridization conditions to a nucleic acid molecule having the nucleotide sequence of SEQ ID NO:1, 2, 31, 32, 33, 34, 35, 36, 37, 38, 43, 44, 45, 46, 47, 48, 49, 50, 51, 52, 53, 54, 55, 56, 57, 58, 66 or 67, a complement thereof, or the non-coding strand of a EpCD2000 cDNA of ATCC® patent deposit Number PTA-2267, wherein the polypeptides also exhibit at least one structural and/or functional feature of a CD2000 polypeptide of the invention.

The invention features polypeptides which comprise at least 15, preferably at least 25, at least 30, at least 35, at least 40, at least 45, at least 50, at least 55, at least 60, at least 65, at least 75, at least 80, at least 85, at least 90, at least 95, at least 100, at least 125, at least 150, at least 175, at least 200, at least 225, at least 250, at least 275, at least 300, at least 325, or at least 330 contiguous amino acid residues of the amino acid sequence of SEQ ID NO:3, or the amino acid sequence encoded by the cDNA insert of a EpCD2000 cDNA of ATCC® patent deposit Number PTA-2267. In preferred embodiments, the polypeptides represent N-terminal and/or C-terminal truncations of the amino acid sequence of SEQ ID NO:3, the amino acid sequence of the mature CD2000 polypeptide, or the amino acid sequence encoded by the cDNA insert of a EpCD2000 cDNA of ATCC® patent deposit Number PTA-2267. In one embodiment, such a polypeptide of the invention represents an antigenic polypeptide comprising at least 15 (preferably at least 25, at least 50, at least 75, at least 100, at least 125, at least 150, or at least 175) contiguous amino acids of the amino acid sequence of SEQ ID NO:3, 4, 5, 6, 7, 8, 9, 10, 12, 13, 14, 15, 16, 17, 18, 19, 21, 22, 39, 40, 41, or 42, or the amino acid sequence encoded by a EpCD2000 cDNA insert deposited with the ATCC® as patent deposit Number PTA-2267, wherein antibodies generated against the polypeptide bind to a native CD2000.

The invention also includes polypeptides which are naturally occurring variants, e.g., naturally occupying allelic variants, of a polypeptide that includes the amino acid sequence of SEQ ID NO:3, 4, 5, 6, 7, 8, 9, 10, 12, 13, 14, 15, 16, 17, 18, 19, 21, 22, 39, 40, 41, or 42, or the amino acid sequence encoded by a EpCD2000 cDNA of ATCC® patent deposit Number PTA-2267, wherein the polypeptide is encoded by a nucleic acid molecule which hybridizes to a nucleic acid molecule having the sequence of SEQ ID NO:1, 2, 31, 32, 33, 34, 35, 36, 37, 38, 43, 44, 45, 46, 47, 48, 49, 50, 51, 52, 53, 54, 55, 56, 57, 58, 66 or 67, or a complement thereof, under stringent conditions.

The invention also features isolated CD2001 polypeptides of the invention. In one embodiment, a CD2001 protein includes at least one or more of the following domains: a signal sequence, an immunoglobulin-like domain, an immunoglobulin domain, or an amino acid sequence sufficiently identical to an identified domain of a CD2001 polypeptide of the invention.

The invention features isolated polypeptides comprising the amino acid sequence of SEQ ID NO:76, 77, 78, 79, 80, 81, 82, 88, 89, 90, 91, 96 or 97. The invention also includes isolated polypeptides comprising an amino acid sequence that is at least about 70%, at least 75%, at least 80%, at least 85%, at least 90%, at least 95% or at least 98% identical to the amino acid sequence of SEQ ID NO:76 or 77. The invention also includes isolated polypeptides comprising an amino acid sequence that is at least about 90%, preferably 95% identical to the amino acid sequence of SEQ ID NO:79 or 82.

The invention also features isolated polypeptides comprising an amino acid sequence that is at least about 70%, preferably at least 75%, at least 80%, at least 85%, at least 90%, at least 95% or at least 98% identical to the amino acid sequence of SEQ ID NO:76, 77, 78, 79, 80, 81, 82, 88, 89, 90, 91, 96 or 97, wherein the polypeptides also exhibit at least one structural and/or functional feature of a CD2001 polypeptide of the invention.

The invention also includes isolated polypeptides which are encoded by a nucleic acid molecule having a nucleotide sequence that is at least about 80%, preferably at least 85%, at least 90%, at least 95% or at least 98% identical to the nucleic acid sequence encoding SEQ ID NO:76, 77, 79, 82, 88, 89, 90, 91, 96 or 97. The invention also includes isolated polypeptides which are encoded by a nucleic acid molecule having a nucleotide sequence which hybridizes under stringent hybridization conditions to a nucleic acid molecule having the nucleotide sequence of SEQ ID NO:74, 75, 84, 87, 92, 93, 94 or 95, or a complement thereof.

The invention also features isolated polypeptides which are encoded by a nucleic acid molecule having a nucleotide sequence that is at least about 70%, preferably at least 75%, at least 80%, at least 85%, at least 90%, at least 95% or 98% identical to a nucleic acid sequence encoding SEQ ID NO:76, 77, 78, 79, 80, 81, 82, 88, 89, 90, 91, 96 or 97, wherein the polypeptides also exhibit at least one structural and/or functional feature of a CD2001 polypeptide of the invention. The invention also features isolated polypeptides which are encoded by a nucleic acid molecule having a nucleotide sequence which hybridizes under stringent hybridization conditions to a nucleic acid molecule having the nucleotide sequence of SEQ ID NO:74, 75, 83, 84, 85, 86, 87, 92, 93, 94 or 95, or a complement thereof, wherein the polypeptides also exhibit at least one structural and/or functional feature of a CD2001 polypeptide of the invention.

The invention features polypeptides which comprise at least 15, preferably at least 25, at least 50, at least 75, at least 100, at least 125, at least 150, at least 175, at least 180, at least 190, or more contiguous amino acids of SEQ ID NO:76, 77, 79, 82, 88, 89, 90, 91, 96 or 97. The invention also includes nucleic acid molecules comprising a nucleotide sequence that encodes a polypeptide comprising at least 15, preferably at least 25, at least 50, at least 75, at least 100, at least 125, at least 150, at least 175, at least 180, at least 190, or more contiguous amino acids of SEQ ID NO:76, 77, 79, 82, 88, 89, 90, 91, 96 or 97, wherein the polypeptide encoded by the nucleotide sequence also exhibits at least one structural and/or functional feature of a CD2001 polypeptide of the invention. In preferred embodiments, the polypeptides represent N-terminal and/or C-terminal truncations of the amino acid sequence of SEQ ID NO:76, or the amino acid sequence of the mature CD2001 polypeptide.

In one embodiment, a polypeptide of the invention represents an antigenic polypeptide comprising at least 15 (preferably at least 25, at least 50, at least 75, at least 100, at least 125, at least 150, or at least 175) contiguous amino acids of the amino acid sequence of SEQ ID NO:76, 77, 78, 79, 80, 81, 82, 88, 89, 90, 91, 96 or 97, wherein antibodies generated against the polypeptide bind to a native CD2001. In another embodiment, a polypeptide of the invention represents an antigenic polypeptide comprising at least 15 (preferably at least 25, at least 50, at least 75, at least 100, at least 125, at least 150, or at least 175) contiguous amino acids of the amino acid sequence of SEQ ID NO:76, 77, 79, 82, 88, 89, 90, 91, 96 or 97, and wherein the antibodies generated against the polypeptide bind to a native CD2001.

The invention also includes polypeptides which are naturally occurring variants, *e.g.*, naturally occupying allelic variants, of a polypeptide that includes the amino acid sequence of SEQ ID NO:76, 77, 78, 79, 80, 81, 82, 88, 89, 90, 91, 96 or 97, wherein the polypeptide is encoded by a nucleic acid molecule which hybridizes to a nucleic acid molecule having the sequence of SEQ ID NO:74, 75, 83, 84, 85, 86, 87, 92, 93, 94 or 95, or a complement thereof, under stringent conditions.

The polypeptides of the present invention can be operably linked to a heterologous amino acid sequence to form fusion proteins. In one embodiment, the heterologous amino acid sequence is fused to the N-terminus of the polypeptide of the invention. In another embodiment, the heterologous amino acid sequence is fused to the C-terminus of the polypeptide of the invention.

In addition, the polypeptides of the invention can be incorporated into pharmaceutical compositions, which optionally include pharmaceutically acceptable carriers.

Preferred proteins and polypeptides of the invention possess at least one biological activity possessed by the corresponding naturally-occurring human polypeptide. A biological activity of a polypeptide of the invention refers, first, to an activity exerted by a protein or polypeptide of the invention on a responsive cell, protein, or nucleic acid as determined *in vivo* or *in vitro,* according to standard techniques. Such activities can be a direct activity, such as an association with, or an enzymatic activity on, a protein or modulation of gene expression via association with a nucleic acid regulatory sequence, or an indirect activity, such as a cellular signaling activity, mediated by interaction of the protein with a second protein. Such biological activities can also be considered to be functional features of the polypeptides of the invention. A biological activity of a polypeptide of the invention also refers to an antigenic or immunogenic activity of a polypeptide of the invention, *e.g.,* the ability of the polypeptide to act as an antigen for production of antibodies, and the ability of the polypeptide to act as an immunogen for mounting an immune response directed against the polypeptide. Such a biological activity is one, non-limiting example of a structural feature of the polypeptides of the invention.

For CD2000, biological activities include, *e.g.,* (1) the ability to modulate (*e.g.,* stabilize, increase or promote, suppress, decrease, inhibit, or disrupt) and/or track (*e.g.,* identify, follow, monitor, or measure) protein-protein interactions (*e.g*., homophilic and/or heterophilic), and protein-ligand interactions, *e.g.,* in receptor-ligand recognition; (2) the ability to modulate and/or track intracellular signaling cascades (*e.g.*, p59fyn phosphorylation, ZAP-70 phosphorylation, Syk phosphorylation, p561yn phosphorylation, p561ck phosphorylation, intracellular Ca²⁺, and NF-κB activation) or intercellular signaling cascades *(e.g.,* immune system signaling); (3) the ability to modulate and/or track hematopoietic processes, *e.g.,* the ability to modulate T cell activation; (4) the ability to modulate and/or track the immunoregulatory functions, such as host immune response, *e.g.,* by modulating TH1 or TH2 development, activity, function and/or proliferation; (5) the ability to modulate and/or track the development, differentiation, maturation, morphology, migration or chemotaxis, proliferation and/or activity *(e.g.,* the production of cytokines such as IFN-γ, IL-2, IL-3, IL-4, IL-6, IL-10, IL-12, and IL-15) of immune cells *(e.g.,* B cells, T cells, eosinophils, granulocytes, dendritic cells and macrophages); (6) the ability to modulate and/or track ligand-receptor interactions; (7) the ability to modulate and/or track lymphocyte selection (such as modulation of B-cell receptor or T-cell receptor stimulation in developing lymphocytes, *e.g.,* through modulation of ligand *(e.g.,* antigen) interaction with immunoglobulin domains of the receptors); (8) the ability to modulate and/or track autoimmunity (*e.g.,* as associated with multiple sclerosis, psoriasis, arthritis, and lupus); (9) the ability to modulate and/or track inflammatory functions *e.g.,* by modulating leukocyte adhesion to extracellular matrix and/or endothelial cells; (10) the ability to modulate and/or track homeostasis; (11) the ability to modulate T cell receptor-peptide major histocompatibility (MHC) binding and/or signaling; (12) the ability to modulate *(e.g.,* inhibit or stimulate) and/or track the expression of molecules, *e.g.,* the expression of T cell activation markers such as ICOS and CD28 and the expression of cytokines such as IFN-α, IFN-β, IFN-γ, IL-2, IL-4, IL-5, IL-6, IL-10, IL-12, IL-15, and IL-18; (13) the ability to alter, *e.g*., increase, expression in response to stimuli and pathophysiological stimuli relevant to inflammatory processes and disorders; (14) the ability to modulate and/or track lymphoproliferative disorders *(e.g.,* X-linked lymphoproliferative disease); (15) the ability to bind to, *e.g.,* SAP, EAT-2, and/or SHP-2; and (16) the ability to modulate cell adhesion.

For CD2001, biological activities include, *e.g*., (1) the ability to modulate *(e.g.,* stabilize, increase or promote, suppress, decrease, inhibit, or disrupt) and/or track *(e.g.,* identify, follow, monitor, or measure) protein-protein interactions *(e.g.,* homophilic and/or heterophilic), and protein-ligand interactions, *e.g.,* in receptor-ligand recognition; (2) the ability to modulate and/or track intracellular signaling cascades *(e.g.,* p59fyn phosphorylation, ZAP-70 phosphorylation, Syk phosphorylation, p56lyn phosphorylation, p561ck phosphorylation, intracellular Ca²⁺, and NF-κB activation) or intercellular signaling cascades *(e.g.,* immune system signaling); (3) the ability to modulate and/or track hematopoietic processes, *e.g.,* the ability to modulate T cell activation; (4) the ability to modulate and/or track the immunoregulatory functions, such as host immune response, *e.g.*, by modulating TH1 or TH2 development, activity, function and/or proliferation; (5) the ability to modulate and/or track the development, differentiation, maturation, morphology, migration or chemotaxis, proliferation and/or activity (*e.g*., the production of cytokines such as IFN-γ, IL-2, IL-3, IL-4, IL-6, IL-10, IL-12, and IL-15) of immune cells *(e.g.,* B cells, T cells, eosinophils, granulocytes, dendritic cells and macrophages); (6) the ability to modulate and/or track ligand-receptor interactions; (7) the ability to modulate and/or track lymphocyte selection (such as modulation of B-cell receptor or T-cell receptor stimulation in developing lymphocytes, *e.g.,* through modulation of ligand *(e.g.,* antigen) interaction with immunoglobulin domains of the receptors); (8) the ability to modulate and/or track autoimmunity *(e.g.,* as associated with multiple sclerosis, psoriasis, arthritis, and lupus); (9) the ability to modulate and/or track inflammatory functions *e.g.,* by modulating leukocyte adhesion to extracellular matrix and/or endothelial cells; (10) the ability to modulate and/or track homeostasis; (11) the ability to modulate T cell receptor-peptide major histocompatibility (MHC) binding and/or signaling; (12) the ability to modulate (*e.g*., inhibit or stimulate) and/or track the expression of molecules, *e.g*., the expression of T cell activation markers such as ICOS and CD28 and the expression of cytokines such as IFN-α, IFN-β, IFN-γ, IL-2, IL-4, IL-5, IL-6, IL-10, IL-12, IL-15, and IL-18; (13) the ability to alter, *e.g*., increase, expression in response to stimuli and pathophysiological stimuli relevant to inflammatory processes and disorders; (14) the ability to modulate and/or track lymphoproliferative disorders; and (15) the ability to bind to, *e.g.*, SHP-2; and (16) the ability to modulate cell adhesion.

The invention further features antibodies, such as monoclonal or polyclonal antibodies, that specifically bind a polypeptide of the invention. Thus, in one aspect, the invention provides antibodies or fragments thereof, preferably substantially purified antibodies or fragments thereof, including human, humanized, chimeric and non-human antibodies or fragments thereof, which antibodies or fragments immunospecifically bind to a polypeptide comprising an amino acid sequence of SEQ ID NO:3, 4, 5, 6, 7, 8, 9, 10, 12, 13, 14, 15, 16, 17, 18, 19, 21, 22, 39, 40, 41, or 42, or the amino acid sequence encoded by a EpCD2000 cDNA insert deposited with the ATCC® as patent deposit Number PTA-2267.

In another aspect, the invention provides antibodies or fragments thereof, preferably substantially purified antibodies or fragments thereof, including, *e.g.*, human, non-human, chimeric and humanized antibodies, which antibodies or fragments thereof immunospecifically bind to a polypeptide comprising at least 95% identical to the amino acid sequence of SEQ ID NO:3, 4, 5, 6, 7, 8, 9, 10, 12, 13, 14, 15, 16, 17, 18, 19, 21, 22, 39, 40, 41, or 42, or the amino acid sequence encoded by a EpCD2000 cDNA insert deposited with the ATCC® as patent deposit Number PTA-2267, wherein the percent identity is determined using a sequence alignment program such as ALIGN program of the GCG software package with a PAM120 weight residue table, a gap length penalty of 12, and a gap penalty of 4.

In another aspect, the invention provides antibodies or fragments thereof, preferably substantially purified antibodies or fragments thereof, including, e.g., human, non-human, chimeric and humanized antibodies, which antibodies or fragments thereof immunospecifically bind to a polypeptide encoded by a nucleic acid molecule which hybridizes to the nucleic acid molecule of SEQ ID NO:1, 2, 31, 32, 33, 34, 35, 36, 37, 38, 43, 45, 46, 47, 48, 49, 50, 51, 52, 53, 54, 55, 56, 57, 58, 66 or 67, or the nucleotide sequence of a EpCD2000 cDNA insert deposited with the ATCC® as patent deposit Number PTA-2267 under conditions of hybridization of 6X SSC at 45°C and washing in 0.2 X SSC, 0.1% SDS at 50°C, 55°C, 60°C or 65°C, or 6X SSC at 45°C and washing in 0.1 X SSC, 0.2% SDS at 68°C.

In another aspect, the invention provides antibodies or fragments thereof, preferably substantially purified antibodies or fragments thereof, including human, humanized, chimeric and non-human antibodies or fragments thereof, which antibodies or fragments immunospecifically bind to a polypeptide comprising an amino acid sequence of SEQ ID NO:76, 77, 78, 79, 80, 81, 82, 88, 89, 90, 91, 96 or 97.

In another aspect, the invention provides antibodies or fragments thereof, preferably substantially purified antibodies or fragments thereof, including, *e.g.*, human, non-human, chimeric and humanized antibodies, which antibodies or fragments thereof immunospecifically bind to a polypeptide comprising at least 95% identical to the amino acid sequence of SEQ ID NO:76, 77, 78, 79, 80, 81, 82, 88, 89, 90, 91, 96 or 97, wherein the percent identity is determined using a sequence alignment program such as ALIGN program of the GCG software package with a PAM120 weight residue table, a gap length penalty of 12, and a gap penalty of 4.

In another aspect, the invention provides antibodies or fragments thereof, preferably substantially purified antibodies or fragments thereof, including, e.g., human, non-human, chimeric and humanized antibodies, which antibodies or fragments thereof immunospecifically bind to a polypeptide encoded by a nucleic acid molecule which hybridizes to the nucleic acid molecule of SEQ ID NO:74, 75, 83, 84, 85, 86, 87, 92, 93, 94 or 95 under conditions of hybridization of 6X SSC at 45°C and washing in 0.2 X SSC, 0.1% SDS at 50°C, 55°C, 60°C or 65°C, or 6X SSC at 45°C and washing in 0.1 X SSC, 0.2% SDS at 68°C.

Any of the antibodies of the invention or fragments thereof can be conjugated to a therapeutic moiety or to a detectable substance. Non-limiting examples of detectable substances that can be conjugated to the antibodies of the invention are an enzyme, a prosthetic group, a fluorescent material, a luminescent material, a bioluminescent material, and a radioactive material.

The invention also provides a kit containing an antibody of the invention or fragment thereof conjugated to a detectable substance, and instructions for use. Still another aspect of the invention is a pharmaceutical composition comprising an antibody of the invention or a fragment thereof and a pharmaceutically acceptable carrier. In preferred embodiments, the pharmaceutical composition contains an antibody of the invention or fragment thereof, a therapeutic moiety, and a pharmaceutically acceptable carrier.

Still another aspect of the invention is a method of making an antibody that specifically recognizes CD2000 or CD2001, the method comprising immunizing a mammal with a polypeptide. In one embodiment, the polypeptide used as an immunogen comprises an amino acid sequence selected from the group consisting of: the amino acid sequence of SEQ ID NO:3, 4, 5, 6, 7, 8, 9, 10, 12, 13, 14, 15, 16, 17, 18, 19, 21, 22, 39, 40, 41, or 42, or the amino acid sequence encoded by a EpCD2000 cDNA insert deposited with the ATCC® as patent deposit Number PTA-2267; a fragment of at least 15 (preferably at least 25, at least 50, at least 75, at least 100, at least 125, at least 150, or at least 175) contiguous amino acid residues of the amino acid sequence of SEQ ID NO:3, 4, 5, 6, 7, 8, 9, 10, 12, 13, 14, 15, 16, 17, 18, 19, 21, 22, 39, 40, 41, or 42; an amino acid sequence which is at least 50%, at least 55%, at least 60%, at least 65%, at least 70%, at least 75%, at least 80%, at least 85%, at least 90%, at least 95% or at least 98% identical to the amino acid sequence of SEQ ID NO:3, 4, 5, 6, 7, 8, 9, 10, 12, 13, 14, 15, 16, 17, 18, 19, 21, 22, 39, 40, 41, or 42, wherein the percent identity is determined using a sequence alignment program such as ALIGN program of the GCG software package with a PAM120 weight residue table, a gap length penalty of 12, and a gap penalty of 4; and an amino acid sequence which is encoded by a nucleic acid molecule which hybridizes to the nucleic acid molecule consisting of SEQ ID NO:1, 2, 31, 32, 33, 34, 35, 36, 37, 38, 43, 44, 45, 46, 47, 48, 49, 50, 51, 52, 53, 54, 55, 56, 57, 58, 66 or 67 under conditions of hybridization of 6X SSC at 45°C and washing in 0.2 X SSC, 0.1% SDS at 50°C, 55°C, 60°C or 65°C, or 6X SSC at 45°C and washing in 0.1 X SSC, 0.2% SDS at 68°C.

In another embodiment, the polypeptide used as an immunogen comprises an amino acid sequence selected from the group consisting of: the amino acid sequence of SEQ ID NO:76, 77, 78, 79, 80, 81, 82, 88, 89, 90, 91, 96 or 97; a fragment of at least 15 (preferably at least 25, at least 50, at least 75, at least 100, at least 125, at least 150, or at least 175) contiguous amino acid residues of the amino acid sequence of SEQ ID NO:76, 77, 78, 79, 80, 81, 82, 88, 89, 90, 91, 96 or 97; a fragment of at least 15 (preferably at least 25, at least 50, at least 75, at least 100, at least 125, at least 150, or at least 175) contiguous amino acid residues of the amino acid sequence of SEQ ID NO:76, 77, 79, 82, 88, 89, 90, 91, 96 or 97; an amino acid sequence which is at least 70%, at least 75%, at least 80%, at least 85%, at least 90%, at least 95% or at least 98% identical to the amino acid sequence of SEQ ID NO:76, 77, 78, 79, 80, 81, 82, 88, 89, 90, 91, 96 or 97, wherein the percent identity is determined using a sequence alignment program such as ALIGN program of the GCG software package with a PAM120 weight residue table, a gap length penalty of 12, and a gap penalty of 4; and an amino acid sequence which is encoded by a nucleic acid molecule which hybridizes to the nucleic acid molecule consisting of SEQ ID NO:74, 75, 83, 84, 85, 86, 87, 92, 93, 94 or 95 under conditions of hybridization of 6X SSC at 45°C and washing in 0.2 X SSC, 0.1% SDS at 50°C, 55°C, 60°C or 65°C, or 6X SSC at 45°C and washing in 0.1 X SSC, 0.2% SDS at 68°C.

After immunization, a sample is collected from the mammal that contains an antibody that specifically recognizes CD2000 or CD2001. Preferably, the polypeptide is recombinantly produced using a non-human host cell. Optionally, the antibodies can be further purified from the sample using techniques well known to those of skill in the art. The method can further comprise producing a monoclonal antibody-producing cell from the cells of the mammal. Optionally, antibodies are collected from the antibody-producing cell.

In another aspect, the present invention provides methods for detecting the presence, activity or expression of a polypeptide of the invention in a biological sample by contacting the biological sample with an agent capable of being detected as an indicator of the presence, activity or expression of a polypeptide of the invention in the biological sample.

In another aspect, the invention provides methods for modulating activity of a polypeptide of the invention comprising contacting a cell or a polypeptide of the invention with an agent that modulates (*e.g.*, inhibits or stimulates) the activity or expression of a polypeptide of the invention such that activity or expression in the cell is modulated. In one embodiment, the agent is an antibody that specifically binds to a polypeptide of the invention. In another embodiment, the agent modulates expression of a polypeptide of the invention by modulating transcription, splicing, or translation of an mRNA encoding a polypeptide of the invention. In yet another embodiment, the agent is a nucleic acid molecule having a nucleotide sequence that is antisense to the coding strand of an mRNA encoding a polypeptide of the invention.

The present invention also provides methods to treat a subject having a disorder characterized by aberrant activity of a polypeptide of the invention or aberrant expression of a nucleic acid of the invention by administering an agent which is a modulator of the activity of a polypeptide of the invention or a modulator of the expression of a nucleic acid of the invention to the subject. In one embodiment, the modulator is a protein of the invention. In another embodiment, the modulator is a nucleic acid of the invention. In other embodiments, the modulator is a peptide, peptidomimetic, or other small molecule.

The present invention also provides diagnostic assays for identifying the presence or absence of a genetic lesion or mutation characterized by at least one of: (i) aberrant modification or mutation of a gene encoding a polypeptide of the invention; (ii) misregulation of a gene encoding a polypeptide of the invention; and (iii) aberrant post-translational modification of the invention wherein a wild-type form of the gene encodes a protein having the activity of the polypeptide of the invention.

In another aspect, the invention provides a method for identifying a compound that binds to a polypeptide of the invention. In general, such methods comprise contacting a test compound with a polypeptide of the invention for a time sufficient for the test compound to bind the polypeptide, removing unbound test compound, and assaying for the presence of bound test compound. Preferentially, the test compound is one that selectively binds a polypeptide of the invention.

As used herein, the term " selectively binds" refers to a compound (*e.g.*, an antibody or small organic molecule) that binds to a polypeptide of the invention preferentially relative to heterologous polypeptides (i.e., polypeptides that are different from the polypeptides of the invention). A compound selectively binds to a polypeptide preferentially relative to a heterologous polypeptide if it has at least a 10%, preferably at least a 25%, at least a 50%, at least a 75%, at least a 90%, at least a 95%, or at least a 100% higher affinity and/or avidity for a CD2000 polypeptide of the invention than a heterologous polypeptide as determined by an assay well known in the art or described herein.

The term "immunospecifically" and analogous terms as used herein refer to peptides, polypeptides, and antibodies or fragments thereof that specifically bind to an antigen or a fragment and do not specifically bind to other antigens. Antibodies or fragments that immunospecifically bind to a polypeptide of the invention can be identified, for example, by immunoassays, BIAcore, or other techniques known to those of skill in the art. An antibody or fragment thereof binds specifically to a polypeptide when it binds to a polypeptide of the invention with higher affinity than to any cross-reactive antigen as determined using experimental techniques, such as radioimmunoassays (RIA) and enzyme-linked immunosorbent assays (ELISAs). See, e.g., Paul, ed., 1989,Fundamental Immunology Second Edition, Raven Press, New York at pages 332-336 for a discussion regarding antibody specificity. Preferably, antibodies or fragments that immunospecifically bind to an antigen do not cross-react with other antigens.

In yet another aspect, the invention provides a method for identifying a compound that modulates the activity of a polypeptide of the invention. In general, such methods comprise measuring a biological activity of a polypeptide of the invention in the presence of a test compound, comparing the activity of the polypeptide to the biological activity of the polypeptide in the absence of the test compound, and identifying a test compound that alters the biological activity or the polypeptide.

The invention also features methods for identifying a compound which modulates the expression of a polypeptide or nucleic acid of the invention by measuring the expression of the polypeptide or nucleic acid in the presence of the compound and comparing the expression to that measured in the absence of the compound.

Other features and advantages of the invention will be apparent from the following detailed description and claims.

### Brief Description of the Drawings

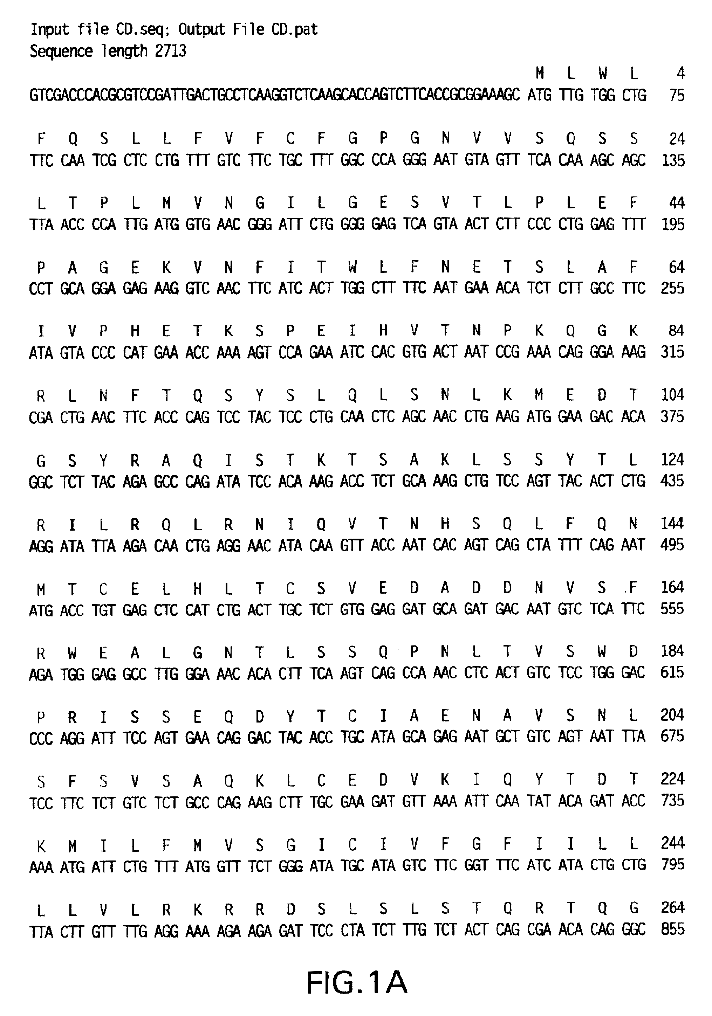

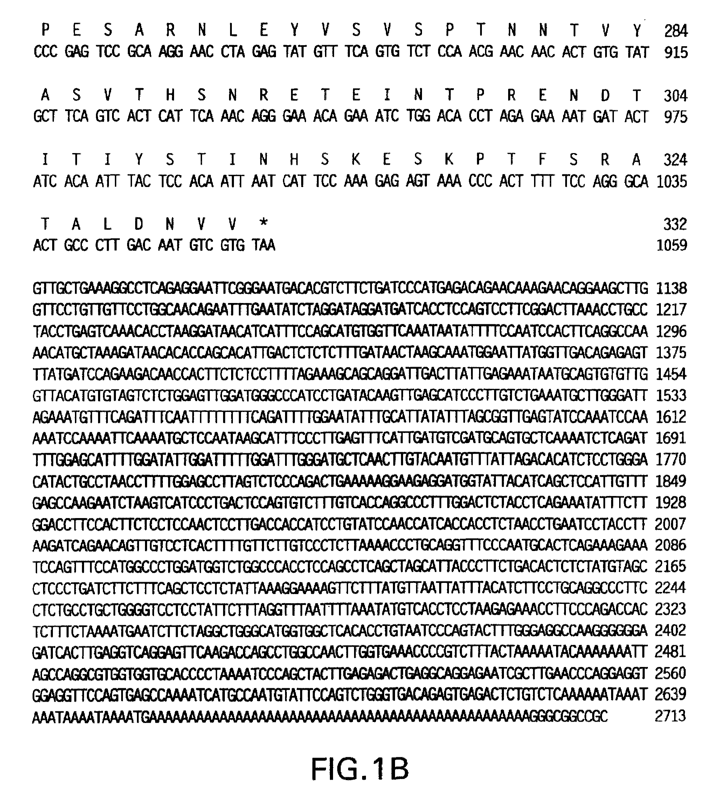

FIGURES 1A-1B depict the cDNA sequence of human CD2000 (SEQ ID NO:1) and the predicted amino acid sequence of human CD2000 (SEQ ID NO:3). The open reading frame of SEQ ID NO:1 extends from nucleotide 64 to nucleotide 1056 of SEQ ID NO:1 (SEQ ID NO:2).

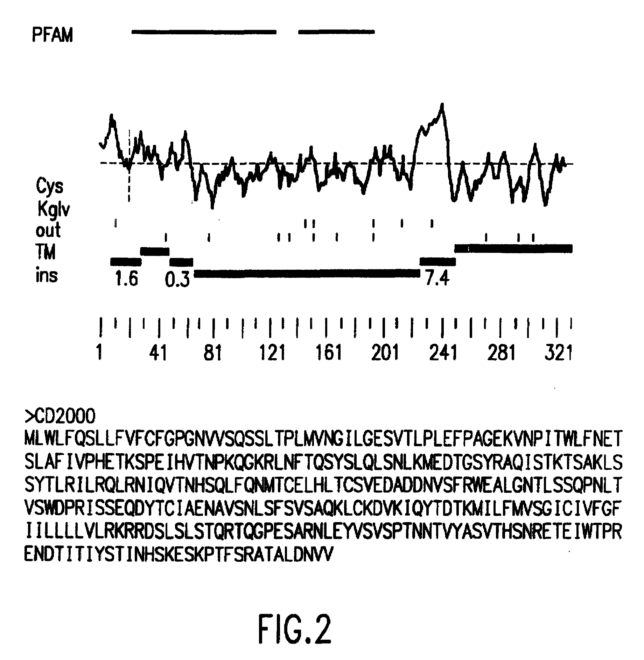

FIGURE 2 depicts a hydropathy plot of human CD2000. Relatively hydrophobic regions of the protein are above the dashed horizontal line, and relatively hydrophilic regions of the protein are below the dashed horizontal line. The cysteine residues (cys) are indicated by short vertical lines just below the hydropathy trace. The dashed vertical line separates the signal sequence (amino acids 1 to 22 of SEQ ID NO:3; SEQ ID NO:4) on the left from the mature protein (amino acids 23 to 331 of SEQ ID NO:3; SEQ ID NO:5) on the right.

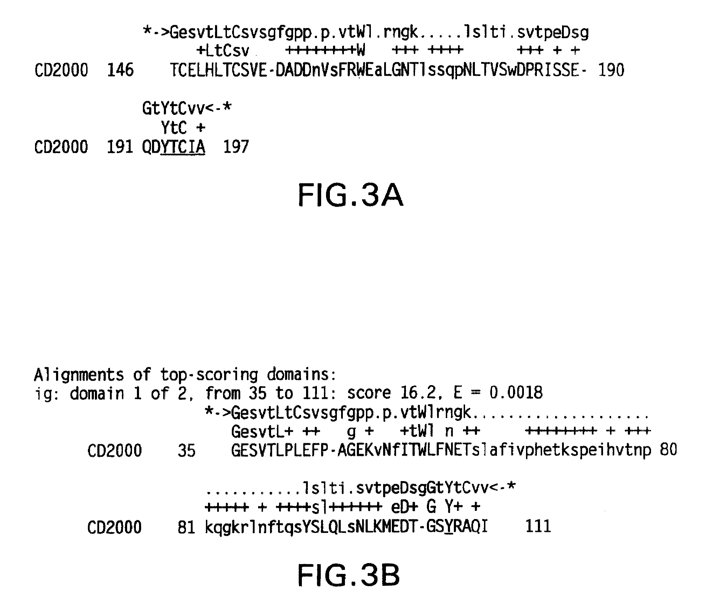

FIGURES 3A-3B depict alignments of a typical immunoglobulin domain and regions of human CD2000. Figure 3A depicts an alignment of the amino acid sequence of a typical immunoglobulin domain (SEQ ID NO:20; GenBank Accession Number PF00047) and amino acid residues 146 to 197 of human CD2000 (SEQ ID NO:18). Figure 3B depicts an alignment of the amino acid sequence of a typical immunoglobulin domain (SEQ ID NO:20; GenBank Accession Number PF00047) and amino acid residues 35 to 111 of human CD2000 (SEQ ID NO:19). These alignments were performed using the ALIGN alignment program with a PAM120 scoring matrix, a gap length penalty of 12, and a gap penalty of 4.

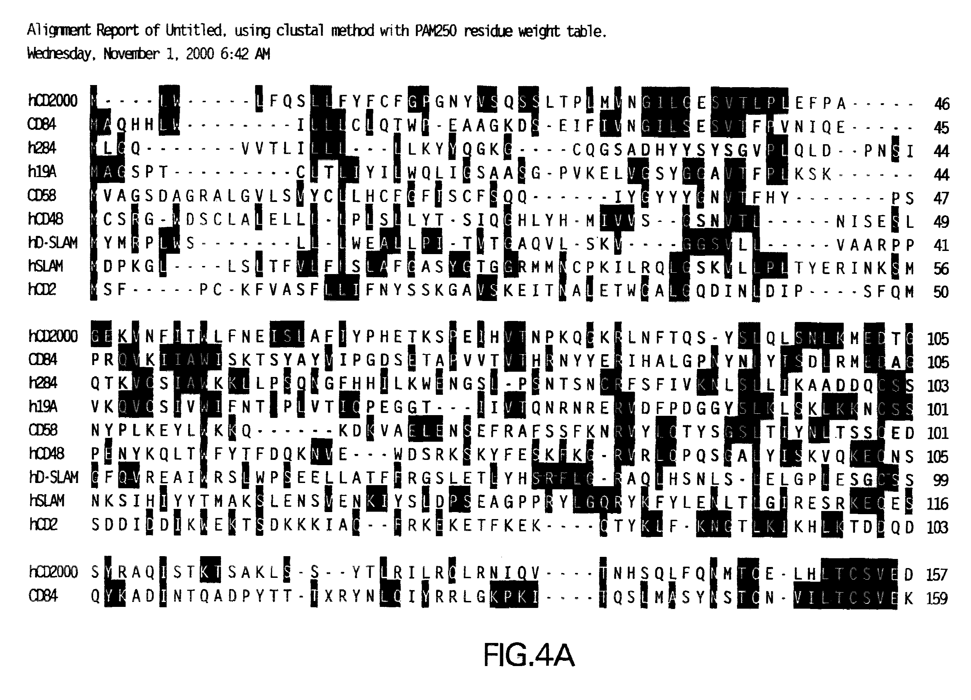

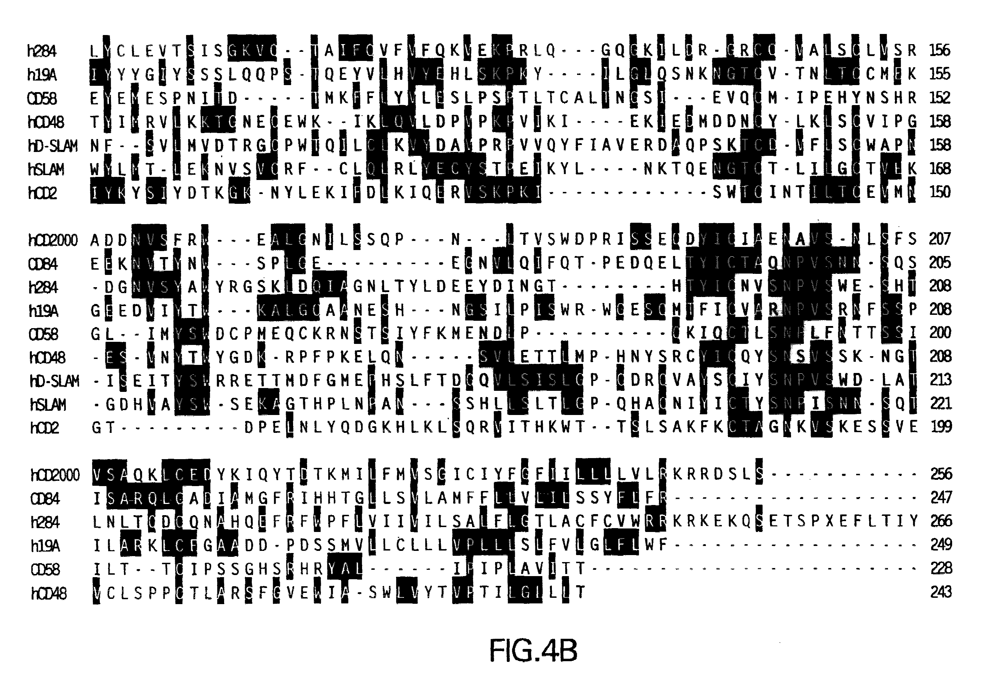

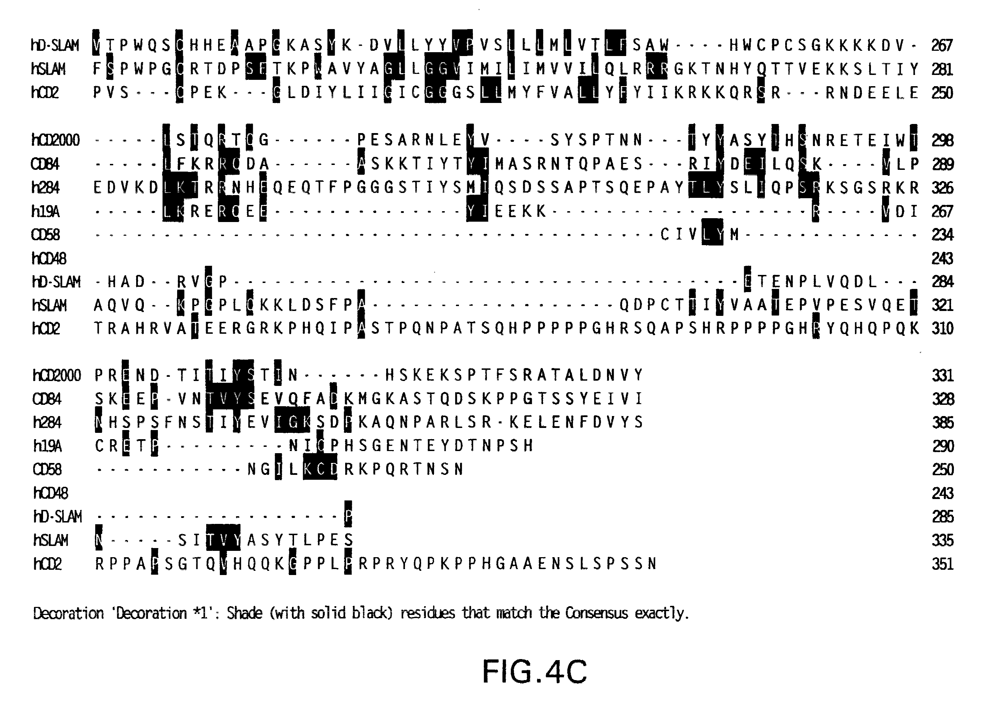

FIGURES 4A-4B depict alignments of the amino acid sequence for human CD2000 and the amino acid sequences for human CD84 (SEQ ID NO:30; GenBank Accession No. AAB84364), human 2B4 (SEQ ID NO:59; GenBank Accession No. AAD32538), human 19A (SEQ ID NO:60; GenBank Accession No. CAB81950), human CD58 (SEQ ID NO:61; GenBank Accession No. NP_001770), human CD48 (SEQ ID NO:62; GenBank Accession No. CAC00578), human D-SLAM (T195; SEQ ID NO:63; GenBank Accession No. NP_064510), human SLAM (SEQ ID NO:64; GenBank Accession No. NP_003028), and human CD2 (SEQ ID NO:65; GenBank Accession No. AA51946). The amino acid residues shaded with solid black comprise the consensus sequence.

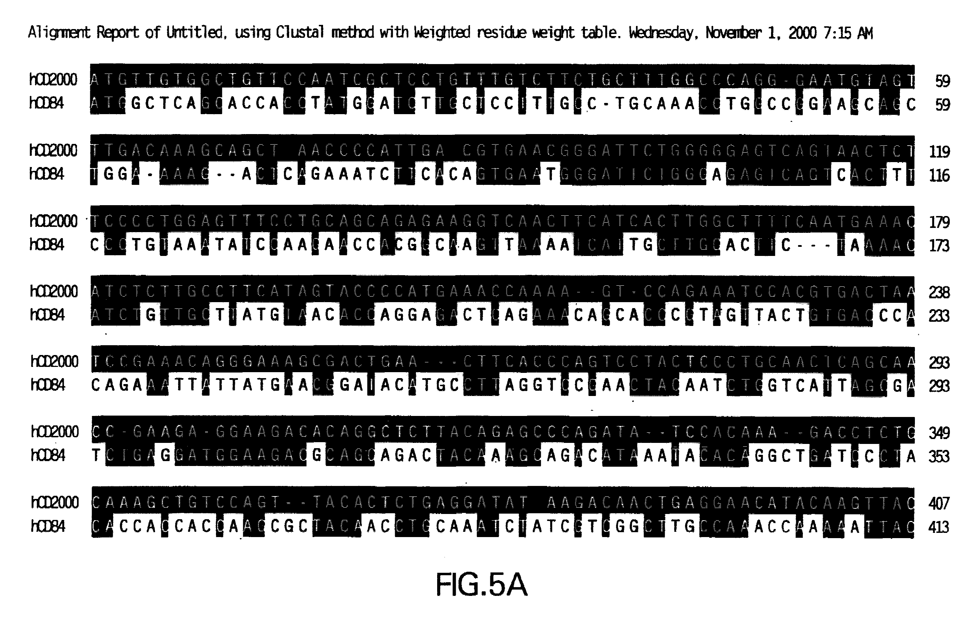

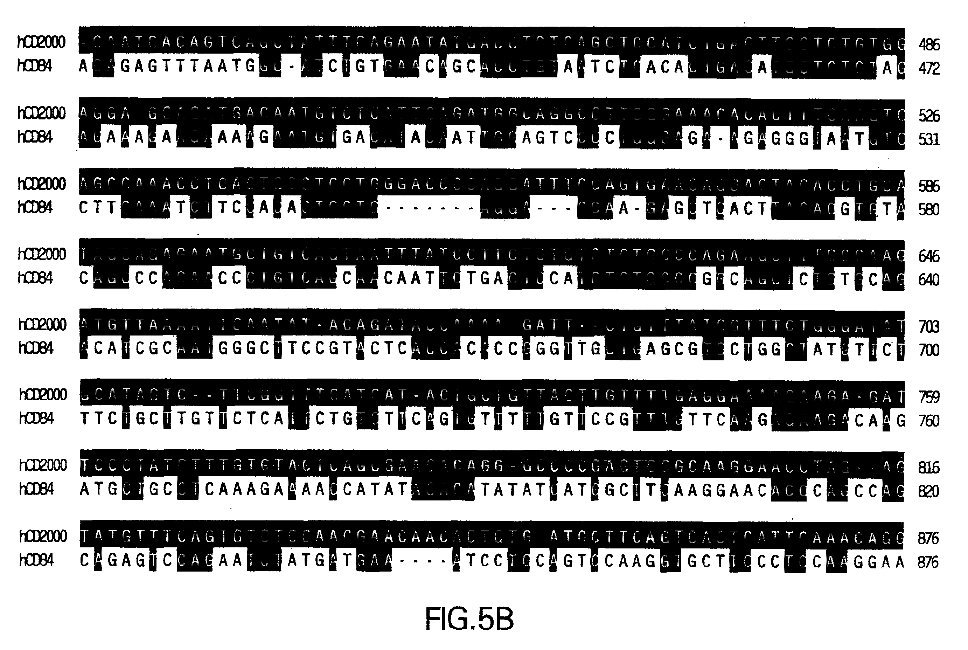

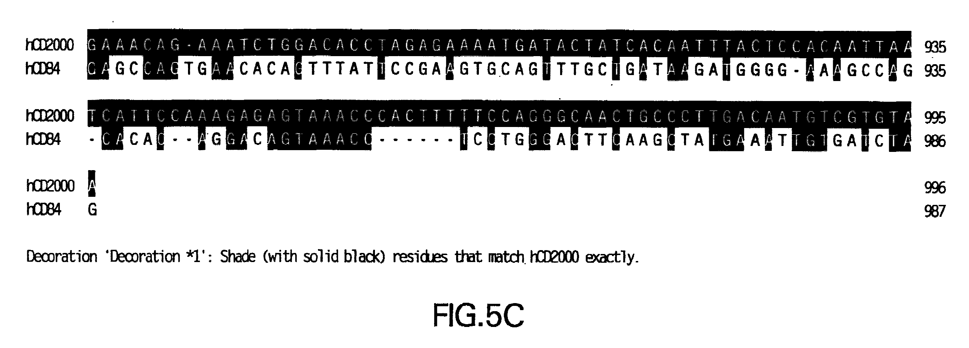

FIGURE 5A-5B depict an alignment of the nucleotide sequence of the open reading frame for human CD84 (SEQ ID NO:29; GenBank Accession No. U82988) and the nucleotide sequence of the open reading frame for human CD2000 (SEQ ID NO:2). The nucleotide sequences of coding regions of human CD84 and human CD2000 are 34.1% identical. This alignment was performed using the ALIGN alignment program with a PAM120 scoring matrix, a gap length penalty of 12, and a gap penalty of 4.

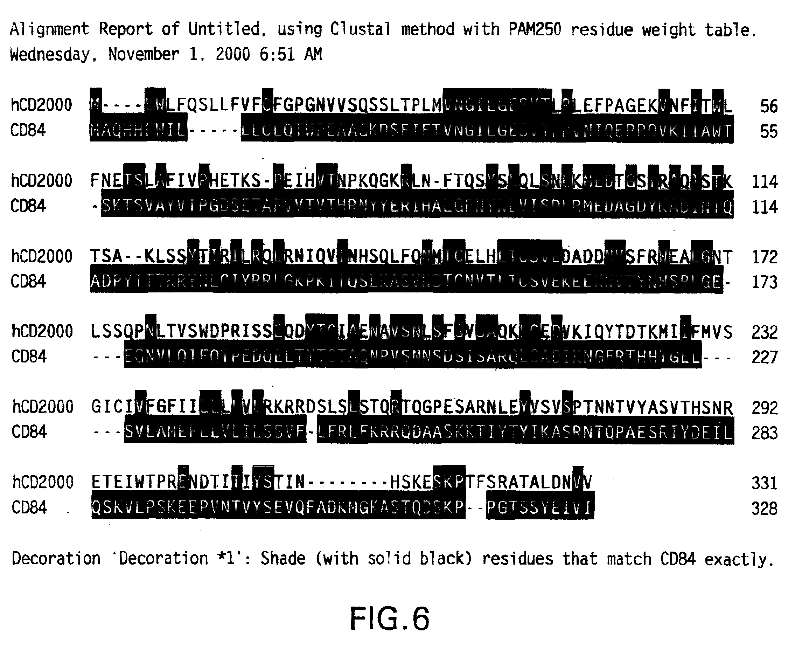

FIGURES 6 depicts an alignment of the amino acid sequence of the alpha subunit of human CD84 (SEQ ID NO:30; GenBank Accession No. AAB84364) and the amino acid sequence of human CD2000 (SEQ ID NO:3). The amino acid sequences of human CD84 and human CD2000 are 22% identical. This alignment was performed using the ALIGN alignment program with a PAM120 scoring matrix, a gap length penalty of 12, and a gap penalty of 4.

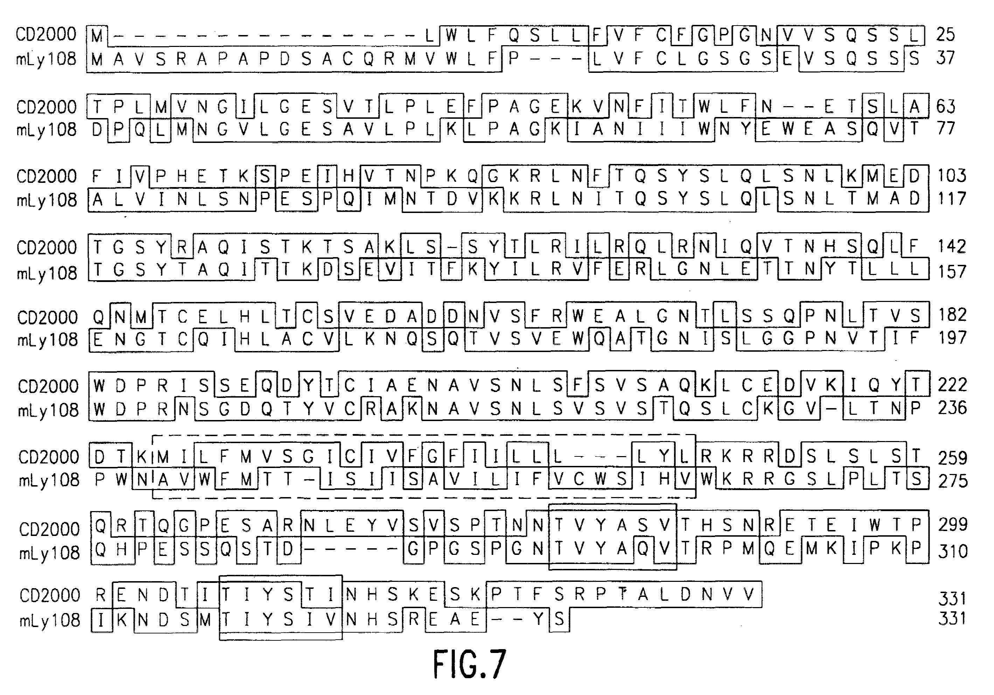

FIGURE 7 depicts a sequence alignment of the amino acid sequence of human CD2000 (SEQ ID NO:3) and the amino acid sequence of murine Ly108 (SEQ ID NO:11). The putative transmembrane domains are outlined with a dotted line and predicted immunoglobulin domains underlined. SAP binding motifs in the cytoplasmic tail are indicated by bold.

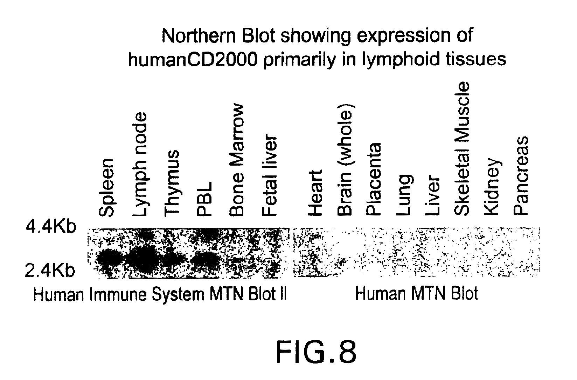

FIGURE 8: Northern blot analysis of human tissues. A 3.1 kb transcript was observed in the spleen, lymph node, thymus, peripheral blood leucocytes (PBL), bone marrow, fetal liver, liver, and lung. No signal was observed in heart, brain (whole), skeletal muscle, kidney, or pancreas.

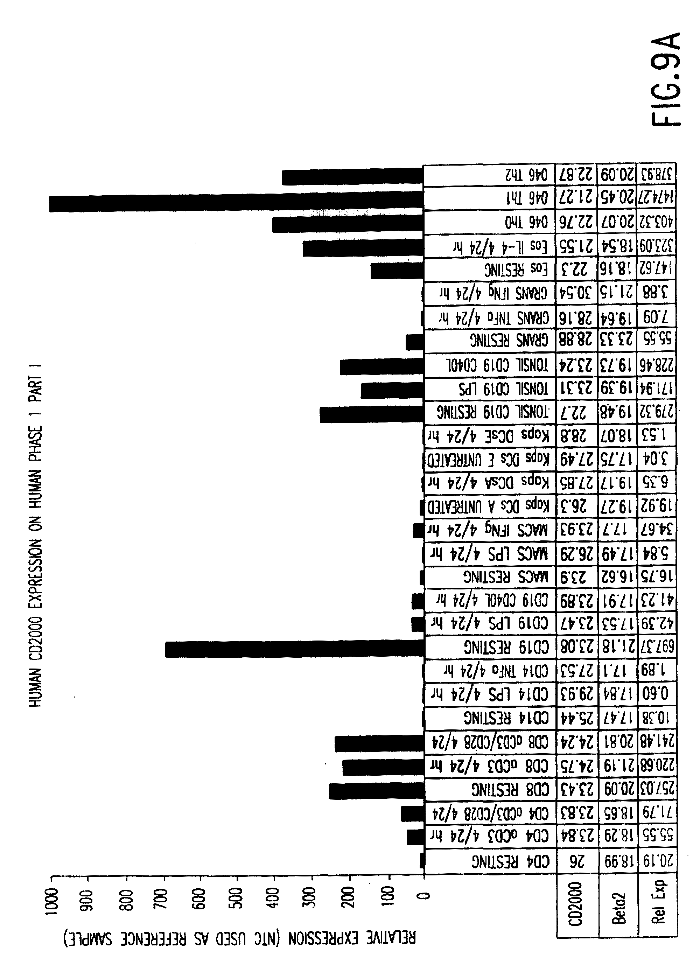

FIGURES 9A-9B: Human CD2000 expression by cells and tissues as measured by TaqMan® quantitative PCR.

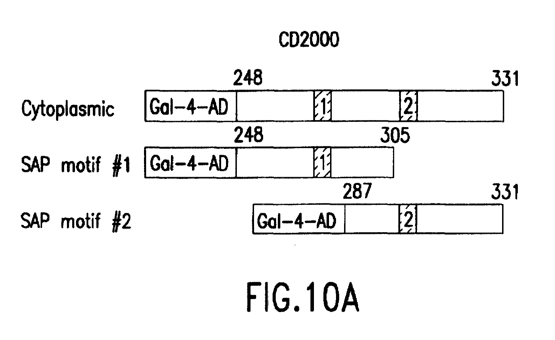

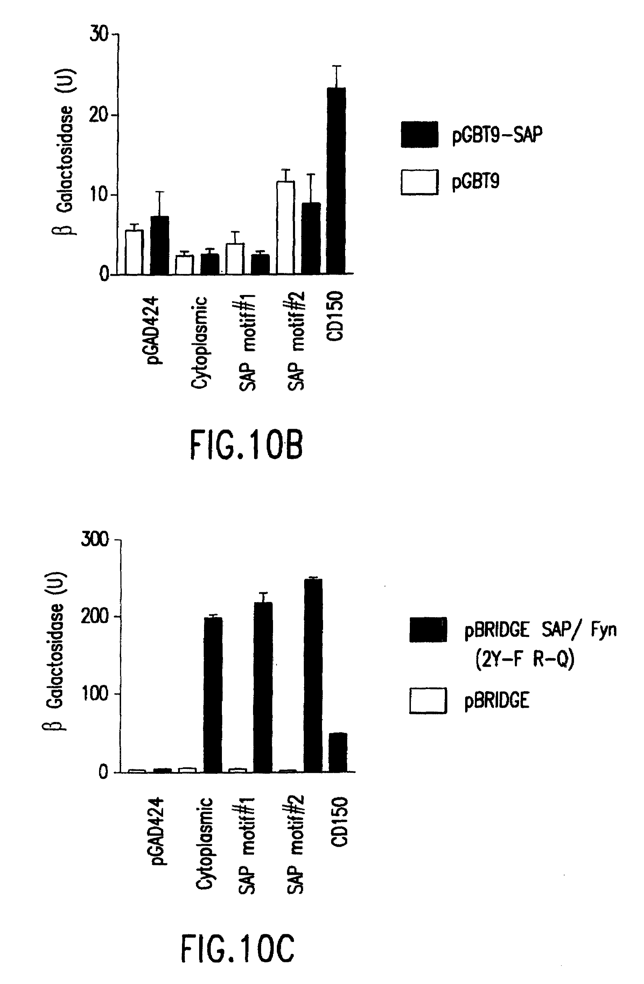

FIGURES 10A-10C: Analysis of SAP binding to CD2000 using yeast two hybrid systems. Figure 10A is a diagram depicts the Gal-4 activation domain-CD2000 fusion constructs used for two-hybrid analysis. The cytoplasmic construct encodes the entire cytoplasmic region of CD2000 (amino acid residues 248 to 331 of the human CD2000 polypeptide) fused to the Gal-4 activation domain. The SAP motif #1 construct encodes the cytoplasmic domain up to the second SAP binding motif (amino acid residues 248 to 305 of the human CD2000 polypeptide) fused to the Gal-4 activation domain. The SAP motif #2 construct encodes the cytoplasmic region C-terminal to the first SAP binding motif, including the second SAP binding motif, (amino acid residues 287 to 331 of the human CD2000 polypeptide) fused to the Gal-4 activation domain. Figure 10B: Yeast strain Y190 was co-transfected with a Gal-4 activation domain-CD2000 fusion construct (*i.e*., pGAD24-cytoplamsic, pGAD24-SAP motif #1, or pGAD24-SAP motif #2), a Gal-4 activation domain-CD150 fusion construct (pGAD24-CD150) or control construct (pGAD24) and a Gal-4 binding domain-SAP fusion construct (pGTB9-SAP) or control construct (pGBT9). β-galactosidase reporter activity was assayed using ONPG as a substrate, as described in materials and methods. Figure 10C: Yeast strain Y190 was co-transfected with a Gal-4 activation domain-CD2000 fusion construct (*i.e*., pGAD24-cytoplamsic, pGAD24-SAP motif #1, or pGAD24-SAP motif #2), a Gal-4 activation domain-CD150 fusion construct (pGAD24-CD150), or control construct (pGAD24), and a Gal-4 binding domain-SAP fusion and modified *c-fyn* construct (pBRIDGE-SAP/fyn^{2Y-F R-Q}) or control construct (pBRDIGE). β-galactosidase reporter activity was assayed using ONPG as a substrate, as described in materials and methods.

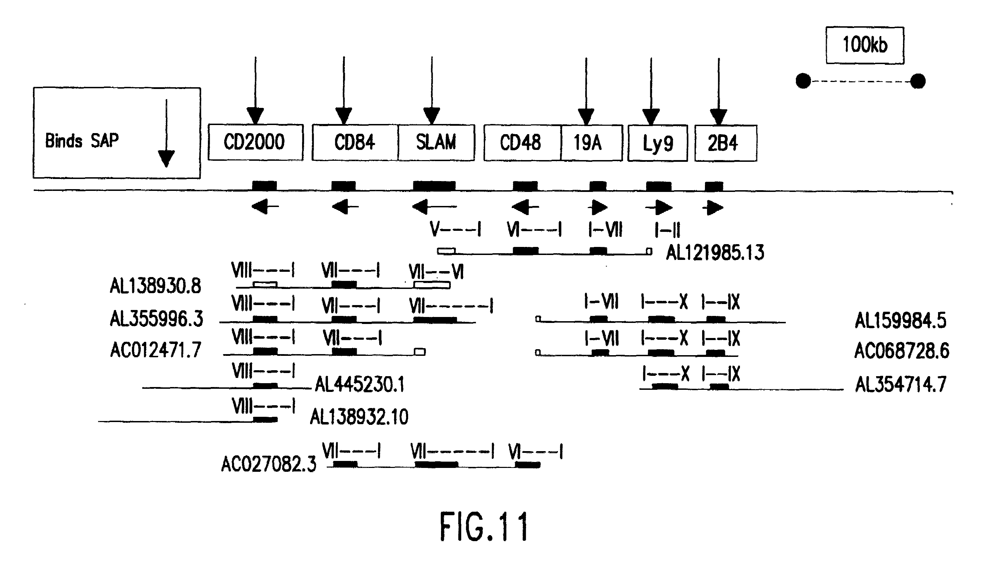

FIGURE 11 depicts the location of CD2000 in the SLAM-related gene cluster in chromosome 1q23. GenBank accession numbers of the genomic fragments are included in the figure. The filled boxes indicate complete genomic sections containing the entire gene with the number of exons and orientation as indicated. The empty boxes indicate partial gene fragments. Seven genes in the SLAM family cluster in approximately 300 kb of contiguous genomic region.

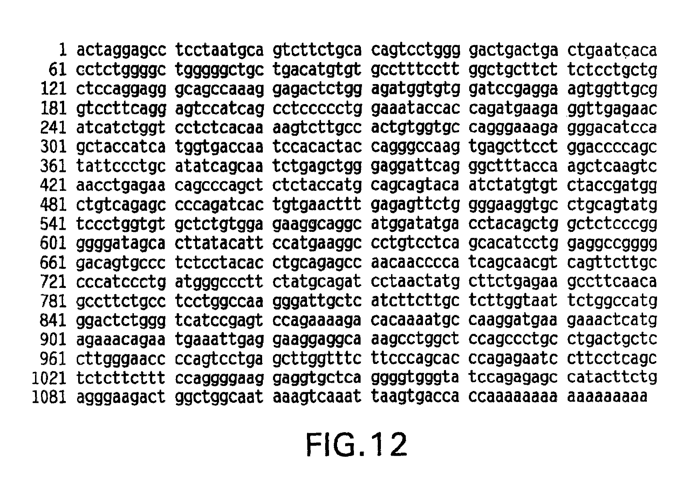

FIGURE 12 depicts the cDNA sequence of human CD2001 (SEQ ID NO:74). The open reading frame of SEQ ID NO:74 extends from nucleotide 85 to nucleotide 954 of SEQ ID NO:74 (SEQ ID NO:75).

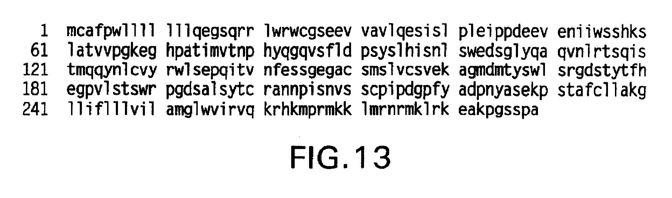

FIGURE 13 depicts the amino acid sequence of human CD2001 (SEQ ID NO:76).

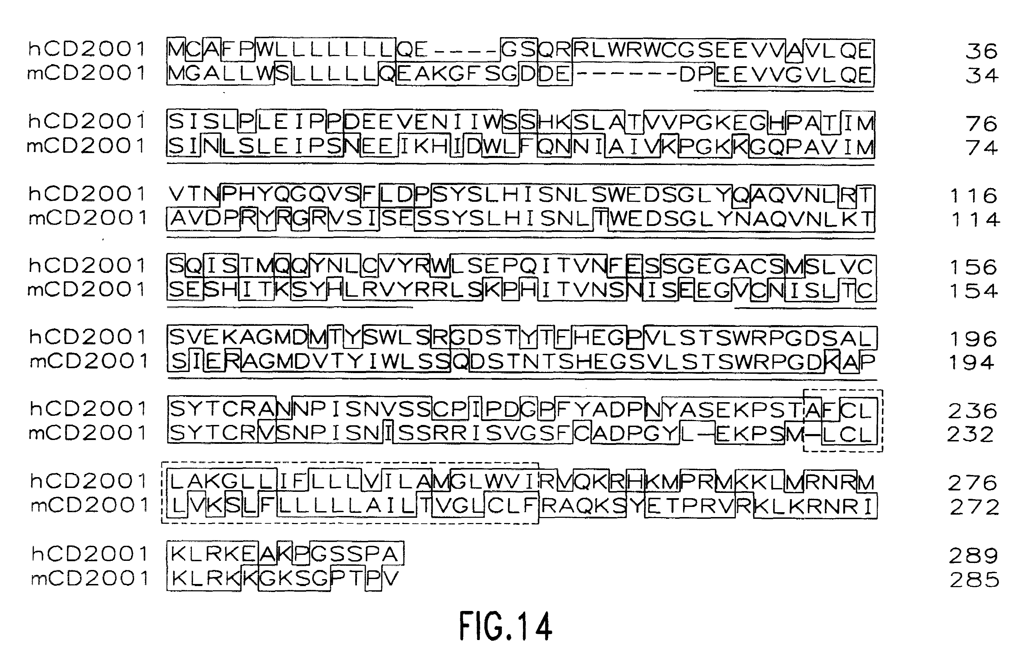

FIGURE 14 depicts an alignment of the amino acid sequence of human CD2001 (SEQ ID NO:76) and the amino acid sequence encoded by the mouse gene BAB26328 (GenBank Accession No. AK009505; SEQ ID NO:100). The putative transmembrane domains are outlined with a dotted line and the predicted Ig domains are underlined.

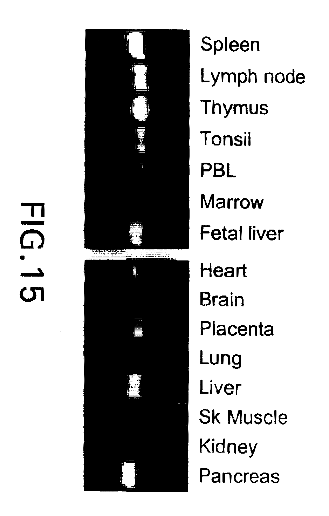

FIGURE 15 depicts the expression of CD2001 in multiple human tissues as determined by RT-PCR.

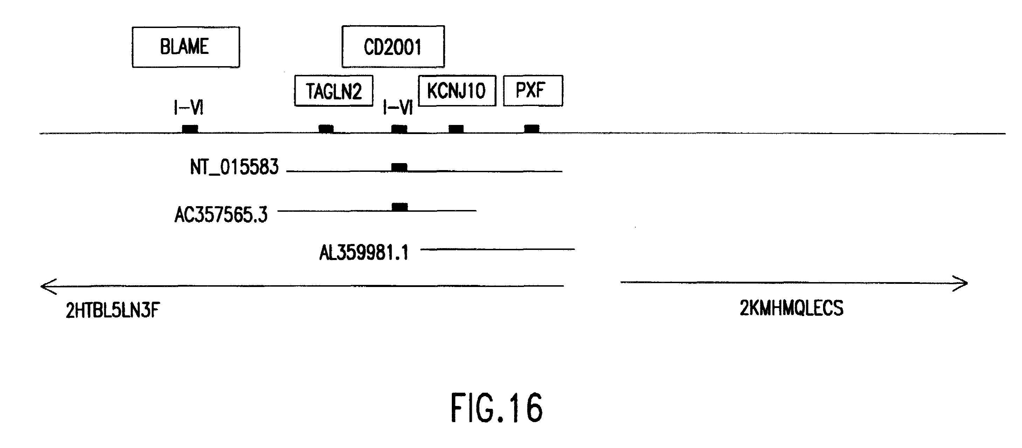

FIGURE 16 depicts the chromosomal location of CD2001. Accession numbers of genomic fragments are included. Filled boxes indicate complete genomic sections containing the entire gene with the number of exons and orientation as indicated.

### Detailed Description of the Invention

The CD2000 proteins and nucleic acid molecules comprise a family of molecules having certain conserved structural and functional features. As used herein, the term "family" is intended to mean two or more proteins or nucleic acid molecules having a common structural domain and having sufficient amino acid or nucleotide sequence identity as defined herein. Family members can be from either the same or different species. For example, a family can comprise two or more proteins of human origin, or can comprise one or more proteins of human origin and one or more of non-human origin. Members of the same family may also have common structural domains.

For example, CD2000 proteins of the invention have signal sequences. As used herein, a "signal sequence" includes a peptide of at least about 15 or 20 amino acid residues in length which occurs at the N-terminus of secretory and membrane-bound proteins and which contains at least about 70% hydrophobic amino acid residues such as alanine, leucine, isoleucine, phenylalanine, proline, tyrosine, tryptophan, or valine. In a preferred embodiment, a signal sequence contains at least about 10 to 40 amino acid residues, preferably about 19-34 amino acid residues, and has at least about 60-80%, more preferably 65-75%, and more preferably at least about 70% hydrophobic residues. A signal sequence serves to direct a protein containing such a sequence to a lipid bilayer. Thus, in one embodiment, a CD2000 protein contains a signal sequence at about amino acids 1 to 22 of SEQ ID NO:3 (SEQ ID NO:4). The signal sequence is cleaved during processing of the mature protein.

A CD2000 family member comprises one or more of the following domains: (1) an extracellular domain; (2) a transmembrane domain; and (3) a cytoplasmic domain. In one embodiment, a CD2000 protein contains extracellular domains at about amino acid residues 23 to 49 and amino acids 249 to 331 of SEQ ID NO:3 (SEQ ID NO:6 and SEQ ID NO:10, respectively), transmembrane domains at about amino acid residues 50 to 66 and amino acid residues 226 to 248 of SEQ ID NO:3 (SEQ ID NO:7 and SEQ ID NO:9, respectively), and a cytoplasmic domain at about amino acid residues 67 to 225 of SEQ ID NO:3 (SEQ ID NO:8). In another embodiment, a CD2000 protein contains an extracellular domain at about amino acid residues 249 to 331 of SEQ ID NO:3 (SEQ ID NO:10), a transmembrane domain at about amino acid residues 226 to 248 of SEQ ID NO:3 (SEQ ID NO:9), and a cytoplasmic domain at about amino acid residues 23 to 225 of SEQ ID NO:3 (SEQ ID NO:16). In a preferred embodiment, a CD2000 protein contains an extracellular domain at about amino acid residues 23 to 225 of SEQ ID NO:3 (SEQ ID NO:16), a transmembrane domain at about amino acid residues 226 to 248 of SEQ ID NO:3 (SEQ ID NO:9), and a cytoplasmic domain at about amino acid residues 249 to 331 of SEQ ID NO:3 (SEQ ID NO:17). In these embodiments, the mature CD2000 protein corresponds to amino acids 23 to 331 of SEQ ID NO:3 (SEQ ID NO:5).

In another embodiment, a CD2000 protein contains extracellular domains at about amino acid residues 31 to 49 and amino acid residues 249 to 331 of SEQ ID NO:3 (SEQ ID NO:14 and SEQ ID NO:10, respectively), transmembrane domains at about amino acid residues 8 to 30, amino acid residues 50 to 66, and amino acid residues 226 to 248 of SEQ ID NO:3 (SEQ ID NO:13, SEQ ID NO:7, and SEQ ID NO:9, respectively), and cytoplasmic domains at about amino acid residues 1 to 7 and amino acid residues 67 to 225 of SEQ ID NO:3 (SEQ ID NO:12 and SEQ ID NO:8, respectively). In another embodiment, a CD2000 protein contains an extracellular domain at about amino acid residues 31 to 225 of SEQ ID NO:3 (SEQ ID NO: 15), transmembrane domains at about amino acid residues 8 to 30 and amino acid residues 226 to 248 of SEQ ID NO:3 (SEQ ID NO:13 and SEQ ID NO:9, respectively), and cytoplasmic domains at about amino acid residues 1 to 7 and amino acid residues 249 to 331 of SEQ ID NO:3 (SEQ ID NO:12 and 17, respectively). In these embodiments, the mature CD2000 protein corresponds to amino acids 1 to 331 of SEQ ID NO:3.

A CD2000 family member can include a signal sequence. Thus, in one embodiment, a CD2000 family member comprises a signal sequence, an extracellular domain, a transmembrane, and a cytoplasmic domain. In certain embodiments, a CD2000 family member has the amino acid sequence of SEQ ID NO:3, and the signal sequence is located at amino acids 1 to 24, 2 to 24, 1 to 23, 2 to 23, 1 to 22, 2 to 22, 1 to 21, 2 to 21, 1 to 20, or 2 to 20. In such embodiments of the invention, the extracellular domain and the mature protein resulting from cleavage of such signal peptides are also included herein. For example, the cleavage of a signal sequence consisting of amino acids 1 to 24 results in an extracellular domain consisting of amino acids 25 to 225 of SEQ ID NO:3 and the mature CD2000 protein corresponding to amino 25 to 331 of SEQ ID NO:3.

A CD2000 family member can include one or more immunoglobulin (Ig) domains and/or Ig-like domains. In one embodiment, a CD2000 family member comprises a signal sequence, an extracellular domain, an Ig domain, an Ig-like domain, a transmembrane, and a cytoplasmic domain. An Ig domain typically has the following consensus sequence, beginning at about 1 to 15 amino acid residues, more preferably at about 3 to 10 amino acid residues, and most preferably at about 5 amino acid residues from the C-terminal end of a protein: (FY)-Xaa-C-Xaa-(VA)-COO-, wherein (FY) is either a phenylalanine or a tyrosine residue (preferably tyrosine), where "Xaa" is any amino acid, C is a cysteine residue, (VA) is either valine or an alanine residue (preferably alanine), and COO- is the protein C-terminus. In one embodiment, a CD2000 family member comprises one or more Ig domains having an amino acid sequence that is at least about 55%, preferably at least about 65%, more preferably at least 75%, yet more preferably at least about 85%, and most preferably at least about 95% identical to amino acids 146 to 197 of SEQ ID NO:3, which is the Ig domain of human CD2000 (this Ig domain is also represented as SEQ ID NO:18).

In another embodiment, a CD2000 family member comprises one or more Ig domains having an amino acid sequence that is at least about 55%, preferably at least about 65%, more preferably at least about 75%, yet more preferably at least about 85%, and most preferably at least about 95% identical to amino acids 146 to 197 of SEQ ID NO:3, which is the Ig domain of human CD2000 (this Ig domain are also represented as SEQ ID NO:18), includes a conserved cysteine residue about 8 residues downstream from the N-terminus of the Ig domain, and has one or more Ig domain consensus sequences as described herein.

In another embodiment, a CD2000 family member comprises one or more Ig domains having an amino acid sequence that is at least 55%, preferably at least about 65%, more preferably at least about 75%, yet more preferably at least about 85%, and most preferably at least about 95% identical to amino acids 146 to 197 of SEQ ID NO:3, which is the Ig domain of human CD2000 (this Ig domain are also represented as SEQ ID NO:18), includes a conserved cysteine residue 8 residues downstream from the N-terminus of the Ig domain, has one or more Ig domain consensus sequences as described herein, and has a conserved cysteine within the consensus sequence that forms a disulfide with said first conserved cysteine.

In yet another embodiment, a CD2000 family member comprises one or more Ig domains having an amino acid sequence that is at least 55%, preferably at least about 65%, more preferably at least about 75%, yet more preferably at least about 85%, and most preferably at least about 95% identical to amino acids 146 to 197 of SEQ ID NO:3, which is the Ig domain of human CD2000 (this Ig domain are also represented as SEQ ID NO:18), includes a conserved cysteine residue 8 residues downstream from the N-terminus of the Ig domain, has one or more Ig domain consensus sequences described herein, has a conserved cysteine within the consensus sequence that forms a disulfide with said first conserved cysteine, and has at least one CD2000 biological activity as described herein.

In a preferred embodiment, a CD2000 family member has the amino acid sequence of SEQ ID NO:3, wherein the aforementioned Ig domain conserved residues are located as follows: the N-terminal conserved cysteine residue is located at amino acid residue position 153 (within the Ig domain of SEQ ID NO:3) and the C-terminal conserved cysteine residue is located at amino acid position 195 (within the Ig domain of SEQ ID NO:3).

An Ig-like domain of CD2000 as described herein has the following consensus sequence, beginning at about 1 to 15 amino acid residues, more preferably at about 3 to 10 amino acid residues, and most preferably at about 5 amino acid residues from the domain C-terminus: (FY)-Xaa, wherein (FY) is either a phenylalanine or a tyrosine residue (preferably tyrosine) and where "Xaa" is any amino acid. In one embodiment, a CD2000 family member comprises one or more Ig-like domains having an amino acid sequence that is at least about 55%, preferably at least about 65%, more preferably at least 75%, yet more preferably at least about 85%, and most preferably at least about 95% identical to amino acid residues 35 to 111 of SEQ ID NO:3, which are the Ig-like domains of human CD2000 (this Ig-like domain is also represented as SEQ ID NO:19).

In another embodiment, a CD2000 family member comprises one or more Ig-like domains having an amino acid sequence that is at least about 55%, preferably at least about 65%, more preferably at least about 75%, yet more preferably at least about 85%, and most preferably at least about 95% identical to amino acid residues 35 to 111 of SEQ ID NO:3, which are the Ig-like domains of human CD2000 (this Ig-like domain is also represented as SEQ ID NO:19), includes a conserved cysteine residue about 8 residues downstream from the N-terminus of the Ig-like domain, and has one or more Ig-like domain consensus sequences as described herein.

In yet another embodiment, a CD2000 family member comprises one or more Ig-like domains having an amino acid sequence that is at least 55%, preferably at least about 65%, more preferably at least about 75%, yet more preferably at least about 85%, and most preferably at least about 95% identical to amino acid residues 35 to 111 of SEQ ID NO:3, which are the Ig-like domains of human CD2000 (this Ig-like domain is also represented as SEQ ID NO:19), includes a conserved cysteine residue 8 residues downstream from the N-terminus of the Ig-like domain, has one or more Ig-like domain consensus sequences described herein, and has at least one CD2000 biological activity as described herein.

In another embodiment, the Ig-like domains of a CD2000 family member are Ig domains, which have the following consensus sequence at the C-terminus of the domain: (FY)-Xaa-C-Xaa-(VA)-COO-, wherein (FY) is either a phenylalanine or a tyrosine residue (preferably tyrosine), where "Xaa" is any amino acid, C is a cysteine residue, (VA) is a valine or alanine residue, and COO- is the C-terminus of the domain.

In a preferred embodiment, a CD2000 family member has the amino acid sequence of SEQ ID NO:3, wherein the aforementioned Ig-like domain has an N-terminal conserved cysteine residue located at amino acid residue position 43 (within the Ig-like domain of SEQ ID NO:3).

A CD2000 family member can include one or more SLAM-associated protein (SAP) domains. In one embodiment, a CD2000 family member comprises a signal sequence, an extracellular domain, two SAP domains, an Ig domain, an Ig-like domain, a transmembrane, and a cytoplasmic domain. A SAP binding domain as described herein has the following consensus sequence: T-(IXaa)-Y-Xaa-Xaa-(VI), wherein T is threonine, (IXaa) is either isoleucine or any amino acid, Y is tyrosine, "Xaa" is any amino acid, and (VI) is valine or isoleucine. In one embodiment, a CD2000 family member comprises one or more SAP binding domains having an amino acid sequence that comprises a sequence at least 50%, at least 55%, at least 60%, at least 65%, at least 75%, at least 85%, at least 95%, or at least 99% identical to amino acid residues 282 to 287 and/or amino acid residues 306 to 311 of SEQ ID NO:3 (SEQ ID NO:21 and SEQ ID NO:22, respectively). In one embodiment, a CD2000 family member comprises one or more SAP binding domains having an amino acid sequence that comprises a sequence at least 50%, preferably at least 55%, at least 60%, at least 65%, at least 75%, at least 85%, at least 95%, or at least 99% identical to amino acid residues 282 to 287 and/or amino acid residues 306 to 311 of SEQ ID NO:3 (SEQ ID NO:21 and SEQ ID NO:22, respectively), and has at least one biological activity of CD2000 as described herein.

In a preferred embodiment, a CD2000 family member comprises one or more SAP binding domains having an amino acid sequence that comprises amino acid residues 282 to 287 and/or amino acid residues 306 to 311 of SEQ ID NO:3 (SEQ ID NO:21 and SEQ ID NO:22, respectively). In another preferred embodiment, a CD2000 family member comprises one or more SAP binding domains having an amino acid sequence that comprises amino acid residues 282 to 287 and/or amino acid residues 306 to 311 of SEQ ID NO:3 (SEQ ID NO:21 and SEQ ID NO:22, respectively), and has at least one biological activity of CD2000 as described herein. In another preferred embodiment, a CD2000 family member comprises SAP binding domains comprising amino acid residues 282 to 287 and amino acid residues 306 to 311 of SEQ ID NO:3 (SEQ ID NO:21 and SEQ ID NO:22, respectively) in which tyrosine residues 284 and 308 are phosphorylated. In yet another preferred embodiment, a CD2000 family member comprises SAP binding domains comprising amino acid residues 282 to 287 and amino acid residues 306 to 311 of SEQ ID NO:3 (SEQ ID NO:21 and SEQ ID NO:22, respectively) in which tyrosine residues 284 and 308 are phosphorylated, and said CD2000 family member has at least one biological activity of CD2000 as described herein.

The CD2001 proteins and nucleic acid molecules comprise a family of molecules having certain conserved structural and functional features. A CD2001 family member comprises one or more of the following domains: (1) an extracellular domain; (2) a transmembrane domain; and (3) a cytoplasmic domain. In one embodiment, a CD2001 protein contains an extracellular domain at about amino acid residues 20-236 of SEQ ID NO:76 (SEQ ID NO:79), a transmembrane domain at about amino acid residues 237 to 263 of SEQ ID NO:76 (SEQ ID NO:80), and a cytoplasmic domain at about amino acid residues 264 to 289 of SEQ ID NO:76 (SEQ ID NO:81). In this embodiment, the mature CD2001 protein corresponds to amino acids 20 to 289 of SEQ ID NO:76 (SEQ ID NO:77). A CD2001 family member can include a signal sequence. Thus, in one embodiment, a CD2001 family member comprises a signal sequence, an extracellular domain, a transmembrane, and a cytoplasmic domain. In certain embodiments, a CD2001 family member has the amino acid sequence of SEQ ID NO:76, and the signal sequence is located at amino acids 1 to 17, 2 to 17, 1 to 18, 2 to 18, 1 to 19, 2 to 19, 1 to 20, 2 to 20, 1 to 21, or 2 to 21. In a preferred embodiment, a CD2001 protein contains a signal sequence at about amino acids 1 to 19 of SEQ ID NO:76 (SEQ ID NO:78). In such embodiments of the invention, the extracellular domain and the mature protein resulting from cleavage of such signal peptides are also included herein. For example, the cleavage of a signal sequence consisting of amino acids 1 to 19 results in an extracellular domain consisting of amino acids 20 to 236 of SEQ ID NO:76 and the mature CD2001 protein corresponding to amino 20 to 289 of SEQ ID NO:76.

A CD2001 family member can include one or more immunoglobulin (Ig) domains and/or Ig-like domains. In one embodiment, a CD2001 family member comprises a signal sequence, an extracellular domain, an Ig-like domain, an Ig domain, a transmembrane, and a cytoplasmic domain.

An Ig-like domain of CD2001 as described herein has the following consensus sequence, beginning at about 1 to 15 amino acid residues, more preferably at about 3 to 10 amino acid residues, and most preferably at about 5 amino acid residues from the domain C-terminus: (FY)-Xaa, wherein (FY) is either a phenylalanine or a tyrosine residue (preferably tyrosine) and where "Xaa" is any amino acid. In one embodiment, a CD2001 family member comprises one or more Ig-like domains having an amino acid sequence that is at least about 55%, preferably at least about 65%, more preferably at least 75%, yet more preferably at least about 85%, and most preferably at least about 95% identical to amino acid residues 35 to 132 of SEQ ID NO:76, which is the Ig-like domain of human CD2001 (this Ig-like domain is also represented as SEQ ID NO:96).

In another embodiment, a CD2001 family member comprises one or more Ig-like domains having an amino acid sequence that is at least about 55%, preferably at least about 65%, more preferably at least about 75%, yet more preferably at least about 85%, and most preferably at least about 95% identical to amino acid residues 35 to 132 of SEQ ID NO:76, which is the Ig-like domain of human CD2001 (this Ig-like domain is also represented as SEQ ID NO:96), includes a conserved cysteine residue about 8 residues downstream from the N-terminus of the Ig-like domain, and has one or more Ig-like domain consensus sequences as described herein.

In yet another embodiment, a CD2001 family member comprises one or more Ig-like domains having an amino acid sequence that is at least 55%, preferably at least about 65%, more preferably at least about 75%, yet more preferably at least about 85%, and most preferably at least about 95% identical to amino acid residues 35 to 132 of SEQ ID NO:76, which is the Ig-like domain of human CD2001 (this Ig-like domain is also represented as SEQ ID NO:96), includes a conserved cysteine residue 8 residues downstream from the N-terminus of the Ig-like domain, has one or more Ig-like domain consensus sequences described herein, and has at least one CD2001 biological activity as described herein.

In another embodiment, a CD2001 family member comprises one or more Ig domains having an amino acid sequence that is at least about 55%, preferably at least about 65%, more preferably at least 75%, yet more preferably at least about 85%, and most preferably at least about 95% identical to amino acid residues 135 to 204 of SEQ ID NO:76, which is the Ig domain of human CD2001 (this Ig domain is also represented as SEQ ID NO:97).

In another embodiment, the Ig-like domains of a CD2001 family member are Ig domains, which have the following consensus sequence at the C-terminus of the domain: (FY)-Xaa-C-Xaa-(VA)-COO-, wherein (FY) is either a phenylalanine or a tyrosine residue (preferably tyrosine), where "Xaa" is any amino acid, C is a cysteine residue, (VA) is a valine or alanine residue, and COO- is the C-terminus of the domain.

In another embodiment, a CD2001 family member comprises one or more Ig domains having an amino acid sequence that is at least about 55%, preferably at least about 65%, more preferably at least about 75%, yet more preferably at least about 85%, and most preferably at least about 95% identical to amino acid residues 135 to 204 of SEQ ID NO:76, which is the Ig domain of human CD2001 (this Ig domain is also represented as SEQ ID NO:97), includes a conserved cysteine residue about 8 residues downstream from the N-terminus of the Ig-like domain, and has one or more Ig domain consensus sequences as described herein.

In yet another embodiment, a CD2001 family member comprises one or more Ig domains having an amino acid sequence that is at least 55%, preferably at least about 65%, more preferably at least about 75%, yet more preferably at least about 85%, and most preferably at least about 95% identical to amino acid residues 135 to 204 of SEQ ID NO:76, which is the Ig domain of human CD2001 (this Ig domain is also represented as SEQ ID NO:97), includes a conserved cysteine residue 8 residues downstream from the N-terminus of the Ig domain, has one or more Ig-like domain consensus sequences described herein, and has at least one CD2001 biological activity as described herein.

Various features of human CD2000 and human CD2001 are summarized below.

### Human CD2000

A cDNA encoding human CD2000 was identified by analyzing the sequences of clones present in a human cDNA library from peripheral blood lymphocytes from a mixed lymphocyte reaction. This analysis led to the identification of a clone, jthLa118e01, encoding full-length human CD2000. The human CD2000 cDNA of this clone is 2713 nucleotides long (Figures 1A-1B; SEQ ID NO:1). The open reading frame of this cDNA, nucleotides 64 to 1056 of SEQ ID NO:1 (SEQ ID NO:2), encodes a 331 amino acid transmembrane protein (Figures 1A-B; SEQ ID NO:3) that, as discussed below, represents a CD2 family member.

The signal peptide prediction program SIGNALP (Nielsen, et al., 1997, *Protein Engineering* 10:1-6) predicted that human CD2000 includes a 22 amino acid signal peptide (amino acid 1 to about amino acid 22 of SEQ ID NO:3; SEQ ID NO:4) preceding the mature human CD2000 protein (corresponding to about amino acid 23 to amino acid 331 of SEQ ID NO:3; SEQ ID NO:5). The molecular weight of human CD2000 without post-translational modifications is 37.273 kDa prior to the cleavage of the signal peptide, 34.758 kDa after cleavage of the signal peptide.

Human CD2000 is a transmembrane protein that is a CD2 family member expressed on lymphocytes which consists of one or more of the following domains: (1) an extracellular domain; (2) a transmembrane domain; and (3) a cytoplasmic domain. The mature human CD2000 protein contains an extracellular domain at amino acid residues 23 to 225 of SEQ ID NO:3 (SEQ ID NO:16), a transmembrane domain at amino acid residues 226 to 248 of SEQ ID NO:3 (SEQ ID NO:9), and a cytoplasmic domain at amino acid residues 249 to 331 of SEQ ID NO:3 (SEQ ID NO:17).

Figure 2 depicts a hydropathy plot of human CD2000. Relatively hydrophobic regions of the protein are shown above the horizontal line, and relatively hydrophilic regions of the protein are below the horizontal line. The cysteine residues (cys) and potential N-glycosylation sites (Ngly) are indicated by short vertical lines just below the hydropathy trace. The dashed vertical line separates the signal sequence (amino acids 1 to 22 of SEQ ID NO:3; SEQ ID NO:4) on the left from the mature protein (amino acids 23 to 331 of SEQ ID NO:3; SEQ ID NO:5) on the right.

Human CD2000 comprises an immunoglobulin domain sequence at amino acid residues 146 to 197 of SEQ ID NO:3 (SEQ ID NO:18) and an immunoglobulin-like sequence at amino acid residues 35 to 111 of SEQ ID NO:3 (SEQ ID NO:19). Ten N-glycosylation sites are present in human CD2000. The first N-glycosylation site has the amino acid sequence NETS (at amino acid residues 58 to 61 of SEQ ID NO:3), the second N-glycosylation site has the amino acid sequence NFTQ (at amino acid residues 87 to 90 of SEQ ID NO:3), the third N-glycosylation site has the amino acid sequence NHSQ (at amino acid residues 137 to 140 of SEQ ID NO:3), the fourth N-glycosylation site has the amino acid sequence NMTC (at amino acid residues 144 to 147 of SEQ ID NO:3), the fifth N-glycosylation site has the amino acid sequence NVSF (at amino acid residues 161 to 164 of SEQ ID NO:3), the sixth N-glycosylation site has the amino acid sequence NLTV (at amino acid residues 178 to 181 of SEQ ID NO:3), the seventh N-glycosylation site has the amino acid sequence NLSF (at amino acid residues 203 to 206 of SEQ ID NO:3), the eighth N-glycosylation site has the amino acid sequence NNTV (at amino acid residues 280 to 283 of SEQ ID NO:3), the ninth N-glycosylation site has the amino acid sequence NDTI (at amino acid residues 302 to 305 of SEQ ID NO:3), and the tenth N-glycosylation site has the amino acid sequence NHSK (at amino acid residues 312 to 315 of SEQ ID NO:3). Human CD2000 has a single cAMP phosphorylation site having the amino acid sequence RRDS at amino acid residues 251 to 254 of SEQ ID NO:3.

Nine protein kinase C (PKC) phosphorylation sites are present in human CD2000. The first PKC phosphorylation site has the amino acid sequence SYR (at amino acid residues 106 to 108 of SEQ ID NO:3), the second PKC phosphorylation site has the amino acid sequence STK (at amino acid residues 112 to 114 of SEQ ID NO:3), the third PKC phosphorylation site has the amino acid sequence SAK (at amino acid residues 116 to 118 of SEQ ID NO:3), the fourth PKC phosphorylation site has the amino acid sequence TLR (at amino acid residues 123 to 125 of SEQ ID NO:3), the fifth PKC phosphorylation site has the amino acid sequence SFR (at amino acid residues 163 to 165 of SEQ ID NO:3), the sixth PKC phosphorylation site has the amino acid sequence TQR (at amino acid residues 259 to 261 of SEQ ID NO:3), the seventh PKC phosphorylation site has the amino acid sequence SAR (at amino acid residues 267 to 269 of SEQ ID NO:3), the eighth PKC phosphorylation site has the amino acid sequence SNR (at amino acids 290 to 292 of SEQ ID NO:3), and the ninth PKC phosphorylation site has the amino acid sequence TPR (at amino acid residues 298 to 300 of SEQ ID NO:3). Five casein kinase II phosphorylation sites are present in human CD2000. The first casein kinase II phosphorylation site has the amino acid sequence SVED (at amino acid residues 154 to 157 of SEQ ID NO:3), the second casein kinase II phosphorylation site has the amino acid sequence SEQD (at amino acid residues 189 to 192 of SEQ ID NO:3), the third casein kinase II phosphorylation site has the amino acid sequence SNRE (at amino acid residues 290 to 293 of SEQ ID NO:3), the fourth casein kinase II phosphorylation site has the amino acid sequence TPRE (at amino acid residues 298 to 301 of SEQ ID NO:3), and the fifth casein kinase II phosphorylation site has the amino acid sequence TALD (at amino acid residues 325 to 328 of SEQ ID NO:3).

Human CD2000 has two tyrosine kinase phosphorylation sites having the amino acid sequences KMEDTGSY (at amino acid residues 100 to 107 of SEQ ID NO:3) and RISSEQDY (at amino acid residues 186 to 193 of SEQ ID NO:3). Human CD2000 has three N-myristylation sites. The first N-myristylation site has the amino acid sequence GNVVSQ (at amino acid residues 17 to 22 of SEQ ID NO:3), the second N-myristylation site has the amino acid sequence GSYRAQ (at amino acid residues 105 to 110 of SEQ ID NO:3), and the third N-myristylation site has the amino acid sequence GNTLSS (at amino acid residues 170 to 175 of SEQ ID NO:3). Human CD2000 has a single amidation site having the amino acid sequence QGKR (at amino acid residues 82 to 85 of SEQ ID NO:3).

Figure 3A depicts the alignment between an immunoglobulin domain of human CD2000 (from amino acid residues 146 to 197 of SEQ ID NO:3; SEQ ID NO:18) and a typical immunoglobulin domain (SEQ ID NO:20; Accession Number PF00047). Figure 3B depicts the alignment between an immunoglobulin-like domain of human CD2000 (from amino acid residues 35 to 111 of SEQ ID NO:3; SEQ ID NO: 19) and a typical immunoglobulin domain (SEQ ID NO:20; Accession Number PF00047).

Human CD2000 has two SAP binding domain comprising amino acid residues 282 to 287 and amino acid residues 306 to 311 of SEQ ID NO:3 (SEQ ID NO: 21 and SEQ ID NO:22, respectively). SAP recognizes the binding motif TI-Xaa-Y-Xaa-Xaa-(VI) found in the cytoplasmic domains of a number of T cell, B cell, and NK cell surface receptors, including CD2 family members such as SLAM. Mutations in SAP are associated with X-linked lymphoproliferative syndrome (XPL). SAP consists of a single SH2 domain and has been suggested to function as a signaling inhibitor by blocking or regulating the binding of other signaling molecules such as, *e.g.,* SHP-2 phosphatase to a receptor *(e.g.,* SLAM; Poy et al., 1999, Molecular Cell 4:555-561 and Sayos et al., 1998, Nature 395:462-469)

Human CD2000 has homology to CD2 family members (Figures 4A-4B). (See, *e.g.*, Wingren et al., 1995, Crit. Rev. Immunol. 15(3-4):235-253 for a review of CD2 family members.) Human CD2000 has the highest homology to human CD84. Figure 5 shows an alignment of the human CD2000 coding region (SEQ ID NO:2) and the human CD84 coding region (SEQ ID NO:29; GenBank Accession No. U82988) The nucleotide sequences of coding regions of human CD84 and human CD2000 are 34.1 % identical. Figure 6 shows that there is an overall 22 % identity between the amino acid sequence of the human CD2000 protein (SEQ ID NO:3) and the amino acid sequence of the CD84 (SEQ ID NO:30).

Human CD2000 also has homology to the murine CD2 family member Ly108. The Ly108 gene encodes a 331 amino acid polypeptide that includes an extracellular membrane domain, two Ig-like domains, a transmembrane domain, and a cytoplasmic domain (Peck et al., 2000, Immunogenetics 52(1-2):63-72). Figure 7 depicts an alignment of the amino acid sequence of human CD2000 and the amino acid sequence of murine Ly108. There is 36.6% identity between the amino acid sequence of the human CD2000 protein (SEQ ID NO:3) and the amino acid sequence of the murine Ly108 protein (SEQ ID NO:11).

CD2 family members are cell adhesion molecules expressed on the surface of immune cells which function as co-stimulatory molecules. In particular, CD2 is expressed on T cells and binds to LFA-3, which is expressed by antigen presenting cells (APCs). The CD2/LFA-3 interaction induces a strong antigen-independent cell adhesion and results in T cell proliferation and the production of cytokines such as IFN-γ and IL-2 (Wingren et al., 1995, Crit. Rev. Immunol. 15(3-4):235-253). Signaling lymphocyte activation molecule (SLAM or CDw150) is expressed on the surface of activated T cells and seems to act only on memory cells (Aversa et al., 1997, J. Immunol. 158:4036-4044). Human CD84 is expressed predominantly by mature B lymphocytes and monocytes (Angel del al Fuente et al., 1997, Blood 90:2398-2405).

Northern blot analysis of human CD2000 expression demonstrates expression of a single, 3.1 kb transcript in the spleen, lymph node, thymus, peripheral blood leukocytes, bone marrow, fetal liver, lung, and liver (Figure 8). Human CD2000 RNA expression was low to undetectable in the following tissues: heart, brain, placenta, skeletal muscle, kidney, or pancreas (Figure 8). The highest level of human CD2000 RNA expression was detected in the peripheral blood, spleen and lymph node. The results from the Northern blot analysis indicate that CD2000 is expressed primarily in lymphoid tissues.

Clone EpCD2000, which encodes human TANGO CD2000, was deposited with the American Type Culture Collection (10801 University Boulevard, Manassas, VA 20110-2209) on July 17, 2000 and assigned patent deposit Number PTA-2267. This deposit will be maintained under the terms of the Budapest Treaty on the International Recognition of the Deposit of Microorganisms for the Purposes of Patent Procedure. This deposit was made merely as a convenience for those of skill in the art and is not an admission that a deposit is required under 35 U.S.C. §112.

### CD2000 Gene Expression Analysis

Total RNA was prepared from various human tissues by a single step extraction method using RNeasy Mini Kit according to the manufacturer's instructions (Qiagen, Inc.). Each RNA preparation was treated with DNase I (Qiagen) at room temperature for 15 minutes. DNAse I treatment was determined to be complete if the sample required at least 38 PCR amplification cycles to reach a threshold level of fluorescence using ÿ-2 microglobulin as an internal amplicon reference. The integrity of the RNA samples following DNase I treatment was confirmed by agarose gel electrophoresis and ethidium bromide staining. Next, the cDNA was prepared from the sample using the SUPERSCRIPT™ Choice System following the manufacturer's instructions (Qiagen, Inc.). A negative control of RNA without reverse transcriptase was mock reverse transcribed for each RNA sample.

CD2000 expression was measured by TaqMan® quantitative PCR (Perkin Elmer Applied Biosystems) in cDNA prepared from the following human tissues and cells: normal kidney; normal lymph node; normal spleen; normal brain; normal lung; normal liver; normal tonsil; normal colon; normal heart; Clonetech spleen; Clonetech thymus; Clonetech lung; resting CD4⁺ cells; CD4⁺ cells stimulated 4 or 24 hr with anti-CD3 antibody or anti-CD3 in combination with anti-CD28 antibody; resting CD8⁺ cells; CD8⁺ cells stimulated 4 or 24 hr with anti-CD3 antibody or anti-CD3 in combination with anti-CD28 antibody; resting CD14⁺ cells; CD14⁺ cells stimulated 4 or 24 hrs with TNF-α or lipopolysaccharide (LPS); resting macrophages (MACs); MACs stimulated 4 or 24 hr with TNF-α or LPS; normal dendritic cells (Kaps DC) from donors A and E untreated or treated for 4 or 24 hr TNF-α; resting tonsillar CD19⁺ cells; CD19⁺ cells stimulated with LPS or CD40 ligand; resting granulocytes (Grans); Grans stimulated 4 or 24 hr with TNF-α or IFN-γ; resting eosinophils (Eos) or Eos stimulated 4 or 24 hr with IL-4; Th0, Th1, and Th2 cells; resting fibroblastic synoviocytes from rheumatoid arthritis (RA synovio type B) or RA synovio type B stimulated 4 or 24 hr with TNF-α, IFN-γ, or IL-1; resting normal human bronchial epithelium (NHBE) or NHBE stimulated 4 or 24 hr with IL-4, IL-13, or the combination of IL-4 and IL-13; resting bronchial smooth muscle cells (BSMC) or stimulated 4 or 24 hr with TNF-α, IFN-γ, or IL-1; resting human microvascular endothelial cells (HMVEC) or stimulated 4 or 24 hr with TNF-α, IFN-γ, or IL-1; resting normal human lung fibroblasts (NHLF) or stimulated 4 or 24 hr with TNF-α or TGF-β; colons from patients with Crohn's disease; colon's from patients with colitis; resting normal human dermal fibroblasts (NHDF) or stimulated 4 or 24 hr with TNF-α or TGF-β; resting normal human dermal fibroblasts (HDF) or stimulated 4 or 24 hr with TNF-α or IL-1; normal synovium; synovium from rheumatoid arthritis patients; lung tissue pooled from patients with chronic obstructive pulmonary disease (COPD-1 and COPD-2); and lung tissue pooled from patients with idiopathic pulmonary fibrosis (IPF). PCR probes were designed by PrimerExpress software (PE Biosystems) based on the disclosed sequence of CD2000. The primers and probes used were as follows:

CD2000 Forward Primer: AGAGACTGAGGCAGGAGAATCG (SEQ ID NO:23)

CD2000 Reverse Primer: TGGAATACATTGGCATGATTTTG (SEQ ID NO:24)

CD2000 TaqMan Probe: TTGAACCCAGGAGGTGGAGGTTCCAG (SEQ ID NO:25)

β2-microglobulin Forward Primer: CACCCCCACTGAAAAAGATGA (SEQ ID NO:26)

β2-microglobulin Reverse Primer: CTTAACTATCTTGGGCTGTGACAAAG (SEQ ID
NO:27)

β2-microglobulin TaqMan Probe: ATGCCTGCCGTGTGAACCACGTG (SEQ ID NO:28)

The CD2000 gene probe was labeled using FAM (6-carboxyfluorescein), and the β2-microglobulin reference probe was labeled with a different fluorescent dye, VIC. The differential labeling of the target gene and internal reference gene thus enabled measurement in same well. Forward and reverse primers and the probes for both β2-microglobulin and the CD2000 gene were added to the TaqMan® Universal PCR Master Mix (PE Applied Biosystems). Although the final concentration of primer and probe could vary, each was internally consistent within a given experiment. A typical experiment contained 200 nM of forward and reverse primers plus 100 nM probe for β2-microglobulin and 600 nM forward and reverse primers plus 200 nM probe for the target gene. TaqMan® matrix experiments were carried out using an ABI PRISM 7700 Sequence Detection System (PE Applied Biosystems). The thermal cycler conditions were as follows: hold for 2 min at 50°C and 10 min at 95°C, followed by two-step PCR for 40 cycles of 95°C for 15 sec followed by 60°C for 1 min.

The following method was used to quantitatively calculate CD2000 gene expression in the various tissues relative to β2-microglobulin expression in the same tissue. The threshold cycle (Ct) value is defined as the cycle at which a statistically significant increase in flourescence is detected. A lower Ct value is indicative of a higher mRNA concentration. The Ct value of the kinase gene is normalized by subtracting the Ct value of the β2-microglobulin gene to obtain a _{Δ}Ct value using the following formula: _{Δ}Ct=Ctₖᵢₙₐₛₑ - Ct_{β2} _{microglobulin}. Expression is then calibrated against a well containing a nontemplate control, *e.g*., water. The _{Δ}Ct value for the calibrator sample is then subtracted from Ct for each tissue sample according to the following formula: _{Δ}Ct=_{Δ}Ct-ₛₐₘₚₗₑ - _{Δ}Ct-_{calibrator}. Relative expression is then calculated using the arithmetic formula given by 2-Ct. Expression of the CD2000 gene in each of the tissues tested is then graphically represented in Figures 8A-8B.

CD2000 expression was detected in the following cell types: resting CD4⁺ cells; CD4⁺ cells stimulated with anti-CD3 antibody; CD4⁺ cells stimulated with anti-CD3 antibody and anti-CD28 antibody; resting CD8⁺ cells; CD8⁺ cells stimulated with anti-CD3 antibody; CD8⁺ cells stimulated with anti-CD3 antibody and anti-CD28 antibody; resting CD14⁺ cells; resting CD19⁺ cells; CD19⁺ cells stimulated with LPS; CD19⁺ cells stimulated with CD40L; resting MACs; MACS stimulated with IFN-γ; untreated Kaps DCs A; resting tonsillar CD19⁺ cells; tonsillar CD19⁺ cells stimulated with LPS; tonsillar CD19⁺ cells stimulated with CD40L; resting GRANS; GRANS stimulated with TNF-α; GRANS stimulated with IFN-γ; resting eosinophils; eosinophils stimuated with IL-4; TH0 cells; TH1 cells; and TH2 cells (Figure 9A). Higher levels of CD2000 were detected on activated CD4⁺ T cells than resting CD4⁺, resting CD19⁺ B cells than activated CD19⁺ B cells, activated eosinophils than resting eosinophils, resting granulocytes than activated granulocytes, and TH1 cells than TH0 or TH2 cells. These results suggest that CD2000 expression is upregulated on CD4⁺ cells and eosinophils following stimulation. In particular, these results suggest that the expression of CD2000 is upregulated on CD4⁺ cells following activation of the T cell receptor. These results suggest also that CD2000 expression is downregulated on CD19⁺ cells and granulocytes following stimulation. Further, these results suggest that CD2000 is differentially expressed on TH1, TH2 and TH0 cells.

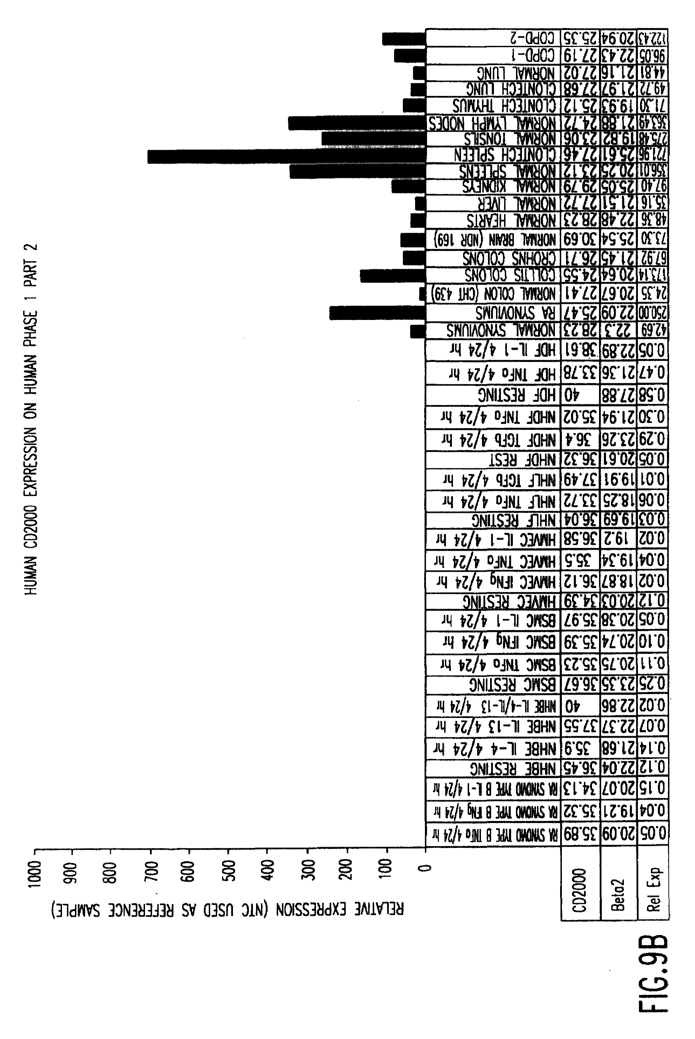

CD2000 expression was detected in the following tissues: normal synovium; rheumatoid arthritis synovium; normal colon; colons from patients with colitis; colons from patients with Crohn's disease; normal brain; normal hearts; normal liver; normal kidneys; normal spleens; Clonetech spleen; normal tonsils; normal lymph node; Clonetech thymus; Clonetech lung; normal lung; and lung tissue pooled from patients with chronic obstructive pulmonary disease (COPD-1 and COPD-2) (Figure 9B). Higher levels of CD2000 expression were detected in synovium from rheumatoid arthritis patients than normal synovium, and higher levels of CD2000 expression were detected in colons from patients with Crohn's disease and colitis than in normal colons. These results suggest that CD2000 expression is upregulated in patients with rheumatoid arthritis, Crohn's disease, and colitis.

### CD2000 is Phosphorylated by c-fyn and Binds to SAP in a Phosphotyrosine-Dependent Manner

Since CD2000 contains two SAP binding motifs in the cytoplasmic tail, the ability of these motifs to bind to SAP was assessed using yeast two-hybrid systems. The following results demonstrate that CD2000 is phosphorylated by *c-fyn* and that the two SAP motifs in the cytoplasmic tail of CD2000 bind to the SH2 domain of SLAM associated protein (SAP) in a phosphotyrosine-dependent manner.

### Materials & Methods:

Yeast two and three-hybrid system plasmid construction: CD2000 truncation constructs were generated by PCR using full length cDNA as a template. Three PCR products generated as follows: (1) a 'cytoplasmic' PCR encoding the entire cytoplasmic region of CD2000 was generated using the forward primer: 5'CCCGAATTCGGCAGGAAAAGAAGAGAT-3' (SEQ ID NO::68) and reverse primer: 
5'-CCCGGATCCTTACACGACATTGTCAAG-3' (SEQ ID NO:69); (2) a 'SAP motif #1'
PCR product was generated with the forward primer:
 5'-CCCGAATTCGGCAGGAAAAGAAGAGAT-3' (SEQ ID NO:68) and reverse primer: 
5'-CCCGGATCCTTAGATAGTATCATTTTC-3' (SEQ ID NO:70); and (3) a 'SAP motif
#2" PCR product was generated with the forward primer:
 
5'-CCCGAATTCGGCACTCATTCAAACAGG-3'(SEQ ID NO:71) and
reverse primer: 5'-CCCGGATCCTTACACGACATTGTCAAG-3'(SEQ ID NO:69).
 All forward primers incorporated an EcoR1 restriction site and a 'hinge' glycine codon 'GGC' upstream of the coding sequence. All reverse primers incorporated a BamHl restriction site and a stop codon 'TTA' prior to the coding sequence. The three PCR products were cloned into the multiple cloning site of pGAD424 a yeast two hybrid GAL-4 activation domain vector (Clontech) utilizing the incorporated BamHl and EcoR1 sites and sequenced to confirm their identity.

Human SAP was amplified by PCR from cDNA and cloned in the EcoR1/BamH1 sites in the first multiple cloning site of a bicistronic vector termed pBRIDGE (Clontech), or the unique cloning site of pGBT9 (Clontech) for two hybrid analysis. The following primers were utilized to generate SAP cDNA: forward primer 5'-ATCGAATTCATGGACGCAGTGGCTGTGTAT-3' (SEQ ID NO:72) and reverse primer: 5' ATCGGATCCTCATGGGGCTTTCAGGCAGAC 3' (SEQ ID NO:73). For 'three hybrid' analysis a modified *c-fyn* sequence was inserted into the second multiple cloning site of pBRIDGE. *c-fyn* was modified by PCR-mediated mutagenesis by mutating two regulatory tyrosine residues, Y420 and Y531, and the SH2 domain R-Q 176 as previously described (Sayos etal., 1998, Nature 395:462; and Sayos et al., 2001, Blood 97:3867-3874). The mutations in the two regulatory tyrosine residues allow expression of *c-fyn* in yeast without inducing cell death, and the SH2 mutation inhibits possible *c-fyn* competition with the GAL-4 activation domain fusion protein. Conditional expression of the resultant *c-fyn* mutant was driven by the MET25 promoter in response to methionine levels in the medium. In the presence of 1 mM methionine the expression of the protein was repressed, while the absence of methionine in the medium induces protein expression. Mutated *c-fyn* still phosphorylated endogenous yeast proteins and its capability to become autophosphorylated was maintained.

Yeast two hybrid and yeast three hybrid β-galactosidase assay: Yeast strain Y190 (Clontech) was co-transfected with CD2000 constructs in pGAD424 and SAP in pGBT9 (two hybrid) or SAP + *c-fyn* ^{2Y-F R-Q} in pBRIDGE (three hybrid). SLAM (CDw150) and SAP co-transfections were performed as a control for SAP binding. Co-transfectants were selected by growth on solid media lacking tryptophan and leucine (two hybrid) or tryptophan, leucine and methionine (three hybrid) for two days. Selected yeast colonies were then grown in liquid selection media overnight, harvested and liquid β-galactosidase assays were performed as per the Clontech yeast protocols handbook.

### Results:

A classical yeast two-hybrid system, including SAP as 'bait' and the cytoplasmic region of CD2000, the CD2000 SAP motif #1, or the CD2000 SAP motif #2 as 'prey', was used to investigate the phosphotyrosine-dependent interaction between cytoplasmic regions of CD2000 and SAP (Figure 10B). A modified yeast two-hybrid system, including SAP as 'bait', the cytoplasmic region of CD2000, the CD2000 SAP motif #1, or the CD2000 SAP motif #2 as 'prey', and modified *c-fyn* as a kinase to phosphorylate CD2000, was used to investigate the phosphotyrosine-dependent interaction between cytoplasmic regions of CD2000 and SAP (Figure 10C). The ability of the cytoplasmic region of CD2000 or SAP binding motifs present in CD2000 to interact with SAP was measured by determining β-galactosidase reporter expression. In both yeast two-hybrid systems, SAP was unable to bind to the cytoplasmic region of CD2000 or either CD2000 SAP motif in the absence of tyrosine phosphorylation (Figures 10B-C). In contrast, under the same conditions SAP binds to SLAM (CD150; Figure 10B). Following the introduction of a modified *c-fyn* into the yeast cells, CD2000 binds to SAP with high affinity (Figure 10C). In particular, following the introduction of a modified *c-fyn* into the yeast cells, SAP binds to the cytoplasmic region of CD2000 and both SAP motifs present in CD2000 with high affinity. Higher β-galactosidase reporter expression was detected in response to the SAP/CD2000 interaction than the SAP/SLAM interaction when *c-fyn* was introduced into the yeast cells (Figure 10C). These results indicate that SAP binds to phosphorylated CD2000 and that SAP binds to phosphorylated CD2000 with a higher affinity than to phosphorylated SLAM.

### CD2000 Binds to EAT-2 in Phosphorylation-Dependent

The following results demonstrate that the cytoplasmic region of CD2000 binds to the SH2 domain of EAT-2 in a phosphorylation-dependent manner.

### Materials & Methods:

Yeast two and three-hybrid system plasmid construction: The pGAD424-cytoplasmic, pGAD424-SAP motif #1, pGAD424-SAP motif #2 constructs were generated as described above. Human EAT-2 was amplified by PCR from cDNA and cloned in the multiple cloning site 1 of a bicistronic vector termed pBRIDGE (Clontech), or the unique cloning site of pGBT9 (Clontech) for two hybrid analysis. See Morra et al., 2001, EMBO J. 20:5840-5852 for a brief description of the assay. For 'three hybrid' analysis a modified *c-fyn* sequence was inserted into the second multiple cloning site of pBRIDGE. *c-fyn* was modified by PCR-mediated mutagenesis by mutating two regulatory tyrosine residues, Y420 and Y531, and the SH2 domain R-Q 176 as previously described (Sayos etal., 1998, Nature 395:462; and Sayos et al., 2001, Blood 97:3867-3874). Conditional expression of the resultant *c-fyn* mutant was driven by the MET25 promoter in response to methionine levels in the medium. In the presence of 1 mM methionine the expression of the protein was repressed, while the absence of methionine in the medium induces protein expression.

Yeast two/three hybrid β-galactosidase assay: Yeast strain Y190 (Clontech) was co-transfected with pGAD424-cytoplasmic, pGAD424-SAP motif#1, or pGAD424-SAP motif #2 and pBRIDGE-EAT-2+ *c-fyn* ^{2Y-F R-Q}. Co-transfectants were selected by growth on solid media lacking tryptophan, leucine (two hybrid assays) and methionine (three hybrid assays) for two days. Selected yeast colonies were then grown in liquid selection media overnight, harvested and liquid β-galactosidase assays were performed as per the Clontech yeast protocols handbook.

### Results:

A modified yeast two-hybrid system, including EAT-2 as 'bait', the cytoplasmic region of CD2000, the CD2000 SAP motif #1, or the CD2000 SAP motif #2 as 'prey', and modified *c-fyn* as a kinase to phosphorylate CD2000, was used to investigate the phosphotyrosine-dependent interaction between cytoplasmic regions of CD2000 and EAT-2. The ability of cytoplasmic regions of CD2000 to interact with EAT-2 was measured by determining β-galactosidase reporter expression. EAT-2 was unable to bind to cytoplasmic regions of CD2000 in the absence of tyrosine phosphorylation. Following the introduction of a modified *c-fyn* into the yeast cells, cytoplasmic regions of CD2000 bind to EAT-2 with high affinity. These results demonstrate that unphosphorylated CD2000 does not bind to EAT-2, but that phosphorylated CD2000 does bind to EAT-2. Thus, CD2000, like SLAM, requires phosphorylation to bind to EAT-2 at detectable levels, as measured by the modified yeast two-hybrid system described above.

### CD2000 Maps to the Cluster of SLAM Related Genes

Alignment of finished and unfinished genomic sequences from BAC clones has allowed a map of the locus surrounding the SLAM gene on chromosome 1q23 to be characterized (Morra et al., 2001, J. Immunol. 151:4614). CD2000 is located next to and in the same orientation as CD84 and then SLAM (Figure 12). The genomic region covers approximately 300 kb and now includes 7 of the known CD2 family members. Of the 7 genes located in this region, 6 contain cytoplasmic tails with potential SAP binding motifs. The exception is CD48, which is the ligand for 2B4, also located in this gene cluster. CD2000 has eight exons and spans 37kb, exon 1 begins approximately 23 kb from the end of CD84's exon VII.

### Uses of CD2000 Nucleic acids, Polypeptides, and Modulators Thereof

CD2000 nucleic acids and polypeptides can be utilized as a marker *(e.g.,* an *in situ* marker) for identification, isolation, depletion, and/or tracking of immune cells *(e.g.,* T cells, (TH1 cells), B cells, dendritic cells, granulocytes, eosinophils, and macrophages) in a sample. CD2000 proteins and nucleic acids encoding CD2000 proteins can be utilized as antigens to make antibodies that can, in turn, be used for identification, isolation, depletion, and/or tracking of the immune cells (*e.g*., T cells, B cells, dendritic cells, granulocytes, eosinophils, and macrophages) or CD2000 in a sample, or to track immune disorders such as immune proliferative disorders (*e.g*., carcinoma, X-linked lymphoproliferative syndrome, and lymphoma, *e.g.*, follicular lymphoma), disorders associated with fighting pathogenic infections (*e.g*., bacterial (*e.g*., chlamydia) infection, parasitic infection, and viral infection (*e.g.,* HSV or HIV infection)), pathogenic disorders *(e.g.,* immunodeficiency disorders, such as HIV), autoimmune disorders (*e.g.*, arthritis, graft rejection, multiple sclerosis, Grave's disease, and Hashimoto's disease), T cell disorders (*e.g.*, AIDS), septicemia, cerebral malaria, inflammatory bowel disease (*e.g.*, Crohn's disease and ulcerative colitis), inflammatory disorders (*e.g.*, rheumatoid arthritis and osteoarthritis), allergic inflammatory disorders *(e.g.,* IgE mediated disorders, asthma and psoriasis), apoptotic disorders *(e.g.,* rheumatoid arthritis, systemic lupus erythematosus, and insulin-dependent diabetes mellitus), cytotoxic disorders, septic shock, chronic obstructive pulmonary disease (COPD; *e.g*., emphysema and chronic bronchitis, bronchial asthma, and bronchiectasis), and cachexia. Further, CD2000 nucleic acids can also be utilized for chromosomal mapping, or as chromosomal markers, *e.g.*, in radiation hybrid mapping.

As CD2000 was originally found in a cDNA library from peripheral blood lymphocytes from a mixed lymphocyte reaction, CD2000 nucleic acids, polypeptides and modulators thereof can be used to modulate the proliferation, development, differentiation, and/or function of immune cells (*e.g*. T cells, B cells, dendritic cells, eosinophils, granulocytes and macrophages), and/or immune function. CD2000 nucleic acids, proteins and modulators thereof can be utilized to modulate immune-related processes, *e.g*., the host immune response by, for example, modulating the formation of and/or binding to immune complexes and immune surveillance for rapid removal or pathogens.

CD2000 nucleic acids, polypeptides and modulators thereof can be utilized to modulate or treat immune disorders that include, but are not limited to, immune proliferative disorders (*e.g*., carcinoma, X-linked lymphoproliferative syndrome, and lymphoma, *e.g*., follicular lymphoma), disorders associated with fighting pathogenic infections *(e.g.,* bacterial *(e.g.,* chlamydia) infection, parasitic infection, and viral infection *(e.g.,* HSV or HIV infection)), pathogenic disorders (*e.g*., immunodeficiency disorders, such as HIV), autoimmune disorders (*e.g.*, arthritis, graft rejection, multiple sclerosis, Grave's disease, and Hashimoto's disease), T cell disorders (*e.g.*, AIDS), septicemia, cerebral malaria, inflammatory bowel disease (*e.g*., Crohn's disease and ulcerative colitis), inflammatory disorders (*e.g*., rheumatoid arthritis and osteoarthritis), allergic inflammatory disorders (*e.g.*, IgE mediated disorders, asthma and psoriasis), apoptotic disorders *(e.g.,* rheumatoid arthritis, systemic lupus erythematosus, and insulin-dependent diabetes mellitus), cytotoxic disorders, septic shock, chronic obstructive pulmonary disease (COPD; *e.g*., emphysema and chronic bronchitis, bronchial asthma, and bronchiectasis), and cachexia.

In view of the importance of immune cells in a subject's immune response to an infection and that CD2000 is expressed by immune cells, CD2000 nucleic acids, polypeptides and modulators thereof can be used to treat or ameliorate symptoms associated with infections by, *e.g.,* viruses, fungi, parasites, and bacteria. Examples of viral infections include, but are not limited, infections by DNA viruses such as herpesviruses *(e.g.,* herpes simplex virus type 1 and 2, varicella-zoster virus, cytomegalovirus, and Epstein-Barr virus), hepatitis B virus, and adenovirus, and viruses such as orthomyxoviruses *(e.g.,* influenza virus A, B and C), paramyxoviruses (*e.g*., respiratory syncytial virus (RSV), parainfluenza virus, measles virus, and mumps virus), togaviruses (*e.g.*, rubella virus), rhabdoviruses *(e.g.,* rabies virus), polioviruses, echoviruses, hepatitis A viruses, hepatitis C viruses, rhinoviruses, reoviruses *(e.g.,* rotaviruses), and retroviruses (*e.g*., HTLV and HIV). Examples of fungal infections include, but are not limited to, infections by *Coccidioides, Histoplasma, Blastomyces, Paracoccidioides, Cryptococcus, Aspergillus,* and *Candida.* Examples of parasitic infections include, but are not limited to, infections by *Entamoeba, Giardia, Cryptosporidium, Trichomonas, Trypanosoma T cruzi, Leishmania, Plasmodium, Toxoplasma*, and *Pneumocystis.* Examples of bacterial infections include, but are not limited to, infections by *Streptococcus pneumoniae, Streptococcus pyogenes, Staphylococcus aureus, Neisseria meingitidis, Bacillus anthracis, Heamophilus influenzae, Klebsiella pneumoniae, Escherichia coli, Salmonella typhi, Yersinia pestis, Corynebacterium diphtheriae, Clostridium tetani, Clostridium botiulinum, Clostridium perfringens, Vibro cholerae, Bordella pertussis, Shigella dysenteriae, Pseudomonas aeruginosa, Legionella pneumophila, Bordetella pertussis, and Mycobacterium tuberculosis.*

As CD2000 has homology to CD2 family members, CD2000 nucleic acids, polypeptides and modulators thereof can be used to modulate cell adhesion between T cells and APCs. Further, as CD2000 has homology to CD2 family members, CD2000 nucleic acids, proteins and modulators thereof can be utilized to modulate immune activation *(e.g.,* activation of T-cells or B cells). For example, antagonists of CD2000 action, such as peptides, antibodies or small molecules that decrease or block CD2000 activity *(e.g.,* by binding to immunoglobulin domains or SAP binding domain motifs), or that prevent CD2000 signaling, can be used as immune system activation blockers. In another example, agonists that mimic or partially mimic CD2000 activity, such as peptides, antibodies or small molecules, can be used to induce immune system activation. Antibodies may activate or inhibit cell adhesion, proliferation and activation, and may help in treating infection, autoimmunity, inflammation, and cancer by affecting these cellular processes. CD2000 nucleic acids, proteins and modulators thereof can also be utilized to modulate intercellular signaling in the immune system, *e.g*., by modulating intercellular signal transduction in immune stimulation or suppression, or by modulating immune cell membrane adhesion to extracellular matrix (ECM) components.

As CD2000 is a transmembrane protein, CD2000 nucleic acids, polypeptides and modulators thereof can be utilized to modulate intracellular signaling cascades (*e.g.*,the association of CD2000 with SLAM-associated protein (SAP), EAT-2, and SHP-2 phosphatase, and the activation of Syk, Fyn, Lyn, phosphatidylinositol 3-kinase, protein kinase C (PKC), and NF-κB).

As CD2000 is expressed at higher levels on TH1 than TH0 or TH2 cells, CD2000 nucleic acids, polypeptides and modulators thereof can be utilized to modulate and/or track the proliferation, development, differentiation, and/or function of TH1 cells. Also, CD2000 nucleic acids, proteins, and modulators thereof can be used to treat and/or ameliorate one or more symptoms associated with a TH1 or TH1-like disorder such as, for example, chronic inflammatory diseases and disorders (*e.g.*, Crohn's disease, reactive arthritis, and Lyme disease), insulin-dependent diabetes, organ specific autoimmunity (including multiple sclerosis, Hashimoto's thryroiditis, and Grave's disease), contact dermatitis, psoriasis, graft rejection, graft versus host disease, and sarcoidosis.

CD2000 nucleic acids, polypeptides and modulators thereof, can be used to modulate the function, morphology, proliferation and/or differentiation of cells in the tissues in which it is expressed (*e.g*., the spleen, liver, lung, lymph nodes, thymus, peripheral blood lymphocytes and bone marrow). Thus, CD2000 nucleic acids, polypeptides and modulators thereof can be used to treat disorders such as asthma, bronchitis, bronchiolitis, cystic fibrosis, sacrcoidosis, idiopathic pulmonary fibrosis, hypersensitivity pneumontis, pneumonia, emphysema, lung cancer, jaundice, hepatic failure, hereditary hyperbiliruinemias *(e.g.,* Gilbert's syndrome, Crigler-Naijar syndromes and Dubin-Johnson and Rotor's syndromes), hepatic circulatory disorders (*e.g*., hepatic vein thrombosis and portal vein obstruction and thrombosis), hepatitis *(e.g.,* chronic active hepatitis, acute viral hepatitis, and toxic and drug-induced hepatitis), cirrhosis *(e.g.,* alcoholic cirrhosis, biliary cirrhosis, and hemochromatosis), malignant tumors (*e.g*., primary carcinoma, hepatoma, hepatoblastoma, liver cysts, and angiosarcoma), hepatic vein thrombosis, lymphoma, leukemia, colon cancer, amyloidosis, scleroderma, mastocytosis, ulcerative colitis, Crohn's disease, splenic lymphoma, splenomegaly, phagocytotic disorders, acute myeloid leukemia, hemophilia, leukemia, anemia (*e.g.*, sickle cell anemia), and thalassemia.

Further, as CD2000 includes immunoglobulin domains, CD2000 nucleic acids, polypeptides and modulators thereof can be used to treat disorders involving an immune, allergic or autoimmune response (*e.g.*, arthritis, multiple sclerosis, meningitis, encephalitis, atherosclerosis, or infection).

### Human CD2001

The cDNA encoding human CD2001 is 1139 nucleotides long (Figures 12; SEQ ID NO:74). The open reading frame of this cDNA, nucleotides 85 to 954 of SEQ ID NO:74 (SEQ ID NO:75), encodes a 289 amino acid transmembrane protein (Figures 13; SEQ ID NO:76) that, as discussed below, represents a CD2 family member.

The signal peptide prediction program SIGNALP (Nielsen, et al., 1997, *Protein Engineering* 10:1-6) predicted that human CD2001 includes a 19 amino acid signal peptide (amino acid 1 to about amino acid 19 of SEQ ID NO:76; SEQ ID NO:78) preceding the mature human CD2001 protein (corresponding to about amino acid 20 to amino acid 198 of SEQ ID NO:76; SEQ ID NO:77).

Human CD2001 is a transmembrane protein that is a CD2 family member expressed on lymphocytes which consists of one or more of the following domains: (1) an extracellular domain; (2) a transmembrane domain; and (3) a cytoplasmic domain. The mature human CD2001 protein contains an extracellular domain at about amino acid residues 20 to 236 of SEQ ID NO:76 (SEQ ID NO:79), a transmembrane domain at about amino acid residues 237 to 263 of SEQ ID NO:76 (SEQ ID NO:80), and a cytoplasmic domain at about amino acid residues 264 to 289 of SEQ ID NO:76 (SEQ ID NO:81). Human CD2001 comprises an immunoglobulin-like domain at about amino acid residues 35 to 132 of SEQ ID NO:76 (SEQ ID NO:96) and an immunoglobulin domain at about amino acid residues 135 to 204 of SEQ IDN O:76 (SEQ ID NO:97).

Human CD2001 has homology to CD2 family members. In particular, CD2001 exhibits homology to CD2000. The amino acid sequence of human CD2001 is 28.9% identical to the amino acid sequence of human CD84. The amino acid sequence of human CD2001 is 54.7% identical to the amino acid sequence encoded by the mouse gene BAB26328 (GenBank Accession No. AK009505; Figure 14).

The nucleotide and amino acid sequences of human CD2001 (SEQ ID NOs: 74 and 76, respectively) are identical to the nucleotide and amino acid sequences of CD84-H1 (GenBank Accession Nos. AF275725 and AAK69052, respectively) described by Zhang et al., 2001, Clin. Cancer Res. 7(3 Suppl):822s-829s. Human CD84-H1 is expressed by immune cells, including dendritic cells, B cells and T cells (Zhang et al., 2001, Clin. Cancer Res. 7(3 Suppl):822s-829s) and has been suggested to important in immune responses.

RT-PCR analysis was performed using the following primers to detect human CD2001 expression in various human tissue samples:
forward primer 5' ATGTGTGCCTTTCCTTGGCTGCTTCTTC 3' (SEQ ID NO:98) and reverse primer 5' CCG TTT CGG ACC GAG GTC GGG ACG GAC T 3' (SEQ ID NO: 99). Human CD2001 expression was detected in fetal liver, liver, placenta, pancreas and the following immune tissues: the spleen, lymph node, thymus, and tonsil. Low levels of CD2001 were detected in peripheral blood leukocytes (PBLs), bone marrow, heart, brain, lung, skeletal muscle, and kidney (Figure 14).

### CD2001 Maps to the Cluster of SLAM Related Genes

CD2001 is contained within three human pBAC clones (AL513485, AL513302, and AC357565) in the proximity of BLAME, a SLAM family member (Kingsbury et al., 2001, J. Immunol. 166:5675-5680). The five exons of BLAME map to pBAC AL590560 and exons 2 to five are found at the end of pBAC AL513485, which contains the complete human CD2001. CD2001 has four exons and the intron/exon boundaries are similar to the murine gene BAB26328. The region that contains CD2001 and BLAME has been mapped to chromosome 1q21 (Figure 15).

### Uses of CD2001 Nucleic acids, Polypeptides, and Modulators Thereof

CD2001 nucleic acids and polypeptides can be utilized as a marker *(e.g.,* an *in situ* marker) for identification, isolation, depletion, and/or tracking of immune cells *(e.g.,* T cells, (TH1 cells), B cells, dendritic cells, granulocytes, eosinophils, and macrophages) in a sample. CD2001 proteins and nucleic acids encoding CD2001 proteins can be utilized as antigens to make antibodies that can, in turn, be used for identification, isolation, depletion, and/or tracking of the immune cells (*e.g*., T cells, B cells, dendritic cells, granulocytes, eosinophils, and macrophages) or CD2001 in a sample, or to track immune disorders such as immune proliferative disorders *(e.g.,* carcinoma, X-linked lymphoproliferative syndrome, and lymphoma, *e.g*., follicular lymphoma), disorders associated with fighting pathogenic infections (*e.g*., bacterial (*e.g*., chlamydia) infection, parasitic infection, and viral infection *(e.g.,* HSV or HIV infection)), pathogenic disorders (*e.g*., immunodeficiency disorders, such as HIV), autoimmune disorders (*e.g*., arthritis, graft rejection, multiple sclerosis, Grave's disease, and Hashimoto's disease), T cell disorders (*e.g*., AIDS), septicemia, cerebral malaria, inflammatory bowel disease (*e.g*., Crohn's disease and ulcerative colitis), inflammatory disorders (*e.g*., rheumatoid arthritis and osteoarthritis), allergic inflammatory disorders *(e.g.,* IgE mediated disorders, asthma and psoriasis), apoptotic disorders *(e.g.,* rheumatoid arthritis, systemic lupus erythematosus, and insulin-dependent diabetes mellitus), cytotoxic disorders, septic shock, chronic obstructive pulmonary disease (COPD; e.g., emphysema and chronic bronchitis, bronchial asthma, and bronchiectasis), and cachexia. Further, CD2001 nucleic acids can also be utilized for chromosomal mapping, or as chromosomal markers, *e.g.*, in radiation hybrid mapping.

CD2001 nucleic acids, polypeptides and modulators thereof can be used to modulate the proliferation, development, differentiation, and/or function of immune cells (*e.g.* T cells, B cells, dendritic cells, eosinophils, granulocytes and macrophages), and/or immune function. CD2001 nucleic acids, proteins and modulators thereof can be utilized to modulate immune-related processes, *e.g.*, the host immune response by, for example, modulating the formation of and/or binding to immune complexes and immune surveillance for rapid removal or pathogens.

CD2001 nucleic acids, polypeptides and modulators thereof can be utilized to modulate or treat immune disorders that include, but are not limited to, immune proliferative disorders *(e.g.,* carcinoma, X-linked lymphoproliferative syndrome, and lymphoma, *e.g.,* follicular lymphoma), disorders associated with fighting pathogenic infections (*e.g.*, bacterial (*e.g*., chlamydia) infection, parasitic infection, and viral infection *(e.g.,* HSV or HIV infection)), pathogenic disorders (*e.g.*, immunodeficiency disorders, such as HIV), autoimmune disorders (*e.g.*, arthritis, graft rejection, multiple sclerosis, Grave's disease, and Hashimoto's disease), T cell disorders (*e.g.*, AIDS), septicemia, cerebral malaria, inflammatory bowel disease (*e.g*., Crohn's disease and ulcerative colitis), inflammatory disorders *(e.g.,* rheumatoid arthritis and osteoarthritis), allergic inflammatory disorders (*e.g*., IgE mediated disorders, asthma and psoriasis), apoptotic disorders (*e.g*., rheumatoid arthritis, systemic lupus erythematosus, and insulin-dependent diabetes mellitus), cytotoxic disorders, septic shock, chronic obstructive pulmonary disease (COPD; *e.g.*, emphysema and chronic bronchitis, bronchial asthma, and bronchiectasis), and cachexia.

In view of the importance of immune cells in a subject's immune response to an infection and that CD2001 is expressed by immune cells, CD2001 nucleic acids, polypeptides and modulators thereof can be used to treat or ameliorate symptoms associated with infections by, *e.g.*, viruses, fungi, parasites, and bacteria. Examples of viral infections include, but are not limited, infections by DNA viruses such as herpesviruses (*e.g.*, herpes simplex virus type 1 and 2, varicella-zoster virus, cytomegalovirus, and Epstein-Barr virus), hepatitis B virus, and adenovirus, and viruses such as orthomyxoviruses *(e.g.,* influenza virus A, B and C), paramyxoviruses (*e.g*., respiratory syncytial virus (RSV), parainfluenza virus, measles virus, and mumps virus), togaviruses *(e.g.,* rubella virus), rhabdoviruses *(e.g.,* rabies virus), polioviruses, echoviruses, hepatitis A viruses, hepatitis C viruses, rhinoviruses, reoviruses (*e.g*., rotaviruses), and retroviruses *(e.g.,* HTLV and HIV). Examples of fungal infections include, but are not limited to, infections by *Coccidioides, Histoplasma, Blastomyces, Paracoccidioides, Cryptococcus, Aspergillus*, and *Candida.* Examples of parasitic infections include, but are not limited to, infections by *Entamoeba, Giardia, Cryptosporidium, Trichomonas, Trypanosoma T cruzi, Leishmania, Plasmodium, Toxoplasma,* and *Pneumocystis*. Examples of bacterial infections include, but are not limited to, infections by *Streptococcus pneumoniae, Streptococcus pyogenes, Staphylococcus aureus, Neisseria meingitidis, Bacillus anthracis, Heamophilus influenzae, Klebsiella pneumoniae, Escherichia coli, Salmonella typhi, Yersinia pestis, Corynebacterium diphtheriae, Clostridium tetani, Clostridium botiulinum, Clostridium perfringens, Vibro cholerae, Bordella penussis, Shigella dysenteriae, Pseudomonas aeruginosa, Legionella pneumophila, Bordetella pertussis, and Mycobacterium tuberculosis.*

As CD2001 has homology to CD2 family members, CD2001 nucleic acids, polypeptides and modulators thereof can be used to modulate cell adhesion between T cells and APCs. Further, as CD2000 has homology to CD2 family members, CD2001 nucleic acids, proteins and modulators thereof can be utilized to modulate immune activation *(e.g.,* activation of T-cells or B cells). For example, antagonists of CD2001 action, such as peptides, antibodies or small molecules that decrease or block CD2001 activity *(e.g.,* by binding to immunoglobulin-like domains), or that prevent CD2001 signaling, can be used as immune system activation blockers. In another example, agonists that mimic or partially mimic CD2001 activity, such as peptides, antibodies or small molecules, can be used to induce immune system activation. Antibodies may activate or inhibit cell adhesion, proliferation and activation, and may help in treating infection, autoimmunity, inflammation, and cancer by affecting these cellular processes. CD2001 nucleic acids, proteins and modulators thereof can also be utilized to modulate intercellular signaling in the immune system, *e.g.,* by modulating intercellular signal transduction in immune stimulation or suppression, or by modulating immune cell membrane adhesion to extracellular matrix (ECM) components.

As CD2001 is a transmembrane protein, CD2001 nucleic acids, polypeptides and modulators thereof can be utilized to modulate intracellular signaling cascades (*e.g.*, the association of CD2001 with SHP-2 phosphatase, and the activation of Syk, Fyn, Lyn, phosphatidylinositol 3-kinase, protein kinase C (PKC), and NF-κB).

CD2001 nucleic acids, polypeptides and modulators thereof, can be used to modulate the function, morphology, proliferation and/or differentiation of cells in the tissues in which it is expressed *(e.g.,* the spleen, liver, lung, lymph nodes, thymus, peripheral blood lymphocytes and bone marrow). Thus, CD2001 nucleic acids, polypeptides and modulators thereof can be used to treat disorders such as asthma, bronchitis, bronchiolitis, cystic fibrosis, sacrcoidosis, idiopathic pulmonary fibrosis, hypersensitivity pneumontis, pneumonia, emphysema, lung cancer, jaundice, hepatic failure, hereditary hyperbiliruinemias *(e.g.,* Gilbert's syndrome, Crigler-Naijar syndromes and Dubin-Johnson and Rotor's syndromes), hepatic circulatory disorders *(e.g.,* hepatic vein thrombosis and portal vein obstruction and thrombosis), hepatitis *(e.g.,* chronic active hepatitis, acute viral hepatitis, and toxic and drug-induced hepatitis), cirrhosis *(e.g.,* alcoholic cirrhosis, biliary cirrhosis, and hemochromatosis), malignant tumors *(e.g.,* primary carcinoma, hepatoma, hepatoblastoma, liver cysts, and angiosarcoma), hepatic vein thrombosis, lymphoma, leukemia, colon cancer, amyloidosis, scleroderma, mastocytosis, ulcerative colitis, Crohn's disease, splenic lymphoma, splenomegaly, phagocytotic disorders, acute myeloid leukemia, hemophilia, leukemia, anemia *(e.g.,* sickle cell anemia), and thalassemia.

Further, as CD2001 includes immunoglobulin and immunoglobulin-like domains, CD2001 nucleic acids, polypeptides and modulators thereof can be used to treat disorders involving an immune, allergic or autoimmune response *(e.g.,* arthritis, multiple sclerosis, meningitis, encephalitis, atherosclerosis, or infection).

### Assays for the Detection of CD2000 & CD2001 Expression or Activity

The expression of CD2000 and/or CD2001 can be readily detected, *e.g.,* by quantifying CD2000 or CD2001 protein and/or RNA. Many methods standard in the art can be thus employed, including, but not limited to, immunoassays to detect and/or visualize gene expression (*e.g*., Western blot, immunoprecipitation followed by sodium dodecyl sulfate polyacrylamide gel electrophoresis (SDS-PAGE), immunocytochemistry, etc.) and/or hybridization assays to detect gene expression by detecting and/or visualizing, respectively, mRNA encoding a gene (*e.g*., Northern assays, dot blots, *in situ* hybridization, etc.), etc. The ability of CD2000 or CD2001 to bind to antibodies or fragments thereof (*e.g*., Fab fragments) can readily be detected using techniques known to those of skill in the art.

The activity of a CD2000 protein or CD2001 protein can be measured by employing methods known to those of skill in the art. For example, the activity of a CD2000 protein can be analyzed by treating T cells or CD2000-transfected cells with an antibody or a fragment thereof *(e.g.,* a Fab fragment) and measuring the effect of such treatment on the level of tyrosine phosphorylation of signaling molecules, such as CD2000, Syk, Lck, Fyn, Zap-70, PLC-γ1 phosphatidylinositol 3-kinase, and Lyn *(e.g.,* tyrosine phosphorylation can be detected by immunoprecipitation followed by SDS-PAGE, kinase assays, etc.). The activity of a CD2000 protein can also be analyzed by treating T cells or CD2000-transfected cells with an antibody or fragment thereof and measuring NF-κB activiation using techniques known to those of skill in the art (*e.g.*, electrophoretic mobility shift assays). Further, the activity of a CD2000 protein can be analyzed by measuring changes in the concentration of free intracellular Ca²⁺ induced by the treatment of T cells or CD2000-transfected cells with antibody or a fragment thereof *(e.g.,* a Fab fragment). Briefly, T cells or CD2000-transfected cells are incubated with fura-2 fluorescence at 37°C and, then incubated with 2mM CaCl₂ prior to incubation with antibody or a fragment thereof (*e.g.*, a Fab fragment). The cells are lysed in lysis buffer, and the concentration of free intracellular Ca²⁺ is measured by fluorescence at 37°C using a spectrophotometer (see, *e.g.,* Jandrot-Perrus et al., 1997, *J*. *of Biol. Chem.* 272:27035-27041).

The activity of a CD2000 protein or CD2001 protein can also be analyzed by measuring T cell activation and proliferation using techniques known to those of skill in the art. For example, T cell activation can be measured by assessing the expression of T cell activation markers such as CD28 and ICOS, and T cell proliferation can be measured using ³H-thymidine uptake assays. Further, the activity of a CD2000 protein or CD2001 protein can be analyzed by measuring the expression of such molecules as cytokines (*e.g*., IFN-α, IFN-β, IFN-γ, IL-2, IL-4, IL-5, IL-6, IL-10, IL-12, IL-15, and IL-18) using techniques known to those of skill in the art. For example, the expression of T cell activation markers and cytokines can be measured by Northern blot analysis, RT-PCR and immunoassays such as, *e.g.,* ELISAs and Western blot analysis.

Such assays maybe utilized as part of CD2000 or CD2001 diagnostic assays. In addition, such assays may be utilized as part of screening methods for identifying compounds that modulate the activity and/or expression of CD2000 or CD2001.

### Assays for Analysis of CD2000 & CD2001 Modulators

A variety of assays can be utilized to analyze a CD2000 protein, nucleic acid or modulator thereof, or a CD2001 protein, nucleic acid or modulator thereof. Such assays include, *e.g*., *in vivo, ex vivo* and *in vitro* assays, as described herein.

For example, in view of the fact that CD2000 and CD2001 are cell surface receptors, in particular, CD2 family members, standard quantitative binding studies can be utilized to measure modulator binding to immune cells. (See, *e.g.,* Horton (ed), 1995, Adhesion Receptors as Therapeutic Targets, Chapter 15, CRC Press, Inc.: London, United Kingdom.) Such binding assays can also be utilized to perform receptor blockade studies to measure the number of cellular sites available for binding modulator by comparing the number of molecules of labeled modulator molecules (*e.g.,* labeled anti-CD2000 antibodies or labeled anti-CD2001 antibodies) bound per immune cell *(e.g.,* T cell) at a series of concentrations with the number of modulator molecules bound at saturation.

The activation of signal transduction pathways can be analyzed in *in vitro* and *ex vivo* assays using assays known to those of skill in the art. For example, the activation of signal transduction pathways *in vitro* can be analyzed by contacting immune cells such as T cells or CD2000-transfected cells with a CD2000 modulator (*e.g.*, an antibody or a fragment thereof) or a control *(e.g.,* PBS) and measuring the effect of such treatment on the level of tyrosine phosphorylation of signaling molecules, such as CD2000, Syk, Lck, Fyn, Zap-70, PLC-γ1, phosphatidylinositol 3-kinase, and PKC (*e.g.*, tyrosine phosphorylation can be detected by immunoprecipitation followed by SDS-PAGE, kinase assays, etc.). Further, the activation of signal transduction pathways *in vitro* can be analyzed by contacting immune cells such as T cells or CD2000-transfected cells with a CD2000 modulator *(e.g.,* an antibody or a fragment thereof) or a control *(e.g.,* PBS) and measuring the effect of such treatment on the association of SLAM-associated protein (SAP), EAT-2 and/or SHP-2 phosphatase with CD2000. In an *ex vivo* assay, the activation of signal transduction pathways can be analyzed by contacting immune cells (*e.g*., T cells) obtained from individuals treated with a CD2000 modulator or a placebo with antibodies and measuring the effect of such treatment on the level of tyrosine phosphorylation of signaling molecules, such as CD2000, Syk, Lck, Fyn, Zap-70, PLC-γ1, phosphatidylinositol 3-kinase, and PKC (*e.g*., tyrosine phosphorylation can be detected by immunoprecipitation followed by SDS-PAGE, kinase assays, etc.). The effect of CD2000 modulators on cytokine expression and T cell activation markers can also be analyzed, e.g., by RT-PCR, Western blot analysis, ELISA, etc. Further, the effect of CD2000 modulators on calcium mobilization can be analyzed by measuring changes in the concentration of free intracellular Ca²⁺ induced in *in vitro* and *ex vivo* assays using assays known to those of skill in the art.

The efficacy of CD2000 modulators or CD2001 modulators can be assessed in a variety of animal models including, but not limited to, mice overexpressing a particular type of T cell activation marker (*e*.*g*., CD28, SLAM, BLAME, Ly9, CD84, CD48, CD58, D-SLAM, 2B4, or 19A), mice deficient in a particular type of T cell activation marker (*e.g*., CD28, SLAM, BLAME, Ly9, CD84, CD48, CD58, D-SLAM, 2B4, or 19A), mice overexpressing a signaling molecule (*e.g*., Fyn, Lck, SHP-2 phosphatase, SAP, EAT-2, PI 3-kinase), mice deficient in a signaling molecule *(e.g.,* Fyn, Lck, SHP-2 phosphatase, SAP, EAT-2, PI 3-kinase), and mice deficient in a particular type of immune cell *(e.g.,* T cells, natural killer cells, B cells, granulocytes, eosinophils, monocytes, or macrophages). The efficacy of CD2000 modulators or CD2001 modulators can also be tested in such autoimmune disorder models as an experimental allergic encephalomyelitis (EAE) model. EAE is an experimental autoimmune disease of the central nervous system (CNS) (Zamvil et al, 1990, Ann. Rev, Immunol. 8:579) and is a disease model for the human autoimmune condition, multiple sclerosis (MS). EAE is an example of a cell-mediated autoimmune disorder that is mediated via T cells. EAE is readily induced in mammalian species by immunizations of myelin basic protein (MBP) purified from the CNS or an encephalitogenic proteolipid (PLP). SJL/J mice are a susceptible strain of mice (H-2^{u}) and, upon induction of EAE, these mice develop an acute paralytic disease and an acute cellular infiltrate is identifiable within the CNS. EAE spontaneously develops in MBP₁₋₁₇ peptide-specific T cell receptor (TCR) transgenic mice (TgMBP⁺) of a RAG-1-deficient background (Lafaille et al., 1994, Cell 78:399).

A collagen-induced arthritis (CIA) model can also be utilized to determine the efficacy of CD2000 modulators or CD2001 modulators. CIA is an animal model for the human autoimmune disease rheumatoid arthritis (RA) (Trenthorn et al., 1977, J. Exp. Med.146:857). This disease can be induced in many species by the administration of heterologous type II collagen (Courtenay et al., 1980, Nature 283:665; and Cathcart et at, 1986, Lab. Invest.54:26). With respect to animal models of arthritis see, in addition, *e.g.*, Holmdahl, R., 1999, Curr. Biol. 15:R528-530.

Animal models for chronic obstructive pulmonary disease (COPD) can also be used to determine the efficacy of CD2000 modulators or CD2001 modulators (for review of COPD animal models see, *e.g.,* Shapiro, 2000, Am. J. Respir. Cell Mol. Biol. 22:4-7; and Shapiro, 2000, Chest 117:2223S-227S). COPD is a generic term for several clinical syndromes including, but not limit to, emphysema and chronic bronchitis. Emphysema can be induced in animals such as mice by the administration or overexpression of elasolytic enzymes including, but not limited to pancreatic elastase, neutrophil elastase, and proteinase 3. Emphysema can also be induced by the administration of a variety of chemicals and irritants including, but not limited to, lipopolysaccharides (LPS), cadmium chloride, nitrogen dioxide, inorganic dust, and ozone. Further, emphysema can be induced by overexpression of interferon-γ (IFN-γ). Cigarette smoke-related COPD in mice can be induced by chronic exposure to cigarette smoke. Pulmonary fibrosis can be induced by the administration of bleomycin to mice. Further, mutant mouse strains such as tight skin (Tsk^{+/-}), pallid (pa/pa), blotchy (Blo), mice transgenic for collagenase, and PDGF-A^{-/-} that spontaneously develop enlarged airspaces can also be used to determine the therapeutic efficacy of CD2000 modulators or CD2001 modulators.

Animal models for inflammatory bowel disease (IBD) can also be used to determine the efficacy of CD2000 modulators or CD2001 modulators (for review see, *e.g.*, Kim et al., 1992, Scand. J. Gastroentrol. 27:529-537; and Strober, 1985, Dig. Dis. Sci. 30(12 Suppl.):3S-10S. Crohn's disease and ulcerative colitis are two types of human IBDs. IBD can be induced in animals by oral administration of sulfated polysaccharides including, but not limited to, carrageenan, amylopectin sulfate, and dextran sulfate. IBD can also be induced by the administration of chemical irritants such as trinitrobenzenesulphonic acid (TNBS) and acetic acid.

Further, animal models such as the adoptive transfer model described, *e.g.,* in L. Cohn et al., 1997, J. Exp. Med. 186:1737-1747 can be used to determine the efficacy of CD2000 modulators or CD2001 modulators. In such an animal system, aeroallergen provocation of TH1 or TH2 recipient mice results in TH effector cell migration to the airways and is associated with an intense neutrophilic (TH1) and eosinophilic (TH2) lung mucosal inflammatory response. The animal model represents an accepted model for asthma.

Still further, animal models for type 1 diabetes, thyroid autoimmunity or systemic lupus erythematosus, including glomerulonephritis can be utilized to determine the efficacy of CD2000 modulators or CD2001 modulators (see, *e.g.,* Flanders et al., 1999, Autoimmunity 29:235-246; Krogh et al., 1999, Biochimie 81:511-515; and Foster, N.H., 1999, Semin. Nephrol. 19:12-24, respectively).

Tables 1 and 2 below provide a summary of the sequence information for CD2000.

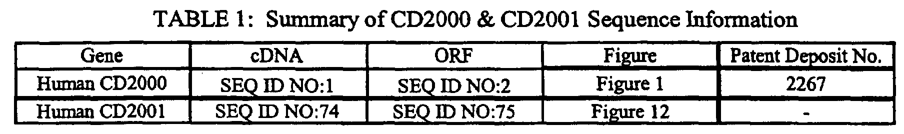

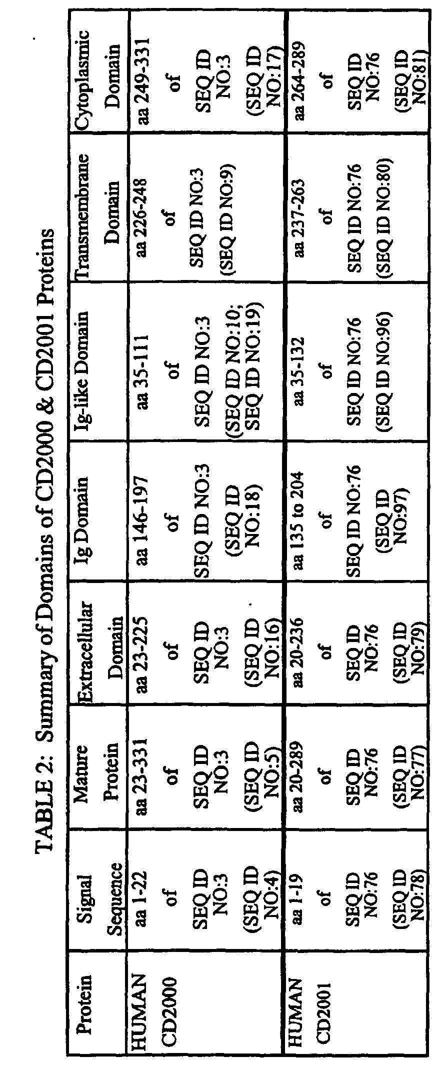

Various aspects of the invention are described in further detail in the following subsections:

### I. Isolated Nucleic Acid Molecules

One aspect of the invention pertains to isolated nucleic acid molecules that encode a polypeptide of the invention, as well as nucleic acid molecules sufficient for use as hybridization probes to identify the presence or level of nucleic acid molecules encoding a polypeptide of the invention, including nucleic acid molecules suitable for use as PCR primers for the amplification or mutation of nucleic acid molecules.

As used herein, the term "nucleic acid molecule" is intended to include DNA molecules (*e.g*., cDNA or genomic DNA), RNA molecules *(e.g.,* mRNA), combinations of DNA and RNA molecules or hybrid DNA/RNA molecules, and analogs of DNA or RNA molecules. Such analogs can be generated using, for example, nucleotide analogs, which include, but are not limited to, inosine or tritylated bases. Such analogs can also comprise DNA or RNA molecules comprising modified backbones that lend beneficial attributes to the molecules such as, for example, nuclease resistance or an increased ability to cross cellular membranes. The nucleic acid molecule can be single-stranded, double-stranded, may contain both single-stranded and double-stranded portions, and may contain triple-stranded portions, but preferably is double-stranded DNA. In one embodiment, the nucleic acid molecules of the invention comprise a contiguous open reading frame encoding a polypeptide of the invention, *e.g.,* a cDNA molecule, or the open reading frame of a polypeptide of the invention as present on a cDNA molecule.

An "isolated" nucleic acid molecule is one which is separated from other nucleic acid molecules which are present in the natural source of the nucleic acid molecule. Preferably, an "isolated" nucleic acid molecule is free of sequences (preferably protein encoding sequences) which naturally flank the nucleic acid *(i.e.,* sequences located at the 5' and 3' ends of the nucleic acid) in the genomic DNA of the organism from which the nucleic acid is derived. For example, in various embodiments, the isolated nucleic acid molecule can contain less than about 5 kB, 4 kB, 3 kB, 2 kB, 1 kB, 0.5 kB or 0.1 kB of nucleotide sequences which naturally flank the nucleic acid molecule in genomic DNA of the cell from which the nucleic acid is derived. The term "isolated" nucleic acid molecule can refer to a nucleic acid molecule of the invention that lacks intron sequences, such as a cDNA molecule. Moreover, an "isolated" nucleic acid molecule can be substantially free of other cellular material, or culture medium when produced by recombinant techniques, or substantially free of chemical precursors or other chemicals when chemically synthesized. As used herein, the term "isolated"when referring to a nucleic acid molecule does not include an isolated chromosome.

A nucleic acid molecule of the present invention, *e.g.*, a nucleic acid molecule comprising the nucleotide sequence of SEQ ID NO:1, 2, 31, 32, 33, 34, 35, 36, 37, 38, 43, 44, 45, 46, 47, 48, 49, 50, 51, 52, 53, 54, 55, 56, 57, 58, 66, 67, 74, 75, 83, 84, 85, 86, 87, 92, 93, 94 or 95, or a complement thereof, can be isolated using standard molecular biology techniques and the sequence information provided herein. Using all or a portion of the nucleic acid sequences of SEQ ID NO:1, 2, 31, 32, 33, 34, 35, 36, 37, 38, 43, 44, 45, 46, 47, 48, 49, 50, 51, 52, 53, 54, 55, 56, 57, 58, 66, 67, 74, 75, 83, 84, 85, 86, 87, 92, 93, 94 or 95 as a hybridization probe, nucleic acid molecules of the invention can be isolated using standard hybridization and cloning techniques (*e.g.*, as described in Sambrook et al., eds., *Molecular Cloning: A Eaboratory Manual, 2nd ed., Cold Spring Harbor Laboratory,* Cold Spring Harbor Laboratory Press, Cold Spring Harbor, NY, 1989).

A nucleic acid molecule of the invention can be amplified using, for example, cDNA, mRNA or genomic DNA as a template and appropriate oligonucleotide primers according to standard PCR amplification techniques. The nucleic acid so amplified can be cloned into an appropriate vector and characterized by DNA sequence analysis. Furthermore, oligonucleotides corresponding to all or a portion of a nucleic acid molecule of the invention can be prepared by standard synthetic techniques, *e.g.*, using an automated DNA synthesizer.

In a preferred embodiment, an isolated nucleic acid molecule of the invention comprises a nucleic acid molecule which is a complement of the nucleotide sequence of SEQ ID NO:1, 2, 31, 32, 33, 34, 35, 36, 37, 38, 43, 44, 45, 46, 47, 48, 49, 50, 51, 52, 53, 54, 55, 56, 57, 58, 66 or 67, or the nucleotide sequence of the cDNA insert of a EpCD2000 clone deposited with the ATCC® as patent deposit Number PTA-2267, or a portion thereof. In another preferred embodiment, an isolated nucleic acid molecule of the invention comprises a nucleic acid molecule which is a complement of the nucleotide sequence of SEQ ID NO:66, 67, 74, 75, 83, 84, 85, 86, 87, 92, 93, 94 or 95. A nucleic acid molecule which is complementary to a given nucleotide sequence is one which is sufficiently complementary to the given nucleotide sequence that it can hybridize to the given nucleotide sequence, thereby forming a stable duplex, under one of the hybridization conditions discussed herein. In one embodiment, the complementary nucleic acid molecule is 100 % complementary to the nucleotide sequence with which it forms the stable duplex. In another embodiment, the complementary nucleic acid molecule is complementary over its entire length to the nucleotide sequence with which it forms the stable duplex. In yet another embodiment, the nucleotide sequence forms a stable duplex over its entire length with the complementary nucleic acid molecule of the invention.

Moreover, a nucleic acid molecule of the invention can comprise only a portion of a nucleic acid sequence encoding a full length polypeptide of the invention, for example, a fragment which can be used as a probe or primer or a fragment encoding a biologically active portion of a polypeptide of the invention. The nucleotide sequence determined from the cloning of one gene allows for the generation of probes and primers designed for use in identifying and/or cloning homologs in other cell types, e.g., from other tissues, as well as homologs from other mammals. The probe/primer typically comprises substantially purified oligonucleotide.

In one embodiment, an oligonucleotide comprises a region of nucleotide sequence that hybridizes under stringent conditions to at least about 12, preferably about 15, more preferably about 25, 50, 75, 100, 125, 150, 175, 200, 250, 300, 350 or 400 consecutive nucleotides of the sense or antisense sequence of SEQ ID NO:1, 2, 31, 32, 33, 34, 35, 36, 37, 38, 43, 44, 45, 46, 47, 48, 49, 50, 51, 52, 53, 54, 55, 56, 57, 58, 66 or 67, or the nucleotide sequence of the cDNA insert of a EpCD2000 clone deposited with the ATCC® as patent deposit Number PTA-2267, or of a naturally occurring mutant of SEQ ID NO:1, 2, 31, 32, 33, 34, 35, 36, 37, 38, 43, 44, 45, 46, 47, 48, 49, 50, 51, 52, 53, 54, 55, 56, 57, 58, 66 or 67. In another embodiment, an oligonucleotide comprises a region of nucleotide sequence that hybridizes under stringent conditions to at least 25, preferably at least 50, 75, 100, 125, 150, 175, 200, 225, 250, 275, 300, 325, 350, 375, 450, 500, 530, 550, 600, 700, 800, 900, 1000 or 1150 consecutive oligonucleotides of the sense or antisense sequence of SEQ ID NO:1, 2, 31, 32, 33, 34, 35, 36, 37, 38, 43, 44, 45, 46, 47, 48, 49, 50, 51, 52, 53, 54, 55, 56, 57, 58, 66 or 67, or the nucleotide sequence of the cDNA insert of a EpCD2000 clone deposited with the ATCC® as patent deposit Number PTA-22 67, or a naturally occurring mutant of SEQ ID NO:1, 2, 31, 32, 33, 34, 35, 36, 37, 38, 43, 44, 45, 46, 47, 48, 49, 50, 51, 52, 53, 54, 55, 56, 57, 58, 66 or 67.

In another embodiment, an oligonucleotide comprises at least 12, preferably about 15, more preferably about 25, 50, 75, 100, 125, 150, 175, 200, 250, 300, 350 or 400 consecutive nucleotides of the sense or antisense sequence of SEQ ID NO:74 or 75. In yet another embodiment, an oligonucleotide comprises at least 12, preferably about 15, more preferably about 25, 50, 75, 100, 125, 150, 175, 200, 250, 300, 350 or 400 consecutive nucleotides of the sense or antisense sequence of SEQ ID NO:74, 75, 82, 92, 93, 94 or 95.

Probes based on the sequence of a nucleic acid molecule of the invention can be used to detect transcripts or genomic sequences encoding the same protein molecule encoded by a selected nucleic acid molecule. The probe comprises a label group attached thereto either directly or indirectly, *e.g.*, a radioisotope, a fluorescent compound, an enzyme, or an enzyme co-factor. Such probes can be used as part of a diagnostic test kit for identifying cells or tissues which express or mis-express the protein, such as by measuring levels of a nucleic acid molecule encoding the protein in a sample of cells from a subject, *e.g.*, detecting mRNA levels or determining whether a gene encoding the protein has been mutated or deleted.

A nucleic acid fragment encoding a biologically active portion of a polypeptide of the invention can be prepared by isolating a portion of any of SEQ ID NO:1, 2, 31, 32, 33, 34, 35, 36, 37, 38, 43, 44, 45, 46, 47, 48, 49, 50, 51, 52, 53, 54, 55, 56, 57, 58, 66, 67, 74, 75, 83, 84, 85, 86, 87, 92, 93, 94 or 95 expressing the encoded portion of the polypeptide protein (*e.g*., by recombinant expression *in vitro)* and assessing the activity of the encoded portion of the polypeptide.

The invention further encompasses nucleic acid molecules that differ from the nucleotide sequence of SEQ ID NO:1, 2, 31, 32, 33, 34, 35, 36, 37, 38, 43, 44, 45, 46, 47, 48, 49, 50, 51, 52, 53, 54, 55, 56, 57, 58, 66, 67, 74, 75, 83, 84, 85, 86, 87, 92, 93, 94 or 95 due to degeneracy of the genetic code and thus encode the same protein as that encoded by the nucleotide sequence of SEQ ID NO:1, 2, 31, 32, 33, 34, 35, 36, 37, 38, 43, 44, 45, 46, 47, 48, 49, 50, 51, 52, 53, 54, 55, 56, 57, 58, 66, 67, 74, 75, 83, 84, 85, 86, 87, 92, 93, 94 or 95.

In addition to the nucleotide sequences of SEQ ID NO:1, 2, 31, 32, 33, 34, 35, 36, 37, 38, 43, 44, 45, 46, 47, 48, 49, 50, 51, 52, 53, 54, 55, 56, 57, 58, 66, 67, 74, 75, 83, 84, 85, 86, 87, 92, 93, 94 or 95 it will be appreciated by those skilled in the art that DNA sequence polymorphisms that lead to changes in the amino acid sequence may exist within a population (*e.g*., the human population). Such genetic polymorphisms may exist among individuals within a population due to natural allelic variation. An allele is one of a group of genes which occur alternatively at a given genetic locus. For example, human CD2000 has been mapped to chromosome 1q23, and therefore CD2000 family members can include nucleotide sequence polymorphisms (*e.g*., nucleotide sequences that vary from SEQ ID NO:1 and SEQ ID NO:2) that map to this chromosomal locus *(e.g.,* region of chromosome 1q23) and such sequences represent CD2000 allelic variants. Human CD2001 has been mapped to chromosome 1q21, and therefore CD2001 family members can include nucleotide sequence polymorphisms (*e.g*., nucleotide sequences that vary from SEQ ID NO:74 and SEQ ID NO:75) that map to this chromosomal locus (*e.g*., region of chromosome 1q21) and such sequences represent CD2001 allelic variants. As used herein, the terms "gene" and "recombinant gene" refer to nucleic acid molecules comprising an open reading frame, *e.g.,* a contiguous open reading frame, encoding a polypeptide of the invention.

As used herein, the phrase "allele" or "allelic variant" refers to a nucleotide sequence which occurs at a given chromosomal locus, to a nucleic acid molecule that encodes a polypeptide encoded by the nucleotide sequence which occurs at the given chromosomal locus *(e.g.,* a cDNA molecule), or to a polypeptide encoded by the nucleotide sequence. Allelic variations can typically result in about 1-5% variance in the nucleotide sequence of a given gene. Alternative alleles can be identified by sequencing the gene of interest in a number of different individuals. This can be readily carried out by using hybridization probes to identify the same genetic locus in a variety of individuals, and to characterize the polymorphisms present at the genetic locus across the individuals. In one embodiment, polymorphisms that are associated with a particular disease and/or disorder are used as markers to diagnose said disease or disorder. In a preferred embodiment, polymorphisms are used as a marker to diagnose abnormal immune function *(e.g.,* immune diseases such as inflammatory bowel disease (*e.g*., Crohn's disease and ulcerative colitis), proliferative disorders, chronic obstructive pulmonary disease, autoimmune diseases (*e.g*., multiple sclerosis), and inflammatory disorders (e.g., rheumatoid arthritis and asthma)).

Moreover, nucleic acid molecules encoding proteins of the invention from other species (homologs), which have a nucleotide sequence which differs from that of the human protein described herein are intended to be within the scope of the invention. Nucleic acid molecules corresponding to natural allelic variants and homologs of a cDNA of the invention can be isolated based on their identity to the human nucleic acid molecule disclosed herein using the human cDNAs, or a portion thereof, as a hybridization probe according to standard hybridization techniques under stringent hybridization conditions.

Accordingly, in another embodiment, an isolated nucleic acid molecule ofthe invention is at least 15, preferably at least 25, at least 50, at least 75, at least 100, at least 125, at least 150, at least 175, at least 200, at least 225, at least 250, at least 275, at least 300, at least 325, at least 350, at least 375, at least 400, at least 425, at least 450, at least 500, at least 600, at least 700, at least 800, at least 900, at least 1000, at least 1100, at least 1200, at least 1300, at least 1400, at least 1500, at least 1600, at least 1700, at least 1800 contiguous nucleotides in length and hybridizes under stringent conditions to the nucleic acid molecule comprising the nucleotide sequence, preferably the coding sequence, of SEQ ID NO:1, or a complement thereof.

Accordingly, in another embodiment, an isolated nucleic acid molecule of the invention is at least 15, preferably at least 25, at least 50, at least 75, at least 100, at least 125, at least 150, at least 175, at least 200, at least 225, at least 250, at least 275, at least 300, at least 325, at least 350 contiguous nucleotides in length and hybridizes under stringent conditions to the nucleic acid molecule comprising the nucleotide sequence of SEQ ID NO:2, or a complement thereof.

Accordingly, in another embodiment, an isolated nucleic acid molecule of the invention is at least 15, preferably at least 25, at least 50, at least 75, at least 100, at least 125, at least 150, at least 175, at least 200, or at least 225 contiguous nucleotides in length and hybridizes under stringent conditions to the nucleic acid molecule comprising the nucleotide sequence of SEQ ID NO:5 or 54, or a complement thereof.

Accordingly, in another embodiment, an isolated nucleic acid molecule of the invention is at least 15 contiguous nucleotides in length and hybridizes under stringent conditions to the nucleic acid molecule comprising the nucleotide sequence of SEQ ID NO:55 or 57, or a complement thereof.

Accordingly, in another embodiment, an isolated nucleic acid molecule of the invention is at least 15, preferably at least 25, at least 35, at least 40, at least 45, at least 50, at least 55, at least 60, at least 65, at least 70, at least 75, or more contiguous nucleotides in length and hybridizes under stringent conditions to the nucleic acid molecule comprising the nucleotide sequence of SEQ ID NO:47 or 58, or a complement thereof.

Accordingly, in another embodiment, an isolated nucleic acid molecule of the invention is at least 15, preferably at least 25, at least 30, at least 35, at least 40, at least 45, at least 50 contiguous nucleotides in length and hybridizes under stringent conditions to the nucleic acid molecule comprising the nucleotide sequence of SEQ ID NO:49, or a complement thereof.

Accordingly, in another embodiment, an isolated nucleic acid molecule of the invention is at least 15, preferably at least 25, at least 50, at least 75, at least 100, at least 125, at least 150, at least 175, at least 200, at least 225, at least 250, at least 275, at least 300, at least 325, at least 350, at least 375, at least 400, at least 425, at least 450, at least 475 at least 500, at least 525, at least 550, at least 575, at least 600, at least 625, at least 650, at least 675, at least 700, at least 725, at least 750, at least 775, at least 800, at least 825, at least 850, at least 875, or more contiguous nucleotides in length and hybridizes under stringent conditions to the nucleic acid molecule comprising the nucleotide sequence of SEQ ID NO:74 or 75, or a complement thereof.

As used herein, the term "hybridizes under stringent conditions" is intended to describe conditions for hybridization and washing under which nucleotide sequences at least 60% (65%, 70%, preferably 75%) identical to each other typically remain hybridized to each other. Such stringent conditions are known to those skilled in the art and can be found in *Current Protocols in Molecular Biology,* John Wiley & Sons, N.Y. (1989), 6.3.1-6.3.6. In one, non-limiting example stringent hybridization conditions are hybridization at 6X sodium chloride/sodium citrate (SSC) at about 45°C, followed by one or more washes in 0.1XSSC, 0.2% SDS at about 68° C. A preferred, non-limiting example stringent hybridization conditions are hybridization in 6XSSC at about 45°C, followed by one or more washes in 0.2 X SSC, 0.1% SDS at 50-65°C (*i.e*., one or more washes at 50°C, 55°C, 60°C or 65°C). It is understood that the nucleic acids of the invention do not include nucleic acid molecules that hybridize under these conditions solely to a nucleotide sequence consisting of only A or T nucleotides.

Preferably, an isolated nucleic acid molecule of the invention that hybridizes under stringent conditions to the sequence of SEQ ID NO:1, 2, 31, 32, 33, 34, 35, 36, 37, 38, 43, 44, 45, 46, 47, 48, 49, 50, 51, 52, 53, 54, 55, 56, 57, 58, 66, 67, 74, 75, 83, 84, 85, 86, 87, 92, 93, 94 or 95, or a complement thereof, corresponds to a naturally-occurring nucleic acid molecule. As used herein, a "naturally-occurring" nucleic acid molecule refers to an RNA or DNA molecule having a nucleotide sequence that occurs in nature *(e.g.,* encodes a natural protein), or to a cDNA molecule corresponding to (*e.g.*, amplified or reverse-transcribed from) such an RNA or DNA molecule.

In addition to naturally-occurring variants of a nucleic acid molecule of the invention sequence that may exist in the population, the skilled artisan will further appreciate that changes can be introduced by mutation thereby leading to changes in the amino acid sequence of the encoded protein and production of a variant protein, without altering the biological activity of the protein. Said variants can function as either agonists (mimetics) or antagonists, as described below in the discussion of the polypeptides of the invention.

An isolated nucleic acid molecule encoding a variant protein can be created by introducing one or more nucleotide substitutions, additions or deletions into the nucleotide sequence of SEQ ID NO:1, 2, 31, 32, 33, 34, 35, 36, 37, 38, 43, 44, 45, 46, 47, 48, 49, 50, 51, 52, 53, 54, 55, 56, 57, 58, 66, 67, 74, 75, 83, 84, 85, 86, 87, 92, 93, 94 or 95 such that one or more amino acid substitutions, additions or deletions are introduced into the encoded protein. Mutations can be introduced by standard techniques, such as site-directed mutagenesis and PCR-mediated mutagenesis.

Preferably, conservative amino acid substitutions are made at one or more predicted non-essential amino acid residues. A "conservative amino acid substitution" is one in which the amino acid residue is replaced with an amino acid residue having a similar side chain. Families of amino acid residues having similar side chains have been defined in the art. These families include amino acids with basic side chains *(e.g.,* lysine, arginine, histidine), acidic side chains *(e.g.,* aspartic acid, glutamic acid), uncharged polar side chains (*e.g*., glycine, asparagine, glutamine, serine, threonine, tyrosine, cysteine), nonpolar side chains (*e.g.,* alanine, valine, leucine, isoleucine, proline, phenylalanine, methionine, tryptophan), beta-branched side chains (*e.g.,* threonine, valine, isoleucine), and aromatic side chains *(e.g.,* tyrosine, phenylalanine, tryptophan, histidine). Alternatively, mutations can be introduced randomly along all or part of the coding sequence, such as by saturation mutagenesis, and the resultant mutants can be screened for biological activity to identify mutants that retain or antagonize activity. Following mutagenesis, the encoded protein can be expressed recombinantly and the activity of the protein can be determined.

Specific examples of conservative amino acid alterations from the original amino acid sequence of SEQ ID NO:3 are shown in SEQ ID NO:39, 40, 41 and 42. Specific examples of conservative amino acid alterations from the original amino acid sequence of SEQ ID NO:76 are shown in SEQ ID NO:89, 90, 91 and 92. Alternatively, amino acid residues that are conserved among the homologs of various species (e.g., mouse and human) may be essential for activity and thus would not be likely targets for alteration.

Nucleotide substitutions leading to amino acid substitutions at "non-essential" amino acid residues can be introduced. A "non-essential" amino acid residue is a residue that can be altered from the wild-type sequence without altering the biological activity, whereas an "essential" amino acid residue is required for biological activity. For example, amino acid residues that are not conserved or only semi-conserved among homologs of various species can be non-essential for activity and thus would be likely targets for alteration. Discussed below are additional methods by which to routinely identify non-essential amino acid residues.

Accordingly, another aspect of the invention pertains to nucleic acid molecules encoding a polypeptide of the invention that contain changes in amino acid residues that are not essential for activity. Such polypeptides differ in amino acid sequence from SEQ ID NO:3 or SEQ ID NO:76, yet retain biological activity. In one embodiment, the isolated nucleic acid molecule includes a nucleotide sequence encoding a protein that includes an amino acid sequence that is at least about 30%, 35%, 40%, 45%, 50%, 55%, 60%, 65%, 75%, 85%, 95%, or 98% identical to the amino acid sequence of SEQ ID NO:3. In another embodiment, the isolated nucleic acid molecule includes a nucleotide sequence encoding a protein that includes an amino acid sequence that is at least about 70%, 75%, 85%, 95%, or 98% identical to the amino acid sequence of SEQ ID NO:76.

In a preferred embodiment, a polypeptide whose amino acid sequence has been modified can be assayed to determine whether it represents a variant of a polypeptide of the invention that retains a biological activity of a CD2000 polypeptide by, for example, assaying for one or more of the following: (1) the ability to form or modulate protein-protein interactions and/or protein-ligand interactions with proteins in a signaling pathway of a polypeptide of the invention; (2) the ability to bind to an intracellular target of a polypeptide of the invention *(e.g.,* the ability to bind to SAP, EAT-2, or SHP-2 phosphatase); (3) the ability to modulate the activity of an intracellular signal molecule *(e.g.,* the ability to activate Syk, Lyn, Lck, phosphatidylinostiol 3-kinase, PLC- γ1, Zap-70, PKC and/or NF-κB); (4) the ability to modulate the expression of molecules such as, *e.g.,* cytokines (*e.g.*, IFN-γ, IL-2, IL-3, IL-4, IL-5, IL-6, IL-10, IL-12, or IL-15) and T cell activation markers *(e.g.,* CD28, ICOS, CD48, CD58, BLAME, Ly9, CD84, SLAM, D-SLAM, CD2, 19A, or 2B4); (5) the ability to optimize T cell receptor (TCR)-peptide- MHC interactions, *e.g.,* by optimizing the distance between opposing cell membranes; (6) the ability to modulate T cell activation; and (7) the ability to modulate intercellular signaling.

In another preferred embodiment, a polypeptide whose amino acid sequence has been modified can be assayed to determine whether it represents a variant of a polypeptide of the invention that retains a biological activity of a CD2001 polypeptide by, for example, assaying for one or more of the following: (1) the ability to form or modulate protein-protein interactions and/or protein-ligand interactions with proteins in a signaling pathway of a polypeptide of the invention; (2) the ability to bind to an intracellular target of a polypeptide of the invention *(e.g.,* the ability to bind to SHP-2 phosphatase); (3) the ability to modulate the activity of an intracellular signal molecule *(e.g.,* the ability to activate Syk, Lyn, Lck, phosphatidylinostiol 3-kinase, PLC- γ1, Zap-70, PKC and/or NF-κB); (4) the ability to modulate the expression of molecules such as, *e.g.,* cytokines *(e.g.,* IFN-γ, IL-2, IL-3, IL-4, IL-5, IL-6, IL-10, IL-12, or IL-15) and T cell activation markers *(e.g.,* CD28, ICOS, CD48, CD58, BLAME, Ly9, CD84, SLAM, D-SLAM, CD2, 19A, or 2B4); (5) the ability to optimize T cell receptor (TCR)-peptide- MHC interactions, *e.g*., by optimizing the distance between opposing cell membranes; (6) the ability to modulate T cell activation; and (7) the ability to modulate intercellular signaling.

In addition to the above functional assays, a polypeptide whose amino acid sequence has been modified can be assayed to determine whether it represents a variant of a polypeptide of the invention that retains a biological activity of a CD2000 polypeptide or CD2001 polypeptide by, for example, assaying whether it exhibits an antigenicity or immunogenicity of a polypeptide of the invention. For example, antibodies raised against the modified polypeptide can be tested for an ability to compete with antibodies directed against a polypeptide of the invention for binding to the polypeptide of the invention. Variant polypeptides are ones that can be used to generate antibodies that can compete for such binding.

In a specific embodiment, a nucleic acid sequence of the invention, *e.g.,* the nucleotide sequence of SEQ ID NO:1, 2, 31, 32, 33, 34, 35, 36, 37, or 38, or the nucleotide sequence of the cDNA insert of an EpCD2000 clone deposited with the ATCC® as patent deposit number PTA-2267, encodes a CD2 family member which modulates T cell activation. In a specific embodiment, a nucleic acid sequence of the invention, *e.g.,* the nucleotide sequence of SEQ ID NO:74, 75, 92, 93, 94 or 95, encodes a CD2 family member which modulates T cell activation. In accordance with these embodiments, techniques known to those of ordinary skill in the art for assaying the activity of CD2 family members can be utilized to assay the functional activity of the CD2000 polypeptides or CD2001 polypeptides encoded by the nucleic acid sequences of the invention.

The invention also provides nucleic acid molecules that encode chimeric or fusion proteins. As used herein, a "chimeric protein" or "fusion protein" comprises all or part (preferably biologically active) of a polypeptide of the invention operably linked (*e.g.*, joined via a peptide bond) to a heterologous polypeptide (*i.e.*, a polypeptide other than the same polypeptide of the invention). The term "operably linked" is intended to indicate that the nucleic acid molecule encoding the polypeptide portion of the invention and the nucleic acid molecule encoding the heterologous polypeptide portion are fused in-frame to each other. The heterologous polypeptide can be fused to the N-terminus or C-terminus of the polypeptide of the invention.

The present invention encompasses nucleic acid molecules of the invention that are antisense molecules, *i.e.,* molecules which are complementary to a sense nucleic acid encoding a polypeptide of the invention, *e.g.,* complementary to the coding strand of a double-stranded cDNA molecule or complementary to an mRNA sequence. Accordingly, an antisense nucleic acid is one that can form a stable duplex with a sense nucleic acid, or, in the case of an antisense nucleic acid molecule that binds to DNA duplexes, through specific interactions in the major groove of the double helix. The antisense nucleic acid can be complementary to or bind to an entire coding strand, or to only a portion thereof, *e.g.*, all or part of the protein coding open reading frame region. An antisense nucleic acid molecule can also be antisense to or bind to all or part of a non-coding region of the coding strand of a nucleotide sequence encoding a polypeptide of the invention, *e.g.,* the non-coding region of a cDNA molecule. The non-coding regions ("5' and 3' untranslated regions") are the 5' and 3' sequences which flank the coding region and are not translated into amino acids.

An antisense oligonucleotide can be, for example, about 5, 10, 15, 20, 25, 30, 35, 40, 45 or 50 nucleotides or more in length. An antisense nucleic acid of the invention can be constructed using chemical synthesis and enzymatic ligation reactions using procedures known in the art. For example, an antisense nucleic acid *(e.g.,* an antisense oligonucleotide) can be chemically synthesized using naturally occurring nucleotides or variously modified nucleotides designed to increase the biological stability of the molecules or to increase the physical stability of the duplex formed between the antisense and sense nucleic acids, *e.g.,* phosphorothioate derivatives and acridine substituted nucleotides can be used. Examples of modified nucleotides which can be used to generate the antisense nucleic acid include 5-fluorouracil, 5-bromouracil, 5-chlorouracil, 5-iodouracil, hypoxanthine, xanthine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine. Alternatively, the antisense nucleic acid can be produced biologically using an expression vector into which a nucleic acid has been subcloned in an antisense orientation (*i.e.,* RNA transcribed from the inserted nucleic acid will be of an antisense orientation to a target nucleic acid of interest, described further in the following subsection).

The antisense nucleic acid molecules of the invention can be administered to a subject or generated *in situ* such that they hybridize with or bind to cellular mRNA and/or genomic DNA encoding a selected polypeptide of the invention to thereby inhibit expression, *e.g.,* by inhibiting transcription and/or translation. An example of a route of administration of antisense nucleic acid molecules of the invention includes direct injection at a tissue site. Alternatively, antisense nucleic acid molecules can be modified to target selected cells and then administered systemically. For example, for systemic administration, antisense molecules can be modified such that they specifically bind to receptors or antigens expressed on a selected cell surface, *e.g.*, by linking the antisense nucleic acid molecules to peptides or antibodies which bind to cell surface receptors or antigens. The antisense nucleic acid molecules can also be delivered to cells using the vectors described herein. To achieve sufficient intracellular concentrations of the antisense molecules, vector constructs in which the antisense nucleic acid molecule is placed under the control of a strong pol II or pol III promoter are preferred.

An antisense nucleic acid molecule of the invention can be an α-anomeric nucleic acid molecule. An α-anomeric nucleic acid molecule forms specific double-stranded hybrids with complementary RNA in which, contrary to the usual β-units, the strands run parallel to each other (Gaultier et al., 1987, *Nucleic Acids Res.* 15:6625-6641). The antisense nucleic acid molecule can also comprise a 2'-o-methylribonucleotide (Inoue et al., 1987, *Nucleic Acids Res.* 15:6131-6148) or a chimeric RNA-DNA analogue (Inoue et al., 1987, *FEBS Lett*. 215:327-330).

The invention also encompasses ribozymes. Ribozymes are catalytic RNA molecules with ribonuclease activity which are capable of cleaving a single-stranded nucleic acid, such as an mRNA, to which they have a complementary region. Thus, ribozymes (*e.g.*, hammerhead ribozymes (described in Haselhoff and Gerlach, 1988, *Nature* 334:585-591)) can be used to catalytically cleave mRNA transcripts to thereby inhibit translation of the protein encoded by the mRNA. A ribozyme having specificity for a nucleic acid molecule encoding a polypeptide of the invention can be designed based upon the nucleotide sequence of a cDNA disclosed herein. For example, a derivative of a *Tetrahymena* L-19 IVS RNA can be constructed in which the nucleotide sequence of the active site is complementary to the nucleotide sequence to be cleaved in a Cech et al. U.S. Patent No. 4,987,071; and Cech et al. U.S. Patent No. 5,116,742. Alternatively, an mRNA encoding a polypeptide of the invention can be used to select a catalytic RNA having a specific ribonuclease activity from a pool of RNA molecules. *See, e.g.,* Bartel and Szostak, 1993, *Science* 261:1411-1418.

The invention also encompasses nucleic acid molecules which form triple helical structures. For example, expression of a polypeptide of the invention can be inhibited by targeting nucleotide sequences complementary to the regulatory region of the gene encoding the polypeptide (*e.g*., the promoter and/or enhancer) to form triple helical structures that prevent transcription of the gene in target cells. *See generally* Helene, 1991, *Anticancer Drug Des*. 6(6):569-84; Helene, 1992, *Ann*. *N.Y. Acad. Sci.* 660:27-36; and Maher, 1992, *Bioassays* 14(12):807-15.

In various embodiments, the nucleic acid molecules of the invention can be modified at the base moiety, sugar moiety or phosphate backbone to improve, *e.g.*, the stability, hybridization, or solubility of the molecule. For example, the deoxyribose phosphate backbone of the nucleic acids can be modified to generate peptide nucleic acids *(see* Hyrup et al., 1996, *Bioorganic & Medicinal Chemistry* 4(1): 5-23). As used herein, the terms "peptide nucleic acids" or "PNAs" refer to nucleic acid mimics, *e.g*., DNA mimics, in which the deoxyribose phosphate backbone is replaced by a pseudopeptide backbone and only the four natural nucleobases are retained. The neutral backbone of PNAs has been shown to allow for specific hybridization to DNA and RNA under conditions of low ionic strength. The synthesis of PNA oligomers can be performed using standard solid phase peptide synthesis protocols as described in Hyrup et al., 1996, *supra*; and Perry-O'Keefe et al., 1996, *Proc. Natl. Acad. Sci. USA* 93: 14670-675.

PNAs can be used in therapeutic and diagnostic applications. For example, PNAs can be used as antisense or antigene agents for sequence-specific modulation of gene expression by, *e.g.*, inducing transcription or translation arrest or inhibiting replication. PNAs can also be used, *e.g*., in the analysis of single base pair mutations in a gene by, *e.g.,* PNA directed PCR clamping, or as artificial restriction enzymes when used in combination with other enzymes, *e.g.,* S1 nucleases (Hyrup, 1996, *supra;* or as probes or primers for DNA sequence and hybridization (Hyrup, 1996, *supra;* and Perry-O'Keefe et al., 1996, *Proc. Natl. Acad. Sci. USA* 93: 14670-675).

In another embodiment, PNAs can be modified, *e.g.,* to enhance their stability or cellular uptake, by attaching lipophilic or other helper groups to PNA, by the formation of PNA-DNA chimeras, or by the use of liposomes or other techniques of drug delivery known in the art. For example, PNA-DNA chimeras can be generated which may combine the advantageous properties of PNA and DNA. Such chimeras allow DNA recognition enzymes, *e.g.,* RNAse H and DNA polymerases, to interact with the DNA portion while the PNA portion would provide high binding affinity and specificity. PNA-DNA chimeras can be linked using linkers of appropriate lengths selected in terms of base stacking, number of bonds between the nucleobases, and orientation (Hyrup, 1996, *supra).* The synthesis of PNA-DNA chimeras can be performed as described in Hyrup, 1996, *supra,* and Finn et al., 1996, *Nucleic Acids Res*. 24(17):3357-63. For example, a DNA chain can be synthesized on a solid support using standard phosphoramidite coupling chemistry and modified nucleoside analogs. Compounds such as 5'-(4-methoxytrityl)amino-5'-deoxy-thymidine phosphoramidite can be used as a link between the PNA and the 5' end of DNA (Mag et al., 1989, *Nucleic Acids Res.* 17:5973-88). PNA monomers are then coupled in a stepwise manner to produce a chimeric molecule with a 5' PNA segment and a 3' DNA segment (Finn et al., 1996, *Nucleic Acids Res.* 24(17):3357-63). Alternatively, chimeric molecules can be synthesized with a 5' DNA segment and a 3' PNA segment (Peterser et al., 1975, *Bioorganic Med. Chem. Lett.* 5:1119-11124).

In other embodiments, the oligonucleotide may include other appended groups such as peptides *(e.g.,* for targeting host cell receptors *in vivo* ), or agents facilitating transport across the cell membrane *(see, e.g.,* Letsinger et al., 1989, *Proc. Natl. Acad. Sci. USA* 86:6553-6556; Lemaitre et al., 1987, *Proc. Natl. Acad. Sci. USA* 84:648-652; PCT Publication No. W0 88/09810) or the blood-brain barrier *(see, e.g.,* PCT Publication No. W0 89/10134). In addition, oligonucleotides can be modified with hybridization-triggered cleavage agents *(see, e.g.,* Krol et al., 1988, *Bio*/*Techniques* 6:958-976) or intercalating agents *(see, e.g*., Zon, 1988, *Pharm. Res.* 5:539-549). To this end, the oligonucleotide may be conjugated to another molecule, *e.g.,* a peptide, hybridization triggered cross-linking agent, transport agent, hybridization-triggered cleavage agent, etc.

### II. Isolated Proteins and Antibodies

One aspect of the invention pertains to isolated polypeptides of the invention. Such polypeptides can be produced by a variety of means. For example, the polypeptides of the invention can be isolated from natural sources, chemically synthesized, or recombinantly produced. In one embodiment, therefore, a native polypeptide can be isolated from cells or tissue sources by an appropriate purification scheme using standard protein purification techniques. In another embodiment, polypeptides of the invention are produced by recombinant DNA techniques. Alternative to recombinant expression, a polypeptide of the invention can be synthesized chemically using standard peptide synthesis techniques.

An "isolated" or "purified" polypeptide is substantially free of cellular material or other contaminating proteins from the cell or tissue source from which the protein is derived, or substantially free of chemical precursors or other chemicals when chemically synthesized. The language "substantially free of cellular material" includes preparations of protein in which the protein is separated from cellular components of the cells from which it is isolated or recombinantly produced. Thus, protein that is substantially free of cellular material includes preparations of protein having less than about 30%, 20%, 10%, or 5% (by dry weight) of heterologous protein (also referred to herein as a "contaminating protein"). When the protein or biologically active portion thereof is recombinantly produced, it is also preferably substantially free of culture medium, *i.e.,* culture medium represents less than about 20%, 10%, or 5% of the volume of the protein preparation. When the protein is produced by chemical synthesis, it is preferably substantially free of chemical precursors or other chemicals, *i.e.,* it is separated from chemical precursors or other chemicals which are involved in the synthesis of the protein. Accordingly such preparations of the protein have less than about 30%, 20%, 10%, 5% (by dry weight) of chemical precursors or compounds other than the polypeptide of interest.

Preferred polypeptides have the amino acid sequence of SEQ ID NO:3, 4, 5, 6, 7, 8, 9, 10, 12, 13, 14, 15, 16, 17, 18, 19, 21, 22, 39, 40, 41, 42, 76, 77, 78, 79, 80, 81, 82, 88, 89, 90, 91, 96 or 97. Other useful, preferred polypeptides of the invention are ones that are substantially identical *(e.g.,* at least about 45%, preferably 55%, 65%, 75%, 85%, 95%, 98%, or 99%) to any of such polypeptides of the invention and retain a functional and/or structural activity of a polypeptide of a corresponding naturally-occurring protein yet differ in amino acid sequence due to natural allelic variation or mutagenesis.

To determine the percent identity of two amino acid sequences or of two nucleic acids, the sequences are aligned for optimal comparison purposes (*e.g.*, gaps can be introduced in the sequence of a first amino acid or nucleic acid sequence for optimal alignment with a second amino or nucleic acid sequence). The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position. The percent identity between the two sequences is a function of the number of identical positions shared by the sequences (% identity = # of identical positions/total # of positions (e.g., overlapping positions) x 100). In one embodiment, the two sequences are the same length.

The determination of percent identity between two sequences can be accomplished using a mathematical algorithm. A preferred, non-limiting example of a mathematical algorithm utilized for the comparison of two sequences is the algorithm of Karlin and Altschul (1990) *Proc. Natl. Acad. Sci. USA* 87:2264-2268, modified as in Karlin and Altschul (1993) *Proc. Natl. Acad. Sci. USA* 90:5873-5877. Such an algorithm is incorporated into the NBLAST and XBLAST programs of Altschul, et al. (1990) *J. Mol. Biol.* 215:403-410. BLAST nucleotide searches can be performed with the NBLAST program, score = 100, wordlength = 12 to obtain nucleotide sequences homologous to a nucleic acid molecules of the invention. BLAST protein searches can be performed with the XBLAST program, score = 50, wordlength = 3 to obtain amino acid sequences homologous to a protein molecules of the invention. To obtain gapped alignments for comparison purposes, Gapped BLAST can be utilized as described in Altschul et al. (1997) *Nucleic Acids Res.* 25:3389-3402. Alternatively, PSI-Blast can be used to perform an iterated search which detects distant relationships between molecules *(Id.).* When utilizing BLAST, Gapped BLAST, and PSI-Blast programs, the default parameters of the respective programs (e.g., XBLAST and NBLAST) can be used. *See* http://www.ncbi.nlm.nih.gov.

Another preferred, non-limiting example of a mathematical algorithm utilized for the comparison of sequences is the algorithm of Myers and Miller, CABIOS (1989). Such an algorithm is incorporated into the ALIGN program (version 2.0) which is part of the CGC sequence alignment software package. When utilizing the ALIGN program for comparing amino acid sequences, a PAM120 weight residue table, a gap length penalty of 12, and a gap penalty of 4 can be used. Additional algorithms for sequence analysis are known in the art and include ADVANCE and ADAM as described in Torellis and Robotti (1994) *Comput. Appl. Biosci., 10*:3-5; and FASTA described in Pearson and Lipman (1988) *Proc. Natl. Acad. Sci. 85*:2444-8. Within FASTA, ktup is a control option that sets the sensitivity and speed of the search. If ktup=2, similar regions in the two sequences being compared are found by looking at pairs of aligned residues; if ktup=1, single aligned amino acids are examined. ktup can be set to 2 or 1 for protein sequences, or from 1 to 6 for DNA sequences. The default if ktup is not specified is 2 for proteins and 6 for DNA. For a further description of FASTA parameters, see http://bioweb.pasteur.fr/docs/man/man/fasta.1.html#sect2, the contents of which are incorporated herein by reference.

The percent identity between two sequences can be determined using techniques similar to those described above, with or without allowing gaps. In calculating percent identity, typically exact matches are counted.

The invention also provides chimeric or fusion proteins. As used herein, a "chimeric protein" or "fusion protein" comprises all or part (preferably biologically active) of a polypeptide of the invention operably linked (*e.g.*, joined via a peptide bond) to a heterologous polypeptide *(i.e.,* a polypeptide other than the same polypeptide of the invention). The heterologous polypeptide can be fused to the N-terminus or C-terminus of the polypeptide of the invention.

One useful fusion protein is a GST fusion protein in which the polypeptide of the invention is fused to the C-terminus of GST sequences. Such fusion proteins can facilitate the purification of a recombinant polypeptide of the invention.

In another embodiment, the fusion protein contains a heterologous signal sequence at its N-terminus. For example, the native signal sequence of a polypeptide of the invention can be removed and replaced with a signal sequence from another protein. For example, the gp67 secretory sequence of the baculovirus envelope protein can be used as a heterologous signal sequence *(Current Protocols in Molecular Biology,* Ausubel et al., eds., John Wiley & Sons, 1992). Other examples of eukaryotic heterologous signal sequences include the secretory sequences of melittin and human placental alkaline phosphatase (Stratagene; La Jolla, California). In yet another example, useful prokaryotic heterologous signal sequences include the phoA secretory signal (Sambrook et al., *supra)* and the protein A secretory signal (Pharmacia Biotech; Piscataway, New Jersey).

In yet another embodiment, the fusion protein is an immunoglobulin fusion protein in which all or part of a polypeptide of the invention is fused to sequences derived from a member of the immunoglobulin protein family. The immunoglobulin fusion proteins of the invention can be incorporated into pharmaceutical compositions and administered to a subject to inhibit an interaction between a ligand (soluble or membrane-bound) and a protein on the surface of a cell (receptor), to thereby suppress signal transduction *in vivo*. The immunoglobulin fusion protein can be used to affect the bioavailability of a cognate ligand of a polypeptide of the invention. Inhibition of ligand/receptor interaction may be useful therapeutically, both for treating proliferative and differentiative disorders and for modulating (*e.g.,* promoting or inhibiting) cell survival. Moreover, the immunoglobulin fusion proteins of the invention can be used as immunogens to produce antibodies directed against a polypeptide of the invention in a subject, to purify ligands and in screening assays to identify molecules which inhibit the interaction of receptors with ligands.

Chimeric and fusion proteins of the invention can be produced by standard recombinant DNA techniques. For example, a nucleic acid molecule encoding a fusion protein can be synthesized by conventional techniques including automated DNA synthesizers. Alternatively, PCR amplification of gene fragments can be carried out using anchor primers which give rise to complementary overhangs between two consecutive gene fragments which can subsequently be annealed and reamplified to generate a chimeric gene sequence *(see, e.g.,* Ausubel et al., *supra).* Moreover, many expression vectors are commercially available that already encode a fusion moiety *(e.g.,* a GST polypeptide). A nucleic acid encoding a polypeptide of the invention can be cloned into such an expression vector such that the fusion moiety is linked in-frame to the polypeptide of the invention.

A signal sequence of a polypeptide of the invention (*e.g*., SEQ ID NO:4 or SEQ ID NO:78) can be used to facilitate secretion and isolation of the secreted protein or other proteins of interest. Signal sequences are typically characterized by a core of hydrophobic amino acids at the N-terminus of a protein which are generally cleaved from the mature protein during secretion in one or more cleavage events. Such signal peptides contain processing sites that allow cleavage of the signal sequence from the mature proteins as they pass through the secretory pathway. Thus, the invention pertains to the described polypeptides having a signal sequence, as well as to the signal sequence itself and to the polypeptides in the absence of the signal sequence *(i.e.,* the cleavage products). In one embodiment, a nucleic acid sequence encoding a signal sequence of the invention can be operably linked in an expression vector to a protein of interest, such as a protein which is ordinarily not secreted or is otherwise difficult to isolate. The signal sequence directs secretion of the protein, such as from a eukaryotic host into which the expression vector is transformed, and the signal sequence is subsequently or concurrently cleaved. The protein can then be readily purified from the extracellular medium by art recognized methods. Alternatively, the signal sequence can be linked to the protein of interest using a sequence which facilitates purification, such as with a GST domain.

In another embodiment, the signal sequences of the present invention can be used to identify regulatory sequences, *e.g.*, promoters, enhancers, and repressors. Since signal sequences are the most amino-terminal sequences of a peptide, it is expected that the nucleic acids which flank the signal sequence on its amino-terminal side will be regulatory sequences which affect transcription. Thus, a nucleotide sequence which encodes all or a portion of a signal sequence can be used as a probe to identify and isolate signal sequences and their flanking regions, and these flanking regions can be studied to identify regulatory elements therein.

The present invention also pertains to variants of the polypeptides of the invention. Such variants have an altered amino acid sequence which can function as either agonists (mimetics) or as antagonists. Variants can be generated by mutagenesis, *e.g*., discrete point mutation or truncation. An agonist can retain substantially the same, or a subset, of the biological activities of the naturally occurring form of the protein. An antagonist of a protein can inhibit one or more of the activities of the naturally occurring form of the protein by, for example, competitively binding to a downstream or upstream member of a cellular signaling cascade which includes the protein of interest. Thus, specific biological effects can be elicited by treatment with a variant of limited function. Treatment of a subject with a variant having a subset of the biological activities of the naturally occurring form of the protein can have fewer side effects in a subject relative to treatment with the naturally occurring form of the protein.

Variants of a polypeptide of the invention which function as either agonists (mimetics) or as antagonists can be identified, for example, by screening combinatorial libraries of mutants, *e.g*., truncation mutants, of the protein of the invention for agonist or antagonist activity. In one embodiment, a variegated library of variants is generated by combinatorial mutagenesis at the nucleic acid level and is encoded by a variegated gene library. A variegated library of variants can be produced by, for example, enzymatically ligating a mixture of synthetic oligonucleotides into gene sequences such that a degenerate set of potential protein sequences is expressible as individual polypeptides, or alternatively, as a set of larger fusion proteins *(e.g.,* for phage display). There are a variety of methods which can be used to produce libraries of potential variants of the polypeptides of the invention from a degenerate oligonucleotide sequence. Methods for synthesizing degenerate oligonucleotides are known in the art *(see, e.g.,* Narang, 1983, *Tetrahedron* 39:3; Itakura et al., 1984, *Annu. Rev. Biochem*. 53:323; Itakura et al., 1984, *Science* 198:1056; and Ike et al., 1983, *Nucleic Acid Res.* 11:477).

In addition, libraries of fragments of the coding sequence of a polypeptide of the invention can be used to generate a variegated population of polypeptides for screening and subsequent selection of variants. For example, a library of coding sequence fragments can be generated by treating a double stranded PCR fragment of the coding sequence of interest with a nuclease under conditions wherein nicking occurs only about once per molecule, denaturing the double stranded DNA, renaturing the DNA to form double stranded DNA which can include sense/antisense pairs from different nicked products, removing single stranded portions from reformed duplexes by treatment with S1 nuclease, and ligating the resulting fragment library into an expression vector. By this method, an expression library can be derived which encodes N-terminal and internal fragments of various sizes of the protein of interest.

Several techniques are known in the art for screening gene products of combinatorial libraries made by point mutations or truncation, and for screening cDNA libraries for gene products having a selected property. The most widely used techniques, which are amenable to high through-put analysis, for screening large gene libraries typically include cloning the gene library into replicable expression vectors, transforming appropriate cells with the resulting library of vectors, and expressing the combinatorial genes under conditions in which detection of a desired activity facilitates isolation of the vector encoding the gene whose product was detected. Recursive ensemble mutagenesis (REM), a technique which enhances the frequency of functional mutants in the libraries, can be used in combination with the screening assays to identify variants of a protein of the invention (Arkin and Yourvan, 1992, *Proc. Natl. Acad. Sci. USA 89*:7811-7815; and Delgrave et al., 1993, *Protein Engineering* 6(3):327-331).

The polypeptides of the invention can be used in protein arrays to: (1) screen for proteins that interact with a polypeptide of the invention; (2) identify substrates of a polypeptide of the invention; and (3) identify small molecules that interact with a polypeptide of the invention. Methods of constructing protein arrays are known in the art (see, *e.g.,* Fodor *et al*., 1996, "Photolabile nucleoside and peptide protecting groups", US Patent No. 5,489,678; Barret *et al*., 1993, "Spatially-addressable immobilization of anti-ligands on surfaces", US Patent No. 5,252,743; Blawas and Reichert, 1998, "Protein patterning", Biomaterials 19:595-609; Blawas *et al*., 1996, "Patterning antibodies for multiple analyte sensor via photodeprotection chemistry", San Jose: SPIE; Delamarche *et al*., 1996, "Immobilization of antibodies on a photoactive self-assembled monolayer on gold", Langmuir 12: 1997-2006; Firestone *et al*., 1996, "Film architecture in biomolecular assemblies, Effect of linker on the orientation of genetically engineered surface-bound proteins", J. Amer. Chem. Soc.18: 9033-9041; Mooney *et al*., 1996, Patterning of functional antibodies and other proteins by photolithography of silane monolayers, Proc. Natl. Acad. Sci. 93: 12287-12291; Pirrung *et al*., 1996, "A general method for the spatially defined immobilization of biomolecules on glass surfaces using 'caged' biotin", Bioconjugate Chem. 7: 317-321; Gao *et al*., 1995, "Immunosensing with photoimmobilized immunoreagents on planar optical wave guides", Biosensors Bioelectron 10: 317-328; Schena *et al*., 1995, "Quantitative monitoring of gene expression patterns with a complementary DNA microarray", Science 270: 467-470; Lom *et al*., 1993, "A versatile technique for patterning biomolecules onto glass coverslips", J. Neurosci. Methods 385-397; Pope *et al*., 1993, "New applications of silane coupling agents for covalently binding antibodies to glass and cellulose solid phase surfaces", Bioconjugate Chem. 4: 116-171; Schramm *et al*., 1992, "Antibody-antigen complex formation with immobilized immunoglobulins", Anal. Biochem. 205: 47-56; Gombotz *et al*., 1991, Protein adsorption to poly(ethylene oxide) surfaces, J. Biomed. Mater. Res. 25: 1547-1562; Alarie *et al*., 1990, "Evaluation of antibody immobilization techniques for fiber optic-based fluoroimmunosensing", Analy. Chim. Acta 229: 169-176; Owaku *et al*., 1993, Optical immunosensing for IgG, Sensors Actuators B, 13-14: 723-724; Bhatia *et al*., 1989, "Use of thiol-terminal silanes and heterobifunctional cross linkers for immobilization of antibodies on silica surfaces", Analy. Biochem. 178: 408-413; Lin *et al*., 1988, "Characterization of immobilized antibodies on silica surfaces", IEEE Trans. Biomed. Engng., 35(6): 466-471; and MacBeath et al., 2000, "Printing Proteins as Microarrays for High-Throughput Function Determination", Science 289: 1760-1762).

The polypeptides of the invention can exhibit post-translational modifications, including, but not limited to glycosylations, *(e.g.,* N-linked or O-linked glycosylations), myristylations, palmitylations, acetylations and phosphorylations (*e.g*., serine/threonine or tyrosine). In one embodiment, the CD2000 polypeptides of the invention or CD2001 polypeptides of the invention exhibit reduced levels of O-linked glycosylation and/or N-linked glycosylation relative to endogenously expressed CD2000 polypeptides or CD2001 polypeptides. In another embodiment, the CD2000 polypeptides of the invention or CD2001 polypeptides of the invention do not exhibit O-linked glycosylation or N-linked glycosylation. Further, post-translational modifications of polypeptides of the invention such as glycosylation can be prevented by treating cells, *e.g.*, with tunicamycin, or by expressing nucleic acid molecules of the invention in host cells lacking lacking the capacity to post-translational modify the polypeptides of the invention.

An isolated polypeptide of the invention can be used as an antigen or immunogen to generate antibodies using standard techniques for polyclonal and monoclonal antibody preparation. The full-length polypeptide or protein, or antigenic peptide fragments can be used for use as immunogens. In one embodiment, an isolated polypeptide or fragment thereof which lacks N- and/or O-linked glycosylation is used as an immunogen to generate antibodies using standard techniques known to those of skill in the art. The antigenic peptide of a protein of the invention comprises at least 8 (preferably at least 10, at least 15, at least 20, at least 30, at least 50, at least 75, at least 100, at least 125, at least 150, or at least 175) contiguous amino acid residues of the amino acid sequence of SEQ ID NO:3, 4, 5, 6, 7, 8, 9, 10, 12, 13, 14, 15, 16, 17, 18, 19, 21, 22, 39, 40, 41, 42, 76, 77, 78, 79, 80, 81, 82, 88, 89, 90, 91, 96 or 97, and encompasses an epitope of the protein such that an antibody raised against the peptide forms a specific immune complex with the protein.

In one embodiment, a polypeptide used as an antigen or immungen comprises an amino acid sequence of SEQ ID NO:3, 4, 5, 6, 7, 8, 9, 10, 12, 13, 14, 15, 16, 17, 18, 19, 21, 22, 39, 40, 41, or 42, or the amino acid sequence encoded by the cDNA insert of EpCD2000 deposited with ATCC® as patent deposit Number PTA-2267. In another embodiment, a polypeptide used as an antigen or immunogen comprises a fragment of at least 8, preferably at least 10, at least 15, at least 25, at least 30, at least 50, at least 75, at least 100, at least 125, at least 150, at least 175, at least 200 or more contiguous amino acid residues of the amino acid sequence of SEQ ID NO:3, 4, 5, 6, 7, 8, 9, 10, 12, 13, 14, 15, 16, 17, 18, 19, 21, 22, 39, 40, 41, or 42. In another embodiment, a polypeptide used as an antigen or immungen comprises an amino acid sequence which is at least 50%, preferably at least 65%, at least 75%, at least 85%, at least 95% or at least 99% identical to the amino acid sequence of SEQ ID NO:3, 4, 5, 6, 7, 8, 9, 10, 12, 13, 14, 15, 16, 17, 18, 19, 21, 22, 39, 40, 41, or 42, wherein the percent identity is determined using a sequence alignment program such as the ALIGN program of the GCG software package with a PAM120 weight residue table, a gap length penalty of 12, and a gap penalty of 4. In yet another embodiment, a polypeptide used as an antigen or immunogen comprises an amino acid sequence which is encoded by a nucleic acid molecule which hybridizes to the nucleic acid molecule consisting of SEQ ID NO:1, 2, 31, 32, 33, 34, 35, 36, 37, 38, 43, 44, 45, 46, 47, 48, 49, 50, 51, 52, 53, 54, 55, 56, 57, 58, 66 or 67 under conditions of hybridization of 6X SSC at 45°C and washing in 0.2 X SSC, 0.1% SDS at 50°C, 55°C, 60°C or 65°C, or 6X SSC at 45°C and washing in 0.1 X SSC, 0.2% SDS at 68°C.

In another embodiment, a polypeptide used as an antigen or immungen comprises an amino acid sequence of SEQ ID NO:76, 77, 78, 79, 80, 81, 82, 88, 89, 90, 91, 96 or 97. In another embodiment, a polypeptide used as an antigen or immunogen comprises a fragment of at least 8, preferably at least 10, at least 15, at least 25, at least 30, at least 50, at least 75, at least 100, at least 125, at least 150, at least 175, at least 200 or more contiguous amino acid residues of the amino acid sequence of SEQ ID NO:76, 77, 78, 79, 80, 81, 82, 88, 89, 90, 91, 96 or 97. In another embodiment, a polypeptide used as an antigen or immunogen comprises a fragment of at least 8, preferably at least 10, at least 15, at least 25, at least 30, at least 50, at least 75, at least 100, at least 125, at least 150, at least 175, at least 200 or more contiguous amino acid residues of the amino acid sequence of SEQ ID NO:76, 77, 79, 82, 88, 89, 90, 91, 96 or 97. In another embodiment, a polypeptide used as an antigen or immungen comprises an amino acid sequence which is at least 70%, preferably at least 75%, at least 85%, at least 95% or at least 99% identical to the amino acid sequence of SEQ ID NO:76, 77, 79, 82, 88, 89, 90, 91, 96 or 97, wherein the percent identity is determined using a sequence alignment program such as the ALIGN program of the GCG software package with a PAM120 weight residue table, a gap length penalty of 12, and a gap penalty of 4. In yet another embodiment, a polypeptide used as an antigen or immunogen comprises an amino acid sequence which is encoded by a nucleic acid molecule which hybridizes to the nucleic acid molecule consisting of SEQ ID NO:74, 75, 83, 84, 85, 86, 87, 92, 93, 94 or 95 under conditions of hybridization of 6X SSC at 45°C and washing in 0.2 X SSC, 0.1% SDS at 50°C, 55°C, 60°C or 65°C, or 6X SSC at 45°C and washing in 0.1 X SSC, 0.2% SDS at 68°C.

The term "epitopes" as used herein refers to portions of a polypeptide having an antigenic or immunogenic activity in an animal, preferably a mammal, and most preferably a human. An epitope having immunogenic activity is a fragment of a polypeptide that elicits an antibody response in an animal. An epitope having antigenic activity is a fragment of a polypeptide to which an antibody immunospecifically binds *in vivo* or*in vitro* as determined by any method well known to those of skill in the art, for example, by the immunoassays described herein. Epitopes encompassed by the antigenic peptide are regions that are located on the surface of the protein, *e.g.*, hydrophilic regions. Alternatively, epitopes encompassed by the antigenic peptides are regions that are located within the proteins, and/or epitopes exposed in denatured or partially denatured forms of the polypeptides of the invention. Figure 2 is a hydropathy plot of the CD2000 protein of the invention. This plot or similar analyses can be used to identify hydrophilic regions. In addition, an epitope can encompass, in addition to a polypeptide or polypeptides of the invention, a post-translational modification (*e.g*., glycosylation, such as, for example, N- and/or O-linked glycosylation of the polypeptide or polypeptides).

An immunogen typically is used to prepare antibodies by immunizing a suitable subject (*e.g*., rabbit, goat, mouse or other mammal). An appropriate immunogenic preparation can contain, for example, recombinantly expressed or chemically synthesized polypeptide. The preparation can further include an adjuvant, such as Freund's complete or incomplete adjuvant, or similar immunostimulatory agent.

Accordingly, another aspect of the invention pertains to antibodies directed against a polypeptide of the invention. The term "antibody" as used herein refers to immunoglobulin molecules and immunologically active portions of immunoglobulin molecules, *i.e*., molecules that contain an antigen binding site which specifically binds an antigen, such as a polypeptide of the invention, *e.g*., an epitope of a polypeptide of the invention. Antibodies of the invention include, but are not limited to, monoclonal antibodies, polyclonal antibodies, multispecific antibodies, human antibodies, humanized antibodies, chimeric antibodies, single chain Fv (scFv), single chain antibodies, anti-idiotypic (anti-Id) antibodies, F(ab) fragments, F(ab')₂ fragments, and epitope-binding fragments of any of the above. A molecule which specifically or immunospecifically binds to a given polypeptide of the invention or fragment thereof is a molecule which binds the polypeptide, but does not substantially bind other molecules in a sample, *e.g.,* a biological sample, which naturally contains the polypeptide.

The antibodies of the invention may be from any animal origin including birds and mammals (*e.g.*, human, mouse, donkey, rabbit, sheep, guinea pigs, camel, horse or chicken). In one embodiment, the antibodies of the invention originate from non-human mammals such as mice, rats, sheep, and goat. In another embodiment, the antibodies of the invention are human or humanized antibodies. As used herein, "human" antibodies include antibodies having the amino acid sequence of a human immunoglobulin and include antibodies isolated from human immunoglobulin libraries. In a preferred embodiment, the antibodies of the invention are human or humanized monoclonal antibodies. The term "monoclonal antibodies", "monoclonal antibody" or "monoclonal antibody composition", as used herein, refers to a population of antibody molecules that contain only one species of an antigen binding site capable of immunoreacting with a particular epitope.

The antibodies of the present invention may be monospecific, bispecific, trispecific or of greater multispecificity. Multispecific antibodies may be specific for different epitopes of a polypeptide of the invention or may be specific for both a polypeptide of the invention as well as for a heterologous epitope, such as a heterologous polypeptide or solid support material. See, *e.g.,* PCT publications WO 93/177 *15;* WO 92/08802; WO 91/00360; WO 92/05793; Tutt, *et al*., J. Immunol. 147:60-69(1991); U.S. Patent Nos. 4,474,893; 4,714,681; 4,925,648; 5,573,920; 5,601,819; and Kostelny *et al*., J. Immunol. 148:1547-1553 (1992).

In a specific embodiment, an antibody of the invention has a dissociation constant or K_{d} of less than 10⁻² M, less than 5 X 10⁻² M, less than 10⁻³ M, less than 5 X 10⁻³ M, less than 10⁻⁴ M, less than 5 X 10⁻⁴ M, less than 10⁻⁵ M, less than 5 X 10⁻⁵ M, less than 10⁻⁶ M, less than 5 X 10⁻⁶ M, less than 10⁻⁷ M, less than 5 X 10⁻⁷ M, less than 10⁻⁸ M, less than 5 X 10⁻⁸ M, less than 10⁻⁹ M, less than 5 X 10⁻⁹ M, less than 10⁻¹⁰ M, less than 5 X 10⁻¹⁰ M, less than 10⁻¹¹ M, less than 5 X 10⁻¹¹ M, less than 10⁻¹² M, less than 5 X 10⁻¹² M, less than 10⁻¹³ M, less than 5 X 10⁻¹³ M, less than 10⁻¹⁴ M, less than 5 X 10⁻¹⁴ M, less than 10⁻¹⁵ M, or less than 5 X 10⁻¹⁵ M.

Polyclonal antibodies can be prepared as described above by immunizing a suitable subject with a polypeptide of the invention as an immunogen. Preferred polyclonal antibody compositions are ones that have been selected for antibodies directed against a polypeptide or polypeptides of the invention. Particularly preferred polyclonal antibody preparations are ones that contain only antibodies directed against a polypeptide or polypeptides of the invention. Particularly preferred immunogen compositions are those that contain no other human proteins such as, for example, immunogen compositions made using a non-human host cell for recombinant expression of a polypeptide of the invention. In such a manner, the only human epitope or epitopes recognized by the resulting antibody compositions raised against this immunogen will be present as part of a polypeptide or polypeptides of the invention.

The antibody titer in the immunized subject can be monitored over time by standard techniques, such as with an enzyme linked immunosorbent assay (ELISA) using immobilized polypeptide. If desired, the antibody molecules can be isolated from the mammal *(e.g.,* from the blood) and further purified by well-known techniques, such as protein A chromatography to obtain the IgG fraction. Alternatively, antibodies specific for a protein or polypeptide of the invention can be selected for *(e.g.,* partially purified) or purified by, *e.g.,* affinity chromatography. For example, a recombinantly expressed and purified (or partially purified) protein of the invention is produced as described herein, and covalently or non-covalently coupled to a solid support such as, for example, a chromatography column. The column can then be used to affinity purify antibodies specific for the proteins of the invention from a sample containing antibodies directed against a large number of different epitopes, thereby generating a substantially purified antibody composition, *i.e*., one that is substantially free of contaminating antibodies. By a substantially purified antibody composition is meant, in this context, that the antibody sample contains at most only 30% (by dry weight) of contaminating antibodies directed against epitopes other than those on the desired protein or polypeptide of the invention, and preferably at most 20%, yet more preferably at most 10%, and most preferably at most 5% (by dry weight) of the sample is contaminating antibodies. A purified antibody composition means that at least 99% of the antibodies in the composition are directed against the desired protein or polypeptide of the invention.

At an appropriate time after immunization, *e.g.*, when the specific antibody titers are highest, antibody-producing cells can be obtained from the subject and used to prepare monoclonal antibodies by standard techniques, such as the hybridoma technique originally described by Kohler and Milstein, 1975, *Nature* 256:495-497, the human B cell hybridoma technique (Kozbor et al., 1983, *Immunol. Today* 4:72), the EBV-hybridoma technique (Cole et al. (1985), *Monoclonal Antibodies and Cancer Therapy*, Alan R. Liss, Inc., pp. 77-96) or trioma techniques. The technology for producing hybridomas is well known *(see generally Current Protocols in Immunology* (1994) Coligan et al. (eds.) John Wiley & Sons, Inc., New York, NY). Hybridoma cells producing a monoclonal antibody of the invention are detected by screening the hybridoma culture supernatants for antibodies that bind the polypeptide of interest, *e.g.*, using a standard ELISA assay.

Alternative to preparing monoclonal antibody-secreting hybridomas, a monoclonal antibody directed against a polypeptide of the invention can be identified and isolated by screening a recombinant combinatorial immunoglobulin library *(e.g.,* an antibody phage display library) with the polypeptide of interest. Kits for generating and screening phage display libraries are commercially available *(e.g.,* the Pharmacia *Recombinant Phage Antibody System,* Catalog No. 27-9400-01; and the Stratagene *SurfZAP™ Phage Display Kit,* Catalog No. 240612). Additionally, examples of methods and reagents particularly amenable for use in generating and screening antibody display library can be found in, for example, U.S. Patent No. 5,223,409; PCT Publication No. WO 92/18619; PCT Publication No. WO 91/17271; PCT Publication No. WO 92/20791; PCT Publication No. WO 92/15679; PCT Publication No. WO 93/01288; PCT Publication No. WO 92/01047; PCT Publication No. WO 92/09690; PCT Publication No. WO 90/02809; Fuchs et al., 1991, *Bio*/*Technology* 9:1370-1372; Hay et al., 1992, *Hum. Antibod. Hybridomas* 3:81-85; Huse et al., 1989, *Science* 246:1275-1281; and Griffiths et al., 1993, *EMBO J*. 12:725-734.

Additionally, recombinant antibodies, such as chimeric and humanized monoclonal antibodies, comprising both human and non-human portions, which can be made using standard recombinant DNA techniques, are within the scope of the invention. A chimeric antibody is a molecule in which different portions are derived from different animal species, such as those having a variable region derived from a murine mAb and a human immunoglobulin constant region. (See, *e.g.*, Cabilly et al., U.S. Patent No. 4,816,567; and Boss et al., U.S. Patent No. 4,816397, which are incorporated herein by reference in their entirety.) Humanized antibodies are antibody molecules from non-human species having one or more complementarily determining regions (CDRs) from the non-human species and a framework region from a human immunoglobulin molecule. (See, *e.g*., Queen, U.S. Patent No. 5,585,089, which is incorporated herein by reference in its entirety.) Such chimeric and humanized monoclonal antibodies can be produced by recombinant DNA techniques known in the art, for example using methods described in PCT Publication No. WO 87/02671; European Patent Application 184,187; European Patent Application 171,496; European Patent Application 173,494; PCT Publication No. WO 86/01533; U.S. Patent No. 4,816,567; European Patent Application 125,023; Better et al., 1988, *Science* 240:1041-1043; Liu et al., 1987, *Proc. Natl. Acad. Sci. USA* 84:3439-3443; Liu et al., 1987, *J. Immunol.* 139:3521-3526; Sun et al., 1987, *Proc. Natl. Acad. Sci. USA* 84:214-218; Nishimura et al., 1987, *Canc. Res.* 47:999-1005; Wood et al., 1985, *Nature* 314:446-449; and Shaw et al., 1988, *J. Natl. Cancer Inst.* 80:1553-1559); Morrison, 1985, *Science* 229:1202-1207; Oi et al., 1986, *Bio*/*Techniques* 4:214; U.S. Patent 5,225,539; Jones et al., 1986, *Nature* 321:552-525; Verhoeyan et al., 1988, *Science* 239:1534; and Beidler et al., 1988, *J. Immunol.* 141:4053-4060.

Completely human antibodies are particularly desirable for therapeutic treatment of human patients. Such antibodies can be produced, for example, using transgenic mice which are incapable of expressing endogenous immunoglobulin heavy and light chains genes, but which can express human heavy and light chain genes. The transgenic mice are immunized in the normal fashion with a selected antigen, *e.g.*, all or a portion of a polypeptide of the invention. Monoclonal antibodies directed against the antigen can be obtained using conventional hybridoma technology. The human immunoglobulin transgenes harbored by the transgenic mice rearrange during B cell differentiation, and subsequently undergo class switching and somatic mutation. Thus, using such a technique, it is possible to produce therapeutically useful IgG, IgM, IgA and IgE antibodies. For an overview of this technology for producing human antibodies, see Lonberg and Huszar (1995, *Int. Rev. Immunol.* 13:65-93). For a detailed discussion of this technology for producing human antibodies and human monoclonal antibodies and protocols for producing such antibodies, *see, e.g.,* U.S. Patent 5,625,126; U.S. Patent 5,633,425; U.S. Patent 5,569,825; U.S. Patent 5,661,016; and U.S. Patent 5,545,806. In addition, companies such as Abgenix, Inc. (Fremont, CA), can be engaged to provide human antibodies directed against a selected antigen using technology similar to that described above.

Completely human antibodies which recognize a selected epitope can be generated using a technique referred to as "guided selection." In this approach a selected non-human monoclonal antibody, *e.g*., a mouse antibody, is used to guide the selection of a completely human antibody recognizing the same epitope. (Jespers et al. (1994) *Bio*/*technology* 12:899-903).

Antibody fragments which recognize specific CD2000 or CD2001 epitopes may be generated by any technique known to those of skill in the art. For example, Fab and F(ab')₂ fragments of the invention may be produced by proteolytic cleavage of immunoglobulin molecules, using enzymes such as papain (to produce Fab fragments) or pepsin (to produce F(ab')₂ fragments). F(ab')₂ fragments contain the variable region, the light chain constant region and the CH1 domain of the heavy chain.

Further, the antibodies of the present invention can also be generated using various phage display methods known in the art. In phage display methods, functional antibody domains are displayed on the surface of phage particles which carry the polynucleotide sequences encoding them. In particular, DNA sequences encoding VH and VL domains are amplified from animal cDNA libraries (*e.g.*, human or murine cDNA libraries of lymphoid tissues). The DNA encoding the VH and VL domains are recombined together with an scFv linker by PCR and cloned into a phagemid vector (*e.g*., pCANTAB6 or pComb3HSS). The vector is electroporated in *E. coli* and the *E. coli* is infected with helper phage. Phage used in these methods are typically filamentous phage including fd and M13 and the VH and VL domains are usually recombinantly fused to either the phage gene III or gene VIII. Phage expressing an antigen binding domain that binds to a CD2000 or CD2001 antigen can be selected or identified with antigen, *e.g.,* using labeled antigen or antigen bound or captured to a solid surface or bead. Examples of phage display methods that can be used to make the antibodies of the present invention include those disclosed in Brinkman *et al*., J. Immunol. Methods 182:41-50 (1995); Ames *et al*., J. Immunol. Methods 184:177-186 (1995); Kettleborough *et al*., Eur. J. Immunol. 24:952-958 (1994); Persic *et al*., Gene 187 9-18 (1997); Burton *et al*., Advances in Immunology 57:191-280(1994); PCT application No. PCT/GB91/O1 134; PCT publications WO 90/02809; WO 91/10737; WO 92/01047; WO 92/18619; WO 93/1 1236; WO 95/15982; WO 95/20401; WO97/13844; and U.S. Patent Nos. 5,698,426; 5,223,409; 5,403,484; 5,580,717; 5,427,908; 5,750,753; 5,821,047; 5,571,698; 5,427,908; 5,516,637; 5,780,225; 5,658,727; 5,733,743 and 5,969,108; each of which is incorporated herein by reference in its entirety.

As described in the above references, after phage selection, the antibody coding regions from the phage can be isolated and used to generate whole antibodies, including human antibodies, or any other desired antigen binding fragment, and expressed in any desired host, including mammalian cells, insect cells, plant cells, yeast, and bacteria, *e.g*., as described below. Techniques to recombinantly produce Fab, Fab' and F(ab')₂ fragments can also be employed using methods known in the art such as those disclosed in PCT publication WO 92/22324; Mullinax *et al*., BioTechniques 12(6):864-869 (1992); and Sawai *et al*., AJRI 34:26-34 (1995); and Better *et al*., Science 240:1041-1043 (1988) (said references incorporated by reference in their entireties).

To generate whole antibodies (*i.e.,* IgG antibodies) or Fab fragments, PCR primers including VH or VL nucleotide sequences, a restriction site, and a flanking sequence to protect the restriction site can be used to amplify the VH or VL sequences in scFv clones. Utilizing cloning techniques known to those of skill in the art, the PCR amplified VH domains can be cloned into vectors expressing a VH constant region, *e.g.,* the human gamma 4 constant region, and the PCR amplified VL domains can be cloned into vectors expressing a VL constant region, *e.g.,* human kappa or lamba constant regions. Preferably, the vectors for expressing the VH or VL domains in eukaryotic cells comprise pcDNA3 vectors containing CMV or EF-1a promoters, a secretion signal, a cloning site for the variable domain, constant domains, and a selection marker such as neomycin or DHFR. Vectors for expressing VH or VL domains in *E. coli* comprise promoters, the constant domain of human IgG (CH1 and CL), leader sequences (pelB, ompA or gIII), a cloning site for the variable domain, and a selection marker such as kanimycin. The VH and VL domains may also cloned into one vector expressing the necessary constant regions. The heavy chain conversion vectors and light chain conversion vectors are then co-transfected into *E*. *coli* or eukaryotic cell lines to generate stable or transient cell lines that express full-length antibodies, *e.g.*, IgG or Fab fragments using techniques known to those of skill in the art. Active Fab fragments produced by stable or transient transfected cell lines will be recovered by Protein A chromatography. IgG antibody produced by stable or transient transfected cell lines will purified using Protein A chromatography. IgG produced by this method may be subjected to enzymatic digestion (*e.g.*, papain) to release Fab or (Fab')2 fragments. The digested Fc fragment would be captured using Protein G affinity chromatography and the Fab or (Fab')₂ will be collected in the flow-through. The specificity and activity of antibodies produced can be analyzed in using assays described herein such as immunoassays.

The present invention also provides for antibodies that have a half-life in an animal, preferably a mammal and most preferably a human, of greater than 10 days, preferably greater than 15 days, greater than 25 days, greater than 30 days, greater than 35 days, greater than 40 days, greater than 45 days, greater than 2 months, greater than 3 months, greater than 4 months, or greater than 5 months. To prolong the serum circulation of antibodies *(e.g.,* monoclonal antibodies, single chain antibodies and Fab fragments) *in vivo*, for example, inert polymer molecules such as high molecular weight polyethyleneglycol (PEG) can be attached to the antibodies with or without a multifunctional linker either through site-specific conjugation of the PEG to the N- or C-terminus of the antibodies or via epsilon-amino groups present on lysine residues. Linear or branched polymer derivatization that results in minimal loss of biological activity will be used. Degree of conjugation will be closely monitored by SDS-PAGE and mass spectrometry to ensure proper conjugation of PEG molecules to the antibodies. Unreacted PEG will be separated from antibody-PEG conjugates by size-exclusion or by ion-exchange chromatography. PEG-derivatized antibodies can be tested for binding activity as well as for *in vivo* efficacy using methods known to those of skill in the art, for example, by immunoassays described herein. Further, antibodies having an increased half-life *in vivo* can be generated as described in PCT Publication No. WO 97/34631 and U.S. Patent No. 6,277,375.

In one aspect, the invention provides substantially purified antibodies or fragments thereof, including human, non-human, chimeric, and humanized antibodies, which antibodies or fragments thereof immunospecifically bind to a polypeptide comprising an amino acid sequence of any one of SEQ ID NO:3, 4, 5, 6, 7, 8, 9, 10, 12, 13, 14, 15, 16, 17, 18, 19, 21, 22, 39, 40, 41, or 42, or an amino acid sequence encoded by the cDNA insert of a EpCD2000 clone deposited with the ATCC® as patent deposit Number PTA-2267. In a preferred embodiment, the invention provides substantially purified human or humanized monoclonal antibodies which immunospecifically bind to a polypeptide comprising an amino acid sequence of any one of SEQ ID NO:3, 4, 5, 6, 7, 8, 9, 10, 12, 13, 14, 15, 16, 17, 18, 19, 21, 22, 39, 40, 41, or 42, or an amino acid sequence encoded by the cDNA insert of a EpCD2000 clone deposited with the ATCC® as patent deposit Number PTA-2267.

In another aspect, the invention provides substantially purified antibodies or fragments thereof, including human, non-human, chimeric and humanized antibodies, which antibodies or fragments thereof immunospecifically bind to a polypeptide comprising a fragment of at least 8 contiguous amino acid residues, preferably at least 10 or at least 15 contiguous amino acid residues, of the amino acid sequence of any one of SEQ ID NO:3, 4, 5, 6, 7, 8, 9, 10, 12, 13, 14, 15, 16, 17, 18, 19, 21, 22, 39, 40, 41, or 42, or an amino acid sequence encoded by the cDNA insert of a EpCD2000 clone deposited with the ATCC® as patent deposit Number PTA-2267. In a preferred embodiment, the invention provides substantially purified human or humanized monoclonal antibodies which immunospecifically bind to a polypeptide comprising a fragment of at least 8 contiguous amino acid residues, preferably at least 15, at least 10, at least 15, at least 20, at least 30, at least 50, at least 75, at least 100, at least 125, at least 150, or at least 175 contiguous amino acid residues, of the amino acid sequence of any one of SEQ ID NO:3, 4, 5, 6, 7, 8, 9, 10, 12, 13, 14, 15, 16, 17, 18, 19, 21, 22, 39, 40, 41, or 42, or an amino acid sequence encoded by the cDNA insert of a EpCD2000 clone deposited with the ATCC® as patent deposit Number PTA-2267.

In another aspect, the invention provides substantially purified antibodies or fragments thereof, including human, non-human, chimeric and humanized antibodies, which antibodies or fragments thereof immunospecifically bind to a polypeptide comprising an amino acid sequence which is at least 65%, preferably at least 75%, at least 85%, at least 95%, or at least 98% identical to the amino acid sequence of any one of SEQ ID NO:3, 4, 5, 6, 7, 8, 9, 10, 12, 13, 14, 15, 16, 17, 18, 19, 21, 22, 39, 40, 41, or 42, wherein the percent identity is determined using a sequence alignment program such as the ALIGN program of the GCG software package with a PAM120 weight residue table, a gap length penalty of 12, and a gap penalty of 4. In a specific embodiment, the invention provides substantially purified human or humanized monoclonal antibodies which immunospecifically bind to a polypeptide comprising an amino acid sequence which is at least 65%, preferably at least 75%, at least 85%, at least 95%, or at least 98% identical to the amino acid sequence of any one of SEQ ID NO:3, 4, 5, 6, 7, 8, 9, 10, 12, 13, 14, 15, 16, 17, 18, 19, 21, 22, 39, 40, 41, or 42, wherein the percent identity is determined using the ALIGN program of the GCG software package with a PAM120 weight residue table, a gap length penalty of 12, and a gap penalty of 4.

In another aspect, the invention provides substantially purified antibodies or fragments thereof, including human, non-human, chimeric and humanized antibodies, which antibodies or fragments thereof immunospecifically bind to a polypeptide comprising and an amino acid sequence which is encoded by a nucleic acid molecule which hybridizes to the nucleic acid molecule consisting of any one of SEQ ID NO:1, 2, 31, 32, 33, 34, 35, 36, 37, 38, 43, 44, 45, 46, 47, 48, 49, 50, 51, 52, 53, 54, 55, 56, 57, 58, 66 or 67, or the cDNA insert of a clone deposited as ATCC® as patent deposit Number PTA-2267, or a complement thereof, under conditions of hybridization of 6X SSC at 45°C and washing in 0.2 X SSC, 0.1% SDS at 50°C, 55°C, 60°C or 65°C, or 6 X SSC at 45°C and washing in 0.1 X SSC, 0.2% SDS at 68°C. In a specific embodiment, the invention provides substantially purified human or humanized monoclonal antibodies which immunospecifically bind to a polypeptide comprising an amino acid sequence which is encoded by a nucleic acid molecule which hybridizes to the nucleic acid molecule consisting of any one of SEQ ID NO:1, 2, 31, 32, 33, 34, 35, 36, 37, 38, 43, 44, 45, 46, 47, 48, 49, 50, 51, 52, 53, 54, 55, 56, 57, 58, 66 or 67, or the cDNA insert of a clone deposited as ATCC® as patent deposit Number PTA-2267, under conditions of hybridization of 6 X SSC at 45°C and washing in 0.2 X SSC, 0.1% SDS at 50°C, 55°C, 60°C or 65°C, or 6 X SSC at 45°C and washing in 0.1 X SSC, 0.2% SDS at 68°C.

In one aspect, the invention provides substantially purified antibodies or fragments thereof, including human, non-human, chimeric, and humanized antibodies, which antibodies or fragments thereof immunospecifically bind to a polypeptide comprising an amino acid sequence of any one of SEQ ID NO:76, 77, 78, 79, 80, 81, 82, 88, 89, 90, 91, 96 or 97. In a preferred embodiment, the invention provides substantially purified human or humanized monoclonal antibodies which immunospecifically bind to a polypeptide comprising an amino acid sequence of any one of SEQ ID NO:76, 77, 78, 79, 80, 81, 82, 88, 89, 90, 91, 96 or 97.

In another aspect, the invention provides substantially purified antibodies or fragments thereof, including human, non-human, chimeric and humanized antibodies, which antibodies or fragments thereof immunospecifically bind to a polypeptide comprising a fragment of at least 8 contiguous amino acid residues, preferably at least 10 or at least 15 contiguous amino acid residues, of the amino acid sequence of any one of SEQ ID NO:76, 77, 78, 79, 80, 81, 82, 88, 89, 90, 91, 96 or 97. In a preferred embodiment, the invention provides substantially purified human or humanized monoclonal antibodies which immunospecifically bind to a polypeptide comprising a fragment of at least 8 contiguous amino acid residues, preferably at least 15, at least 10, at least 15, at least 20, at least 30, at least 50, at least 75, at least 100, at least 125, at least 150, or at least 175 contiguous amino acid residues, of the amino acid sequence of any one of SEQ ID NO:76, 77, 79, 82, 88, 89, 90, 91, 96 or 97.

In another aspect, the invention provides substantially purified antibodies or fragments thereof, including human, non-human, chimeric and humanized antibodies, which antibodies or fragments thereof immunospecifically bind to a polypeptide comprising an amino acid sequence which is at least 70%, preferably at least 70%, at least 75%, at least 85%, at least 95%, or at least 98% identical to the amino acid sequence of any one of SEQ ID NO:76, 77, 78, 79, 80, 81, 82, 88, 89, 90, 91, 96 or 97, wherein the percent identity is determined using a sequence alignment program such as the ALIGN program of the GCG software package with a PAM120 weight residue table, a gap length penalty of 12, and a gap penalty of 4. In a specific embodiment, the invention provides substantially purified human or humanized monoclonal antibodies which immunospecifically bind to a polypeptide comprising an amino acid sequence which is at least 70%, preferably at least 75%, at least 75%, at least 85%, at least 95%, or at least 98% identical to the amino acid sequence of any one of SEQ ID NO:76, 77, 78, 79, 80, 81, 82, 88, 89, 90, 91, 96 or 97, and wherein the percent identity is determined using the ALIGN program of the GCG software package with a PAM120 weight residue table, a gap length penalty of 12, and a gap penalty of 4.

In another aspect, the invention provides substantially purified antibodies or fragments thereof, including human, non-human, chimeric and humanized antibodies, which antibodies or fragments thereof immunospecifically bind to a polypeptide comprising and an amino acid sequence which is encoded by a nucleic acid molecule which hybridizes to the nucleic acid molecule consisting of any one of SEQ ID NO:74, 75, 83, 84, 85, 86, 87, 92, 93, 94 or 95, or a complement thereof, under conditions of hybridization of 6X SSC at 45°C and washing in 0.2 X SSC, 0.1% SDS at 50°C, 55°C, 60°C or 65°C, or 6 X SSC at 45°C and washing in 0.1 X SSC, 0.2% SDS at 68°C. In a specific embodiment, the invention provides substantially purified human or humanized monoclonal antibodies which immunospecifically bind to a polypeptide comprising an amino acid sequence which is encoded by a nucleic acid molecule which hybridizes to the nucleic acid molecule consisting of any one of SEQ ID NO:74, 75, 84, 87, 92, 93, 94 or 95, or a complement thereof, under conditions of hybridization of 6 X SSC at 45°C and washing in 0.2 X SSC, 0.1% SDS at 50°C, 55°C, 60°C or 65 °C, or 6 X SSC at 45°C and washing in 0.1 X SSC, 0.2% SDS at 68°C.

In various embodiments, the substantially purified antibodies or fragments thereof of the invention are polyclonal, monoclonal, Fab fragments, single chain antibodies, or F(ab')2 fragments. The non-human antibodies or fragments thereof of the invention can be goat, mouse, sheep, horse, chicken, rabbit or rat antibodies or antibodies fragments. In a preferred embodiment, the antibodies of the invention are monoclonal antibodies that specifically bind to a polypeptide of the invention.

The substantially purified antibodies or fragments thereof specifically bind to a signal peptide, a secreted sequence, an extracellular domain, a transmembrane or a cytoplasmic domain cytoplasmic membrane of a polypeptide of the invention. In a particularly preferred embodiment, the substantially purified antibodies or fragments thereof of the invention immunospecifically bind to a secreted sequence or an extracellular domain of the amino acid sequence of SEQ ID NO:3, 4, 5, 6, 7, 8, 9, 10, 12, 13, 14, 15, 16, 17, 18, 19, 21, 22, 39, 40, 41, or 42, or the amino acid sequence encoded by the cDNA insert of EpCD2000 deposited with the ATCC® as patent deposit Number PTA-2267. In a particularly preferred embodiment, the substantially purified antibodies or fragments thereof of the invention immunospecifically bind to a secreted sequence or an extracellular domain of the amino acid sequence of SEQ ID NO:76, 77, 79, 82, 88, 89, 90, 91, 96 or 97. In another embodiment, the extracellular domain to which the antibody or antibody fragment binds comprises at least 8 contiguous amino acid residues, preferably at least 15, at least 10, at least 15, at least 20, at least 30, at least 50, at least 75, at least 100, at least 125, at least 150, or at least 175 contiguous amino acid residues, of amino acid residues 23 to 225 of SEQ ID NO:3 (SEQ ID NO:16). In another embodiment, the extracellular domain to which the antibody or antibody fragment binds comprises at least 8 contiguous amino acid residues, preferably at least 15, at least 10, at least 15, at least 20, at least 30, at least 50, at least 75, at least 100, or at least 120 contiguous amino acid residues, of amino acid residues 20 to 236 of SEQ ID NO:76 (SEQ ID NO:79).

In one embodiment, antibodies of the invention are ones that immunospecifically bind to a native polypeptide of the invention or fragment thereof (*e.g.*, a glycosylated polypeptide of the invention or fragment thereof). In another embodiment, antibodies of the invention are ones that immunospecifically bind to a polypeptide of the invention comprising the native primary amino acid sequence, or fragment thereof, lacking post-translational modifications such as glycosylation.

The antibodies of the invention maybe assayed for immunospecific binding to a polypeptide of the invention or fragments thereof and cross-reactivity with other antigens by any method known in the art. Immunoassays which can be used to analyze immunospecific binding and cross-reactivity include, but are not limited to, competitive and non-competitive assay systems using techniques such as western blots, radioimmunoassays, ELISA (enzyme linked immunosorbent assay), "sandwich" immunoassays, immunoprecipitation assays, precipitin reactions, gel diffusion precipitin reactions, immunodiffusion assays, agglutination assays, complement-fixation assays, immunoradiometric assays, fluorescent immunoassays, protein A immunoassays, to name but a few. Such assays are routine and well known in the art (see, *e.g*., Ausubel et al, eds, 1994, Current Protocols in Molecular Biology, Vol. 1, John Wiley & Sons, Inc., New York, which is incorporated by reference herein in its entirety). In a preferred embodiment, the immunospecificity of an antibody is measured in a competitive binding assay. One example of a competitive binding assay is a radioimmunoassay comprising the incubation of labeled polypeptide of the invention *(e.g.,* ³H or ¹²⁵I) with the antibody of interest in the presence of increasing amounts of unlabeled antigen, and the detection of the antibody bound to the labeled polypeptide of the invention.

In a preferred embodiment, BIAcore kinetic analysis is used to determine the binding on and off rates of antibodies to a polypeptide of the invention. BIAcore kinetic analysis is a well known technique that comprises, in this instance, analyzing the binding and dissociation of a polypeptide of the invention from chips with immobilized antibodies on their surface.

An antibody directed against a polypeptide of the invention (*e.g.*, monoclonal antibody) can be used to isolate the polypeptide by standard techniques, such as affinity chromatography or immunoprecipitation. Moreover, such an antibody can be used to detect the protein (*e.g*., in a cellular lysate or cell supernatant) in order to evaluate the abundance and pattern of expression of the polypeptide. The antibodies can also be used diagnostically to monitor protein levels in tissue as part of a clinical testing procedure, *e.g.*, to, for example, determine the efficacy of a given treatment regimen. Detection can be facilitated by coupling the antibody to a detectable substance. Examples of detectable substances include various enzymes, prosthetic groups, fluorescent materials, luminescent materials, bioluminescent materials, and radioactive materials. Examples of suitable enzymes include horseradish peroxidase, alkaline phosphatase, beta-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin, and examples of suitable radioactive material include ¹²⁵I, ¹³¹I, ³⁵S or ³H.

Further, an antibody (or fragment thereof) can be conjugated to a therapeutic moiety such as a cytotoxin, a therapeutic agent or a radioactive metal ion. A cytotoxin or cytotoxic agent includes any agent that is detrimental to cell viability or function. Examples include taxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, and puromycin and analogs or homologs thereof. Therapeutic agents include, but are not limited to, antimetabolites (*e.g*., methotrexate, 6-mercaptopurine, 6-thioguanine, cytarabine, 5-fluorouracil decarbazine), alkylating agents *(e.g.,* mechlorethamine, thioepa chlorambucil, melphalan, carmustine (BSNU) and lomustine (CCNU), cyclothosphamide, busulfan, dibromomannitol, streptozotocin, mitomycin C, and cis-dichlorodiamine platinum (II) (DDP) cisplatin), anthracyclines *(e.g.,* daunorubicin (formerly daunomycin) and doxorubicin), antibiotics (*e.g.*, dactinomycin (formerly actinomycin), bleomycin, mithramycin, and anthramycin (AMC)), and anti-mitotic agents *(e.g.,* vincristine and vinblastine).

Although the conjugates of the invention can be used for modifying a given biological response, the drug moiety is not to be construed as limited to classical chemical therapeutic agents. For example, the drug moiety may be a protein or polypeptide possessing a desired biological activity. Such proteins may include, for example, a toxin such as abrin, ricin A, pseudomonas exotoxin, or diphtheria toxin; a protein such as tumor necrosis factor, interferon-α, interferon-β, nerve growth factor, platelet derived growth factor, tissue plasminogen activator; or, biological response modifiers such as, for example, lymphokines, interleukin-1 ("IL-1"), interleukin-2 ("IL-2"), interleukin-4 ("IL-4"), interleukin-5 ("IL-5"), interleukin-6 ("IL-6"), interleukin-10 ("IL-10"), interleukin-12 ("IL-12"), granulocyte macrophase colony stimulating factor ("GM-CSF"), granulocyte colony stimulating factor ("G-CSF"), or other growth factors.

Techniques for conjugating a therapeutic moiety to antibodies are well known, see, *e.g*., Amon et al., "Monoclonal Antibodies For Immunotargeting Of Drugs In Cancer Therapy", in Monoclonal Antibodies And Cancer Therapy, Reisfeld et al. (eds.), pp. 243-56 (Alan R. Liss, Inc. 1985); Hellstrom et al., "Antibodies For Drug Delivery", in Controlled Drug Delivery (2nd Ed.), Robinson et al. (eds.), pp. 623-53 (Marcel Dekker, Inc. 1987); Thorpe, "Antibody Carriers Of Cytotoxic Agents In Cancer Therapy: A Review", in Monoclonal Antibodies '84: Biological And Clinical Applications, Pinchera et al. (eds.), pp. 475-506 (1985); "Analysis, Results, And Future Prospective Of The Therapeutic Use Of Radiolabeled Antibody In Cancer Therapy", in Monoclonal Antibodies For Cancer Detection And Therapy, Baldwin et al. (eds.), pp. 303-16 (Academic Press 1985), and Thorpe et al., "The Preparation And Cytotoxic Properties Of Antibody-Toxin Conjugates", Immunol. Rev., 62:119-58 (1982).

Alternatively, an antibody can be conjugated to a second antibody to form an antibody heteroconjugate as described by Segal in U.S. Patent No. 4,676,980.

The invention also provides a kit containing an antibody of the invention conjugated to a detectable substance, and instructions for use. Still another aspect of the invention is a pharmaceutical composition comprising an antibody of the invention and a pharmaceutically acceptable carrier. In preferred embodiments, the pharmaceutical composition contains an antibody of the invention, a therapeutic moiety, and a pharmaceutically acceptable carrier.

### III. Recombinant Expression Vectors and Host Cells

Another aspect of the invention pertains to vectors, preferably expression vectors, containing a nucleic acid encoding a polypeptide of the invention (or a fragment thereof). As used herein, the term "vector" refers to a nucleic acid molecule capable of transporting another nucleic acid to which it has been linked. One type of vector is a "plasmid", which refers to a circular double stranded DNA loop into which additional DNA segments can be ligated. Another type of vector is a viral vector, wherein additional DNA segments can be ligated into the viral genome. Certain vectors are capable of autonomous replication in a host cell into which they are introduced (*e.g*., bacterial vectors having a bacterial origin of replication and episomal mammalian vectors). Other vectors (*e.g*., non-episomal mammalian vectors) are integrated into the genome of a host cell upon introduction into the host cell, and thereby are replicated along with the host genome. Moreover, certain vectors, expression vectors, are capable of directing the expression of genes to which they are operably linked. In general, expression vectors of utility in recombinant DNA techniques are often in the form of plasmids (vectors). However, the invention is intended to include such other forms of expression vectors, such as viral vectors *(e.g.,* replication defective retroviruses, adenoviruses and adeno-associated viruses), which serve equivalent functions.

The recombinant expression vectors of the invention comprise a nucleic acid of the invention in a form suitable for expression of the nucleic acid in a host cell. This means that the recombinant expression vectors include one or more regulatory sequences, selected on the basis of the host cells to be used for expression, which is operably linked to the nucleic acid sequence to be expressed. Within a recombinant expression vector, "operably linked" is intended to mean that the nucleotide sequence of interest is linked to the regulatory sequence(s) in a manner which allows for expression of the nucleotide sequence (*e.g*., in an *in vitro* transcription/translation system or in a host cell when the vector is introduced into the host cell). The term "regulatory sequence" is intended to include promoters, enhancers and other expression control elements (*e.g*., polyadenylation signals). Such regulatory sequences are described, for example, in Goeddel, *Gene Expression Technology: Methods in Enzymology* 185, Academic Press, San Diego, CA (1990). Regulatory sequences include those which direct constitutive expression of a nucleotide sequence in many types of host cell and those which direct expression of the nucleotide sequence only in certain host cells (*e.g*., tissue-specific regulatory sequences). It will be appreciated by those skilled in the art that the design of the expression vector can depend on such factors as the choice of the host cell to be transformed, the level of expression of protein desired, etc. The expression vectors of the invention can be introduced into host cells to thereby produce proteins or peptides, including fusion proteins or peptides, encoded by nucleic acids as described herein.

The recombinant expression vectors of the invention can be designed for expression of a polypeptide of the invention in prokaryotic (*e.g., E. coli*) or eukaryotic cells (*e.g*., insect cells (using baculovirus expression vectors), yeast cells, or mammalian cells). Suitable host cells are discussed further in Goeddel, *supra.* Alternatively, the recombinant expression vector can be transcribed and translated *in vitro,* for example using T7 promoter regulatory sequences and T7 polymerase.

Expression of proteins in prokaryotes is most often carried out in *E. coli* with vectors containing constitutive or inducible promoters directing the expression of either fusion or non-fusion proteins. Fusion vectors add a number of amino acids to a protein encoded therein, usually to the amino terminus of the recombinant protein. Such fusion vectors typically serve three purposes: 1) to increase expression of recombinant protein; 2) to increase the solubility of the recombinant protein; and 3) to aid in the purification of the recombinant protein by acting as a ligand in affinity purification. Often, in fusion expression vectors, a proteolytic cleavage site is introduced at the junction of the fusion moiety and the recombinant protein to enable separation of the recombinant protein from the fusion moiety subsequent to purification of the fusion protein. Such enzymes, and their cognate recognition sequences, include Factor Xa, thrombin and enterokinase. Typical fusion expression vectors include pGEX (Pharmacia Biotech Inc; Smith and Johnson, 1988, *Gene* 67:31-40), pMAL (New England Biolabs, Beverly, MA) and pRIT5 (Pharmacia, Piscataway, NJ) which fuse glutathione S-transferase (GST), maltose E binding protein, or protein A, respectively, to the target recombinant protein.

Examples of suitable inducible non-fusion *E. coli* expression vectors include pTrc (Amann et al., 1988, *Gene* 69:301-315) and pET 11d (Studier et al., *Gene Expression Technology: Methods in Enzymology* 185, Academic Press, San Diego, California (1990) 60-89). Target gene expression from the pTrc vector relies on host RNA polymerase transcription from a hybrid trp-lac fusion promoter. Target gene expression from the pET 11d vector relies on transcription from a T7 gn10-lac fusion promoter mediated by a coexpressed viral RNA polymerase (T7 gnl). This viral polymerase is supplied by host strains BL21(DE3) or HMS174(DE3) from a resident λ prophage harboring a T7 gn1 gene under the transcriptional control of the lacUV 5 promoter.

One strategy to maximize recombinant protein expression in *E. coli* is to express the protein in a host bacteria with an impaired capacity to proteolytically cleave the recombinant protein (Gottesman, *Gene Expression Technology: Methods in Enzymology* 185, Academic Press, San Diego, California (1990) 119-128). Another strategy is to alter the nucleic acid sequence of the nucleic acid to be inserted into an expression vector so that the individual codons for each amino acid are those preferentially utilized in *E. coli* (Wada et al., 1992, *Nucleic Acids Res.* 20:2111-2118). Such alteration of nucleic acid sequences of the invention can be carried out by standard DNA synthesis techniques.

In another embodiment, the expression vector is a yeast expression vector. Examples of vectors for expression in yeast *S. cerivisae* include pYepSecl (Baldari et al., 1987, *EMBO J*. 6:229-234), pMFa (Kurjan and Herskowitz, 1982, *Cell* 30:933-943), pJRY88 (Schultz et al., 1987, *Gene* 54:113-123), pYES2 (Invitrogen Corporation, San Diego, CA), and pPicZ (Invitrogen Corp, San Diego, CA).

Alternatively, the expression vector is a baculovirus expression vector. Baculovirus vectors available for expression of proteins in cultured insect cells (*e.g*., Sf 9 cells) include the pAc series (Smith et al., 1983, *Mol. Cell Biol.* 3:2156-2165) and the pVL series (Lucklow and Summers, 1989, *Virology* 170:31-39).

In yet another embodiment, a nucleic acid of the invention is expressed in mammalian cells using a mammalian expression vector. Examples of mammalian expression vectors include pCDM8 (Seed, 1987, *Nature* 329:840) and pMT2PC (Kaufman et al., 1987, *EMBO J*. 6:187-195). When used in mammalian cells, the expression vector's control functions are often provided by viral regulatory elements. For example, commonly used promoters are derived from polyoma, Adenovirus 2, cytomegalovirus and Simian Virus 40. For other suitable expression systems for both prokaryotic and eukaryotic cells see chapters 16 and 17 of Sambrook et al., *supra.*

In another embodiment, the recombinant mammalian expression vector is capable of directing expression of the nucleic acid preferentially in a particular cell type (*e.g.*, tissue-specific regulatory elements are used to express the nucleic acid). Tissue-specific regulatory elements are known in the art. Non-limiting examples of suitable tissue-specific promoters include the albumin promoter (liver-specific; Pinkert et al., 1987, *Genes Dev*. 1:268-277), lymphoid-specific promoters (Calame and Eaton, 1988, *Adv. Immunol*. 43:235-275), in particular, promoters of T cell receptors (Winoto and Baltimore, 1989, *EMBO J*. 8:729-733) and immunoglobulins (Banerji et al., 1983), Cell 33:729-740; Queen and Baltimore, 1983), Cell 33:741-748), neuron-specific promoters (*e.g*., the neurofilament promoter; Byrne and Ruddle, 1989, *Proc. Natl. Acad. Sci. USA* 86:5473-5477), pancreas-specific promoters (Edlund et al., 1985, *Science* 230:912-916), and mammary gland-specific promoters (*e.g*., milk whey promoter; U.S. Patent No. 4,873,316 and European Application Publication No. 264,166). Developmentally-regulated promoters are also encompassed, for example, the mouse hox promoters (Kessel and Gruss, 1990, *Science* 249:374-379) and the beta-fetoprotein promoter (Campes and Tilghman, 1989, *Genes Dev.* 3:537-546).

The invention further provides a recombinant expression vector comprising a DNA molecule of the invention cloned into the expression vector in an antisense orientation. That is, the DNA molecule is operably linked to a regulatory sequence in a manner which allows for expression (by transcription of the DNA molecule) of an RNA molecule which is antisense to the mRNA encoding a polypeptide of the invention. Regulatory sequences operably linked to a nucleic acid cloned in the antisense orientation can be chosen which direct the continuous expression of the antisense RNA molecule in a variety of cell types. For instance, viral promoters and/or enhancers, or regulatory sequences can be chosen which direct constitutive, tissue specific, or cell type specific expression of antisense RNA. The antisense expression vector can be in the form of a recombinant plasmid, phagemid or attenuated virus in which antisense nucleic acids are produced under the control of a high efficiency regulatory region, the activity of which can be determined by the cell type into which the vector is introduced. For a discussion of the regulation of gene expression using antisense genes see Weintraub et al. (*Reviews - Trends in Genetics*, Vol. 1(1) 1986).

Another aspect of the invention pertains to host cells into which a recombinant expression vector of the invention has been introduced. The terms "host cell" and "recombinant host cell" are used interchangeably herein. It is understood that such terms refer not only to the particular subject cell but to the progeny or potential progeny of such a cell. Because certain modifications may occur in succeeding generations due to either mutation or environmental influences, such progeny may not, in fact, be identical to the parent cell, but are still included within the scope of the term as used herein.

A host cell can be any prokaryotic (*e.g.*, *E. coli*) or eukaryotic cell (*e.g*., insect cells, yeast or mammalian cells). Host cells intended to be part of the invention include ones that comprise a nucleic acid molecule of the invention that has been engineered to be present within the host cell (*e.g*., as part of a vector), and ones that comprise nucleic acid regulatory sequences that have been engineered to be present in the host cell such that a nucleic acid molecule of the invention is expressed within the host cell.

Vector DNA can be introduced into prokaryotic or eukaryotic cells via conventional transformation or transfection techniques. As used herein, the terms "transformation" and "transfection" are intended to refer to a variety of art-recognized techniques for introducing foreign nucleic acid into a host cell, including calcium phosphate or calcium chloride co-precipitation, DEAE-dextran-mediated transfection, lipofection, or electroporation. Suitable methods for transforming or transfecting host cells can be found in Sambrook, et al. (*supra*), and other laboratory manuals.

For stable transfection of mammalian cells, it is known that, depending upon the expression vector and transfection technique used, only a small fraction of cells may integrate the foreign DNA into their genome. In order to identify and select these integrants, a gene that encodes a selectable marker (*e.g*., for resistance to antibiotics) is generally introduced into the host cells along with the gene of interest. Preferred selectable markers include those which confer resistance to drugs, such as G418, hygromycin and methotrexate. Cells stably transfected with the introduced nucleic acid can be identified by drug selection (*e.g*., cells that have incorporated the selectable marker gene will survive, whereas cells that have not die).

In another embodiment, the expression characteristics of an endogenous gene of interest within a cell, cell line, or microorganism may be modified by inserting a DNA regulatory element heterologous to the endogenous gene of interest into the genome of a cell, stable cell line, or cloned microorganism such that the inserted regulatory element is operatively linked with the endogenous gene of interest and controls, modulates or activates. For example, endogenous CD2000 genes which are normally "transcriptionally silent", *i.e*., CD2000 genes which is normally not expressed, or are expressed only at very low levels in a cell line or microorganism, may be activated by inserting a regulatory element which is capable of promoting the expression of a normally expressed gene product in that cell line or microorganism. Alternatively, transcriptionally silent, endogenous CD2000 genes may be activated by insertion of a promiscuous regulatory element that works across cell types.

A heterologous regulatory element may be inserted into a stable cell line or cloned microorganism, such that it is operatively linked with and activates expression of endogenous CD2000 or CD2001 genes, using techniques, such as targeted homologous recombination, which are well known to those of skill in the art, and described, *e.g*., in Chappel, U.S. Patent No. 5,272,071; and PCT publication No. WO 91/06667, published May 16, 1991.

A host cell of the invention, such as a prokaryotic or eukaryotic host cell in culture, can be used to produce a polypeptide of the invention. Accordingly, the invention further provides methods for producing a polypeptide of the invention using the host cells of the invention. In one embodiment, the method comprises culturing the host cell of invention (into which a recombinant expression vector encoding a polypeptide of the invention has been introduced) in a suitable medium such that the polypeptide is produced. In another embodiment, the method further comprises isolating the polypeptide from the medium or the host cell.

The host cells of the invention can also be used to produce nonhuman transgenic animals. For example, in one embodiment, a host cell of the invention is a fertilized oocyte or an embryonic stem cell into which a sequence encoding a polypeptide of the invention has been introduced. Such host cells can then be used to create non-human transgenic animals in which exogenous sequences encoding a polypeptide of the invention have been introduced into their genome. Further, such host cells can be used to create or homologous recombinant animals in which endogenous gene sequences encoding a polypeptide of the invention have been altered. Such animals are useful for studying the function and/or activity of the polypeptide and for identifying and/or evaluating modulators of polypeptide activity. As used herein, a "transgenic animal" is a non-human animal, preferably a mammal, more preferably a rodent such as a rat or mouse, in which one or more of the cells of the animal includes a transgene. Other examples of transgenic animals include non-human primates, sheep, dogs, cows, goats, chickens, amphibians, etc. A transgene is exogenous DNA which is integrated into the genome of a cell from which a transgenic animal develops and which remains in the genome of the mature animal, thereby directing the expression of an encoded gene product in one or more cell types or tissues of the transgenic animal. As used herein, a "homologous recombinant animal" is a non-human animal, preferably a mammal, more preferably a mouse, in which an endogenous gene has been altered by homologous recombination between the endogenous gene and an exogenous DNA molecule introduced into a cell of the animal, *e.g.*, an embryonic cell of the animal, prior to development of the animal.

A transgenic animal of the invention can be created by introducing a nucleic acid molecule encoding a polypeptide of the invention (or a homologue thereof) into the male pronuclei of a fertilized oocyte, *e.g.,* by microinjection, retroviral infection, and allowing the oocyte to develop in a pseudopregnant female foster animal. Intronic sequences and polyadenylation signals can also be included in the transgene to increase the efficiency of expression of the transgene. A tissue-specific regulatory sequence(s) can be operably linked to the transgene to direct expression of the polypeptide of the invention to particular cells. Methods for generating transgenic animals via embryo manipulation and microinjection, particularly animals such as mice, have become conventional in the art and are described, for example, in U.S. Patent Nos. 4,736,866 and 4,870,009, U.S. Patent No. 4,873,191, in Hogan, *Manipulating the Mouse Embryo,* (Cold Spring Harbor Laboratory Press, Cold Spring Harbor, N.Y., 1986) and in Wakayama *et al*., 1999, *Proc. Natl. Acad. Sci. USA*, 96:14984-14989. Similar methods are used for production of other transgenic animals. A transgenic founder animal can be identified based upon the presence of the transgene in its genome and/or expression of mRNA encoding the transgene in tissues or cells of the animals. A transgenic founder animal can then be used to breed additional animals carrying the transgene. Moreover, transgenic animals carrying the transgene can further be bred to other transgenic animals carrying other transgenes.

To create an homologous recombinant animal, a vector is prepared which contains at least a portion of a gene encoding a polypeptide of the invention into which a deletion, addition or substitution has been introduced to thereby alter, *e.g*., functionally disrupt, the gene. In a preferred embodiment, the vector is designed such that, upon homologous recombination, the endogenous gene is functionally disrupted (*i.e*., no longer encodes a functional protein; also referred to as a "knock out" vector). Alternatively, the vector can be designed such that, upon homologous recombination, the endogenous gene is mutated or otherwise altered but still encodes functional protein (*e.g*., the upstream regulatory region can be altered to thereby alter the expression of the endogenous protein). In the homologous recombination vector, the altered portion of the gene is flanked at its 5' and 3' ends by additional nucleic acid of the gene to allow for homologous recombination to occur between the exogenous gene carried by the vector and an endogenous gene in an embryonic stem cell. The additional flanking nucleic acid sequences are of sufficient length for successful homologous recombination with the endogenous gene. Typically, several kilobases of flanking DNA (both at the 5' and 3' ends) are included in the vector (*see, e.g.,* Thomas and Capecchi, 1987, *Cell* 51:503 for a description of homologous recombination vectors). The vector is introduced into an embryonic stem cell line (*e.g*., by electroporation) and cells in which the introduced gene has homologously recombined with the endogenous gene are selected *(see, e.g*., Li et al., 1992, *Cell* 69:915). The selected cells are then injected into a blastocyst of an animal (*e.g*., a mouse) to form aggregation chimeras *(see, e.g*., Bradley in *Teratocarcinomas and Embryonic Stem Cells: A Practical Approach,* Robertson, ed. (IRL, Oxford, 1987) pp. 113-152). A chimeric embryo can then be implanted into a suitable pseudopregnant female foster animal and the embryo brought to term. Progeny harboring the homologously recombined DNA in their germ cells can be used to breed animals in which all cells of the animal contain the homologously recombined DNA by germline transmission of the transgene. Methods for constructing homologous recombination vectors and homologous recombinant animals are described further in Bradley, 1991, *Current Opinion in Bio*/*Technology* 2:823-829 and in PCT Publication Nos. WO 90/11354, WO 91/01140, WO 92/0968, and WO 93/04169.

In another embodiment, transgenic non-human animals can be produced which contain selected systems which allow for regulated expression of the transgene. One example of such a system is the *cre*/*loxP* recombinase system of bacteriophage P1. For a description of the *cre*/*loxP* recombinase system, *see, e.g.,* Lakso et al., 1992, *Proc. Natl. Acad. Sci. USA* 89:6232-6236. Another example of a recombinase system is the FLP recombinase system of *Saccharomyces cerevisiae* (O'Gorman et al., 1991, *Science* 251:1351-1355. If a *cre*/*loxP* recombinase system is used to regulate expression of the transgene, animals containing transgenes encoding both the *Cre* recombinase and a selected protein are required. Such animals can be provided through the construction of "double" transgenic animals, *e.g*., by mating two transgenic animals, one containing a transgene encoding a selected protein and the other containing a transgene encoding a recombinase.

Clones of the non-human transgenic animals described herein can also be produced according to the methods described in Wilmut et al., 1997, *Nature* 385:810-813 and PCT Publication NOS. WO 97/07668 and WO 97/07669.

### IV. Pharmaceutical Compositions

The nucleic acid molecules, polypeptides, antibodies, CD2000 modulators, and CD2001 modulators (also referred to herein as "active compounds") of the invention can be incorporated into pharmaceutical compositions suitable for administration. Such compositions typically comprise the nucleic acid molecule, polypeptide, antibody, or modulator and a pharmaceutically acceptable carrier. As used herein, the language "pharmaceutically acceptable carrier" is intended to include any and all solvents, dispersion media, coatings, antibacterial and antifungal agents, isotonic and absorption delaying agents, and the like, compatible with pharmaceutical administration. The use of such media and agents for pharmaceutically active substances is well known in the art. Except insofar as any conventional media or agent is incompatible with the active compound, use thereof in the compositions is contemplated. Supplementary active compounds can also be incorporated into the compositions.

The invention includes methods for preparing pharmaceutical compositions for modulating the expression or activity of a polypeptide or nucleic acid of the invention. Such methods comprise formulating a pharmaceutically acceptable carrier with an agent which modulates expression or activity of a polypeptide or nucleic acid of the invention. Such compositions can further include additional active agents. Thus, the invention further includes methods for preparing a pharmaceutical composition by formulating a pharmaceutically acceptable carrier with an agent which modulates expression or activity of a polypeptide or nucleic acid of the invention and one or more additional active compounds.

A pharmaceutical composition of the invention is formulated to be compatible with its intended route of administration. Examples of routes of administration include parenteral, *e.g*., intravenous, intradermal, subcutaneous, oral (*e.g*., inhalation), intranasal, transdermal (topical), transmucosal, and rectal administration. Solutions or suspensions used for parenteral, intradermal, intratumoral or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersions. For intravenous administration, suitable carriers include physiological saline, bacteriostatic water, Cremophor EL™ (BASF; Parsippany, NJ) or phosphate buffered saline (PBS). In all cases, the composition must be sterile and should be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as mannitol, sorbitol, sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound (*e.g.*, a polypeptide or antibody) in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed.

Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from a pressurized container or dispenser which contains a suitable propellant, *e.g.*, a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The compounds can also be prepared in the form of suppositories (*e.g.*, with conventional suppository bases such as cocoa butter and other glycerides) or retention enemas for rectal delivery.

In one embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation (Mountain View, CA) and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Patent No. 4,522,811.

It is especially advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals.

For antibodies, the preferred dosage is 0.1 mg/kg to 100 mg/kg of body weight (generally 10 mg/kg to 20 mg/kg). If the antibody is to act in the brain, a dosage of 50 mg/kg to 100 mg/kg is usually appropriate. Generally, partially human antibodies and fully human antibodies have a longer half-life within the human body than other antibodies. Accordingly, lower dosages and less frequent administration is often possible. Modifications such as lipidation can be used to stabilize antibodies and to enhance uptake and tissue penetration (*e.g.,* into the brain). A method for lipidation of antibodies is described by Cruikshank et al. ((1997) *J. Acquired Immune Deficiency Syndromes and Human Retrovirology* 14:193).

As defined herein, a therapeutically effective amount of protein or polypeptide *(i.e.,* an effective dosage) ranges from about 0.001 to 30 mg/kg body weight, preferably about 0.01 to 25 mg/kg body weight, more preferably about 0.1 to 20 mg/kg body weight, and even more preferably about 1 to 10 mg/kg, 2 to 9 mg/kg, 3 to 8 mg/kg, 4 to 7 mg/kg, or 5 to 6 mg/kg body weight.

The skilled artisan will appreciate that certain factors may influence the dosage required to effectively treat a subject, including but not limited to the severity of the disease or disorder, previous treatments, the general health and/or age of the subject, and other diseases present. Moreover, treatment of a subject with a therapeutically effective amount of a protein, polypeptide, or antibody can include a single treatment or, preferably, can include a series of treatments. In a preferred example, a subject is treated with antibody, protein, or polypeptide in the range of between about 0.1 to 20 mg/kg body weight, one time per week for between about 1 to 10 weeks, preferably between 2 to 8 weeks, more preferably between about 3 to 7 weeks, and even more preferably for about 4, 5, or 6 weeks. It will also be appreciated that the effective dosage of antibody, protein, or polypeptide used for treatment may increase or decrease over the course of a particular treatment. Changes in dosage may result and become apparent from the results of diagnostic assays as described herein.

A "subject" as used herein in the context of diagnostic and therapeutic uses described herein refers to an animal, preferably a mammal *(e.g.,* a dog, cat, pig, horse, cow, rabbit, and mouse), and most preferably a human.

Preferably, administration of the CD2000 modulator or CD2001 modulator is at or near the site of the cells or tissue to be treated, *e.g.*, administration is at or near the site of a disorder such as one of those described herein.

In certain embodiments, the CD2000 modulator or CD2001 modulator is administered or co-administered with at least one other desirable agent, *e.g.*, a chemotherapeutic agent, an immunomodulatory agent such as, *e.g.*, anti-CD40L antibody, CTLA4Ig, or anti-CD2 antibody, or an anti-inflammatory agent such as, *e.g.*, aspirin or a corticosteroid.

In certain instances, it is preferred that administration of a CD2000 modulator or CD2001 modulator comprises an initial bolus followed by continuous infusion. For example, such instances will generally include those wherein the modulator exhibits appreciable reversibility in platelet binding, as, *e.g.,* assayed via the techniques described herein.

The present invention encompasses agents which modulate expression or activity of CD2000. The present invention also encompasses agents which modulate expression or activity of CD2001. An agent may, for example, be a small molecule. For example, such small molecules include, but are not limited to, peptides, peptidomimetics, amino acids, amino acid analogs, polynucleotides, polynucleotide analogs, nucleotides, nucleotide analogs, organic or inorganic compounds *(i.e,.* including heteroorganic and organometallic compounds) having a molecular weight less than about 10,000 grams per mole, organic or inorganic compounds having a molecular weight less than about 5,000 grams per mole, organic or inorganic compounds having a molecular weight less than about 1,000 grams per mole, organic or inorganic compounds having a molecular weight less than about 500 grams per mole, and salts, esters, and other pharmaceutically acceptable forms of such compounds. With respect to small organic molecules, such compounds are generally ones that are orally active (that is, can be administered orally).

It is understood that appropriate doses of small molecule agents depends upon a number of factors within the ken of the ordinarily skilled physician, veterinarian, or researcher. The dose(s) of the small molecule will vary, for example, depending upon the identity, size, and condition of the subject or sample being treated, further depending upon the route by which the composition is to be administered, if applicable, and the effect which the practitioner desires the small molecule to have upon the nucleic acid or polypeptide of the invention. Exemplary doses include milligram or microgram amounts of the small molecule per kilogram of subject or sample weight *(e.g.,* about 1 microgram per kilogram to about 500 milligrams per kilogram, about 100 micrograms per kilogram to about 5 milligrams per kilogram, or about 1 microgram per kilogram to about 50 micrograms per kilogram. It is furthermore understood that appropriate doses of a small molecule depend upon the potency of the small molecule with respect to the expression or activity to be modulated. Such appropriate doses may be determined using the assays described herein. When one or more of these small molecules is to be administered to an animal *(e.g.,* a human) in order to modulate expression or activity of a polypeptide or nucleic acid of the invention, a physician, veterinarian, or researcher may, for example, prescribe a relatively low dose at first, subsequently increasing the dose until an appropriate response is obtained. In addition, it is understood that the specific dose level for any particular animal subject will depend upon a variety of factors including the activity of the specific compound employed, the age, body weight, general health, gender, and diet of the subject, the time of administration, the route of administration, the rate of excretion, any drug combination, and the degree of expression or activity to be modulated.

The nucleic acid molecules of the invention can be inserted into vectors and used as gene therapy vectors. Gene therapy vectors can be delivered to a subject by, for example, intravenous injection, local administration (U.S. Patent 5,328,470) or by stereotactic injection *(see, e.g.,* Chen et al. (1994) *Proc. Natl. Acad. Sci. USA* 91:3054-3057). The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells, *e.g.,* retroviral vectors, the pharmaceutical preparation can include one or more cells which produce the gene delivery system.

The pharmaceutical compositions can be included in a container, pack, or dispenser together with instructions for administration.

### V. Uses and Methods of the Invention

The nucleic acid molecules, proteins, protein homologs, antibodies, CD2000 modulators, and CD2001 modulators described herein can be used in one or more of the following methods: a) screening assays; b) detection assays *(e.g.,* chromosomal mapping, tissue typing, forensic biology); c) predictive medicine *(e.g.,* diagnostic assays, prognostic assays, monitoring clinical trials, and pharmacogenomics); and d) methods of treatment (*e.g.*, therapeutic and prophylactic).

Further, the activity of polypeptides of the invention, as well as the effect of CD2000 modulators on CD2000 or CD2001 modulators on CD2001 can routinely be ascertained via use of a number of different *in vitro* or cell-based systems well known to those of skill in the art. For example, the intracellular signaling molecules activated in response to the binding of CD2000 or CD2001 to its ligand can be measured *in vitro* using standard assays known to those of skill in the art such as immunoprecipitation followed by Western blot analysis. The activity of polypeptides of the invention, including the therapeutic, *e.g.*, clinical, efficacy of the polypeptides, as well as the effect, including the therapeutic, *e.g.*, clinical, efficacy, of CD2000 modulators or CD2001 modulators can routinely be ascertained via use of a number of different animal models well known to those of skill in the art. Such methods of ascertaining the activity of polypeptides of the invention, as wells as screening assays for ascertaining the modulatory activity of test compounds are described below.

The isolated nucleic acid molecules of the invention can be used to express proteins (*e.g.,* via a recombinant expression vector in a host cell in gene therapy applications), to detect mRNA *(e.g.,* in a biological sample) or a genetic lesion, and to modulate activity of a polypeptide of the invention. In addition, the polypeptides of the invention can be used to screen drugs or compounds which modulate activity or expression of a polypeptide of the invention as well as to treat disorders characterized by insufficient or excessive production of a protein of the invention or production of a form of a protein of the invention which has decreased or aberrant activity compared to the wild type protein. In addition, the antibodies of the invention can be used to detect and isolate a protein of the invention, isolate any protein of interest when such protein is present as a fusion protein with a polypeptide of the invention, or to modulate activity of a protein of the invention.

This invention further pertains to novel agents (modulators) identified by the above-described screening assays and uses thereof for treatments as described herein.

### A. Screening Assays

The invention provides a method (also referred to herein as a "screening assay") for identifying modulators, *i.e.*, candidate or test compounds or agents *(e.g.,* peptides, peptidomimetics, small molecules or other drugs) which bind to polypeptide of the invention or have a modulatory (*e.g*., stimulatory or inhibitory) effect on, for example, expression or activity of a polypeptide of the invention.

In one embodiment, the invention provides assays for screening candidate or test compounds which bind to or modulate the activity of the membrane-bound form of a polypeptide of the invention or biologically active portion thereof. The test compounds of the present invention can be obtained using any of the numerous approaches in combinatorial library methods known in the art, including: biological libraries; spatially addressable parallel solid phase or solution phase libraries; synthetic library methods requiring deconvolution; the "one-bead one-compound" library method; and synthetic library methods using affinity chromatography selection. The biological library approach is limited to peptide libraries, while the other four approaches are applicable to peptide, non-peptide oligomer or small molecule libraries of compounds (Lam (1997) *Anticancer Drug Des*. 12:145).

Examples of methods for the synthesis of molecular libraries can be found in the art, for example in: DeWitt et al. (1993) *Proc. Natl. Acad. Sci. USA* 90:6909; Erb et al. (1994) *Proc. Natl. Acad. Sci. USA* 91:11422; Zuckermann et al. (1994). *J. Med. Chem.* 37:2678; Cho et al. (1993) *Science* 261:1303; Carrell et al. (1994) *Angew. Chem. Int. Ed. Engl.* 33:2059; Carell et al. (1994) *Angew. Chem. Int. Ed. Engl.* 33:2061; and Gallop et al. (1994) *J. Med. Chem*. 37:1233.

Libraries of compounds may be presented in solution *(e.g.,* Houghten (1992) *Bio*/*Techniques* 13:412-421), or on beads (Lam (1991) *Nature* 354:82-84), chips (Fodor (1993) *Nature 364:555-556),* bacteria (U.S. Patent No. 5,223,409), spores (Patent NOS. 5,571,698; 5,403,484; and *5,223,409),* plasmids (Cull et al. (1992) *Proc. Natl. Acad. Sci. USA* 89:1865-1869) or phage (Scott and Smith (1990) *Science* 249:386-390; Devlin (1990) *Science* 249:404-406; Cwirla et al. (1990) *Proc. Natl. Acad. Sci. USA* 87:6378-6382; and Felici (1991) *J. Mol. Biol.* 222:301-310).

### Cell-Free Assays

In one embodiment, an assay of the present invention is a cell-free assay comprising contacting a polypeptide of the invention or biologically active portion thereof with a test compound and determining the ability of the test compound to bind to the polypeptide or biologically active portion thereof. Binding of the test compound to the polypeptide can be determined either directly or indirectly as described above. In a preferred embodiment, the assay includes contacting the polypeptide of the invention or biologically active portion thereof with a known compound which binds the polypeptide to form an assay mixture, contacting the assay mixture with a test compound, and determining the ability of the test compound to interact with the polypeptide, wherein determining the ability of the test compound to interact with the polypeptide comprises determining the ability of the test compound to preferentially bind to the polypeptide or biologically active portion thereof as compared to the known compound.

In another embodiment, an assay is a cell-free assay comprising contacting a polypeptide of the invention or biologically active portion thereof with a test compound and determining the ability of the test compound to modulate (*e.g.*, stimulate or inhibit) the activity of the polypeptide or biologically active portion thereof. Determining the ability of the test compound to modulate the activity of the polypeptide can be accomplished, for example, by determining the ability of the polypeptide to bind to a target molecule by one of the methods described above for determining direct binding. In an alternative embodiment, determining the ability of the test compound to modulate the activity of the polypeptide can be accomplished by determining the ability of the polypeptide of the invention to further modulate the target molecule. For example, the catalytic/enzymatic activity of the target molecule on an appropriate substrate can be determined as previously described.

In yet another embodiment, the cell-free assay comprises contacting a polypeptide of the invention or biologically active portion thereof with a known compound which binds the polypeptide to form an assay mixture, contacting the assay mixture with a test compound, and determining the ability of the test compound to interact with the polypeptide, wherein determining the ability of the test compound to interact with the polypeptide comprises determining the ability of the polypeptide to preferentially bind to or modulate the activity of a target molecule.

The cell-free assays of the present invention are amenable to use of both a soluble form or the membrane-bound form of a polypeptide of the invention. In the case of cell-free assays comprising the membrane-bound form of the polypeptide, it may be desirable to utilize a solubilizing agent such that the membrane-bound form of the polypeptide is maintained in solution. Examples of such solubilizing agents include non-ionic detergents such as n-octylglucoside, n-dodecylglucoside, n-octylmaltoside, octanoyl-N-methylglucamide, decanoyl-N-methylglucamide, Triton X-100, Triton X-114, Thesit, Isotridecypoly(ethylene glycol ether)n, 3-[(3-cholamidopropyl)dimethylamminio]-1-propane sulfonate (CHAPS), 3-[(3-cholamidopropyl)dimethylamminio]-2-hydroxy-1-propane sulfonate (CHAPSO), and N-dodecyl=N,N-dimethyl-3-ammonio-1-propane sulfonate.

In more than one embodiment of the above assay methods of the present invention, it may be desirable to immobilize either the polypeptide of the invention or its target molecule to facilitate separation of complexed from uncomplexed forms of one or both of the proteins, as well as to accommodate automation of the assay. Binding of a test compound to the polypeptide, or interaction of the polypeptide with a target molecule in the presence and absence of a candidate compound, can be accomplished in any vessel suitable for containing the reactants. Examples of such vessels include microtitre plates, test tubes, and micro-centrifuge tubes. In one embodiment, a fusion protein can be provided which adds a domain that allows one or both of the proteins to be bound to a matrix. For example, glutathione-S-transferase (GST) fusion proteins can be adsorbed onto glutathione sepharose beads (Sigma Chemical; St. Louis, MO) or glutathione derivatized microtitre plates, which are then combined with the test compound or the test compound and either the non-adsorbed target protein or A polypeptide of the invention, and the mixture incubated under conditions conducive to complex formation (*e.g.*, at physiological conditions for salt and pH). Following incubation, the beads or microtitre plate wells are washed to remove any unbound components and complex formation is measured either directly or indirectly, for example, as described above. Alternatively, the complexes can be dissociated from the matrix, and the level of binding or activity of the polypeptide of the invention can be determined using standard techniques.

Other techniques for immobilizing proteins on matrices can also be used in the screening assays of the invention. For example, either the polypeptide of the invention or its target molecule can be immobilized utilizing conjugation of biotin and streptavidin. Biotinylated polypeptide of the invention or target molecules can be prepared from biotin-NHS (N-hydroxy-succinimide) using techniques well known in the art (*e.g.*, biotinylation kit, Pierce Chemicals; Rockford, IL), and immobilized in the wells of streptavidin-coated 96 well plates (Pierce Chemical). Alternatively, antibodies reactive with the polypeptide of the invention or target molecules but which do not interfere with binding of the polypeptide of the invention to its target molecule can be derivatized to the wells of the plate, and unbound target or polypeptide of the invention trapped in the wells by antibody conjugation. Methods for detecting such complexes, in addition to those described above for the GST-immobilized complexes, include immunodetection of complexes using antibodies reactive with the polypeptide of the invention or target molecule, as well as enzyme-linked assays which rely on detecting an enzymatic activity associated with the polypeptide of the invention or target molecule.

This invention further pertains to novel agents identified by the above-described screening assays and uses thereof for treatments as described herein.

### Cell-based Assays

In one embodiment, an assay is a cell-based assay in which a cell which expresses a membrane-bound form of a polypeptide of the invention, or a biologically active portion thereof, on the cell surface is contacted with a test compound, and the ability of the test compound to bind to the polypeptide determined. The cell, for example, can be a yeast cell or a cell of mammalian origin. Determining the ability of the test compound to bind to the polypeptide can be accomplished, for example, by coupling the test compound with a radioisotope or enzymatic label such that binding of the test compound to the polypeptide or biologically active portion thereof can be determined by detecting the labeled compound in a complex. For example, test compounds can be labeled with ¹²⁵I, ³⁵S, ¹⁴C, or ³H, either directly or indirectly, and the radioisotope detected by direct counting of radioemmission or by scintillation counting. Alternatively, test compounds can be enzymatically labeled with, for example, horseradish peroxidase, alkaline phosphatase, or luciferase, and the enzymatic label detected by determination of conversion of an appropriate substrate to product. In a preferred embodiment, the assay comprises contacting a cell which expresses a membrane-bound form of a polypeptide of the invention, or a biologically active portion thereof, on the cell surface with a known compound which binds the polypeptide to form an assay mixture, contacting the assay mixture with a test compound, and determining the ability of the test compound to interact with the polypeptide, wherein determining the ability of the test compound to interact with the polypeptide comprises determining the ability of the test compound to preferentially bind to the polypeptide or a biologically active portion thereof as compared to the known compound.

In another embodiment, an assay is a cell-based assay comprising contacting a cell expressing a membrane-bound form of a polypeptide of the invention, or a biologically active portion thereof, on the cell surface with a test compound and determining the ability of the test compound to modulate *(e.g.,* stimulate or inhibit) the activity of the polypeptide or biologically active portion thereof. Determining the ability of the test compound to modulate the activity of the polypeptide or a biologically active portion thereof can be accomplished, for example, by determining the ability of the polypeptide protein to bind to or interact with a target molecule.

Determining the ability of a polypeptide of the invention to bind to or interact with a target molecule can be accomplished by one of the methods described above for determining direct binding. As used herein, a "target molecule" is a molecule with which a selected polypeptide *(e.g.,* a polypeptide of the invention) binds or interacts with in nature, for example, a molecule on the surface of a cell which expresses the selected protein, a molecule on the surface of a second cell, a molecule in the extracellular milieu, a molecule associated with the internal surface of a cell membrane or a cytoplasmic molecule. A target molecule can be a polypeptide of the invention or some other polypeptide or protein. For example, a target molecule can be a component of a signal transduction pathway which facilitates transduction of an extracellular signal (*e.g.,* a signal generated by binding of a compound to a polypeptide of the invention) through the cell membrane and into the cell or a second intercellular protein which has catalytic activity or a protein which facilitates the association of downstream signaling molecules with a polypeptide of the invention. Determining the ability of a polypeptide of the invention to bind to or interact with a target molecule can be accomplished by determining the activity of the target molecule. For example, the activity of the target molecule can be determined by detecting induction of a cellular second messenger of the target (*e.g*., intracellular Ca²⁺, diacylglycerol, IP3, etc.), detecting catalytic/enzymatic activity of the target on an appropriate substrate, detecting the induction of a reporter gene (*e.g.,* a regulatory element that is responsive to a polypeptide of the invention operably linked to a nucleic acid encoding a detectable marker, *e.g.,* luciferase), or detecting a cellular response, for example, cellular differentiation, or cell proliferation.

In another embodiment, modulators of expression of a polypeptide of the invention are identified in a method in which a cell is contacted with a candidate compound and the expression of the selected mRNA or protein (*i.e*., the mRNA or protein corresponding to a polypeptide or nucleic acid of the invention) in the cell is determined. The level of expression of the selected mRNA or protein in the presence of the candidate compound is compared to the level of expression of the selected mRNA or protein in the absence of the candidate compound. The candidate compound can then be identified as a modulator of expression of the polypeptide of the invention based on this comparison. For example, when expression of the selected mRNA or protein is greater (statistically significantly greater) in the presence of the candidate compound than in its absence, the candidate compound is identified as a stimulator of the selected mRNA or protein expression. Alternatively, when expression of the selected mRNA or protein is less (statistically significantly less) in the presence of the candidate compound than in its absence, the candidate compound is identified as an inhibitor of the selected mRNA or protein expression. The level of the selected mRNA or protein expression in the cells can be determined by methods described herein.

In yet another aspect of the invention, a polypeptide of the invention can be used as "bait proteins" in a two-hybrid assay or three hybrid assay *(see, e.g.,* U.S. Patent No. 5,283,317; Zervos et al. (1993) *Cell* 72:223-232; Madura et al. (1993) *J. Biol. Chem.* 268:12046-12054; Bartel et al. (1993) *Bio*/*Techniques* 14:920-924; Iwabuchi et al. (1993) *Oncogene* 8:1693-1696; and PCT Publication No. WO 94/10300), to identify other proteins, which bind to or interact with the polypeptide of the invention and modulate activity of the polypeptide of the invention. Such binding proteins are also likely to be involved in the propagation of signals by the polypeptide of the inventions as, for example, upstream or downstream elements of a signaling pathway involving the polypeptide of the invention.

This invention further pertains to novel agents identified by the above-described screening assays and uses thereof for treatments as described herein.

In particular, the detection and expression of CD2000 or CD2001 can be readily detected, *e.g.*, by quantifying CD2000 or CD2001 protein and/or RNA. Many methods standard in the art can be thus employed, including, but not limited to, immunoassays to detect and/or visualize gene expression (*e.g*., Western blot, immunoprecipitation followed by sodium dodecyl sulfate polyacrylamide gel electrophoresis (SDS-PAGE), immunocytochemistry, etc.) and/or hybridization assays to detect gene expression by detecting and/or visualizing, respectively, mRNA encoding a gene (*e.g*., Northern assays, dot blots, *in situ* hybridization, etc.), etc. Both wild-type and mutant, including diseaseassociated mutant, genes encoding CD2000 or CD2001 can also be detected using standard techniques such as those described herein. Such detection and predictive medicine-related uses and methods are described below.

### Animal Models

The activity of polypeptides of the invention, including the therapeutic, *e.g.*, clinical, efficacy of the polypeptides, as well as the effect, including the therapeutic, *e.g.*, clinical, efficacy, of CD2000 modulators or CD2001 modulators can routinely be ascertained via use of a number of different accepted animal models well known to those of skill in the art. Such methods of ascertaining the activity of polypeptides of the invention, as well as screening assays for ascertaining the modulatory activity of test compounds are described below.

A number of animal models for autoimmune disorder models can be utilized to assess the activity of CD2000 or CD2001. First, an experimental allergic encephalomyelitis (EAE) model can be utilized. EAE is an experimental autoimmune disease of the central nervous system (CNS) (Zamvil et al, 1990, Ann. Rev, Immunol. 8:579) and is a disease model for the human autoimmune condition, multiple sclerosis (MS). EAE is an example of a cell-mediated autoimmune disorder that is mediated via T cells. EAE is readily induced in mammalian species by immunizations of myelin basic protein (MBP) purified from the CNS or an encephalitogenic proteolipid (PLP). SJL/J mice are a susceptible strain of mice (H-2^{u}) and, upon induction of EAE, these mice develop an acute paralytic disease and an acute cellular infiltrate is identifiable within the CNS. EAE spontaneously develops in MBP₁₋₁₇ peptide-specific T cell receptor (TCR) transgenic mice (TgMBP⁺) of a RAG-1-deficient background (LaFaille etal., 1994, Cell 78:399).

A collagen-induced arthritis (CIA) model can also be utilized CIA is an animal model for the human autoimmune disease rheumatoid arthritis (RA) (Trenthorn et al., 1977, J. Exp. Med., 146:857). This disease can be induced in many species by the administration of heterologous type II collagen (Courtenay et al., 1980, Nature 283:665; Cathcart et at, 1986, Lab. Invest., 54:26). With respect to animal models of arthritis see, in addition, *e.g.,* Holmdahl, R., 1999, Curr. Biol. 15:R528-530.

Animal models for chronic obstructive pulmonary disease (COPD) can also be used to assess the activity of CD2000 or CD2001 (for review of COPD animal models see, *e.g.,* Shapiro, 2000, Am. J. Respir. Cell Mol. Biol. 22:4-7; and Shapiro, 2000, Chest 117:2223S-227S). COPD is a generic term for several clinical syndromes including, but not limit to, emphysema and chronic bronchitis. Emphysema can be induced in animals such as mice by the administration or overexpression of elasolytic enzymes including, but not limited to pancreatic elastase, neutrophil elastase, and proteinase 3. Emphysema can also be induced by the administration of a variety of chemicals and irritants including, but not limited to, lipopolysaccharides (LPS), cadmium chloride, nitrogen dioxide, inorganic dust, and ozone. Further, emphysema can be induced by overexpression of interferon-γ (IFN-γ). Cigarette smoke-related COPD in mice can be induced by chronic exposure to cigarette smoke. Pulmonary fibrosis can be induced by the administration of bleomycin to mice. Further, mutant mouse strains, such as tight skin (Tsk^{+/-}), pallid (pa/pa), blotchy (Blo), mice transgenic for collagenase, and PDGF-A^{-/-}, that spontaneously develop enlarged airspaces can also be used.

Animal models for inflammatory bowel disease (IBD) can also be used to assess the activity of CD2000 or CD2001 (for review see, *e.g.*, Kim et al., 1992, Scand. J. Gastroentrol. 27:529-537; and Strober, 1985, Dig. Dis. Sci. 30(12 Suppl.):3S-10S. Crohn's disease and ulcerative colitis are two types of human IBDs. IBD can be induced in animals by oral administration of sulfated polysaccharides including, but not limited to, carrageenan, amylopectin sulfate, and dextran sulfate. IBD can also be induced by the administration of chemical irritants such as trinitrobenzenesulphonic acid (TNBS) and acetic acid.

Further, animal models such as the adoptive transfer model described, *e.g.*, in L. Cohn et al., 1997, *J. Exp. Med. 186*:1737-1747) can be used to assess the activity of CD2000 or CD2001. In such an animal system, aeroallergen provocation of TH1 or TH2 recipient mice results in TH effector cell migration to the airways and is associated with an intense neutrophilic (TH1) and eosinophilic (TH2) lung mucosal inflammatory response. The animal model represents an accepted model for asthma.

Still further, animal models for type 1 diabetes, thyroid autoimmunity or systemic lupus erythematosus, including glomerulonephritis can be utilized to assess the activity of CD2000 or CD2001 (see, *e.g.*, Flanders et al., 1999, Autoimmunity 29:235-246; Krogh et al., 1999, Biochimie 81:511-515; and Foster, N.H., 1999, Semin. Nephrol. 19:12-24, respectively).

### B. Detection Assays

Portions or fragments of the cDNA sequences identified herein (and the corresponding complete gene sequences) can be used in numerous ways as polynucleotide reagents. For example, these sequences can be used to: (i) map their respective genes on a chromosome and, thus, locate gene regions associated with genetic disease; (ii) identify an individual from a minute biological sample (tissue typing); and (iii) aid in forensic identification of a biological sample. These applications are described in the subsections below.

### 1. Chromosome Mapping

Once the sequence (or a portion of the sequence) of a gene has been isolated, this sequence can be used to map the location of the gene on a chromosome. Accordingly, nucleic acid molecules described herein or fragments thereof, can be used to map the location of the corresponding genes on a chromosome. The mapping of the sequences to chromosomes is an important first step in correlating these sequences with genes associated with disease.

Briefly, genes can be mapped to chromosomes by preparing PCR primers (preferably 15-25 bp in length) from the sequence of a gene of the invention. Computer analysis of the sequence of a gene of the invention can be used to rapidly select primers that do not span more than one exon in the genomic DNA, thus complicating the amplification process. These primers can then be used for PCR screening of somatic cell hybrids containing individual human chromosomes. Only those hybrids containing the human gene corresponding to the gene sequences will yield an amplified fragment. For a review of this technique, see D'Eustachio et al., 1983, *Science* 220:919-924.

PCR mapping of somatic cell hybrids is a rapid procedure for assigning a particular sequence to a particular chromosome. Three or more sequences can be assigned per day using a single thermal cycler. Using the nucleic acid sequences of the invention to design oligonucleotide primers, sublocalization can be achieved with panels of fragments from specific chromosomes. Other mapping strategies which can similarly be used to map a gene to its chromosome include *in situ* hybridization (described in Fan et al. (1990) *Proc. Natl. Acad. Sci. USA* 87:6223-27), pre-screening with labeled flow-sorted chromosomes (CITE), and pre-selection by hybridization to chromosome specific cDNA libraries. Fluorescence *in situ* hybridization (FISH) of a DNA sequence to a metaphase chromosomal spread can further be used to provide a precise chromosomal location in one step. For a review of this technique, see Verma et al., (Human Chromosomes: A Manual of Basic Techniques (Pergamon Press, New York, 1988)).

Reagents for chromosome mapping can be used individually to mark a single chromosome or a single site on that chromosome, or panels of reagents can be used for marking multiple sites and/or multiple chromosomes. Reagents corresponding to noncoding regions of the genes actually are preferred for mapping purposes. Coding sequences are more likely to be conserved within gene families, thus increasing the chance of cross hybridizations during chromosomal mapping. Human CD2000 has been mapped to chromosome 1q23. Human CD2001 has been mapped to chromosome 1q21.

Once a sequence has been mapped to a precise chromosomal location, the physical position of the sequence on the chromosome can be correlated with genetic map data. (Such data are found, for example, in V. McKusick, Mendelian Inheritance in Man, available on-line through Johns Hopkins University Welch Medical Library). The relationship between genes and disease, mapped to the same chromosomal region, can then be identified through linkage analysis (co-inheritance of physically adjacent genes), described in, *e.g.*, Egeland et al., 1987, *Nature* 325:783-787.

Moreover, differences in the DNA sequences between individuals affected and unaffected with a disease associated with a gene of the invention can be determined. If a mutation is observed in some or all of the affected individuals but not in any unaffected individuals, then the mutation is likely to be the causative agent of the particular disease. Comparison of affected and unaffected individuals generally involves first looking for structural alterations in the chromosomes such as deletions or translocations that are visible from chromosome spreads or detectable using PCR based on that DNA sequence. Ultimately, complete sequencing of genes from several individuals can be performed to confirm the presence of a mutation and to distinguish mutations from polymorphisms.

A polypeptide and fragments and sequences thereof and antibodies specific thereto can be used to map the location of the gene encoding the polypeptide on a chromosome. This mapping can be carried out by specifically detecting the presence of the polypeptide in members of a panel of somatic cell hybrids between cells of a first species of animal from which the protein originates and cells from a second species of animal and then determining which somatic cell hybrid(s) expresses the polypeptide and noting the chromosome(s) from the first species of animal that it contains. For examples of this technique, see Pajunen *et al*. (1988) *Cytogenet. Cell Genet. 47*:37-41 and Van Keuren *et al*. (1986) *Hum. Genet. 74*:34-40. Alternatively, the presence of the polypeptide in the somatic cell hybrids can be determined by assaying an activity or property of the polypeptide, for example, enzymatic activity, as described in Bordelon-Riser *et al*., 1979, *Somatic Cell Genetics 5*:597-613 and Owerbach *et al*., 1978, *Proc. Natl. Acad. Sci. USA 75*:5640-5644.

### 2. Tissue Typing

The nucleic acid sequences of the present invention can also be used to identify individuals from minute biological samples. The United States military, for example, is considering the use of restriction fragment length polymorphism (RFLP) for identification of its personnel. In this technique, an individual's genomic DNA is digested with one or more restriction enzymes, and probed on a Southern blot to yield unique bands for identification. This method does not suffer from the current limitations of "Dog Tags" which can be lost, switched, or stolen, making positive identification difficult. The sequences of the present invention are useful as additional DNA markers for RFLP (described in U.S. Patent 5,272,057).

Furthermore, the sequences of the present invention can be used to provide an alternative technique which determines the actual base-by-base DNA sequence of selected portions of an individual's genome. Thus, the nucleic acid sequences described herein can be used to prepare two PCR primers from the 5' and 3' ends of the sequences. These primers can then be used to amplify an individual's DNA and subsequently sequence it.

Panels of corresponding DNA sequences from individuals, prepared in this manner, can provide unique individual identifications, as each individual will have a unique set of such DNA sequences due to allelic differences. The sequences of the present invention can be used to obtain such identification sequences from individuals and from tissue. The nucleic acid sequences of the invention uniquely represent portions of the human genome. Allelic variation occurs to some degree in the coding regions of these sequences, and to a greater degree in the noncoding regions. It is estimated that allelic variation between individual humans occurs with a frequency at about once per each 500 bases. Each of the sequences described herein can, to some degree, be used as a standard against which DNA from an individual can be compared for identification purposes. Because greater numbers of polymorphisms occur in the noncoding regions, fewer sequences are necessary to differentiate individuals. The noncoding sequences of SEQ ID NO:1 can comfortably provide positive individual identification with a panel of perhaps 10 to 1,000 primers which each yield a noncoding amplified sequence of 100 bases. If predicted coding sequences, such as those in SEQ ID NO:1 are used, a more appropriate number of primers for positive individual identification would be 500-2,000.

If a panel of reagents from the nucleic acid sequences described herein is used to generate a unique identification database for an individual, those same reagents can later be used to identify tissue from that individual. Using the unique identification database, positive identification of the individual, living or dead, can be made from extremely small tissue samples.

### 3. Use of Partial Gene Sequences in Forensic Biology

DNA-based identification techniques can also be used in forensic biology. Forensic biology is a scientific field employing genetic typing of biological evidence found at a crime scene as a means for positively identifying, for example, a perpetrator of a crime. To make such an identification, PCR technology can be used to amplify DNA sequences taken from very small biological samples such as tissues, *e.g.,* hair or skin, or body fluids, *e.g.,* blood, saliva, or semen found at a crime scene. The amplified sequence can then be compared to a standard, thereby allowing identification of the origin of the biological sample.

The sequences of the present invention can be used to provide polynucleotide reagents, *e.g*., PCR primers, targeted to specific loci in the human genome, which can enhance the reliability of DNA-based forensic identifications by, for example, providing another "identification marker" (*i.e*. another DNA sequence that is unique to a particular individual). As mentioned above, actual base sequence information can be used for identification as an accurate alternative to patterns formed by restriction enzyme generated fragments. Sequences targeted to noncoding regions are particularly appropriate for this use as greater numbers of polymorphisms occur in the noncoding regions, making it easier to differentiate individuals using this technique. Examples of polynucleotide reagents include the nucleic acid sequences of the invention or portions thereof, *e.g*., fragments derived from noncoding regions having a length of at least 20 or 30 bases, preferably contiguous bases.

The nucleic acid sequences described herein can further be used to provide polynucleotide reagents, *e.g.,* labeled or labelable probes which can be used in, for example, an *in situ* hybridization technique, to identify a specific tissue, *e.g.,* spleen tissue or liver tissue. This can be very useful in cases where a forensic pathologist is presented with a tissue of unknown origin. Panels of such probes can be used to identify tissue by species and/or by organ type.

### C. Predictive Medicine

The present invention also pertains to the field of predictive medicine in which diagnostic assays, prognostic assays, and monitoring clinical trials are used for prognostic (predictive) purposes to thereby treat an individual prophylactically. Accordingly, one aspect of the present invention relates to diagnostic assays for determining CD2000 or CD2001 protein and/or nucleic acid expression as well as CD2000 activity, in the context of a biological sample (*e.g.*, blood, serum, cells, and tissue) to thereby determine whether an individual is afflicted with a disease or disorder, or is at risk of developing a disorder, associated with aberrant or unwanted CD2000 or CD2001 expression or activity. The invention also provides for prognostic (or predictive) assays for determining whether an individual is at risk of developing a disorder associated with CD2000 or CD2001 protein, nucleic acid expression or activity. For example, mutations in a CD2000 gene or CD2001 gene can be assayed in a biological sample. Such assays can be used for prognostic or predictive purpose to thereby prophylactically treat an individual prior to the onset of a disorder characterized by or associated with CD2000 or CD2001 protein and/or nucleic acid expression or activity.

As an alternative to making determinations based on the absolute expression level of selected genes, determinations may be based on the normalized expression levels of these genes. Expression levels are normalized by correcting the absolute expression level of a CD2000 gene or CD2001 gene by comparing its expression to the expression of a gene that is not a CD2000 gene or CD2001 gene, *e.g.,* a housekeeping gene that is constitutively expressed. Suitable genes for normalization include housekeeping genes such as the actin gene. This normalization allows the comparison of the expression level in one sample, *e.g.*, a patient sample, to another sample, *e.g.*, a sample from an individual without a particular disease or disorder, or a sample from a healthy individual, or between samples from different sources.

Alternatively, the expression level can be provided as a relative expression level. To determine a relative expression level of a gene, the level of expression of the gene is determined for 10 or more samples of different cell isolates (*e.g.*, immune cell isolates such as T cells), preferably 50 or more samples, prior to the determination of the expression level for the sample in question. The mean expression level of each of the genes assayed in the larger number of samples is determined and this is used as a baseline expression level for the gene(s) in question. The expression level of the gene determined for the test sample (absolute level of expression) is then divided by the mean expression value obtained for that gene. This provides a relative expression level and aids in identifying extreme cases of diseases and disorders such as immune disorders *(e.g.,* proliferative disorders, inflammatory bowel disease *(e.g.,* Crohn's disease and ulcerative colitis), autoimmune disorders (*e.g*., multiple sclerosis) inflammatory disorders *(e.g.,* rheumatoid arthritis and asthma), and chronic obstructive pulmonary disorders).

Preferably, the samples used in the baseline determination will be from diseased or from non-diseased cells of the appropriate cell type or tissue. The choice of the cell source is dependent on the use of the relative expression level. Using expression found in normal tissues as a mean expression score aids in validating whether the CD2000 gene or CD2001 gene assayed is specific (versus normal cells). Such a use is particularly important in identifying whether a CD2000 gene or CD2001 gene can serve as a target gene. In addition, as more data is accumulated, the mean expression value can be revised, providing improved relative expression values based on accumulated data. Expression data from cells provides a means for grading the severity of the disease or disorder state.

Another aspect of the invention pertains to monitoring the influence of agents (*e.g.*, drugs or compounds) on the expression or activity of CD2000 or CD2001 in clinical trials. These and other agents are described in further detail in the following sections.

### 1. Diagnostic Assays

An exemplary method for detecting the presence or absence of a polypeptide or nucleic acid of the invention in a biological sample involves obtaining a biological sample from a test subject and contacting the biological sample with a compound or an agent capable of detecting a polypeptide or nucleic acid (*e.g.*, mRNA, genomic DNA) of the invention such that the presence of a polypeptide or nucleic acid of the invention is detected in the biological sample. A preferred agent for detecting mRNA or genomic DNA encoding a polypeptide of the invention is a labeled nucleic acid probe capable of hybridizing to mRNA or genomic DNA encoding a polypeptide of the invention. The nucleic acid probe can be, for example, a full-length cDNA, such as the nucleic acid of SEQ ID NO: 1 or 74, or a portion thereof, such as an oligonucleotide of at least 15, 30, 50, 100, 250 or 500 nucleotides in length and sufficient to specifically hybridize under stringent conditions to a mRNA or genomic DNA encoding a polypeptide of the invention. Other suitable probes for use in the diagnostic assays of the invention are described herein.

A preferred agent for detecting a polypeptide of the invention is an antibody capable of binding to a polypeptide of the invention, preferably an antibody with a detectable label. Antibodies can be polyclonal, or more preferably, monoclonal. An intact antibody, or a fragment thereof (*e.g*., Fab or F(ab')₂) can be used. The term "labeled", with regard to the probe or antibody, is intended to encompass direct labeling of the probe or antibody by coupling *(i.e.,* physically linking) a detectable substance to the probe or antibody, as well as indirect labeling of the probe or antibody by reactivity with another reagent that is directly labeled. Examples of indirect labeling include detection of a primary antibody using a fluorescently labeled secondary antibody and end-labeling of a DNA probe with biotin such that it can be detected with fluorescently labeled streptavidin. The term "biological sample" is intended to include tissues, cells and biological fluids isolated from a subject, as well as tissues, cells and fluids present within a subject. That is, the detection method of the invention can be used to detect mRNA, protein, or genomic DNA in a biological sample *in vitro* as well as *in vivo.* For example, *in vitro* techniques for detection of mRNA include Northern hybridizations and *in situ* hybridizations. *In vitro* techniques for detection of a polypeptide of the invention include enzyme linked immunosorbent assays (ELISAs), Western blots, immunoprecipitations and immunofluorescence. *In vitro* techniques for detection of genomic DNA include Southern hybridizations. Furthermore, *in vivo* techniques for detection of a polypeptide of the invention include introducing into a subject a labeled antibody directed against the polypeptide. For example, the antibody can be labeled with a radioactive marker whose presence and location in a subject can be detected by standard imaging techniques.

In one embodiment, the biological sample contains protein molecules from the test subject. Alternatively, the biological sample can contain mRNA molecules from the test subject or genomic DNA molecules from the test subject. A preferred biological sample is a peripheral blood leukocyte sample isolated by conventional means from a subject.

In another embodiment, the methods further involve obtaining a control biological sample from a control subject, contacting the control sample with a compound or agent capable of detecting a polypeptide of the invention or mRNA or genomic DNA encoding a polypeptide of the invention, such that the presence of the polypeptide or mRNA or genomic DNA encoding the polypeptide is detected in the biological sample, and comparing the presence of the polypeptide or mRNA or genomic DNA encoding the polypeptide in the control sample with the presence of the polypeptide or mRNA or genomic DNA encoding the polypeptide in the test sample.

The invention also encompasses kits for detecting the presence of a polypeptide or nucleic acid of the invention in a biological sample (a test sample). Such kits can be used to determine if a subject is suffering from or is at increased risk of developing a disorder associated with aberrant expression of a polypeptide of the invention as discussed, for example, in sections above relating to uses of the sequences of the invention.

For example, kits can be used to determine if a subject is suffering from or is at increased risk of disorders such as proliferative disorders (*e.g.*, X-linked proliferative disorders), cancer, lung disorders (*e.g.*, asthma, bronchitis, bronchiolitis, cystic fibrosis, sacrcoidosis, idiopathic pulmonary fibrosis, hypersensitivity pneumontis, pneumonia, emphysema, and lung cancer), liver disorders *(e.g.,* jaundice, hepatic failure, hereditary hyperbiliruinemias, hepatic circulatory disorders *(e.g.,* hepatic vein thrombosis and portal vein obstruction and thrombosis), hepatitis (*e.g*., chronic active hepatitis, acute viral hepatitis, and toxic and drug-induced hepatitis), cirrhosis (*e.g*., alcoholic cirrhosis, biliary cirrhosis, and hemochromatosis), bone marrow disorders, splenic disorders (*e.g*., splenic lymphoma, splenomegaly, and phagocytotic disorders, *e.g*., those inhibiting macrophage engulfment of bacteria and viruses in the bloodstream), and immune disorders such as inflammatory bowel disease *(e.g.,* Crohn's disease and ulcerative colitis), autoimmune disorders *(e.g.,* multiple sclerosis), psoriasis, graft versus host disease, inflammatory disorders (*e.g*., rheumatoid arthritis and asthma), and chronic obstructive pulmonary disorders, which are associated with aberrant CD2000 or CD2001 expression. The kit, for example, can comprise a labeled compound or agent capable of detecting the polypeptide or mRNA encoding the polypeptide in a biological sample and means for determining the amount of the polypeptide or mRNA in the sample (*e.g.*, an antibody which binds the polypeptide or an oligonucleotide probe which binds to DNA or mRNA encoding the polypeptide). Kits can also include instructions for observing that the tested subject is suffering from or is at risk of developing a disorder associated with aberrant expression of the polypeptide if the amount of the polypeptide or mRNA encoding the polypeptide is above or below a normal level.

For antibody-based kits, the kit can comprise, for example: (1) a first antibody (*e.g.*, attached to a solid support) which binds to a polypeptide of the invention; and, optionally, (2) a second, different antibody which binds to either the polypeptide or the first antibody and is conjugated to a detectable agent.

For oligonucleotide-based kits, the kit can comprise, for example: (1) an oligonucleotide, *e.g.,* a detectably labeled oligonucleotide, which hybridizes to a nucleic acid sequence encoding a polypeptide of the invention or (2) a pair of primers useful for amplifying a nucleic acid molecule encoding a polypeptide of the invention. The kit can also comprise, *e.g.,* a buffering agent, a preservative, or a protein stabilizing agent. The kit can also comprise components necessary for detecting the detectable agent (*e.g*., an enzyme or a substrate). The kit can also contain a control sample or a series of control samples which can be assayed and compared to the test sample contained. Each component of the kit is usually enclosed within an individual container and all of the various containers are within a single package along with instructions for observing whether the tested subject is suffering from or is at risk of developing a disorder associated with aberrant expression of the polypeptide.

### 2. Prognostic Assays

The methods described herein can furthermore be utilized as diagnostic or prognostic assays to identify subjects having or at risk of developing a disease or disorder associated with aberrant expression or activity of a polypeptide of the invention. For example, the assays described herein, such as the preceding diagnostic assays or the following assays, can be utilized to identify a subject having or at risk of developing a disorder associated with aberrant expression or activity of a polypeptide of the invention, *e.g*., an immunologic disorder, or embryonic disorders. Alternatively, the prognostic assays can be utilized to identify a subject having or at risk for developing such a disease or disorder. Thus, the present invention provides a method in which a test sample is obtained from a subject and a polypeptide or nucleic acid (*e.g*., mRNA, genomic DNA) of the invention is detected, wherein the presence of the polypeptide or nucleic acid is diagnostic for a subject having or at risk of developing a disease or disorder associated with aberrant expression or activity of the polypeptide. As used herein, a "test sample" refers to a biological sample obtained from a subject of interest. For example, a test sample can be a biological fluid (*e.g*., serum), cell sample, or tissue.

The prognostic assays described herein, for example, can be used to identify a subject having or at risk of developing disorders such as disorders discussed, for example, in sections above relating to uses of the sequences of the invention. For example, such disorders can include proliferative disorders *(e.g.,* X-linked proliferative disorders), cancer, lung disorders *(e.g.,* asthma, bronchitis, bronchiolitis, cystic fibrosis, sacrcoidosis, idiopathic pulmonary fibrosis, hypersensitivity pneumontis, pneumonia, emphysema, and lung cancer), liver disorders (*e.g*., jaundice, hepatic failure, hereditary hyperbiliruinemias, hepatic circulatory disorders (*e.g*., hepatic vein thrombosis and portal vein obstruction and thrombosis), hepatitis (*e.g.,* chronic active hepatitis, acute viral hepatitis, and toxic and drug-induced hepatitis), cirrhosis (*e.g*., alcoholic cirrhosis, biliary cirrhosis, and hemochromatosis), bone marrow disorders, splenic disorders (e.g., splenic lymphoma, splenomegaly, and phagocytotic disorders, *e.g*., those inhibiting macrophage engulfment of bacteria and viruses in the bloodstream), and immune disorders such as inflammatory bowel disease (*e.g.,* Crohn's disease and ulcerative colitis), autoimmune disorders *(e.g.,* multiple sclerosis), psoriasis, graft versus host disease, inflammatory disorders (*e.g*., rheumatoid arthritis and asthma), and chronic obstructive pulmonary disorders, which are associated with aberrant CD2000 or CD2001 expression.

Furthermore, the prognostic assays described herein can be used to determine whether a subject can be administered an agent (*e.g*., an agonist, antagonist, peptidomimetic, protein, peptide, nucleic acid, small molecule, or other drug candidate) to treat a disease or disorder associated with aberrant expression or activity of a CD2000 or CD2001 polypeptide. For example, such methods can be used to determine whether a subject can be effectively treated with a specific agent or class of agents (*e.g*., agents of a type which decrease activity of the polypeptide). Thus, the present invention provides methods for determining whether a subject can be effectively treated with an agent for a disorder associated with aberrant expression or activity of a CD2000 or CD2001 polypeptide in which a test sample is obtained and the polypeptide or nucleic acid encoding the polypeptide is detected (*e.g.*, wherein the presence of the polypeptide or nucleic acid is diagnostic for a subject that can be administered the agent to treat a disorder associated with aberrant expression or activity of the polypeptide).

The methods of the invention can also be used to detect genetic lesions or mutations in a gene of the invention, thereby determining if a subject with the lesioned gene is at risk for a disorder characterized by aberrant expression or activity of a CD2000 or CD2001 polypeptide. In preferred embodiments, the methods include detecting, in a sample of cells from the subject, the presence or absence of a genetic lesion or mutation characterized by at least one of an alteration affecting the integrity of a gene encoding a CD2000 or CD2001 polypeptide, or the misexpression of the gene encoding a CD2000 or CD2001 polypeptide. For example, such genetic lesions or mutations can be detected by ascertaining the existence of at least one of: 1) a deletion of one or more nucleotides from the gene; 2) an addition of one or more nucleotides to the gene; 3) a substitution of one or more nucleotides of the gene; 4) a chromosomal rearrangement of the gene; 5) an alteration in the level of a messenger RNA transcript of the gene; 6) an aberrant modification of the gene, such as of the methylation pattern of the genomic DNA; 7) the presence of a non-wild type splicing pattern of a messenger RNA transcript of the gene; 8) a non-wild type level of a protein encoded by the gene; 9) an allelic loss of the gene; and 10) an inappropriate post-translational modification of the protein encoded by the gene. As described herein, there are a large number of assay techniques known in the art which can be used for detecting lesions in a gene.

In certain embodiments, detection of the lesion involves the use of a probe/primer in a polymerase chain reaction (PCR) *(see, e.g.,* U.S. Patent Nos. 4,683,195 and 4,683,202), such as anchor PCR or RACE PCR, or, alternatively, in a ligation chain reaction (LCR) (*see*, *e.g.,* Landegran et al. (1988) *Science* 241:1077-1080; and Nakazawa et al. (1994) *Proc. Natl. Acad. Sci. USA* 91:360-364), the latter of which can be particularly useful for detecting point mutations in a gene *(see, e.g.,* Abravaya et al. (1995) *Nucleic Acids Res.* 23:675-682). This method can include the steps of collecting a sample of cells from a patient, isolating nucleic acid *(e.g.,* genomic, mRNA or both) from the cells of the sample, contacting the nucleic acid sample with one or more primers which specifically hybridize to the selected gene under conditions such that hybridization and amplification of the gene (if present) occurs, and detecting the presence or absence of an amplification product, or detecting the size of the amplification product and comparing the length to a control sample. It is anticipated that PCR and/or LCR may be desirable to use as a preliminary amplification step in conjunction with any of the techniques used for detecting mutations described herein.

Alternative amplification methods include: self sustained sequence replication (Guatelli et al. (1990) *Proc. Natl. Acad. Sci. USA* 87:1874-1878), transcriptional amplification system (Kwoh, et al. (1989) *Proc. Natl. Acad. Sci. USA* 86:1173-1177), Q-Beta Replicase (Lizardi et al. (1988) *Bio*/*Technology* 6:1197), or any other nucleic acid amplification method, followed by the detection of the amplified molecules using techniques well known to those of skill in the art. These detection schemes are especially useful for the detection of nucleic acid molecules if such molecules are present in very low numbers.

In an alternative embodiment, mutations in a selected gene from a sample cell can be identified by alterations in restriction enzyme cleavage patterns. For example, sample and control DNA is isolated, amplified (optionally), digested with one or more restriction endonucleases, and fragment length sizes are determined by gel electrophoresis and compared. Differences in fragment length sizes between sample and control DNA indicates mutations in the sample DNA. Moreover, the use of sequence specific ribozymes (*see, e.g*., U.S. Patent No. 5,498,531) can be used to score for the presence of specific mutations by development or loss of a ribozyme cleavage site.

In other embodiments, genetic mutations can be identified by hybridizing a sample and control nucleic acids, *e.g.,* DNA or RNA, to high density arrays containing hundreds or thousands of oligonucleotides probes (Cronin et al. (1996) *Human Mutation* 7:244-255; Kozal et al. (1996) *Nature Medicine* 2:753-759). For example, genetic mutations can be identified in two-dimensional arrays containing light-generated DNA probes as described in Cronin et al., *supra.* Briefly, a first hybridization array of probes can be used to scan through long stretches of DNA in a sample and control to identify base changes between the sequences by making linear arrays of sequential overlapping probes. This step allows the identification of point mutations. This step is followed by a second hybridization array that allows the characterization of specific mutations by using smaller, specialized probe arrays complementary to all variants or mutations detected. Each mutation array is composed of parallel probe sets, one complementary to the wild-type gene and the other complementary to the mutant gene.

In yet another embodiment, any of a variety of sequencing reactions known in the art can be used to directly sequence the selected gene and detect mutations by comparing the sequence of the sample nucleic acids with the corresponding wild-type (control) sequence. Examples of sequencing reactions include those based on techniques developed by Maxim and Gilbert ((1977) *Proc. Natl. Acad. Sci. USA* 74:560) or Sanger ((1977) *Proc. Natl. Acad. Sci. USA* 74:5463). It is also contemplated that any of a variety of automated sequencing procedures can be utilized when performing the diagnostic assays ((1995) *Bio*/*Techniques* 19:448), including sequencing by mass spectrometry ( *see, e.g.,* PCT Publication No. WO 94/16101; Cohen et al. (1996) *Adv. Chromatogr*. 36:127-162; and Griffin et al. (1993) *Appl. Biochem. Biotechnol*. 38:147-159).

Other methods for detecting mutations in a selected gene include methods in which protection from cleavage agents is used to detect mismatched bases in RNA/RNA or RNA/DNA heteroduplexes (Myers et al. (1985) *Science* 230:1242). In general, the technique of "mismatch cleavage" entails providing heteroduplexes formed by hybridizing (labeled) RNA or DNA containing the wild-type sequence with potentially mutant RNA or DNA obtained from a tissue sample. The double-stranded duplexes are treated with an agent which cleaves single-stranded regions of the duplex such as which will exist due to basepair mismatches between the control and sample strands. RNA/DNA duplexes can be treated with RNase to digest mismatched regions, and DNA/DNA hybrids can be treated with S1 nuclease to digest mismatched regions.

In other embodiments, either DNA/DNA or RNA/DNA duplexes can be treated with hydroxylamine or osmium tetroxide and with piperidine in order to digest mismatched regions. After digestion of the mismatched regions, the resulting material is then separated by size on denaturing polyacrylamide gels to determine the site of mutation. *See, e.g*., Cotton et al. (1988) *Proc. Natl. Acad. Sci. USA* 85:4397; Saleeba et al. (1992) *Methods Enzymol.* 217:286-295. In a preferred embodiment, the control DNA or RNA can be labeled for detection.

In still another embodiment, the mismatch cleavage reaction employs one or more proteins that recognize mismatched base pairs in double-stranded DNA (so called "DNA mismatch repair" enzymes) in defined systems for detecting and mapping point mutations in cDNAs obtained from samples of cells. For example, the mutY enzyme of E. coli cleaves A at G/A mismatches and the thymidine DNA glycosylase from HeLa cells cleaves T at G/T mismatches (Hsu et al. (1994) *Carcinogenesis* 15:1657-1662). According to an exemplary embodiment, a probe based on a selected sequence, *e.g*., a wild-type sequence, is hybridized to a cDNA or other DNA product from a test cell(s). The duplex is treated with a DNA mismatch repair enzyme, and the cleavage products, if any, can be detected from electrophoresis protocols or the like. *See, e.g*., U.S. Patent No. 5,459,039.

In other embodiments, alterations in electrophoretic mobility will be used to identify mutations in genes. For example, single strand conformation polymorphism (SSCP) may be used to detect differences in electrophoretic mobility between mutant and wild type nucleic acids (Orita et al. (1989) *Proc. Natl. Acad. Sci. USA* 86:2766; *see also* Cotton (1993) *Mutat. Res.* 285:125-**144**; Hayashi (1992) *Genet. Anal. Tech. Appl*. 9:73-79). Single-stranded DNA fragments of sample and control nucleic acids will be denatured and allowed to renature. The secondary structure of single-stranded nucleic acids varies according to sequence, and the resulting alteration in electrophoretic mobility enables the detection of even a single base change. The DNA fragments may be labeled or detected with labeled probes. The sensitivity of the assay may be enhanced by using RNA (rather than DNA), in which the secondary structure is more sensitive to a change in sequence. In a preferred embodiment, the subject method utilizes heteroduplex analysis to separate double stranded heteroduplex molecules on the basis of changes in electrophoretic mobility (Keen et al. (1991) *Trends Genet.* 7:5).

In yet another embodiment, the movement of mutant or wild-type fragments in polyacrylamide gels containing a gradient of denaturant is assayed using denaturing gradient gel electrophoresis (DGGE) (Myers et al. (1985) *Nature* 313:495). When DGGE is used as the method of analysis, DNA will be modified to insure that it does not completely denature, for example by adding a 'GC clamp of approximately 40 bp of high-melting GC-rich DNA by PCR. In a further embodiment, a temperature gradient is used in place of a denaturing gradient to identify differences in the mobility of control and sample DNA (Rosenbaum and Reissner (1987) *Biophys. Chem.* 265:12753).

Examples of other techniques for detecting point mutations include, but are not limited to, selective oligonucleotide hybridization, selective amplification, or selective primer extension. For example, oligonucleotide primers may be prepared in which the known mutation is placed centrally and then hybridized to target DNA under conditions which permit hybridization only if a perfect match is found (Saiki et al. (1986) *Nature* 324:163); Saiki et al. (1989) *Proc. Natl. Acad. Sci. USA* 86:6230). Such allele specific oligonucleotides are hybridized to PCR amplified target DNA or a number of different mutations when the oligonucleotides are attached to the hybridizing membrane and hybridized with labeled target DNA.

Alternatively, allele specific amplification technology which depends on selective PCR amplification may be used in conjunction with the instant invention. Oligonucleotides used as primers for specific amplification may carry the mutation of interest in the center of the molecule (so that amplification depends on differential hybridization) (Gibbs et al. (1989) *Nucleic Acids Res.* 17:2437-2448) or at the extreme 3' end of one primer where, under appropriate conditions, mismatch can prevent or reduce polymerase extension (Prossner (1993) *Tibtech* 11:238). In addition, it may be desirable to introduce a novel restriction site in the region of the mutation to create cleavage-based detection (Gasparini et al. (1992) *Mol. Cell Probes* 6:1). It is anticipated that in certain embodiments amplification may also be performed using Taq ligase for amplification (Barany (1991) *Proc. Natl. Acad. Sci. USA* 88:189). In such cases, ligation will occur only if there is a perfect match at the 3' end of the 5' sequence making it possible to detect the presence of a known mutation at a specific site by looking for the presence or absence of amplification.

The methods described herein may be performed, for example, by utilizing pre-packaged diagnostic kits comprising at least one probe, nucleic acid or antibody reagent described herein, which maybe conveniently used, *e.g*., in clinical settings to diagnose patients exhibiting symptoms or family history of a disease or illness involving a gene encoding a polypeptide of the invention. Furthermore, any cell type or tissue, *e.g*., chondrocytes, in which the polypeptide of the invention is expressed may be utilized in the prognostic assays described herein.

### 3. Pharmacogenomics

Agents or modulators which have a stimulatory or inhibitory effect on activity or expression of a polypeptide of the invention as identified by a screening assay described herein can be administered to individuals to treat (prophylactically or therapeutically) disorders associated with aberrant activity of the polypeptide. In conjunction with such treatment, the pharmacogenomics (*i.e*., the study of the relationship between an individual's genotype and that individual's response to a foreign compound or drug) of the individual may be considered. Differences in metabolism of therapeutics can lead to severe toxicity or therapeutic failure by altering the relation between dose and blood concentration of the pharmacologically active drug. Thus, the pharmacogenomics of the individual permits the selection of effective agents (*e.g*., drugs) for prophylactic or therapeutic treatments based on a consideration of the individual's genotype. Such pharmacogenomics can further be used to determine appropriate dosages and therapeutic regimens. Accordingly, the activity of a polypeptide of the invention, expression of a nucleic acid of the invention, or mutation content of a gene of the invention in an individual can be determined to thereby select appropriate agent(s) for therapeutic or prophylactic treatment of the individual.

Pharmacogenomics deals with clinically significant hereditary variations in the response to drugs due to altered drug disposition and abnormal action in affected persons. *See, e.g*., Linder (1997) *Clin. Chem.* 43(2):254-266. In general, two types of pharmacogenetic conditions can be differentiated. Genetic conditions transmitted as a single factor altering the way drugs act on the body are referred to as "altered drug action." Genetic conditions transmitted as single factors altering the way the body acts on drugs are referred to as "altered drug metabolism". These pharmacogenetic conditions can occur either as rare defects or as polymorphisms. For example, glucose-6-phosphate dehydrogenase deficiency (G6PD) is a common inherited enzymopathy in which the main clinical complication is haemolysis after ingestion of oxidant drugs (anti-malarials, sulfonamides, analgesics, nitrofurans) and consumption of fava beans.

As an illustrative embodiment, the activity of drug metabolizing enzymes is a major determinant of both the intensity and duration of drug action. The discovery of genetic polymorphisms of drug metabolizing enzymes *(e.g.,* N-acetyltransferase 2 (NAT 2) and cytochrome P450 enzymes CYP2D6 and CYP2C19) has provided an explanation as to why some patients do not obtain the expected drug effects or show exaggerated drug response and serious toxicity after taking the standard and safe dose of a drug. These polymorphisms are expressed in two phenotypes in the population, the extensive metabolizer (EM) and poor metabolizer (PM). The prevalence of PM is different among different populations. For example, the gene coding for CYP2D6 is highly polymorphic and several mutations have been identified in PM, which all lead to the absence of functional CYP2D6. Poor metabolizers of CYP2D6 and CYP2C19 quite frequently experience exaggerated drug response and side effects when they receive standard doses. If a metabolite is the active therapeutic moiety, a PM will show no therapeutic response, as demonstrated for the analgesic effect of codeine mediated by its CYP2D6-formed metabolite morphine. The other extreme are the so called ultra-rapid metabolizers who do not respond to standard doses. Recently, the molecular basis of ultra-rapid metabolism has been identified to be due to CYP2D6 gene amplification.

Thus, the activity of a polypeptide of the invention, expression of a nucleic acid encoding the polypeptide, or mutation content of a gene encoding the polypeptide in an individual can be determined to thereby select appropriate agent(s) for therapeutic or prophylactic treatment of the individual. In addition, pharmacogenetic studies can be used to apply genotyping of polymorphic alleles encoding drug-metabolizing enzymes to the identification of an individual's drug responsiveness phenotype. This knowledge, when applied to dosing or drug selection, can avoid adverse reactions or therapeutic failure and thus enhance therapeutic or prophylactic efficiency when treating a subject with a modulator of activity or expression of the polypeptide, such as a modulator identified by one of the exemplary screening assays described herein.

### 4. Monitoring CD2000 Modulator or CD2001 Modulator Effects

Monitoring the influence of agents (*e.g*., drugs, compounds) on the expression or activity of a polypeptide of the invention (*e.g*., the ability to bind to SAP, EAT-2, and/or SHP-2; the ability to modulate immune cell activation; the ability to activate signaling molecules such as, *e.g*., NF-κB, Syk, Lyn, Fyn, PI3-kinase, and PKC; the ability to modulate immune cell activation and/or differentiation; and the ability to induce cytokine expression) can be applied in basic drug screening, preclinical studies, clinical trials and during therapeutic treatment regimens.

For example, the effectiveness of an agent, as determined by a screening assay as described herein, to increase gene expression, protein levels or protein activity, can be monitored in clinical trials of subjects exhibiting decreased gene expression, protein levels, or protein activity. Alternatively, the effectiveness of an agent, as determined by a screening assay, to decrease gene expression, protein levels or protein activity, can be monitored in clinical trials of subjects exhibiting increased gene expression, protein levels, or protein activity. In such clinical trials, expression or activity of a polypeptide of the invention and preferably, that of other polypeptides that have been implicated in, for example, a cellular proliferation disorder, can be used as a marker of the immune responsiveness of a particular cell.

For example, and not by way of limitation, genes, including those of the invention, that are modulated in cells by treatment with an agent (*e.g*., compound, drug or small molecule) which modulates activity or expression of a polypeptide of the invention (*e.g*., as identified in a screening assay described herein) can be identified. Thus, to study the effect of agents on cellular proliferation disorders, for example, in a clinical trial, cells can be isolated and RNA prepared and analyzed for the levels of expression of a gene of the invention and other genes implicated in the disorder. The levels of gene expression (*i.e*., a gene expression pattern) can be quantified by Northern blot analysis or RT-PCR, as described herein, or alternatively by measuring the amount of protein produced, by one of the methods as described herein, or by measuring the levels of activity of a gene of the invention or other genes. In this way, the gene expression pattern can serve as a marker, indicative of the physiological response of the cells to the agent. Accordingly, this response state maybe determined before, and at various points during, treatment of the individual with the agent.

In a preferred embodiment, the present invention provides a method for monitoring the effectiveness of treatment of a subject with an agent (*e.g*., an agonist, antagonist, peptidomimetic, protein, peptide, nucleic acid, small molecule, or other drug candidate identified by the screening assays described herein) comprising the steps of (i) obtaining a pre-administration sample from a subject prior to administration of the agent; (ii) detecting the level of the polypeptide or nucleic acid of the invention in the preadministration sample; (iii) obtaining one or more post-administration samples from the subject; (iv) detecting the level the of the polypeptide or nucleic acid of the invention in the post-administration samples; (v) comparing the level of the polypeptide or nucleic acid of the invention in the pre-administration sample with the level of the polypeptide or nucleic acid of the invention in the post-administration sample or samples; and (vi) altering the administration of the agent to the subject accordingly. For example, increased administration of the agent may be desirable to increase the expression or activity of the polypeptide to higher levels than detected, *i.e.*, to increase the effectiveness of the agent. Alternatively, decreased administration of the agent may be desirable to decrease expression or activity of the polypeptide to lower levels than detected, *i.e*., to decrease the effectiveness of the agent.

In yet another embodiment, a method of the invention includes a method for determining the therapeutic dosage of a CD2000 or CD2001 modulator to be administered to an individual in need of treatment for a CD2000- or CD2001-related disorder, comprising: administering a dose of a CD2000 or CD2001 modulator to a non-human animal model of a CD2000- or CD2001-related disorder, and assaying CD2000 or CD2001 function and/or assaying a symptom of the CD2000- or CD2001-related disorder in the animal, so that if CD2000 or CD2001 function and/or symptom in the animal is modulated in a manner that more closely resembles a corresponding animal not exhibiting the CD2000 or CD2001 disorder, a therapeutic dosage of the CD2000 or CD2001 modulator is determined, *e.g*., by extrapolating to the corresponding dosage in a human.

### C. Methods of Treatment

The present invention provides for both prophylactic and therapeutic methods of treating a subject at risk of (or susceptible to) a disorder or having a disorder associated with aberrant expression or activity of a CD2000 or CD2001 polypeptide, as discussed, for example, in sections above relating to uses of the sequences of the invention. For example, disorders characterized by aberrant expression or activity of the polypeptides of the invention include proliferative disorders (*e.g*., X-linked proliferative disorders), cancer, lung disorders (*e.g*., asthma, bronchitis, bronchiolitis, cystic fibrosis, sacrcoidosis, idiopathic pulmonary fibrosis, hypersensitivity pneumontis, pneumonia, emphysema, and lung cancer), liver disorders (*e.g*., jaundice, hepatic failure, hereditary hyperbiliruinemias, hepatic circulatory disorders *(e.g.,* hepatic vein thrombosis and portal vein obstruction and thrombosis), hepatitis (*e.g*., chronic active hepatitis, acute viral hepatitis, and toxic and drug-induced hepatitis), cirrhosis (*e.g*., alcoholic cirrhosis, biliary cirrhosis, and hemochromatosis), bone marrow disorders, splenic disorders (*e.g*., splenic lymphoma, splenomegaly, and phagocytotic disorders, *e.g*., those inhibiting macrophage engulfment of bacteria and viruses in the bloodstream), and immune disorders such as inflammatory bowel disease *(e.g.,* Crohn's disease and ulcerative colitis), autoimmune disorders *(e.g.,* multiple sclerosis), psoriasis, graft versus host disease, inflammatory disorders (*e.g*., rheumatoid arthritis and asthma), and chronic obstructive pulmonary disorders. The nucleic acids, polypeptides, and modulators thereof of the invention can be used to treat proliferative disorders, cancer, lung disorders *(e.g.,* asthma, bronchitis, bronchiolitis, cystic fibrosis, sacrcoidosis, idiopathic pulmonary fibrosis, hypersensitivity pneumontis, pneumonia, emphysema, and lung cancer), liver disorders (*e.g*., jaundice, hepatic failure, hereditary hyperbiliruinemias, hepatic circulatory disorders (*e.g*., hepatic vein thrombosis and portal vein obstruction and thrombosis), hepatitis (*e.g*., chronic active hepatitis, acute viral hepatitis, and toxic and drug-induced hepatitis), cirrhosis (*e.g*., alcoholic cirrhosis, biliary cirrhosis, and hemochromatosis), bone marrow disorders, splenic disorders (*e.g*., splenic lymphoma, splenomegaly, and phagocytotic disorders, *e.g*., those inhibiting macrophage engulfment of bacteria and viruses in the bloodstream), and immune disorders such as inflammatory bowel disease (*e.g*., Crohn's disease and ulcerative colitis), autoimmune disorders (*e.g*., multiple sclerosis), psoriasis, graft versus host disease, inflammatory disorders (*e.g*., rheumatoid arthritis and asthma), and chronic obstructive pulmonary disorders. In various embodiments, such modulation can be achieved via administration of CD2000 modulators or CD2001 modulators prior to, during, or subsequent to a given procedure (*e.g*., a transfusion). In a preferred embodiment, such administration can be utilized to prevent immune disorders such as inflammatory bowel disease (*e.g*., Crohn's disease and ulcerative colitis), autoimmune disorders (*e.g*., multiple sclerosis) inflammatory disorders (*e.g*., rheumatoid arthritis and asthma), and chronic obstructive pulmonary disorders, as well as other disorders described herein.

### 1. Prophylactic Methods

In one aspect, the invention provides a method for preventing in a subject, a disease or condition associated with aberrant expression or activity of a CD2000 or CD2001 polypeptide, by administering to the subject an agent which modulates expression or at least one activity of the polypeptide. Subjects at risk for a disease which is caused or contributed to by aberrant expression or activity of a polypeptide of the invention can be identified by, for example, any or a combination of diagnostic or prognostic assays as described herein. Administration of a prophylactic agent can occur prior to the manifestation of symptoms characteristic of the aberrancy, such that a disease or disorder is prevented or, alternatively, delayed in its progression. Depending on the type of aberrancy, for example, an agonist or antagonist agent can be used for treating the subject. For example, an antagonist of a CD2000 or CD2001 protein may be used to treat an immune disorder, *e.g*., inflammatory bowel disease, inflammatory disorders (*e.g*., asthma and rheumatoid arthritis), psoriasis, graft versus host disease, autoimmune disorders (*e.g*., multiple sclerosis), and chronic obstructive pulmonary disease. The appropriate agent can be determined based on screening assays described herein.

### 2. Therapeutic Methods

Another aspect of the invention pertains to methods of modulating expression or activity of a CD2000 or CD2001 polypeptide for therapeutic purposes. The modulatory method of the invention involves contacting a cell with an agent that modulates one or more of the activities of the polypeptide. An agent that modulates activity can be an agent as described herein, such as a nucleic acid or a protein, a naturally-occurring cognate ligand of the polypeptide, or a small molecule such as a peptide, a peptidomimetic, or other small organic molecule. In one embodiment, the agent stimulates one or more of the biological activities of a CD2000 or CD2001 polypeptide. Examples of such stimulatory agents include the active CD2000 or CD2001 polypeptide and a nucleic acid molecule encoding a CD2000 or CD2001 polypeptide that has been introduced into the cell. In another embodiment, the agent inhibits one or more of the biological activities of a CD2000 or CD2001 polypeptide. Examples of such inhibitory agents include antisense nucleic acid molecules and antibodies. These modulatory methods can be performed *in vitro* (*e.g*., by culturing the cell with the agent) or, alternatively, *in vivo* (*e.g*., by administering the agent to a subject). As such, the present invention provides methods of treating an individual afflicted with a disease or disorder characterized by aberrant expression or activity of a CD2000 or CD2001 polypeptide. Such diseases and disorders include those described herein. In one embodiment, the method involves administering an agent (*e.g*., an agent identified by a screening assay described herein), or combination of agents that modulates (*e.g*., upregulates or downregulates) expression or activity. In another embodiment, the method involves administering a CD2000 or CD2001 polypeptide or a nucleic acid molecule of the invention as therapy to compensate for reduced or aberrant expression or activity of the polypeptide.

Stimulation of activity is desirable in situations in which activity or expression is abnormally low or downregulated and/or in which increased activity is likely to have a beneficial effect. Conversely, inhibition of activity is desirable in situations in which activity or expression is abnormally high or upregulated and/or in which decreased activity is likely to have a beneficial effect.

### Deposit of Clones

A clone containing a cDNA molecule encoding human CD2000 was deposited with the American Type Culture Collection, 10801 University Boulevard, Manassas, VA, 20110-2209, on July 21, 2000 as PTA-2267.

### Equivalents

Those skilled in the art will recognize, or be able to ascertain using no more than routine experimentation, many equivalents to the specific embodiments of the invention described herein. Such equivalents are intended to be encompassed by the following claims.

All publications, patents and patent applications mentioned in this specification are herein incorporated by reference into the specification to the same extent as if each individual publication, patent or patent application was specifically and individually indicated to be incorporated herein by reference.

## Claims

1. An isolated nucleic acid molecule selected from the group consisting of:
a) a nucleic acid molecule comprising a nucleotide sequence which is at least 25% identical to the nucleotide sequence of SEQ ID NO:1, the cDNA insert of the plasmid deposited with the ATCC® as patent deposit Number PTA-2267, or a complement thereof;
b) a nucleic acid molecule comprising a nucleotide sequence which is at least 35% identical to the nucleotide sequence of SEQ ID NO:1, the cDNA insert of the plasmid deposited with the ATCC® as patent deposit Number PTA-2267, or a complement thereof;
c) a nucleic acid molecule comprising a fragment of at least 15 nucleotides of the nucleotide sequence of SEQ ID NO: 1 or 2, the cDNA insert of the plasmid deposited with the ATCC® as patent deposit Number PTA-2267, or a complement thereof;
d) nucleic acid molecule which encodes a polypeptide comprising the amino acid sequence of SEQ ID NO:3, or the amino acid sequence encoded by the cDNA insert of the plasmid deposited with the ATCC® as patent deposit Number PTA-2267;
e) a nucleic acid molecule which encodes a fragment of a polypeptide comprising the amino acid sequence of SEQ ID NO:3, or the amino acid sequence encoded by the cDNA insert of the plasmid deposited with the ATCC® as patent deposit Number PTA-2267, wherein the fragment comprises at least 175 contiguous amino acids of SEQ ID NO:3, or the amino acid sequence encoded by the cDNA insert of the plasmid deposited with the ATCC® as patent deposit Number PTA-2267; and
f) a nucleic acid molecule which encodes a fragment of a polypeptide comprising at least 15 contiguous amino acid residues of amino acid residues 174 to 331 of SEQ ID NO:3.

2. The isolated nucleic acid molecule of claim 1, which is selected from the group consisting of:
a) a nucleic acid comprising the nucleotide sequence of SEQ ID NO:1 or 2, the cDNA insert of the plasmid deposited with the ATCC® as patent deposit Number PTA-2267, or a complement thereof; and
b) a nucleic acid molecule which encodes a polypeptide comprising the amino acid sequence of SEQ ID NO:3, or the amino acid sequence encoded by the cDNA insert of the plasmid deposited with the ATCC® as patent deposit Number PTA-2267.

3. The nucleic acid molecule of claim 1 further comprising vector nucleic acid sequences.

4. The nucleic acid molecule of claim 1 further comprising nucleic acid sequences encoding a heterologous polypeptide.

5. A host cell genetically engineered to contain the nucleic acid molecule of claim 1.

6. The host cell of claim 5 which is a mammalian host cell.

7. A non-human mammalian host cell genetically engineered to contain the nucleic acid molecule of claim 1.

8. A host cell genetically engineered to express the nucleic acid molecule of claim 1.

9. The host cell of claim 8 which is a mammalian host cell.

10. A non- host cell genetically engineered to express the nucleic acid molecule of claim 1.

11. An isolated polypeptide selected from the group consisting of:
a) a fragment of a polypeptide comprising the amino acid sequence of SEQ ID NO:3, wherein the fragment comprises at least 175 contiguous amino acids of SEQ ID NO:3;
b) a fragment of a polypeptide comprising at least 15 contiguous amino acids of 174 to 331 SEQ ID NO:3;
c) a naturally occurring allelic variant of a polypeptide comprising the amino acid sequence of SEQ ID NO:3, or the amino acid sequence encoded by the cDNA insert of plasmids deposited with the ATCC® as patent deposit Number PTA-2267, wherein the polypeptide is encoded by a nucleic acid molecule which hybridizes to a nucleic acid molecule comprising SEQ ID NO:2, or a complement thereof under stringent conditions;
d) a polypeptide which is encoded by a nucleic acid molecule comprising a nucleotide sequence which is at least 25 % identical to a nucleic acid comprising the nucleotide sequence of SEQ ID NO:2, or at least 98% to a nucleic acid comprising the nucleotide sequence of SEQ ID NO:2, or a complement thereof; and
e) an amino acid sequence which is encoded by a nucleic acid molecule which hybridizes to the nucleic acid comprising SEQ ID NO:2 under hybridization conditions of hybridization in 6XSSC at 45 °C and washing in 0.2XSSC, 0.1% SDS at 65°C.

12. The isolated polypeptide of claim 11 comprising the amino acid sequence of SEQ ID NO:3.

13. The polypeptide of claim 11 further comprising heterologous amino acid sequences.

14. A substantially purified antibody which immunospecifically binds to a polypeptide of claim 11.

15. The antibody of claim 14, wherein the antibody is a monoclonal antibody.

16. A method for producing a polypeptide selected from the group consisting of:
a) a polypeptide comprising the amino acid sequence of SEQ ID NO:3; or the amino acid sequence encoded by the cDNA insert of the plasmid deposited with the ATCC® as Accession Number patent deposit Number PTA-2267;
b) a polypeptide comprising a fragment of amino acid residues 174 to 331 of SEQ ID NO:3, wherein the fragment comprises at least 15 contiguous amino acid residues;
c) a polypeptide comprising a fragment of the amino acid sequence of SEQ ID NO:3, or the amino acid sequence encoded by the cDNA insert of the plasmid deposited with the ATCC® as patent deposit Number PTA-2267, wherein the fragment comprises at least 175 contiguous amino acids of SEQ ID NO:3, or the amino acid sequence encoded by the cDNA insert of the plasmid deposited with the ATCC® as patent deposit Number PTA-2267; and
d) a naturally occurring allelic variant of a polypeptide comprising the amino acid sequence of SEQ ID NO:3, or the amino acid sequence encoded by the cDNA insert of the plasmid deposited with the ATCC® as patent deposit Number PTA-2267, wherein the polypeptide is encoded by a nucleic acid molecule which hybridizes to a nucleic acid molecule comprising SEQ ID NO:1, or a complement thereof under stringent conditions;
comprising culturing the host cell of claim 5 under conditions in which the nucleic acid molecule is expressed.

17. A method for detecting the presence of a polypeptide of claim 11 in a sample, comprising:
a) contacting the sample with a compound which selectively binds to a polypeptide of claim 11; and
b) determining whether the compound binds to the polypeptide in the sample.

18. The method of claim 17, wherein the compound which binds to the polypeptide is an antibody.

19. A kit comprising a compound in one or more containers which selectively binds to a polypeptide of claim 11, and instructions for use.

20. A kit comprising a compound in one or more containers which selectively hybridizes to a nucleic acid molecule of claim 1, and instructions for use.

21. A kit comprising an antibody as in claim 14 or 15 and instructions for use.

22. A method for detecting the presence of a nucleic acid molecule of claim 1 in a sample, comprising the steps of:
a) contacting the sample with a nucleic acid probe or primer which selectively hybridizes to the nucleic acid molecule; and
b) determining whether the nucleic acid probe or primer binds to a nucleic acid molecule in the sample.

23. The method of claim 22, wherein the sample comprises mRNA molecules and is contacted with a nucleic acid probe.

24. A method for identifying a compound which binds to a polypeptide of claim 11 comprising the steps of:
a) contacting a polypeptide, or a cell expressing a polypeptide of claim 11 with a test compound; and
b) determining whether the polypeptide binds to the test compound.

25. The method of claim 24, wherein the binding of the test compound to the polypeptide is detected by a method selected from the group consisting of:
a) detection of binding by direct detecting of test compound/polypeptide binding;
b) detection of binding using a competition binding assay; and
c) detection of binding using an assay for CD2000-mediated signal transduction.

26. A pharmaceutical composition comprising an antibody as in claim 14 or 15, and a pharmaceutically acceptable carrier.
